(19) Europäisches Patentamt
European Patent Office
Office européen des brevets

(11) EP 3 891 752 B1

(12) **FASCICULE DE BREVET EUROPEEN**

(45) Date de publication et mention de la délivrance du brevet:
**13.08.2025 Bulletin 2025/33**

(21) Numéro de dépôt: **19817270.2**

(22) Date de dépôt: **06.12.2019**

(51) Classification Internationale des Brevets (IPC):
**G16B 40/20** *(2019.01)* **C12Q 1/37** *(2006.01)*

(52) Classification Coopérative des Brevets (CPC):
**G01N 33/86; G16B 40/20;** G01N 2333/745; G01N 2333/96444

(86) Numéro de dépôt international:
**PCT/EP2019/084069**

(87) Numéro de publication internationale:
**WO 2020/115315 (11.06.2020 Gazette 2020/24)**

(54) **METHODE POUR LA DETECTION DE LA PRESENCE, L'IDENTIFICATION ET LA QUANTIFICATION DANS UN ECHANTILLON SANGUIN D'ANTICOAGULANTS INHIBITEURS D'ENZYMES DE LA COAGULATION DU SANG, ET MOYENS POUR SON IMPLEMENTATION**

VERFAHREN ZUM NACHWEIS DER PRÄSENZ, IDENTIFIZIERUNG UND QUANTIFIZIERUNG VON ANTIKOAGULANTIEN IN EINER BLUTPROBE, DIE INHIBITOREN VON BLUTGERINNUNGSENZYMEN SIND, UND MITTEL ZUR DURCHFÜHRUNG DAVON

METHOD FOR DETECTING THE PRESENCE, IDENTIFICATION AND QUANTIFICATION IN A BLOOD SAMPLE OF ANTICOAGULANTS WHICH ARE INHIBITORS OF BLOOD COAGULATION ENZYMES, AND MEANS FOR THE IMPLEMENTATION THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.12.2018 FR 1872562**

(43) Date de publication de la demande:
**13.10.2021 Bulletin 2021/41**

(73) Titulaire: **Diagnostica Stago**
**92600 Asnières-sur- Seine (FR)**

(72) Inventeur: **KERDELO, Sébastien**
**75019 PARIS (FR)**

(74) Mandataire: **Ernest Gutmann - Yves Plasseraud S.A.S.**
**C/o Plasseraud IP**
**104 Rue de Richelieu**
**CS 92104**
**75080 Paris Cedex 02 (FR)**

(56) Documents cités:
**EP-A1- 1 367 135      WO-A1-2014/134223**

- **CUTTE ET AL: "Hematologie De l'automate de NF a l'analyse cellulaire : aboutissement de l'automatisation de l'hematologie ?", IRBM NEWS, ELSEVIER, AMSTERDAM, NL, vol. 29, no. 3-4, 1 October 2008 (2008-10-01), pages 24 - 31, XP026006968, ISSN: 1959-7568, [retrieved on 20081001]**

**Description**

**DOMAINE TECHNIQUE DE L'INVENTION**

La présente demande concerne le domaine de l'hémostase et en particulier celui de la coagulation du sang et fournit des méthodes et moyens permettant une détection "en aveugle", et de façon subséquente et le cas échéant, d'identification et de caractérisation qualitative puis quantitative (dosage), d'anticoagulants, inhibiteurs d'enzymes de la coagulation du sang dans un échantillon analysé.

L'invention met en œuvre des outils d'intelligence artificielle, notamment des modèles d'apprentissage automatique supervisés, à des fins de post-traitement de mesures cinétiques opérées sur des échantillons sanguins.

L'invention concerne ainsi plus particulièrement, une méthode de détection de la présence d'un inhibiteur d'une enzyme de la coagulation du sang, cette dernière étant choisie parmi le facteur Xa et le facteur IIa, comme défini en revendication 1, puis, en cas de présence, d'identification dudit inhibiteur, cette identification incluant sa catégorie comme par exemple défini en revendication 2, sa nature ou sa caractérisation effective comme défini en revendications 3, 5 ou 6, puis après cette identification, son dosage quantitatif comme défini en revendications 4, 7 ou 8. L'invention est en premier lieu destinée à l'application pour la détection *in vitro* de la présence des inhibiteurs ici en cause dans des échantillons biologiques prélevés chez des sujets humains. Les revendications 9 à 12 précisent des caractéristiques des étapes réalisées *in vitro.*

L'invention concerne également des moyens utiles, et adaptés à ces fins: système de traitement de données ou dispositif approprié, programme d'ordinateur, support d'enregistrement ou de données, et kits permettant la mise en œuvre de toutes les étapes des méthodes ici décrites, qu'elles soient expérimentales ou in silico. Lesdits moyens sont tels que définis en revendications 13 à 17.

**ETAT DE LA TECHNIQUE ANTERIEURE**

La coagulation est un phénomène physiologique complexe, procédant en cascade et faisant intervenir plusieurs protéines plasmatiques. Ce processus une fois mis en jeu aboutit à la formation d'un caillot (un clou plaquettaire), qui dans le fonctionnement physiologique non pathologique permet de freiner ou de juguler les hémorragies. A l'inverse, les troubles de la coagulation qui mènent à des risques de saignements plus importants sont appelés hémophilie. La coagulation sanguine naturelle est régulée par la présence de divers facteurs de coagulation qui de concert se chargent d'un équilibre normal entre les tendances naturelles à la coagulation et celles au saignement.

Il est connu, par exemple de EP1367135A1, des méthodes *in vitro* pour déterminer la dynamique de formation de la thrombine, i.e., du facteur IIa, dans un échantillon de sang, au cours du temps. Ce type de méthode permet d'obtenir une cinétique de formation de la thrombine au cours du temps, et de voir l'impact de différents paramètres sur cette cinétique.

Il est également connu de WO2014/134223A1 d'utiliser un test de dosage du facteur X, qui intervient dans la voie commune de la coagulation et est activé en facteur Xa par différents agents, comme biomarqueur pour diagnostiquer une maladie rénale chez un patient. WO2014/134223A1 divulgue que le diagnostic peut être effectué en utilisant des seuils ou des modèles de classification qui peuvent être des sets de règles, des arbres de décision, des méthodes bayesiennes ou des réseaux de neurones. WO2014/134223A1 cependant ne cherche pas à détecter la présence d'un inhibiteur d'une enzyme de la coagulation du sang choisie parmi le facteur Xa et le facteur IIa dans un échantillon de sang d'un patient.

Dans certains cas, notamment dans des situations pathologiques, le processus de coagulation doit d'être corrigé ou modifié à l'aide de l'administration d'un agent anticoagulant, en traitement ou en prévention de certains troubles ou pathologies. Le degré d'effet anticoagulant atteint lors de l'utilisation de ces agents sous forme de médicaments dépend d'un grand nombre de facteurs, souvent peu connus. En cas de surdosage cependant, des saignements inopinés s'extériorisant aux orifices naturels peuvent se produire. De tels traitements impliquent donc un contrôle régulier du degré d'anticoagulation au moyen, généralement, d'un examen sanguin.

Les anticoagulants non physiologiques, par exemple synthétiques, i.e., des anticoagulants médicamenteux, sont ainsi communément prescrits en prévention et/ou en traitement de troubles avérés.

Les agents anticoagulants médicamenteux inhibiteurs d'enzymes de la coagulation disponibles appartiennent à deux grandes familles d'anticoagulants : 1/ les héparines, et 2/ les anticoagulants oraux directs (AOD), les agents des deux familles correspondant à des inhibiteurs du facteur Xa et/ou du facteur IIa de la coagulation.

Les héparines sont des inhibiteurs indirects irréversibles des facteurs Xa et IIa. Elles se déclinent en deux classes : les héparines non fractionnées (HNF) et les héparines de bas poids moléculaires (HBPM). Les HNF ont un ratio (pouvoir anti-Xa / pouvoir anti-IIa) proche de 1 tandis que les HBPM ont un ratio (pouvoir anti-Xa / pouvoir anti-IIa) proche de 2 ou 3.

Les anticoagulants oraux directs (AODs) sont des inhibiteurs réversibles directs spécifiques du facteur Xa ou du facteur IIa. Les AODs principaux, spécifiques du facteur Xa sont le Rivaroxaban, l'apixaban et l'edoxaban. L'AOD principal spécifique du facteur IIa est le dabigatran. Les Tables 18 et 20 détaillent de façon plus complète d'autres inhibiteurs synthétiques connus des facteurs Xa et IIa.

Afin de déterminer le statut hémostatique de sujets dans des situations cliniques l'imposant et afin de surveiller le statut hémostatique de patients sous traitement anticoagulant, différents tests visent à détecter et/ou à doser les paramètres de la coagulation.

On connaît par exemple des tests cliniques permettant de déterminer la présence d'un état d'hypocoagulation, comme en cas d'augmentation du taux de prothrombine ou du temps de Quick. Ces examens de biologie médicale nécessitent cependant un équipement de laboratoire pas toujours disponible en tous lieux.

On connait également des tests qui permettent de détecter la présence d'un anticoagulant précis, le plus souvent unique, dont on soupçonne la présence. Par exemple, le kit STA® - Liquid Anti-Xa commercialisés par le Demandeur sont destinés à être utilisé avec les appareils de la gamme STA-R®, pour une détermination quantitative des taux plasmatiques d'héparines non fractionnées (HNF) ou de faible poids moléculaire (HBPM) en mesurant leur activité anti-Xa dans un test compétitif utilisant un substrat chromogène synthétique. Selon l'expérience, la disponibilité de l'équipement, et la réactivité de l'opérateur, ce test qui peut se réaliser en une dizaine de minutes est bien entendu pertinent, notamment lorsque l'on sait ce que l'on cherche. Ce genre de test requiert cependant toujours la réalisation d'une étape de calibration.

On connait également le kit Stago STA®- Multi-Hep Calibrator qui permet à la fois le dosage des HNF et des HBPM en utilisant une méthodologie commune et une calibration hybride. Ce kit ne permet cependant pas la détection des AOD.

Il existe encore des situations, en particulier des situations cliniques, dans lesquelles le régime médicamenteux du patient considéré n'est pas connu de manière certaine ou ne peut pas être déterminé (par exemple si le patient est inconscient), ce qui complique une tâche de détection. Or il peut être nécessaire ou préférable de connaître le régime anticoagulant d'un patient en identifiant l'anticoagulant ou les anticoagulants qui lui ont été administrés et leur concentration chez le patient et ce de façon éventuellement immédiate après prise en charge médicale du patient, le cas échéant dans des conditions d'urgence, par exemple dans la perspective d'un traitement médicamenteux, d'examens biologiques ou cliniques, ou d'une intervention chirurgicale. De façon générale, la détection de la présence dans un échantillon sanguin d'un des anticoagulants médicamenteux connus ou encore leur identification peut être très utile dans un contexte d'urgence (dans le cadre d'un phénotype hémorragique, de la mise en place d'un antidotage...). Les tests disponibles pour doser les agents anticoagulants sont adaptés au dosage des anticoagulants dont l'identité est connue et a pu être communiquée en temps voulu lors de l'examen du patient et dont il a été vérifié qu'ils ont été prescrits ou administrés au patient examiné. Au contraire, des tests de dosage « en aveugle » qui permettraient dans une première étape d'identifier la présence d'anticoagulant(s) dans un échantillon biologique, puis de déterminer leur catégorie et/ou leur identité et enfin le cas échéant leur concentration, dans un test efficace pour recueillir une information biologique ou clinique ne sont pas disponibles alors qu'ils seraient d'une évidente utilité pour répondre à certaines situations de prise en charge clinique des patients.

Enfin, s'il existe aujourd'hui plusieurs solutions commerciales pour doser les différents anticoagulants susmentionnés, toutes ces solutions imposent nécessairement l'utilisation d'un kit dédié ayant ses propres calibrants et contrôles ainsi que sa propre méthodologie expérimentale.

De fait, sur l'exemple de la mesure de la présence d'un anticoagulant inhibiteur du facteur Xa, l'utilisation des méthodes actuelles impose un minimum de quatre dosages (par exemple un dosage d'héparines en méthodologie hybride et trois dosages d'AODs anti-Xa). L'identification sans information préalable de la molécule anticoagulante est actuellement impossible

L'invention se propose ainsi en particulier de résoudre tout ou partie des problèmes identifiés dans les paragraphes précédents, en particulier le problème d'une détection "en aveugle" de la présence ou non d'un anticoagulant inhibiteur d'une enzyme la coagulation du sang dans un échantillon sanguin. La solution proposée permet notamment, pour la première fois, la mise au point d'un test particulièrement adapté à une situation de prise en charge clinique, en particulier dans un contexte d'urgence, d'incertitude sur le régime de traitement du patient ou de patient inconscient. Si un tel test doit apporter du confort, et faire gagner du temps, à l'utilisateur dans le cadre de la prise en charge clinique, il se doit naturellement d'être également fiable, compte-tenu de l'enjeu pouvant impliquer le pronostic vital. Un tel test devrait permettre l'identification, fiable, d'un agent contenu dans un échantillon de sang, dont on ignore la présence, ou dont on ne peut soupçonner la présence. On observera qu'il n'existe à ce jour aucun test de ce type.

Selon un aspect particulier, l'invention s'intéresse à la détection d'agents anticoagulants exogènes, aussi appelés « synthétiques » dans le présent texte, en particulier la détection de médicaments anticoagulants et de préférence permet aussi une détection en un seul test, permettant d'atteindre une conclusion sans réitérer une multitude de tests séparés.

## DESCRIPTION DETAILLEE

C'est ainsi qu'en réponse à ces problèmes, il a été conçu et mis en œuvre une méthode de détection dans un échantillon biologique, en particulier un échantillon sanguin, de la présence d'un inhibiteur d'une enzyme de la coagulation du sang choisie, indépendamment, parmi le facteur Xa (FXa) et le facteur IIa (FIIa), la méthode comme définie en revendication 1 comprenant les étapes suivantes:

a. Mesure d'une ou de plusieurs cinétique(s) de compétition via la réalisation d'un dosage enzymatique compétitif sur un échantillon sanguin préalablement obtenu d'un sujet, ledit dosage étant adapté à la réalisation d'une cinétique compétitive vis-à-vis, soit d'un inhibiteur du facteur Xa, soit d'un inhibiteur du facteur IIa (indépendamment l'une de l'autre), puis

b. Fourniture en entrée de la ou des cinétique(s) obtenue(s) dans l'étape a. à un modèle décisionnel de classification A obtenu par entraînement d'un modèle d'apprentissage automatique supervisé, par exemple un modèle choisi parmi l'une des familles suivantes : machine à vecteurs de supports, réseaux de neurones, arbres de décision, méthodes d'ensemble, et modèle des k plus proches voisins, en particulier dont les paramètres ont été calculés par entraînement, puis

b.i. Si le modèle décisionnel A exclut la présence d'un inhibiteur de l'enzyme de la coagulation du sang ciblée dans l'échantillon analysé, conclusion de l'absence dudit inhibiteur, optionnellement allocation en sortie par le modèle A, par exemple dans une variable, de l'information correspondante, ou

b.ii. Si le modèle décisionnel A atteste de la présence d'un inhibiteur de l'enzyme de la coagulation du sang ciblée dans l'échantillon analysé, conclusion de la présence dudit inhibiteur, optionnellement allocation en sortie par le modèle A, par exemple dans une variable, de l'information correspondante.

Les revendications 2 à 12 détaillent des caractéristiques plus particulières de cette méthode. L'échantillon sanguin testé est par exemple un échantillon de sang ou un échantillon de plasma. Il peut selon le besoin être dilué ou non.

L'invention sert la problématique dite d'une « détection en aveugle ». Par "détection en aveugle", on indique que selon l'invention, on ne sait pas par avance si un inhibiteur de l'enzyme ciblée est présent dans l'échantillon, ni a fortiori quel inhibiteur pourrait être présent, et que la méthode ici décrite est ainsi adaptée et, en fonction des modèles mis à disposition dans son cadre, peut servir la détection de tout inhibiteur de la coagulation agissant sur les facteurs Xa, et/ou IIa.

L'invention vise plus particulièrement la détection d'un inhibiteur ici appelé médicamenteux (également appelé « synthétique » dans les Tables 17 à 20) du facteur Xa (FXa) ou du facteur IIa (FIIa), par opposition à un inhibiteur dit « naturel » ou physiologique (la distinction étant illustrée sous forme de listes en Tables 17 à 20), l'inhibiteur ayant une fonction d'anticoagulant sélectif - du fait de l'enzyme ciblée. Dans un mode de réalisation particulier de l'invention, un inhibiteur recherché d'une enzyme de la coagulation est ainsi un inhibiteur non physiologique. Un tel inhibiteur est notamment un inhibiteur médicamenteux. Selon un aspect particulier, un inhibiteur médicamenteux peut se trouver dans un échantillon de patient à une concentration sensiblement plus élevée qu'une concentration physiologique « naturelle ». Selon des modes de réalisation particuliers, lesdits inhibiteurs appartiennent à la famille des héparines ou celle des AOD.

Des exemples d'inhibiteurs médicamenteux (synthétiques) du facteur Xa désignés par leur substance active ou DCI sont ainsi: le Rivaroxaban, l'Apixaban, l'Edoxaban, le Bétrixaban, les HNF, par exemple la HNF calcique, la HNF sodique, les HBPM, un Pentasaccharide, le Danaparoïde sodique. La preuve de concept rapportée dans la présente demande vise plus particulièrement : les héparines que sont les héparines non fractionnées (HNF) telles que la HNF calcique ou la HNF sodique, les héparines de bas poids moléculaire (HBPM) telles que celles constituant les principes actifs de Fragmine®, Lovenox®, Innohep®, ou les AODs que sont le Rivaroxaban, l'Apixaban, l'Edoxaban, mais il est entendu que la méthode ici décrite peut être implémentée pour tout type d'inhibiteurs du facteur Xa et ou IIa, pour peu que l'on dispose d'un substrat approprié de l'enzyme, et que des modèles adéquats aient été générés ou puissent l'être en accord avec les principes directeurs ici exposés et l'illustration fournie.

Des exemples d'inhibiteurs médicamenteux (synthétiques) du facteur IIa désignés par leur substance active ou DCI sont ainsi: le Dabigatran, le Melagatran, l'Argatroban, la Bivalirudine, les HNF, les HBPM. L'homme du métier pourra, sur la base de la littérature concernant les algorithmes d'intelligence artificielle et leur implémentation à sa disposition, et suivant le contenu de la partie expérimentale de la présente description, adapter les protocoles ici décrits pour substituer, intégrer ou ajouter d'autres modèles permettant une prise de décision.

Par "enzyme de la coagulation du sang choisie" sont ainsi visés le facteur Xa et le facteur IIa, dont les inhibiteurs sont recherchés indépendamment entre eux, respectivement, dans un même test. La méthode ici décrite se propose de détecter la présence (et dans des étapes subséquentes le cas échéant de détecter l'identité et de préférence de réaliser un dosage quantitatif par exemple pour donner la concentration), soit un inhibiteur du facteur Xa, soit un inhibiteur du facteur IIa, à l'exclusion d'une détection concomitante des deux.

Par "mesure de plusieurs cinétique(s) de compétition" en étape a., il est indiqué que, selon un mode de réalisation particulier, les mesures peuvent être répétées plusieurs fois sur la base d'un même échantillon sanguin prélevé chez un sujet (de préférence, un humain, notamment, un patient), afin d'offrir une meilleure fiabilité. Plusieurs cinétiques pourront ainsi être fournies à toutes les étapes qui le nécessitent, ou une seule cinétique intégrant toutes les autres pourra être fournie. Pour réaliser un dosage enzymatique de façon à obtenir une cinétique de compétition dans le cadre de l'invention, les conditions propres à la réalisation des réactions biochimiques suivantes doivent être réunies : l'échantillon sanguin susceptible de contenir l'inhibiteur de l'enzyme de la coagulation ciblée est mis en présence d'un substrat choisi, de l'enzyme ciblée, de préférence un substrat spécifique de l'enzyme ciblée, et une étape d'incubation est réalisée avant l'ajout de l'enzyme ciblée au mélange réactionnel dans des conditions permettant le déclenchement de la compétition entre la réaction d'inhibition de l'enzyme et la réaction enzymatique de ladite enzyme avec son substrat. Le substrat mis en œuvre est normalement marqué de façon à permettre la libération du marqueur lorsque l'enzyme interagit avec ce substrat pour le transformer en produit de la réaction enzymatique et à permettre subséquemment la mesure dudit marqueur libéré et l'enregistrement de sa variation dans le temps sous la forme d'une cinétique de libération du marqueur qui est impactée lorsque l'inhibiteur est présent dans l'échantillon cet impact étant de plus modulé par la concentration de l'inhibiteur. Des schémas cinétiques des réactions de dosage compétitif sont illustrés dans les Exemples. Des substrats indiqués pour la réaction enzymatique avec l'enzyme ciblée sont connus de l'homme du métier ; on cite à titre d'exemple convenant pour la réalisation de l'invention : le substrat MAPA-Gly-Arg-pNA spécifique du facteur Xa qui une fois transformé par le FXa induit la libération de paranitroalanine (pNA) détectable par colorimétrie lors d'une mesure à 405nm, ou le substrat MAPA- Gly-Arg-AMC ou toute variante connue, qui induit la libération d'AminoMethylCoumarine (AMC) détectable par fluorométrie, ou le substrat EtM-SPro-Arg-pNA spécifique du facteur IIa, ou toute variante connue de l'homme du métier.

La mesure d'une cinétique de compétition selon l'étape a., est une mesure qui s'effectue *in vitro,* selon la pratique usuelle dans le domaine de l'exploration de l'hémostase, et selon des techniques bien connues, dont des exemples sont donnés ci-après. Cette étape expérimentale s'effectue selon les pratiques conventionnelles et bien connues de l'homme du métier.

On comprendra néanmoins qu'il existe un lien indissociable pour la fiabilité des résultats tout au long des chaînes de classification proposées dans la présente invention, entre les jeux de données qui ont été utilisées pour l'entraînement des modèles et les données cinétiques obtenues à partir de l'échantillon sanguin du sujet considéré dès lors que lesdits jeux de données et lesdites données cinétiques du test effectué sur l'échantillon du sujet doivent avoir été obtenus dans des conditions expérimentales proches, sinon identiques. Ainsi, on dira que les modèles de décision utilisés sont liés/associés aux réactifs et instruments utilisés dans le cadre de la collecte de données de test chez le sujet.

Ceci étant, cela ne signifie pas que l'invention doive être conduite sur un modèle particulier d'instrument ou appareil de mesure, ou avec un modèle particulier de réactifs. Il convient simplement de comparer les résultats de tests ou d'expériences conduits(es) dans des conditions expérimentales similaires pour que le résultat ne soit pas faussé, les méthodes algorithmiques ici décrites remplaçant une calibration inter-instruments et permettant de s'affranchir de la nécessité de calibration pour chaque test ou lot de tests effectué(s).

Par « modèle décisionnel de classification A obtenu par entraînement d'un modèle d'apprentissage automatique supervisé », par exemple un modèle choisi parmi l'une des familles suivantes : « machine à vecteurs de supports, réseaux de neurones, arbres de décision, méthodes d'ensemble, et modèle des k plus proches voisins », référence est faite aux multiples possibilités d'implémentation d'un modèle d'apprentissage automatique supervisé, telles que notamment documentées dans la littérature. L'homme du métier, sur la base de ses connaissances générales, pourra moduler le type de modèle utilisé pour adapter les performances observées, à ses besoins et aux objectifs visés. Voir par exemple, de façon non exhaustive [Géron, 2017] ou Bonaccorso, G. (2017).

Un modèle d'apprentissage automatique supervisé a, par définition, ses paramètres qui ont été calculés par entraînement, de façon conventionnelle dans le domaine. Voir par exemple, de façon non exhaustive [Géron, 2017] ou Bonaccorso, G. (2017).

Selon un mode particulier de réalisation, le modèle décisionnel de classification A est une machine à vecteur de supports, en particulier a été obtenu par entraînement d'une machine à vecteurs de supports. Un mode plus particulier de réalisation appliqué à la recherche d'un inhibiteur du facteur Xa est tel que décrit dans la partie expérimentale.

Il découle de l'essence de l'invention que la méthode ici décrite tient compte du résultat d'un entraînement d'un modèle d'apprentissage automatique sur des jeux de données préalablement collectés et établis. Typiquement, un tel modèle décisionnel de classification peut consister en une matrice correspondant à une fonction mathématique empiriquement déterminée, qui, appliquée à un jeu de données en entrée (dont la constitution est, antérieurement à la réalisation de la méthode selon l'invention, inconnue), permet de rendre une solution. S'agissant du modèle A, la solution peut, par exemple, être soit "oui", soit "non" traduisant la présence ou l'absence d'un inhibiteur recherché (pouvant être codé par un entier, comme "1" ou "0").

De fait, l'étape b. rapportée ci-dessus est une étape nécessairement implémentée par ordinateur, du fait de la complexité de la fonction mathématique empiriquement déterminée par apprentissage.

Selon un autre mode particulier de réalisation, le modèle décisionnel de classification A est un réseau de neurones, notamment un perceptron multi-couches, en particulier a été obtenu par entraînement d'un réseau de neurones notamment un perceptron multi-couches. Un mode plus particulier de réalisation d'un tel modèle A, appliqué à la recherche d'un inhibiteur du facteur Xa est tel que décrit dans la partie expérimentale.

Des exemples d'implémentation, en particulier des modèles d'implémentation d'une machine à vecteurs de supports ou d'un réseau de neurones, tel un perceptron multi-couches, sont connus de l'homme du métier, au besoin par référence à la littérature dans le domaine (Voir par exemple, de façon non exhaustive [Géron, 2017] ou Bonaccorso, G. (2017)). Plus précisément, peuvent par exemple être utilisés le package LIBSVM (https://www.csie.ntu.edu.tw/-cjlin/libsvm/) implémenté dans la librairie scikit-learn (https://scikit-learn.org/). Les paramètres que l'homme du métier pensera à fixer sont, par exemple, et indépendamment ou non les uns des autres, le noyau (linéaire, polynomial, gaussien, sigmoidal ...), les coefficients du noyau et le paramètre nu associé au modèle SVM. L'homme du métier peut notamment se référer à Géron, 2017 ou Bonaccorso, G. (2017), pour la mise en œuvre de tels modèles, y compris en ce qui concerne les modalités de fixation des paramètres susmentionnés, incluant au besoin les directives contenues de façon explicite ou implicite dans la présente description. En cas de présence avérée d'un inhibiteur, un mode de réalisation plus poussé de la présente invention inclut à la suite une étape d'identification de l'inhibiteur de ladite enzyme détectée.

L'invention concerne ainsi encore une méthode d'identification dans un échantillon biologique, en particulier un échantillon sanguin, d'un inhibiteur d'une enzyme de la coagulation du sang choisie, indépendamment, parmi le facteur Xa (FXa) et le facteur IIa (FIIa), la méthode comprenant les étapes suivantes:

1. Mise en œuvre des étapes de la méthode de détection décrite plus haut ou de toute mode particulier de cette méthode décrit et dans la présente description, puis

2. Fourniture en entrée de la ou des cinétique(s) obtenue(s) dans l'étape définie au point a. ci-dessus (dans le cadre de la détection) et du résultat obtenu en fin d'étape étape b. ii. ci-dessus (dans le cadre de la détection), à un modèle décisionnel de classification B obtenu par entraînement d'un modèle d'apprentissage automatique supervisé, par exemple un modèle choisi parmi l'une des familles suivantes : machine à vecteurs de supports, réseaux de neurones, arbres de décision, méthodes d'ensemble, et modèle des k plus proches voisins, en particulier dont les paramètres ont été calculés par entraînement, et allocation en sortie par le modèle B de la catégorie de l'inhibiteur à l'une des catégories suivantes: inhibiteur indirect irréversible (héparines), ou inhibiteur direct réversible (AOD), notamment lorsque les jeux de données utilisés pour l'entraînement du modèle B comprennent des données relatives à ces deux catégories d'inhibiteurs, et fourniture en sortie, par exemple par allocation dans une variable, de la catégorie de l'inhibiteur déterminée par le modèle B.

Par « modèle décisionnel de classification B », il est indiqué la même chose que ce qui précède relativement au « modèle A », par analogie.

Selon un mode particulier de réalisation, le modèle décisionnel de classification B est un modèle des k plus proches voisins, en particulier a été obtenu par entraînement d'un modèle des k plus proches voisins. Un mode plus particulier de réalisation appliqué à la recherche d'un inhibiteur du facteur Xa est tel que décrit dans la partie expérimentale.

Par « notamment lorsque les jeux de données utilisés pour l'entraînement du modèle B comprennent des données relatives à ces deux catégories d'inhibiteurs, i.e,, inhibiteur indirect irréversible (héparines), et inhibiteur direct réversible (AOD) » il est entendu qu'il découle de l'essence de l'invention que les jeux de données employés pour l'apprentissage doivent être en corrélation avec le type d'information que l'on cherche à obtenir en aveugle. Des lignes directrices à l'attention de l'homme du métier découlent naturellement des exemples fournis dans la partie expérimentale.

De fait, l'étape d'implémentation du modèle B est une étape nécessairement implémentée par ordinateur, du fait de la complexité de la fonction mathématique empiriquement déterminée par apprentissage.

Selon un autre mode particulier de réalisation, le modèle décisionnel de classification B est un réseau de neurones, notamment un perceptron multi-couches, en particulier a été obtenu par entraînement d'un réseau de neurones notamment un perceptron multi-couches. Un mode plus particulier de réalisation d'un tel modèle B, appliqué à la recherche d'un inhibiteur du facteur Xa est tel que décrit dans la partie expérimentale.

Des exemples d'implémentation, en particulier des modèles d'implémentation d'un modèle des k plus proches voisins ou d'un réseau de neurones, tel un perceptron multi-couches, sont connus de l'homme du métier, au besoin par référence à la littérature dans le domaine (Voir par exemple, de façon non exhaustive [Géron, 2017] ou Bonaccorso, G. (2017)). Plus précisément, peut par exemple être utilisée la librairie scikit-learn (https://scikit-learn.org/). Les paramètres que l'homme du métier pensera à fixer sont, par exemple, et indépendamment ou non les uns des autres, le nombre de voisins ainsi que la métrique de distance. L'homme du métier peut notamment se référer à Géron, 2017 ou Bonaccorso, G.

(2017), pour la mise en œuvre de tels modèles, y compris en ce qui concerne les modalités de fixation des paramètres susmentionnés, incluant au besoin les directives contenues de façon explicite ou implicite dans la présente description.

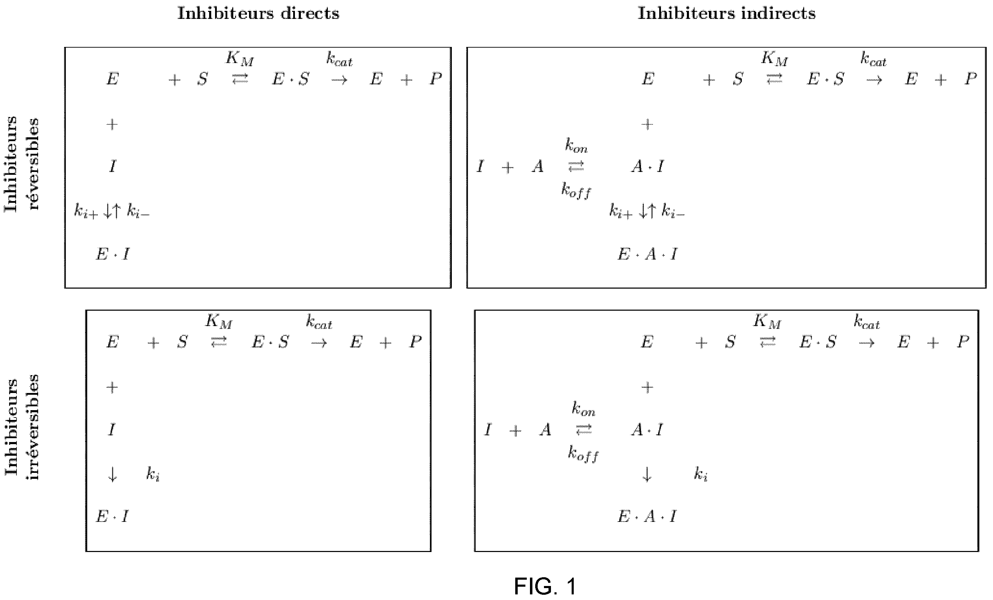

L'implémentation du modèle B rend une réponse relative à une "catégorie d'inhibiteur" choisie parmi deux : inhibiteur direct réversible (alternativement appelée « AOD » dans le présent texte), inhibiteur indirect irréversible (alternativement appelée « héparines » dans le présent texte) (voir explications de ces concepts en Figure 1). De fait, les héparines étant toutes des inhibiteurs indirects irréversibles, et les AOD (anticoagulants oraux directs) étant tous des inhibiteurs directs réversibles, lorsque, selon un mode de réalisation particulier correspondant à la preuve de concept décrite en partie expérimentale, les jeux de données fournis pour l'apprentissage impliquent des principes actifs appartenant à ces deux familles seulement, la méthode selon l'invention pourra distinguer entre ces deux familles particulières, alors indiquées "héparines", et "AOD".

Selon un autre aspect, la méthode d'identification décrite dans les paragraphes précédents (par opposition à la méthode de « détection » décrite plus haut), en partie réalisée *in vitro,* peut encore comprendre une étape additionnelle de caractérisation de l'inhibiteur dont la présence a été détectée dans l'étape b. ii ci-dessus (dans le cadre de la détection), comme suit: fourniture en entrée de la ou des cinétique(s) obtenue(s) dans l'étape a. ci-dessus (dans le cadre de la détection), équivalente à l'étape 1. ci-dessus, et de la donnée déterminée, par exemple la variable allouée, à l'étape 2. ci-dessus implémentant le modèle B concernant la catégorie d'inhibiteur dont la présence a été détectée, à un modèle décisionnel de classification C obtenu par entraînement d'un modèle d'apprentissage automatique supervisé, par exemple un modèle choisi parmi l'une des familles suivantes : machine à vecteurs de supports, réseaux de neurones, arbres de décision, méthodes d'ensemble, et modèle des k plus proches voisins, notamment dont les paramètres ont été calculés par entraînement, et caractérisation en sortie par le modèle C de l'inhibiteur recherché, ce dernier étant identifié parmi :

a. Dans le cas où la catégorie de l'inhibiteur recherché est celle des héparines : la HNF ou la HBPM (pour des inhibiteurs du facteur Xa et/ou IIa), ou

b. Dans le cas où la catégorie de l'inhibiteur recherché est celle des AOD : le Rivaroxaban, l'Apixaban, l'Edoxaban (pour un inhibiteur du facteur Xa) ou le Dabigatran (pour un inhibiteur du facteur IIa),

et fourniture en sortie, par exemple par allocation dans une variable, de la caractérisation de l'inhibiteur déterminée par le modèle C.

Par « modèle décisionnel de classification C », il est indiqué la même chose que ce qui précède relativement au « modèle A » ou au « modèle B », par analogie.

Selon un mode particulier de réalisation, le modèle décisionnel de classification C est un réseau de neurones, notamment un perceptron multi-couches, en particulier a été obtenu par entraînement d'un réseau de neurones notamment un perceptron multi-couches. Un mode plus particulier de réalisation appliqué à la recherche d'un inhibiteur du facteur Xa est tel que décrit dans la partie expérimentale. De fait, l'étape d'implémentation du modèle C est une étape nécessairement implémentée par ordinateur, du fait de la complexité de la fonction mathématique empiriquement déterminée par apprentissage.

Des exemples d'implémentation, en particulier des modèles d'implémentation d'un réseau de neurones, notamment un perceptron multi-couches, sont connus de l'homme du métier, au besoin par référence à la littérature dans le domaine (Voir par exemple, de façon non exhaustive [Géron, 2017] ou Bonaccorso, G. (2017)). Plus précisément, peut par exemple être utilisée la librairie scikit-learn (https://scikit-learn.org/). Les paramètres que l'homme du métier pensera à fixer sont, par exemple, et indépendamment ou non les uns des autres, le nombre de couches de neurones, le nombre de neurones par couche et les fonctions d'activation des différents neurones. Mais l'utilisateur dispose d'une certaine liberté d'implémentation en ce qui concerne l'algorithme d'apprentissage ou les paramètres de régularisation. L'homme du métier peut notamment se référer à Géron, 2017 ou Bonaccorso, G. (2017), pour la mise en œuvre de tels modèles, y compris en ce qui concerne les modalités de fixation des paramètres susmentionnés, incluant au besoin les directives contenues de façon explicite ou implicite dans la présente description.

Selon un autre aspect, la méthode d'identification décrite dans les paragraphes précédents, en partie réalisée *in vitro,* peut encore comprendre, à l'issue de la mise en œuvre du modèle C, une étape additionnelle de dosage quantitatif de l'inhibiteur caractérisé, dans laquelle sont fournies en entrée à un modèle de régression D, notamment un modèle d'apprentissage automatique supervisé, par exemple un modèle choisi parmi l'une des familles suivantes : machines à vecteurs de support, réseaux de neurones, arbres de décision, méthodes d'ensemble, et modèle des k plus proches voisins, la ou des cinétique(s) obtenue(s) dans l'étape a. ci-dessus (dans le cadre de la détection), équivalente à l'étape 1. ci-dessus, et la donnée de caractérisation obtenue suite à l'implémentation du Modèle C identifiant l'inhibiteur présent dans l'échantillon sanguin analysé, ledit modèle de régression ayant été entrainé sur un jeu de données obtenu dans des conditions de mesure identiques à celles de l'étape a. ci-dessus (dans le cadre de la détection), équivalente à l'étape 1. ci-dessus, et permettant de déterminer en sortie la concentration de l'inhibiteur identifié dans l'échantillon analysé et optionnellement fourniture en sortie, par exemple par allocation dans une variable, de la concentration déterminée par le modèle D.

« Permettant de déterminer en sortie la concentration de l'inhibiteur identifié » peut également s'écrire « déterminant en sortie la concentration de l'inhibiteur identifié » ou « retournant en sortie la concentration de l'inhibiteur identifié ».

Par "modèle de régression D », ou « modèle d'apprentissage automatique de type modèle de régression », il est indiqué la même chose que ce qui précède relativement au « modèle A », au « modèle B » ou au « modèle C », par analogie.

Selon un mode particulier de réalisation, le modèle D est un réseau de neurones, notamment un perceptron multi-couches, en particulier a été obtenu par entraînement d'un réseau de neurones notamment un perceptron multi-couches. Un mode plus particulier de réalisation appliqué à la recherche d'un inhibiteur du facteur Xa est tel que décrit dans la partie expérimentale.

De fait, l'étape d'implémentation du modèle D est une étape nécessairement implémentée par ordinateur, du fait de la complexité de la fonction mathématique empiriquement déterminée par apprentissage.

Des exemples d'implémentation, en particulier des modèles d'implémentation d'un réseau de neurones, notamment un perceptron multi-couches, sont connus de l'homme du métier, au besoin par référence à la littérature dans le domaine (Voir par exemple, de façon non exhaustive, [Géron, 2017]). Plus précisément, peut par exemple être utilisée la librairie scikit-learn (https://scikit-learn.org/). Les paramètres que l'homme du métier pensera à fixer sont, par exemple, et indépendamment ou non les uns des autres, le nombre de couches de neurones, le nombre de neurones par couche et les fonctions d'activation des différents neurones. Mais l'utilisateur dispose d'une certaine liberté d'implémentation en ce qui concerne l'algorithme d'apprentissage ou les paramètres de régularisation. L'homme du métier peut notamment se référer à Géron, 2017 ou Bonaccorso, G. (2017), pour la mise en œuvre de tels modèles, y compris en ce qui concerne les modalités de fixation des paramètres susmentionnés, incluant au besoin les directives contenues de façon explicite ou implicite dans la présente description. Il va de soi que les références « A », « B », « C » et « D » pour les modèles discutés dans la présente description ont été indiquées pour faciliter la lecture, mais que ces lettres peuvent être omises sans que la signification de ce qu'elles indiquent ne soit modifiée. En particulier :

- Un modèle auquel il est fait référence sous la lettre « A » sert à exclure ou attester la présence d'un inhibiteur de l'enzyme de la coagulation du sang ciblée dans l'échantillon analysé, selon l'une quelconque des définitions envisagées dans la présente description pour cet inhibiteur ;
- Un modèle auquel il est fait référence sous la lettre « B », si implémenté, sert à déterminer une "catégorie d'inhibiteur" choisie parmi deux : inhibiteur direct réversible (alternativement appelée « AOD » dans le présent texte), inhibiteur indirect irréversible (alternativement appelée « héparines » dans le présent texte) ;
- Un modèle auquel il est fait référence sous la lettre « C », si implémenté, sert à préciser la caractérisation commencée avec le modèle « B » : parmi les héparines, l'inhibiteur recherché peut être classifié entre la HNF ou la HBPM (que l'inhibiteur recherché soit un inhibiteur du facteur Xa et/ou IIa), et parmi les AOD, l'inhibiteur recherché peut être classifié entre : le Rivaroxaban, l'Apixaban, l'Edoxaban (pour un inhibiteur du facteur Xa) ou le Dabigatran (pour un inhibiteur du facteur IIa) ;
- Un modèle auquel il est fait référence sous la lettre « D », si implémenté, sert à déterminer en sortie la concentration de l'inhibiteur identifié. Ceci est également valable pour un modèle appelé « D2 » dans la présente description, détaillé ci-après : ce modèle sert également à déterminer en sortie la concentration d'un inhibiteur.

A ce stade, on notera que la ou les méthodes ici décrites, que la chaîne d'étapes détaillée ci-dessus soit implémentée en totalité ou en partie, et ainsi que démontré par la preuve de concept objet des exemples, apporte(nt) une solution aux problèmes mentionnés plus haut au travers de la réalisation d'un test enzymatique compétitif combiné avec une méthodologie de traitement algorithmique universelle. La ou les méthodes de l'invention ici décrite(s), sont rendue(s) sensible(s) à la présence, le cas échéant à des concentrations modulables, d'agents anticoagulants anti-Xa ou anti-IIa, le test enzymatique compétitif étant couplé à une Intelligence Artificielle implémentée, selon divers modes de réalisations particuliers, par une cascade de modèles d'apprentissage automatique (déroulée en tout ou partie).Les avantages qui en découlent sont :

- la fourniture d'une méthodologie universelle : un seul test peut être sensible à la présence, et à diverses concentrations, de plusieurs types d'anticoagulants dans un échantillon (anticoagulants anti-Xa et/ou anti-IIa), en particulier, les anticoagulants plus particulièrement décrits selon un mode quelconque de réalisation objet de la présente description ;
- pour la détection de cette présence : un seul test est à réaliser versus une pluralité (cinq pour un dosage de la HNF, la HBPM, le Rivaroxaban, l'Apixaban, l'Edoxaban de façon conventionnelle, ou quatre en méthodologie hybride comme discuté plus haut - un seul kit pour les héparines) ;
- l'obsolescence de la calibration : confort d'utilisation pour l'utilisateur ;
- l'identification de la molécule anticoagulante extrinsèque recherchée parmi un certain nombre : ceci est impossible aujourd'hui, le gain clinique pour le patient est évident.

Selon un mode de réalisation particulier de l'invention, la ou les méthodes décrites dans le présent texte sont appliquées à la recherche d'un inhibiteur du facteur Xa (FXa) choisi parmi : la HNF, la HBPM, le Rivaroxaban, l'Apixaban, l'Edoxaban.

Selon un autre mode de réalisation particulier de l'invention, la ou les méthodes décrites dans le présent texte sont appliquées à la recherche d'un inhibiteur du facteur IIa (FIIa) choisi parmi : la HNF, la HBPM, le Dabigatran.

Selon un mode de réalisation plus précis, la méthode de l'invention est appliquée précisément et spécifiquement à la recherche d'un inhibiteur du facteur Xa.

On comprendra sans mal, en se référant notamment à la partie expérimentale faisant état de l'intérêt, dans le cas d'espèce particulier objet des exemples, d'une méthodologie appelée « méthodologie optimisée pour les AOD », que dans certains cas il peut être approprié d'adapter les cycles de décision pour permettre l'introduction d'une nouvelle étape de mesure expérimentale, permettant par exemple, pour la suite de la chaîne de décisions que la présente invention permet, une validation plus fiable, ou l'adaptation à des objectifs plus précis, ou différents (notamment en ce qui concerne la gamme de concentration en inhibiteur(s) que l'on souhaite pouvoir détecter).

En l'espèce, il a été constaté que l'emploi dudit kit STA® - Liquid Anti-Xa décrit ci-dessus pour la recherche d'AOD, dans des conditions de dilution et sur une période de temps de mesure adaptés pour la recherche d'héparines (le kit étant initialement conçu pour cela), ne permettait pas d'obtenir des mesures sur toute la plage de valeurs de concentrations d'inhibiteurs envisagée: ainsi, un ajustement a été proposé en ce qui concerne la dilution et le temps de mesure, afin d'optimiser, moduler, la méthode selon l'invention ici décrite pour remplir l'objectif particulier de pouvoir cibler certaines concentrations. Cet ajustement a été implémenté sous forme d'une « relance expérimentale » (réalisation d'une nouvelle mesure d'une ou de plusieurs cinétique(s) de compétition via la réalisation d'un dosage enzymatique compétitif), intégrée à une méthode par ailleurs déjà démarrée. L'intérêt d'une relance expérimentale peut dépendre de l'activité inhibitrice de l'anticoagulant anti-Xa (Héparines, AODs) dont la recherche est visée, ainsi que de la fenêtre de concentrations que l'on souhaite mesurer, notamment avec précision. L'invention couvre cependant les situations où dès le départ, les conditions employées par exemple soit dans la « méthodologie universelle », soit dans la « méthodologie optimisée pour les AOD » des exemples, sont utilisées.

Les directives données ici permettent à l'utilisateur de déterminer à partir d'un kit quelconque employé pour les mesures cinétiques, notamment tels que connus dans l'art, les conditions de dilution, si nécessaire, et de temps de mesure, à appliquer de façon homogène à la fois pour les mesures affectant les données d'apprentissage, et celles des données effectivement mesurées en aveugle.

De façon générale, ces ajustements tels que décrits dans les exemples, montrent que l'homme du métier est à même d'adapter la manière d'enchaîner les « modèles » ici décrits ou les modalités de classification(s), de décision(s), et les données comparées, pour intégrer une relance expérimentale, suivant les directives implicitement et explicitement détaillées dans la présente demande.

De fait, comme implicite de la partie expérimentale fournissant une preuve de concept, la méthode de l'invention fonctionne également lorsque des données expérimentales initialement obtenues à l'étape a. de « détection » ci-dessus décrite sont employées à la place de celles issues d'une relance expérimentale : la plage de valeurs de concentrations en inhibiteurs pouvant être détectée est alors simplement différente. Il s'agit ici d'optimisation que l'homme du métier peut réaliser, sur la base de ses connaissances générales, éventuellement des directives fournies ici. Par ailleurs, les ajustements ici décrits, le cas échéant avec les méthodes de test indiquées dans la partie expérimentale, peuvent être réalisés par l'homme du métier pour adapter l'invention décrite à différents kits de détection ou de dosage connus, sans que l'essence de la présente invention en soit changée.

Selon un mode de réalisation particulier, la présente invention concerne ainsi également une méthode d'identification, en partie réalisée *in vitro,* d'un inhibiteur du facteur Xa dont la présence a été détectée dans l'étape b. ii ci-dessus (dans le cadre de la détection), comprenant l'étape de caractérisation additionnelle suivante:

I. si la catégorie d'inhibiteur a été allouée, à l'étape 2. où le modèle B a été implémenté, à la catégorie des héparines, alors :

I.i. fourniture en entrée à un modèle décisionnel de classification C obtenu par entraînement d'un modèle d'apprentissage automatique supervisé, par exemple un modèle choisi parmi l'une des familles suivantes : machine à vecteurs de supports, réseaux de neurones (notamment un perceptron multi-couches), arbres de décision, méthodes d'ensemble, et modèle des k plus proches voisins, notamment dont les paramètres ont été calculés par entraînement, de ladite donnée déterminée, par exemple la variable allouée, à l'étape 2. ci-dessus implémentant le modèle B concernant la catégorie d'inhibiteur dont la présence a été détectée, et de la ou des cinétique(s) compétitives vis-à-vis d'un inhibiteur du facteur Xa, obtenue(s) dans l'étape a. ci-dessus (dans le cadre de la détection) équivalente à l'étape 1. ci-dessus, et

I.ii. caractérisation en sortie par le modèle C de l'inhibiteur recherché, ce dernier étant identifié parmi: la HNF ou la HBPM, et fourniture en sortie, par exemple par allocation dans une variable, de la caractérisation de l'inhibiteur déterminée par le modèle C, ou, alternativement,

II. si la catégorie d'inhibiteur a été allouée, à l'étape 2. où le modèle B a été implémenté, à la catégorie des AOD, alors :

II.i. réalisation d'une nouvelle mesure (relance expérimentale) d'une ou de plusieurs cinétique(s) de compétition via la réalisation d'un dosage enzymatique compétitif sur un échantillon sanguin obtenu d'un (du même) sujet, ledit dosage étant adapté à la réalisation d'une cinétique compétitive vis-à-vis d'un inhibiteur du facteur Xa, avec un facteur de dilution de l'échantillon et/ou une durée de mesure adaptés à une situation de compétition impliquant la présence d'AOD inhibant le facteur Xa, en particulier un facteur de dilution et/ou une durée de mesure différent(s) de celui(ceux) employés pour la mesure de la ou des cinétique(s) obtenue(s) dans l'étape a. ci-dessus (dans le cadre de la détection) équivalente à l'étape 1. ci-dessus, puis

II.ii. fourniture en entrée à un modèle décisionnel de classification C obtenu par entraînement d'un modèle d'apprentissage automatique supervisé, par exemple un modèle choisi parmi l'une des familles suivantes : machine à vecteurs de supports, réseaux de neurones (notamment un perceptron multi-couches), arbres de décision, méthodes d'ensemble, et modèle des k plus proches voisins, notamment dont les paramètres ont été calculés par entraînement, de ladite donnée déterminée, par exemple la variable allouée, à l'étape 2. ci-dessus implémentant le modèle B concernant la catégorie d'inhibiteur dont la présence a été détectée, et de la ou des cinétique(s) obtenue(s) dans l'étape i. précédente, et

II.iii. caractérisation en sortie par le modèle C de l'inhibiteur recherché, ce dernier étant identifié parmi: le Rivaroxaban, l'Apixaban, ou l'Edoxaban, et fourniture en sortie, par exemple par allocation dans une variable, de la caractérisation de l'inhibiteur déterminée par le modèle C. Par « modèle décisionnel de classification C », il est cependant indiqué la même chose que ce qui précède relativement au « modèle A » ou au « modèle B », par analogie.

Selon un mode particulier de réalisation, le modèle décisionnel de classification C'est un réseau de neurones, notamment un perceptron multi-couches, en particulier a été obtenu par entraînement d'un réseau de neurones notamment un perceptron multi-couches. Un mode plus particulier de réalisation appliqué à la recherche d'un inhibiteur du facteur Xa est tel que décrit dans la partie expérimentale. De fait, l'étape d'implémentation du modèle C est une étape nécessairement implémentée par ordinateur, du fait de la complexité de la fonction mathématique empiriquement déterminée par apprentissage.

On comprendra que dans le cadre de la réalisation de l'étape ii. du paragraphe précédent, le modèle décisionnel de classification C visé a été entraîné sur des données d'entraînement appropriées au regard du contexte dans lequel il est employé. En particulier, ces données sont adaptées à la réalisation effective d'une classification compte-tenu des circonstances de mesure impliquant une relance expérimentale (au regard des paramètres permettant la réalisation d'une cinétique compétitive dans le contexte de l'étape i., notamment en ce qui concerne le facteur de dilution de l'échantillon et/ou la durée de mesure employé(es) - en d'autres termes et en toute logique, le « contexte » de réalisation des mesures coïncide/correspond en ce qui concerne les données d'apprentissage utilisées, les paramètres de mesure employés (ou kits de mesure employés), et la zone de mesure visée pour l'échantillon cible testé).

Des exemples d'implémentation, en particulier des modèles d'implémentation d'un réseau de neurones, notamment un perceptron multi-couches, sont connus de l'homme du métier, au besoin par référence à la littérature dans le domaine (Voir par exemple, de façon non exhaustive, [Géron, 2017]). Plus précisément, peut par exemple être utilisée la librairie scikit-learn (https://scikit-learn.org/). Les paramètres que l'homme du métier pensera à fixer sont, par exemple, et indépendamment ou non les uns des autres, le nombre de couches de neurones, le nombre de neurones par couche et les fonctions d'activation des différents neurones. Mais l'utilisateur dispose d'une certaine liberté d'implémentation en ce qui concerne l'algorithmes et les paramètres de régularisation. L'homme du métier peut notamment se référer à Géron, 2017 ou Bonaccorso, G. (2017), pour la mise en œuvre de tels modèles, y compris en ce qui concerne les modalités de fixation des paramètres susmentionnés, incluant au besoin les directives contenues de façon explicite ou implicite dans la présente description.

Dans le cadre de la réalisation d'une nouvelle mesure (relance expérimentale) d'une ou de plusieurs cinétique(s) de compétition via la réalisation d'un dosage enzymatique compétitif propre à ce mode de réalisation particulier, on notera qu'avantageusement :

- cette nouvelle mesure est réalisée sur un échantillon sanguin obtenu du même sujet que celui pour lequel une mesure a été initialement réalisée pour la « détection » (mais distinct, celui employé pour la mesure initiale ne pouvant pas être réutilisé),
- ledit dosage propre à la relance expérimentale est adapté à la réalisation d'une cinétique compétitive vis-à-vis d'un inhibiteur du facteur Xa, naturellement avec un facteur de dilution de l'échantillon et/ou une durée de mesure qui est(sont) adapté(s) à une situation de compétition impliquant la présence d'AOD inhibant le facteur Xa, afin que la mesure ait du sens (l'homme du métier, peut, par exemple en employant les méthodes de vérification illustrées en partie expérimentale, vérifier cette adaptation), et
- de préférence, le facteur de dilution de l'échantillon analysé dans la relance expérimentale et/ou la durée de mesure, est(sont) différent(s) de celui(ceux) employés pour la mesure de la ou des cinétique(s) obtenue(s) dans l'étape a. ci-dessus (dans le cadre de la détection).

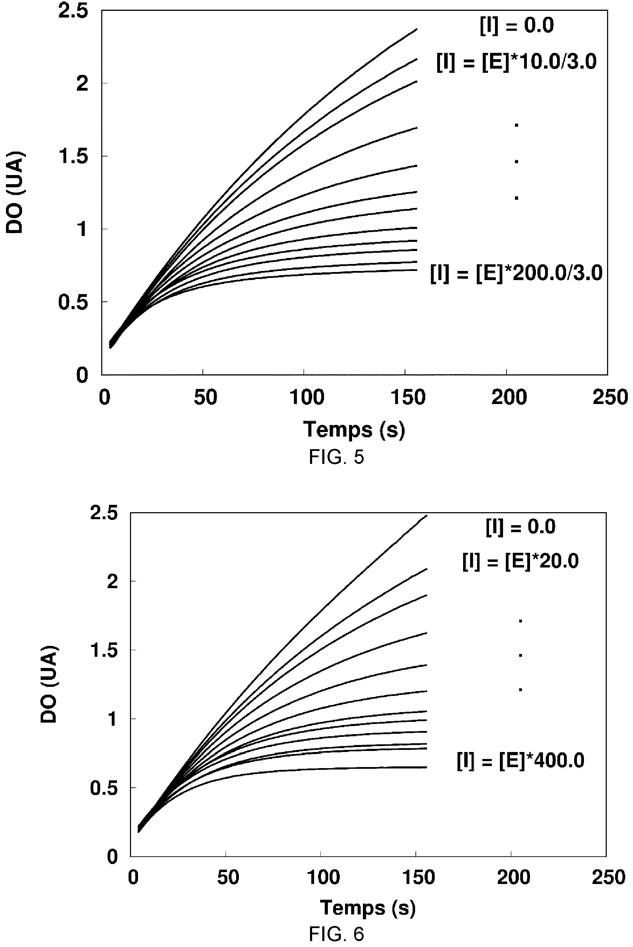

On considère qu'un dosage est adapté à la « réalisation d'une cinétique compétitive vis-à-vis d'un inhibiteur du facteur Xa, naturellement avec un facteur de dilution de l'échantillon et/ou une durée de mesure qui est(sont) adapté(s) à une situation de compétition impliquant la présence d'AOD inhibant le facteur Xa », dès lors qu'il est possible d'obtenir un tracé cinétique conforme aux pratiques conventionnelles dans le domaine. Des exemples de tracés figurent en Figures 5 à 9. L'homme du métier est à même d'adapter les conditions expérimentales d'un test de dosage afin d'obtenir ce type de tracé jugé conforme, dans le sens d'exploitable pour ses besoins.

Comme indiqué ci-après, les paramètres suivants peuvent être pris en compte :

- le substrat employé doit être spécifique du facteur Xa mais peu affin de ce dernier pour ne pas interférer avec la réaction entre l'enzyme et son inhibiteur.
- Par exemple, le substrat employé doit avoir une constante de Michaelis KM considérée élevée, notamment comprise approximativement entre 10 $\mu$M et 1000 $\mu$M ; et/ou

- L'affinité entre l'enzyme et le substrat étant volontairement choisie faible, la constante catalytique kcat de l'enzyme pour le substrat doit être suffisamment élevée, par exemple supérieure à approximativement 10 s-1, ou supérieure à 10 s-1; et/ou
- la concentration initiale en substrat [S]0 doit être suffisante pour permettre la génération du marqueur durant toute la durée d de la mesure, ce qui constitue une adaptation conventionnelle pour l'homme du métier (voir également formule ci-après dans la partie expérimentale). Selon un mode de réalisation, la concentration initiale en substrat [S]0 est inférieure à KM*10 ; et/ou
- La durée de la mesure est choisie suffisamment longue pour permettre à l'anticoagulant, s'il est présent, d'exercer son action inhibitrice sur l'enzyme et que celle-ci soit observable via la mesure ; par exemple une durée comprise entre 10 et 1000 secondes.

Les éléments fournis dans le paragraphe précédent relativement à la situation où le substrat employé est spécifique du facteur Xa, s'appliquent à l'identique à la situation où le substrat employé est spécifique du facteur IIa. De fait, ces éléments s'appliquent de façon générale, à toute réalisation de cinétique envisagée dans la présente description.

Bien qu'il soit indiqué que le facteur de dilution de l'échantillon analysé dans la relance expérimentale et/ou la durée de mesure, est(sont) différent(s) de celui(ceux) employés pour la mesure de la ou des cinétique(s) obtenue(s) dans l'étape a. ci-dessus (dans le cadre de la détection) équivalente à l'étape 1. ci-dessus, dans les deux cas les gammes de valeurs envisageables pour ces deux paramètres peuvent, à titre illustratif uniquement, être :

- Pour le facteur de dilution, l'échantillon est dilué, si nécessaire, dans un intervalle entre 1/2 et 1/50 (les volumes généralement employés pour les mesures cinétiques proprement dites sont comme indiqué ci-dessous dans la présente description, sans que cet aspect ne soit limitatif, puisque les volumes de mesure peuvent varier selon les dispositifs prévus pour de telles mesures), et/ou
- Pour la durée de mesure, elle est comprise entre 10 et 1000 secondes.

Ces paramètres sont des paramètres relativement universels, mais ne sont pas limitatifs car l'homme du métier pourra avoir besoin de les adapter en fonction du matériel utilisé pour le dosage.

La dilution peut être effectuée dans tout tampon conventionnellement utilisé à cette fin, comme connu de l'homme du métier ou indiqué dans des notices de fabricants.

De fait, la présente demande couvre les situations où l'échantillon est dilué, si nécessaire et par rapport à l'échantillon de base obtenu de l'individu analysé, dans un ratio de 1/2, 1/3, 1/4, 1/5, 1/6, 1/7, 1/8, 1/9, 1/10, 1/11, 1/12 ; 1/13, 1/14, 1/15, 1/16, 1/17, 1/18, 1/19, 1/20, 1/21, 1/22 ; 1/23, 1/24, 1/25, 1/26, 1/27, 1/28, 1/29, 1/30, 1/31, 1/32 ; 1/33, 1/34, 1/35, 1/36, 1/37, 1/38, 1/39, 1/40, 1/41, 1/42 ; 1/43, 1/44, 1/45, 1/46, 1/47, 1/48, 1/49, 1/50, ou tout intervalle pris entre l'une quelconque de ces valeurs.

De fait, la présente demande couvre les situations où le volume employé pour les mesures cinétiques proprement dites est de 1, 2, 3, 4, 5, 6, 7, 8, 9, 10, 20, 30, 40, 50, 60, 70, 80, 90, 100, 150, 175, 200, 225, 250, 275, 300, 325, 350, 375, 400 $\mu$L, ou toute valeur intermédiaire comprise dans une gamme de valeur définie comme un intervalle entre l'une quelconque des valeurs juste précisées.

Selon une autre variante de mise en œuvre de l'invention, la méthode ou l'une des méthodes ici décrites, peut être mise en œuvre sur des dispositifs dédiés au point-of-care, par exemple des systèmes pour la réalisation de tests unitaires avec des dispositifs de miniaturisation, tel que par exemple des dispositifs utilisant la microfluidique. Dans ce cas, et de façon conventionnelle, la méthode ou l'une des méthodes ici décrites pourra(ont) être réalisée(s) avec des volumes réactionnels adaptés au dispositif utilisé ou au contexte d'implémentation, lesdits volumes pouvant par exemple, et de façon non limitative, être compris entre 1 et 20 $\mu$L selon le dispositif miniaturisé utilisé. Les temps d'incubation pourront également, le cas échéant et de façon conventionnelle pour l'homme du métier, être adaptés en fonction des caractéristiques dudit dispositif utilisé ou du contexte d'implémentation mis en œuvre. Pour la durée de mesure, et à titre illustratif, elle peut être de 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 secondes, ou toute valeur intermédiaire comprise dans une gamme de valeur définie comme un intervalle entre l'une quelconque des valeurs juste précisées.

L'homme du métier comprendra néanmoins que selon une variante de mise en œuvre de l'invention sur des dispositifs dédiés au point-of-care décrite ci-dessus, ladite durée de mesure pourra être adaptée, de façon conventionnelle pour l'homme du métier

Une relance expérimentale, quand envisagée en fonction des objectifs poursuivis, peut permettre d'optimiser l'identification et/ou le dosage d'un inhibiteur du facteur Xa, présent dans une gamme de concentration plus particulière, ou du moins différente, dans l'échantillon analysé en aveugle. La partie expérimentale fournit des principes aisément reproductibles par l'homme du métier pour vérifier qu'une relance expérimentale est d'intérêt, ou qu'une cinétique peut effectivement être obtenue.

Selon un autre aspect, et de façon analogue à la description qui en est faite ci-dessus, un modèle de régression D pourra être appliqué à la suite du modèle décisionnel de classification C implémenté sur la base d'une relance expérimentale.

Néanmoins, et selon un autre mode de réalisation, la méthode d'identification, en partie réalisée *in vitro,* ici décrite, le cas échéant appliquée à l'identification d'un inhibiteur du facteur Xa, et comprenant une étape additionnelle de dosage quantitatif de l'inhibiteur caractérisé mettant en œuvre un modèle de régression D, peut prendre avantage de la réalisation d'une nouvelle mesure (relance expérimentale) d'une ou de plusieurs cinétique(s) de compétition via la réalisation d'un dosage enzymatique compétitif sur un échantillon sanguin obtenu d'un (du même) sujet, non pas en amont d'un modèle décisionnel de classification C comme décrit ci-dessus, mais en amont de la mise en œuvre dudit modèle de régression D seulement. Dans ce cas, les étapes décrites ci-dessus sont modifiées pour n'intégrer la réalisation d'une nouvelle mesure (relance expérimentale) - selon les mêmes modalités que décrit ci-dessus - qu'avant la mise en œuvre dudit modèle de régression D, en fournissant, de façon analogue aux déroulés ici décrits, audit modèle D la ou les cinétique(s) nouvellement obtenue(s) du fait de la relance expérimentale, et le résultat de l'implémentation du modèle de classification C préalablement mise en œuvre. Dans le cadre d'un tel mode de réalisation, l'apprentissage et la validation du modèle C mis en œuvre d'une part, et l'apprentissage et la validation du modèle D mis en œuvre d'autre part, auront respectivement été effectués sur des jeux de données impliquant des conditions de mesures cinétiques qui permettent une comparaison avec les données obtenues de patients lors de la mise en œuvre de la méthode de l'invention avec un échantillon. C'est-à-dire que, logiquement, dans le cadre d'un tel mode de réalisation, le modèle C aura été entrainé sur la base de mesures cinétiques réalisées dans les mêmes conditions que les mesures « initiales » ici décrites, et le modèle D aura été entrainé sur la base de mesures cinétiques réalisées dans les mêmes conditions que les mesures « de relance expérimentale » ici décrites.

Il va de soi que cette nécessité de comparer des données obtenues dans des conditions comparables (et selon un mode de réalisation particulier, identiques), ou de comparer des données au regard de modèles ayant été obtenus et/ou validés sur la base de mesures effectuées dans des conditions comparables (et selon un mode de réalisation particulier, identiques), sera pris en compte par tout homme du métier mettant en œuvre des modèles de classification ou de régression tels qu'utilisés dans la présente invention. La remarque du présent paragraphe s'applique donc de façon générique à tous les modes de réalisation de la présente description, dans leurs différentes combinaisons.

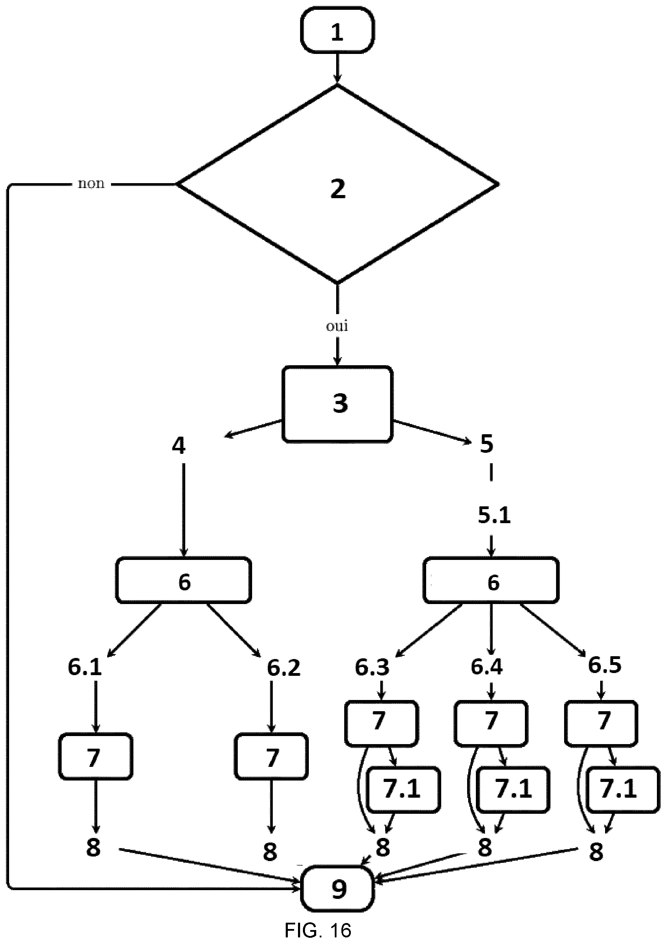

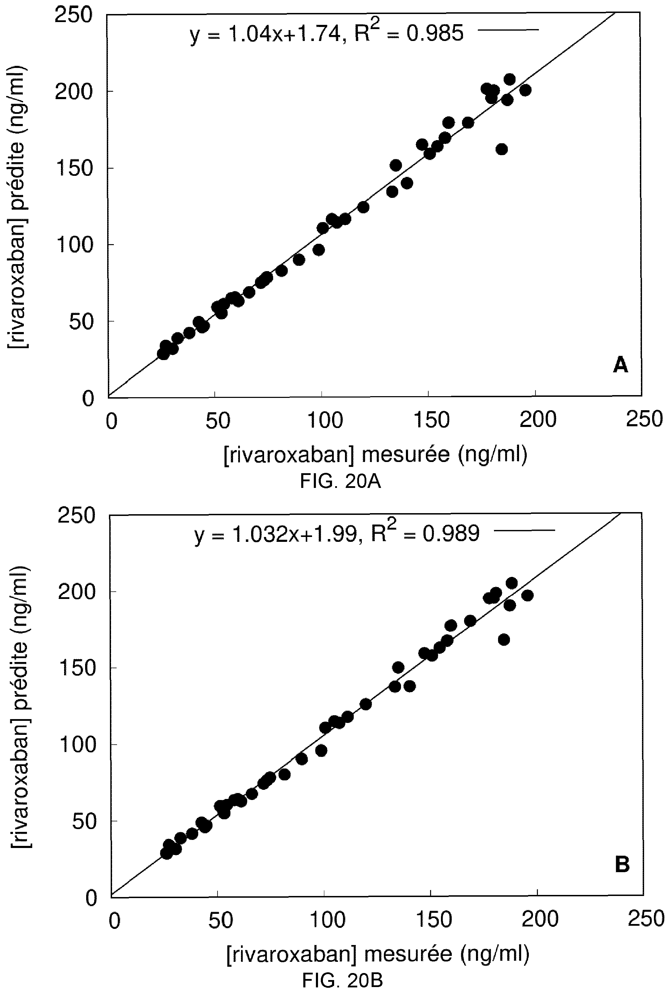

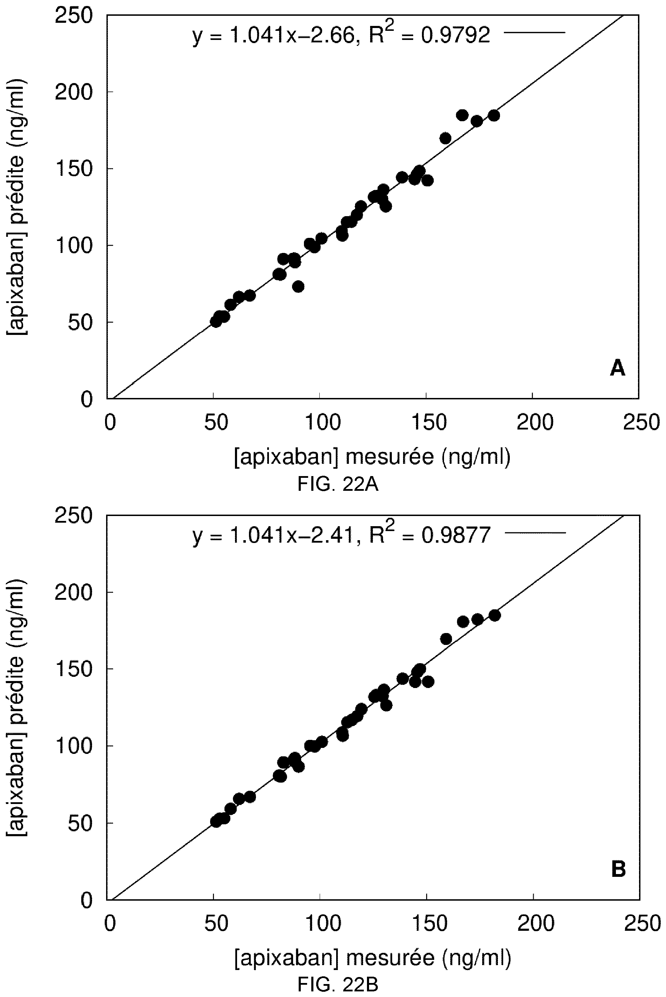

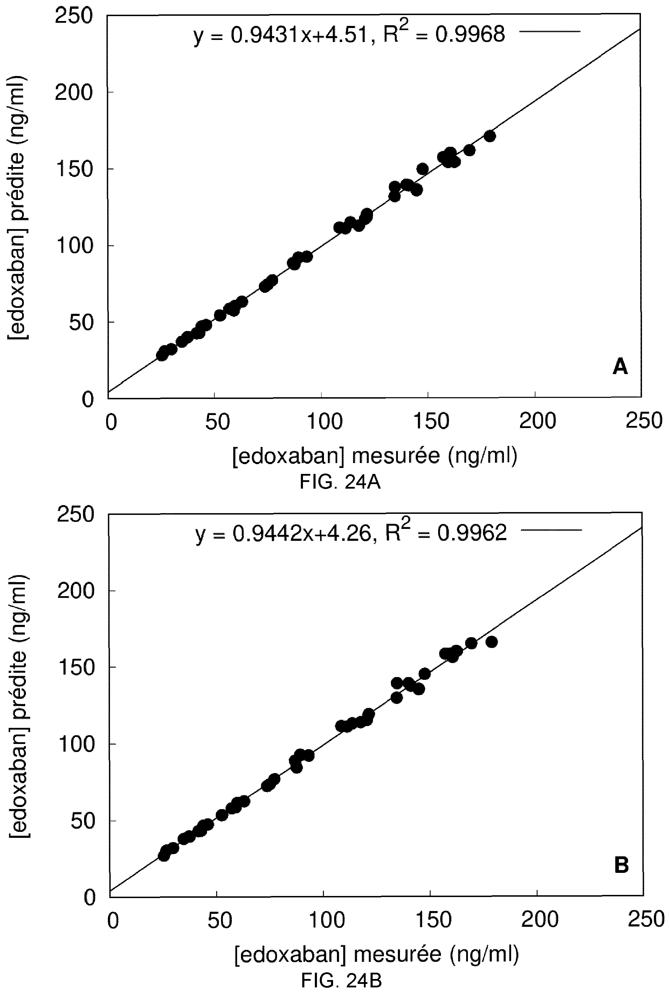

Selon un autre aspect cependant, les inventeurs ont également implémenté un mode de réalisation particulier qui prend avantage d'un recalcul de la concentration en AOD, choisi parmi le rivaroxaban, l'apixaban et l'edoxaban, par la mise en œuvre d'un second modèle de régression, appelé ici modèle de régression D2, utilisant en entrée une cinétique préalablement mesurée par la méthodologie universelle comme ici décrite (plutôt qu'une cinétique mesurée par ladite méthodologie optimisée (pour les AOD), cette dernière ayant généralement été utilisée lors d'un premier calcul de la concentration en AOD choisi parmi le rivaroxaban, l'apixaban et l'edoxaban, ledit calcul employant un premier modèle de régression D). Ce mode de réalisation particulier s'inscrit dans une succession particulière d'étapes préalables représentée dans le schéma de la Figure 16, incluant une étape 7.1. « Recalcul de la concentration en utilisant la cinétique mesurée par la méthodologie universelle ». Les résultats associés à un tel mode de réalisation sont illustrés dans la partie expérimentale qui suit, dans les Sections 3 et 4, sous la terminologie « dosage du rivaroxaban/apixaban/edoxaban mesure universelle », respectivement dans les sections 3.8/4.8 (Figure 20, rivaroxaban), 3.10/4.10 (Figure 22, apixaban), 3.12/4.12 (Figure 24, edoxaban), L'emploi de l'expression « méthodologie universelle » dans ce contexte fait écho au fait que les cinétiques employées pour procéder à un recalcul de la concentration en AOD avec un autre modèle régression, différent du modèle de régression D généralement préalablement utilisé, sont des cinétiques mesurées par une méthodologie universelle comme ici décrite (de fait, de telles cinétiques auront généralement préalablement déjà été mesurées en amont lors des étapes successives d'implémentation de la méthode ici décrite). On pourra substituer, dans ce contexte, l'expression « méthodologie universelle », par « méthodologie améliorée sur la base de la méthodologie universelle », si le contexte ne devait pas suffire à clarifier une différence avec les expressions « méthodologie universelle » et « méthodologie optimisée pour les AODs » employées plus haut et dans la présente description. L'expression « méthodologie améliorée sur la base de la méthodologie universelle » fait plus particulièrement référence à l'ensemble de la chaîne de modèles successivement implémentée pour en arriver à une étape de « recalcul » comme l'étape annotée 7.1 sur la Figure 16.

Comme illustré dans la partie expérimentale, l'intérêt de la mise en œuvre de ce recalcul qui se place à un moment précis en aval de la chaîne de modèles décisionnels comme ici décrits, est l'obtention d'un résultat plus précis pour les concentrations mesurées, pour les faibles concentrations en AOD choisi parmi le rivaroxaban, l'apixaban et l'edoxaban. De ce fait, l'entrée dans ce mode de réalisation particulier est avantageusement réalisée si la concentration en AOD choisi parmi le rivaroxaban, l'apixaban et l'edoxaban, mesurée avec le premier modèle de régression, est inférieure, ou inférieure ou égale à 200 ng/mL.

Ainsi, selon ce mode particulier de réalisation, la méthode de l'invention comprenant une étape additionnelle de dosage quantitatif de l'inhibiteur caractérisé (aussi appelée étape de recalcul de la concentation de l'inhibiteur caractérisé) mettant en œuvre un modèle de régression D2, implémentée à l'issue de la mise en œuvre du modèle de régression D ci-dessus décrit si la concentration en inhibiteur identifié dans l'échantillon analysé, déterminée par le modèle D, est inférieure ou égale à 200 ng/mL, alors fourniture en entrée à un modèle de régression D2, notamment un modèle d'apprentissage automatique supervisé, par exemple un modèle choisi parmi l'une des familles suivantes : machines à vecteurs de support, réseaux de neurones, arbres de décision, méthodes d'ensemble, et modèle des k plus proches voisins, ledit modèle ayant été entrainé sur un jeu de données obtenu dans des conditions de mesure identiques à celles de l'étape a. ci-dessus (dans le cadre de la détection), de la ou des cinétique(s) obtenue(s) dans l'étape a. ci-dessus (dans le cadre de la détection), ledit modèle de régression D2 permettant de calculer (ou plutôt, recalculer par rapport au calcul déjà effectué en implémentant un modèle de régression D) en sortie la concentration en inhibiteur identifié dans l'échantillon analysé et optionnellement fourniture en sortie, par exemple par allocation dans une variable, de la concentration déterminée par le modèle D2.

« Permettant de calculer/recalculer en sortie la concentration de l'inhibiteur identifié » peut également s'écrire « Permettant de déterminer en sortie la concentration de l'inhibiteur identifié » ou « déterminant en sortie la concentration de l'inhibiteur identifié » ou « retournant en sortie la concentration de l'inhibiteur identifié ». Cette donnée pourra venir prendre la place de la donnée déjà calculée et/ou retournée par l'emploi du modèle de régressions D, ou venir en addition, afin d'illustrer par exemple une différence.

Selon un aspect de ce mode de réalisation, si la concentration en AOD choisi parmi le rivaroxaban, l'apixaban et l'edoxaban, mesurée avec le premier modèle de régression D, est supérieure, ou supérieure ou égale, à 200 ng/mL, alors la mise en œuvre d'un modèle de régression D2 n'est pas implémentée, le résultat final de la chaîne de modèles décisionnels ou de régression s'achevant avec la mise en œuvre du modèle de régression D2.

L'homme du métier appréciera que la valeur seuil de 200 ng/mL a été déterminée dans les conditions expérimentales testées pour optimiser les résultats pouvant être obtenus avec la chaîne de modèles décisionnels ou de régression ici décrite.

Un mode de réalisation particulier impliquant un modèle de régression D2 n'est cependant qu'une caractéristique additionnelle, avantageuse, mais dont l'implémentation n'est pas indispensable pour aboutir à des résultats déjà pertinents (voir la section expérimentale qui décrit des implémentations sans cette fonctionnalité additionnelle supplémentaire): il s'agit d'une caractéristique additionnelle à un point particulier d'une chaîne de modèles par ailleurs généralement décrite ici selon ses diverses implémentations possibles.

Les caractéristiques modifiées dans le cadre d'une relance expérimentale par rapport à celles d'une mesure de cinétique(s) réalisée(s) initialement pour la réalisation d'une « détection », illustrée ici par une référence aux cinétique(s) obtenue(s) dans l'étape a. décrite ci-dessus, sont également rappelées plus haut dans la présence description, et illustrées dans la partie expérimentale par le biais d'expériences et de directives à l'attention de l'homme du métier, qui saura ainsi en distinguer les caractéristiques.

Par « modèle de régression D2 », ou « modèle d'apprentissage automatique de type modèle de régression », il est indiqué la même chose que ce qui précède relativement au « modèle A », au « modèle B » ou au « modèle C » ou au « modèle D », par analogie.

Selon un mode particulier de réalisation, le modèle D2 est un réseau de neurones, notamment un perceptron multi-couches, en particulier a été obtenu par entraînement d'un réseau de neurones notamment un perceptron multi-couches. Un mode plus particulier de réalisation appliqué à la recherche d'un inhibiteur du facteur Xa est tel que décrit dans la partie expérimentale.

De fait, l'étape d'implémentation du modèle D2 est une étape nécessairement implémentée par ordinateur, du fait de la complexité de la fonction mathématique empiriquement déterminée par apprentissage. Un modèle de type D2 est entrainé sur des données obtenues en utilisant une méthodologie de type « méthodologie universelle », telle que décrite ici.

Des exemples d'implémentation, en particulier des modèles d'implémentation d'un réseau de neurones, notamment un perceptron multi-couches, sont connus de l'homme du métier, au besoin par référence à la littérature dans le domaine (Voir par exemple, de façon non exhaustive, [Géron, 2017]). Plus précisément, peut par exemple être utilisée la librairie scikit-learn (https://scikit-learn.org/). Les paramètres que l'homme du métier pensera à fixer sont, par exemple, et indépendamment ou non les uns des autres, le nombre de couches de neurones, le nombre de neurones par couche et les fonctions d'activation des différents neurones. Mais l'utilisateur dispose d'une certaine liberté d'implémentation en ce qui concerne l'algorithme d'apprentissage ou les paramètres de régularisation. L'homme du métier peut notamment se référer à Géron, 2017 ou Bonaccorso, G. (2017), pour la mise en œuvre de tels modèles, y compris en ce qui concerne les modalités de fixation des paramètres susmentionnés, incluant au besoin les directives contenues de façon explicite ou implicite dans la présente description. Il va de soi que les références « A », « B », « C », « D » et « D2 » pour les modèles discutés dans la présente description ont été indiquées pour faciliter la lecture, mais que ces lettres peuvent être omises sans que la signification de ce qu'elles indiquent ne soit modifiée. La mise en œuvre d'un modèle de type D2 tel qu'ici décrit se comprend dans une étape de recalcul d'une concentration alors qu'une concentration d'inhibiteur inférieure à 200 ng/mL a préalablement été détectée, comme décrit ici. Selon un mode de réalisation, la mesure *in vitro* d'une cinétique de compétition par dosage enzymatique compétitif sur un échantillon sanguin obtenu d'un sujet, comprend (que ce soit dans le cadre d'une mesure de cinétique initiale ou dans le cadre d'une relance expérimentale) les étapes suivantes:

a. fourniture d'un échantillon sanguin dilué ou non, puis
b. ajout à l'échantillon sanguin d'un substrat spécifique soit du facteur Xa soit du facteur IIa selon l'enzyme ciblée et l'inhibiteur recherché, notamment un substrat porteur d'un marqueur détectable, par exemple visualisable (par exemple un marqueur chromogène, fluorescent ou chemofluorescent) et en particulier un substrat chromogénique ou fluorogénique,
c. incubation avec élévation de la température du mélange obtenu en b. à une température entre (y compris les bornes) 35 et 39 °C, en particulier 37°C,
d. ajout au mélange réactionnel issu de c. de l'enzyme ciblée de la coagulation choisie parmi le facteur Xa et le facteur IIa, selon le substrat ajouté à l'étape b., de façon à déclencher la compétition entre une réaction d'inhibition et la réaction enzymatique provoquée,
e. mesure par un instrument, au cours du temps, de la quantité de produit résultant de la transformation du substrat du fait de l'action de l'enzyme analysée sur ce dernier (facteur Xa ou facteur IIa), le cas échéant via la mesure d'un marqueur associé au substrat, libéré lors de ladite réaction enzymatique, et enregistrement de la cinétique obtenue.

Selon un exemple de réalisation, l'instrument est un appareil STA-R de Stago. Cet exemple n'est cependant pas limitatif. Tout instrument embarquant un spectrophotomètre peut être utilisé. D'autres exemples incluent les appareils connus et commercialisés par Diagnostica Stago sous les noms Compact Max, STA-R ou STA-R Max.

Selon un mode particulier de réalisation, le dosage enzymatique compétitif est spécifique du facteur Xa, et:

a. l'échantillon sanguin utilisé est un échantillon de plasma dilué au 1/2 dans du tampon Owren Koller,
b. dans l'étape b. le substrat est le réactif MAPA-Gly-Arg-pNA,
c. dans l'étape c. la durée d'incubation est de 240 secondes, à 37°C,
d. le facteur Xa ajouté au mélange dans l'étape d. est du facteur Xa bovin (ou alternativement selon un autre mode de réalisation, du facteur Xa humain, ou recombinant, ou toute alternative connue de l'homme du métier),
e. la mesure de la libération de la paranitroaniline (pNA) dans l'étape e. est réalisée par colorimétrie à 405 nm toutes les deux secondes pendant 156 secondes, sur un instrument approprié, de type STA-R commercialisé par Diagnostica Stago (voir variants cités à titre non limitatif ci-dessus). Selon un autre mode particulier de réalisation, le dosage enzymatique compétitif est spécifique du facteur Xa, et:

a. l'échantillon sanguin utilisé est un échantillon de plasma dilué au 1/8ème dans du tampon Owren Koller,

b. dans l'étape b. le substrat est le réactif MAPA-Gly-Arg-pNA,

c. dans l'étape c. la durée d'incubation est de 240 secondes, à 37°C,

d. le facteur Xa ajouté au mélange dans l'étape d. est du facteur Xa bovin (ou alternativement selon un autre mode de réalisation, du facteur Xa humain, ou recombinant, ou toute alternative connue de l'homme du métier),

e. la mesure de la libération de la paranitroaniline (pNA) dans l'étape e. est réalisée par colorimétrie à 405 nm toutes les deux secondes pendant 86 secondes, sur un instrument approprié, de type STA-R commercialisé par Diagnostica Stago (voir variants cités à titre non limitatif ci-dessus).

Selon un mode particulier de réalisation, le dosage enzymatique compétitif est spécifique du facteur IIa, et:

a. l'échantillon sanguin utilisé est un échantillon de plasma dilué au 1/12 dans du tampon TRIS EDTA (notamment ph 8.4),

b. dans l'étape b. le substrat est le réactif EtM-SPro-Arg-pNA,

c. dans l'étape c. la durée d'incubation est de 240 secondes, notamment à 37°C,

d. le facteur IIa ajouté au mélange dans l'étape d. est du facteur IIa bovin (ou alternativement selon un autre mode de réalisation, du facteur IIa humain, ou recombinant, ou toute alternative connue de l'homme du métier),

e. la mesure de la libération de la paranitroaniline (pNA) dans l'étape e. est réalisée par colorimétrie à 405 nm toutes les deux secondes pendant 156 secondes, sur un instrument approprié, de type STA-R commercialisé par Diagnostica Stago (voir variants cités à titre non limitatif ci-dessus). Selon une variante applicable à tous les modes de réalisation décrits de façon générique ou particulière dans la présente demande, le dosage enzymatique compétitif est réalisé sur un dispositif miniaturisé, par exemple un dispositif utilisant la microfluidique, dans un volume réactionnel entre 1 et 20 $\mu$L. La méthode selon l'invention, selon toutes ses variantes ici décrites, peut en effet avantageusement, compte tenu des problèmes qu'elle permet de résoudre, être implémentée via un dispositif miniaturisé. Selon un mode de réalisation, applicable à l'un quelconque des modes de réalisation ici décrit, si le substrat est un substrat fluorométrique, alors la mesure de la libération du fluorophore, par exemple l'AMC, est réalisée par fluorométrie.

Selon un mode de réalisation particulier de l'invention, l'échantillon sanguin analysé est un échantillon de plasma.

Selon un autre mode de réalisation particulier de l'invention, l'échantillon sanguin analysé est un échantillon de sang total.

Dans un mode de réalisation particulier de l'invention, les méthodes ici décrites permettent le dosage quantitatif d'un inhibiteur du facteur Xa sur les plages suivantes, définies par rapport à la concentration finale [E] en molaire dans le test en facteur Xa :

- une concentration en HNF comprise entre approximativement [E]*10.0/3.0 et approximativement [E]*200.0/3.0. Les différents volumes en échantillon, tampon et réactifs sont choisis et ajustés afin de doser idéalement cet inhibiteur du facteur Xa dans l'intervalle [0.1, 2.0] UI/mL ;
- une concentration en HBPM comprise entre approximativement [E]*20.0 et approximativement [E]*400.0. Les différents volumes en échantillon, tampon et réactifs sont choisis et ajustés afin de doser idéalement cet inhibiteur du facteur Xa dans l'intervalle [0.1, 2.0] UI anti-Xa/mL ;
- une concentration en rivaroxaban comprise entre approximativement [E]/6.0 et approximativement [E]*3.0 . Les différents volumes en échantillon, tampon et réactifs sont choisis et ajustés afin de doser idéalement cet inhibiteur du facteur Xa dans l'intervalle [20, 600] ng/mL ;
- une concentration en l'apixaban comprise entre approximativement [E]/6.0 et approximativement [E]*3.0. Les différents volumes en échantillon, tampon et réactifs sont choisis et ajustés afin de doser idéalement cet inhibiteur du facteur Xa dans l'intervalle [20, 600] ng/mL.
- une concentration en edoxaban comprise entre approximativement [E]/8.0 et approximativement [E]*3.0. Les différents volumes en échantillon, tampon et réactifs sont choisis et ajustés afin de doser idéalement cet cet inhibiteur du facteur Xa dans l'intervalle [20, 600] ng/mL.

A noter, l'homme du métier pourra, sur la base des directives ici fournies, déterminer sans difficulté les concentrations en inhibiteurs qu'il est possible de doser en réalisant la méthode de test définie dans les exemples. De plus, par rapport aux indications données ci-dessus, l'homme du métier est en mesure de déterminer sans difficulté qu'une dilution de l'échantillon conduit à l'obtention du dosage d'une concentration cible dans des intervalles de mêmes valeurs, au facteur de dilution près.

Le choix de volumes appropriés en échantillon, tampon et réactifs constitue une mise au point classique dans le domaine des dosages enzymatiques, et de l'invention.

A titre d'exemple particulier de l'invention, obtenu dans les conditions expérimentales précises des exemples, les méthodes ici décrites permettent le dosage quantitatif d'un inhibiteur du facteur Xa sur la plage :

- de 0.0 à 2.0 UI/mL pour les HNF ;
- de 0.0 à 2.0 UI anti-Xa/mL pour les HBPM ;
- de 0.0 à 180.0 ng/mL pour le rivaroxaban ;

- de 0.0 à 190.0 ng/mL pour l'apixaban ;
- de 0.0 à 230.0 ng/mL pour l'edoxaban.

Selon un autre exemple particulier, obtenu dans les conditions expérimentales précises des exemples, impliquant une relance expérimentale telle qu'ici décrite, le dosage quantitatif d'un inhibiteur du facteur Xa est permis sur la plage :

- de 0.0 à 2.0 UI/mL pour les HNF ;
- de 0.0 à 2.0 UI anti-Xa/mL pour les HBPM ;
- de 0.0 à 720.0 ng/mL pour le rivaroxaban ;
- de 0.0 à 760.0 ng/mL pour l'apixaban ;
- de 0.0 à 920.0 ng/mL pour l'edoxaban.

Pour une conversion en molaire des valeurs précitées, on considèrera la règle de conversion suivante : taux d'inhibiteur I en nM = (taux de I en ng/mL) / (poids moléculaire de I en kg/mol) * (volume échantillon) / (volume total).

Dans un mode de réalisation particulier de l'invention la(les) cinétiques de l'échantillon testé sont enregistrées pendant une durée comprise entre 10 et 10000, ou entre 10 et 9000, ou entre 10 et 8000, ou entre 10 et 7000, ou entre 10 et 6000, ou entre 10 et 5000, ou entre 10 et 4000, ou entre 10 et 3000, ou entre 10 et 2000, ou entre 10 et 1000, ou entre 10 et 900, ou entre 10 et 800, ou entre 10 et 700, ou entre 10 et 600, ou entre 10 et 500, ou entre 10 et 400, ou entre 10 et 300, ou entre 10 et 200, ou entre 20 et 200, ou entre 30 et 200, ou entre 40 et 200, ou entre 50 et 200, ou entre 60 et 200, ou entre 70 et 200, ou entre 80 et 150, secondes. Des exemples particuliers sont donnés en partie expérimentale, qui peuvent être appliqués à toute implémentation telle qu'ici décrite. Il découle des explications données ici que l'homme du métier est à même d'ajuster le temps de mesure en fonction de la vérification qu'une cinétique exploitable par lui, peut être obtenue (le cas échéant en suivant les directives de test fournies en partie expérimentale).

Des modèles décisionnels de classification de type A, B, C ou des modèles de régression D, incluant un modèle de régression D2, sont donnés dans la partie expérimentale, à titre d'exemple: les hyper-paramètres qui ont été obtenus suite à l'optimisation sur des données de validation sont indiqués. Il va cependant de soi que les valeurs absolues qui peuvent être affectées à ces hyperparamètres peuvent dépendre des jeux de données utilisés. Ils ne peuvent donc être figés. L'homme du métier peut cependant aisément définir, entraîner et évaluer un modèle d'apprentissage automatique en utilisant les indications fournies ici à titre de guide, et les ouvrages de référence à sa disposition (par exemple, de façon non limitative Géron, A. (2017) ou Bonaccorso, G. (2017), cités ici).

A titre d'exemple :

a. un modèle décisionnel de classification A peut résulter de l'entraînement d'une machine à vecteurs de support, ou d'un réseau de neurones artificiels, notamment un perceptron multi-couches, avec un jeu de données comprenant des cinétiques obtenues dans des conditions identiques à celles implémentées lors de la réalisation de l'étape a. de détection ci-dessus décrite, de façon semi-supervisée, avec le cas échéant des hyper-paramètres résultants qui sont comme indiqués en partie expérimentale, et/ou

b. un modèle décisionnel de classification B peut résulter de l'entraînement d'un modèle des k plus proches voisins, ou d'un réseau de neurones artificiels, notamment un perceptron multi-couches, avec un jeu de données comprenant des cinétiques obtenues dans des conditions identiques à celles implémentées lors de la réalisation de l'étape a. de détection ci-dessus décrite, avec le cas échéant des hyper-paramètres résultants qui sont, par exemple, une valeur k égale à 5, et la métrique étant la distance euclidienne, et/ou

c. un modèle décisionnel de classification C peut résulter de l'entraînement d'un réseau de neurones artificiels, notamment un perceptron multi-couches avec un jeu de données comprenant des cinétiques obtenues dans des conditions identiques à celles implémentées lors de la réalisation de l'étape a. de détection ci-dessus décrite, ou obtenues dans des conditions identiques à celles implémentées lors de la réalisation d'une relance expérimentale., le cas échéant, avec par exemple, des hyper-paramètres résultants qui sont comme indiqués en partie expérimentale, et/ou

d. un modèle de régression D, ou D2, peut résulter de l'entraînement d'un réseau de neurones artificiels, notamment un perceptron multi-couches avec un jeu de données comprenant des cinétiques obtenues dans des conditions identiques à celles implémentées lors de la réalisation de l'étape a. de détection ci-dessus décrite, ou obtenues dans des conditions identiques à celles implémentées lors de la réalisation d'une relance expérimentale (dans le cas d'un modèle de régression D), le cas échéant, avec par exemple, des hyper-paramètres résultants qui sont comme indiqués en partie expérimentale.

De fait, un algorithme d'apprentissage selon l'invention implémente avantageusement un ou des modèle(s) décisionnel(s) qui permettent d'atteindre, lorsque testé(s) sur des données de test (permettant d'évaluer la performance globale et réelle du modèle entraîné et optimisé) une précision dans la justesse du résultat qu'il(s) retourne(nt), supérieure ou égale à 70%, ou 75%, ou 80%, ou 85%, ou 90%, ou 95%, appliquée au cumul des différent(s) modèles(s) utilisé(s) et/ou à chaque modèle utilisé.

Une référence est faite à la partie expérimentale qui indique, à titre d'exemple, une méthode particulière d'évaluation de cette performance.

La précision s'évalue en comparant le résultat prédit par ledit modèle entrainé, aux valeurs réelles. Des exemples illustratifs sont fournis dans les Tables 1 à 6 de la présente description (partie expérimentale). Selon un autre aspect, un modèle de régression, lorsqu'il est utilisé, peut permettre d'atteindre, lorsque testé sur des données de test (permettant d'évaluer la performance globale et réelle du modèle entraîné, voire optimisé) un résultat en sortie qui est caractérisé par une pente de régression linéaire comprise entre 0,9 et 1,1, et un coefficient de détermination R2 supérieur ou égal à 0,70, ou 0,80, ou 0,90, ou 0,95 (selon les critères du CLSI EP9-A2). Une référence est faite à la partie expérimentale qui indique, à titre d'exemple, une méthode particulière d'évaluation de cette performance. La performance indiquée ici étant atteinte, on considèrera que le résultat atteint par la méthode selon l'invention est au moins qualitativement équivalent à un résultat obtenu via une approche dite conventionnelle (c'est-à-dire une méthode de dosage classiquement utilisée à ce jour, notamment en clinique).

Selon un mode de réalisation, les données d'entraînement et de validation utilisées pour l'apprentissage automatique supervisé de l'un au moins des modèles décrits ici, en ce qui concerne les cinétiques fournies aux modèles, sont du type de celles qui peuvent être employées pour des expériences permettant de déterminer des courbes de pré-calibration ou de calibration de cinétiques déjà conventionnellement réalisées dans le domaine, notamment lors de la mise sur le marché de kits utilisables pour la réalisation de telles cinétiques. Le nombre de données et le choix des patients ayant permis de les obtenir permettent avantageusement de s'assurer que les données fournies permettront un apprentissage efficace. La partie expérimentale fournit des exemples quantitatifs du type de données qui peuvent être fournies, avec un résultat probant en terme de fiabilité. L'homme du métier est ainsi, ou de façon générale, à même de déterminer les échantillons de départ nécessaires.

Selon un mode de réalisation, la fourniture en entrée à un modèle d'une cinétique obtenue expérimentalement consiste à fournir les couples de valeurs constitués par chaque valeur mesurée pour chaque point de mesure discret effectué au cours du temps de mesure.

L'invention a également trait à un système de traitement de données ou un dispositif pour la détection, dans un échantillon biologique, de la présence ou l'identification d'un inhibiteur du facteur Xa (FXa) ou du facteur lia (FIIa), ledit système de traitement de données ou dispositif comme défini en revendication 13. Un tel système de traitement de données ou dispositif comprend des moyens pour mettre en œuvre au moins l'étape b. de la méthode « de détection » ici décrite, incluant la mise en œuvre d'un modèle A de de l'une quelconque des méthodes ici décrites, et des moyens pour fournir en entrée une ou plusieurs cinétique(s) de compétition obtenues via la réalisation d'un dosage compétitif vis-à-vis, soit d'un inhibiteur du facteur Xa, soit d'un inhibiteur du facteur IIa, sur un échantillon sanguin préalablement obtenu d'un sujet, notamment via un appareil de mesure, et des moyens pour fournir en sortie les variables générées lors de cette étape. Le cas échéant, le système de traitement de données ou dispositif comprend également des moyens pour mettre en œuvre l'étape mettant en œuvre le modèle B de l'une quelconque des méthodes ici décrites, et optionnellement comprend également des moyens pour fournir en entrée et/ou sortie les variables générées lors de de ces étapes, et optionnellement comprend également des moyens pour mettre en œuvre les étapes impliquant les modèles décisionnels de classification ou de régression C, D, et le cas échéant D2, ici décrits, selon toutes leurs variantes, notamment des moyens permettant de faire usage d'une matrice résultant de l'apprentissage ayant permis de fixer les paramètres de ces modèles, pour retourner le résultat de classification ou de régression prenant en compte les variables fournies au(x) modèle(s).

Selon un mode de réalisation un tel système de traitement de données ou un tel dispositif inclut un appareil de mesure de cinétique(s) tel que décrites ici, notamment un appareil de mesure nécessaire pour réaliser une étape a. de mesure d'une ou plusieurs cinétique(s) de compétition via la réalisation d'un dosage compétitif sur un échantillon sanguin préalablement obtenu d'un sujet, tel qu'ici décrite. Selon un autre mode de réalisation, un tel système de traitement de données ou un tel dispositif fait appel à un tel appareil de mesure, notamment distant. De fait, un appareil de mesure pourrait également exécuter ladite étape a. de mesure, sous commande, notamment d'un système de traitement de données ou d'un dispositif, de façon automatique ou semi-automatique.

A noter, une mesure dite « manuelle », ou du moins semi-automatique si un appareil semi-automatique est employé, n'est pas exclue pour l'implémentation d'une méthode selon l'invention, une telle mesure nécessitant néanmoins, pour sa réalisation, les agents conventionnellement utilisés dans le domaine de la réalisation de mesure d'une ou plusieurs cinétique(s) de compétition via la réalisation d'un dosage compétitif sur un échantillon sanguin préalablement obtenu d'un sujet (voir le contenu d'un kit ici décrit, pour un exemple), en sus d'un appareil de mesure.

L'invention porte également sur un tel système de de traitement de données ou un tel dispositif, comprenant en outre un processeur adapté pour mettre en œuvre les étapes indiquées ci-dessus et dans la présente description.

Notamment, et de façon non limitative, l'invention peut être implémentée via un poste informatique (éventuellement dédié) connecté en réseau ou via une connexion filaire, ou au sein d'un système embarqué (dédié).

De fait, on conçoit que les étapes à partir de l'étape b. de la méthode « de détection » ici décrite, et les étapes subséquentes dès lors qu'elles emploient des modèles de type A, B, C, D ou D2 comme ici décrits, nécessitent un ordinateur pour leur mise en œuvre. L'étape a. de mesure d'une ou plusieurs cinétique(s) de compétition peut être opérée de façon séparée, de façon manuelle, semi-automatique ou automatique, ou des moyens peuvent être intégrés, notamment à un système de traitement de données ou un dispositif décrits ici, pour opérer de façon contrôlée, à partir dudit système de traitement de données ou dudit dispositif, la réalisation de mesure(s) cinétique(s).

L'invention porte encore, comme défini en revendication 14, sur un programme d'ordinateur comprenant des instructions qui, lorsque le programme est exécuté par un ordinateur, conduisent celui-ci à mettre en œuvre au moins l'étape b. de la méthode de « détection » ici décrite. Le cas échéant, ces instructions conduisent également celui-ci à mettre en œuvre l'étape mettant en œuvre le modèle B de l'une quelconque des méthodes ici décrites, et optionnellement le programme comprend des instructions permettant de récupérer en entrée et/ou fournir en sortie les variables générées lors de de ces étapes. Optionnellement le programme comprend des instructions conduisant celui-ci à mettre en œuvre les étapes impliquant les modèles décisionnels de classification ou de régression C, D, et le cas échéant D2, ici décrits, adapté à un mode de réalisation quelconque tel que décrit.

Selon un mode de réalisation particulier, un tel programme d'ordinateur comprend des instructions qui conduisent un système de traitement de données ou un dispositif, notamment un dispositif incluant un appareil de mesure de cinétiques telles que définies et décrites dans la présente demande, ou faisant appel à un tel appareil de mesure, notamment distant, à exécuter les étapes au moins l'étape b. de la méthode de « détection » ici décrite, et le cas échéant également l'étape mettant en œuvre le modèle B de l'une quelconque des méthodes ici décrites, et optionnellement conduisent à exécuter les étapes impliquant les modèles décisionnels de classification ou de régression C, D, et le cas échéant D2, ici décrits.

Selon un mode particulier de réalisation, un programme d'ordinateur comprend en outre des instructions qui, lorsque le programme est exécuté par un ordinateur, conduisent celui-ci à mettre également en œuvre, de façon contrôlée par les instructions du programme, une étape a. de mesure d'une ou plusieurs cinétique(s) de compétition via la réalisation d'un dosage compétitif sur un échantillon sanguin préalablement obtenu d'un sujet, tel qu'ici décrite.

Alternativement, une méthode *in vitro* de détection dans un échantillon biologique de la présence d'un inhibiteur d'une enzyme de la coagulation du sang, selon l'invention dans tous ses modes de réalisation ici exposés, peut voir son étape a. de mesure d'une ou de plusieurs cinétique(s) de compétition, réalisée séparément d'un système de contrôle centralisant la commande des moyens nécessaires aux étapes subséquentes. A l'inverse, selon un autre mode de réalisation, des moyens pour la réalisation de ladite étape a. de façon centralisée, au moins en partie compte-tenu de la spécificité de le mesure réalisée, peuvent également être centralisés par le même organe de commande sous la forme d'un programme est exécuté par un ordinateur tel qu'ici décrit.

L'invention porte encore, comme défini en revendication 15, sur un support d'enregistrement lisible par ordinateur comprenant des instructions qui, lorsqu'elles sont exécutées par un ordinateur, conduisent celui-ci mettre en œuvre au moins l'étape b. de la méthode de « détection » ici décrite. Le cas échéant les instructions conduisent également celui-ci à mettre en œuvre également l'étape mettant en œuvre le modèle B de l'une quelconque des méthodes ici décrites, et optionnellement permettent également de récupérer en entrée et/ou fournir en sortie les variables générées lors de de ces étapes, et optionnellement conduisent celui-ci à mettre en œuvre les étapes impliquant les modèles décisionnels de classification ou de régression C, D, et le cas échéant D2, ici décrits.

Selon un mode particulier de réalisation, un support d'enregistrement lisible par ordinateur comprend en outre des instructions qui, lorsqu'elles sont exécutées par un ordinateur, conduisent celui-ci à mettre également en œuvre, une étape a. de mesure d'une ou plusieurs cinétique(s) de compétition via la réalisation d'un dosage compétitif sur un échantillon sanguin préalablement obtenu d'un sujet, tel qu'ici décrite.

L'invention porte également, comme défini en revendication 16, sur un support de données lisible par ordinateur sur lequel est enregistré le programme d'ordinateur ici décrit, ou un signal d'un support de données portant le programme d'ordinateur ici décrit.

L'invention porte enfin, comme défini en revendication 17, sur un kit, en particulier adapté pour la mise en oeuvre d'une méthode telle qu'ici décrite dans un quelconque mode de réalisation, comprenant:

a. Un substrat spécifique du FXa ou du FIIa, par exemple le substrat MAPA-Gly-Arg-pNA pour le facteur Xa, par exemple le substrat EtM-SPro-Arg-pNA pour le facteur IIa, et
b. Optionnellement, du FXa et/ou du FIIa, par exemple du facteur Xa bovin, par exemple du facteur Xa humain, par exemple du facteur IIa bovin, par exemple du facteur IIa humain, et
c. Optionnellement, un ou plusieurs tampons appropriés, par exemple du tampon Owren Koller, par exemple du tampon Tris EDTA, et
d. Optionnellement des instructions pour la mise en œuvre de mesure(s) d'une ou de plusieurs cinétique(s) de compétition via la réalisation d'un dosage enzymatique compétitif employant le substrat, et

e. un système et/ou dispositif et/ou programme d'ordinateur et/ou support de données lisible par ordinateur ici décrit,

f. et optionnellement des instructions permettant de mettre en œuvre la méthode selon l'invention, le cas échéant en lien avec les instructions du point d. ci-dessus, relativement aux caractéristiques du dosage enzymatique compétitif à mettre en œuvre,

g. et optionnellement des instructions relatives à l'utilisation d'un signal d'un support de données ici décrit, pour la mise en œuvre d'une méthode selon l'un quelconque des modes de réalisation ici décrits.

D'autres caractéristiques et avantages de l'invention apparaissent dans les exemples qui suivent ainsi que dans les Figures qui illustrent la réalisation de différents modes particuliers de l'invention.

## BREVE DESCRIPTION DES FIGURES

**Figure 1. Principe du dosage compétitif** *in vitro* d'inhibiteurs d'enzymes de la coagulation du sang: schémas cinétiques.

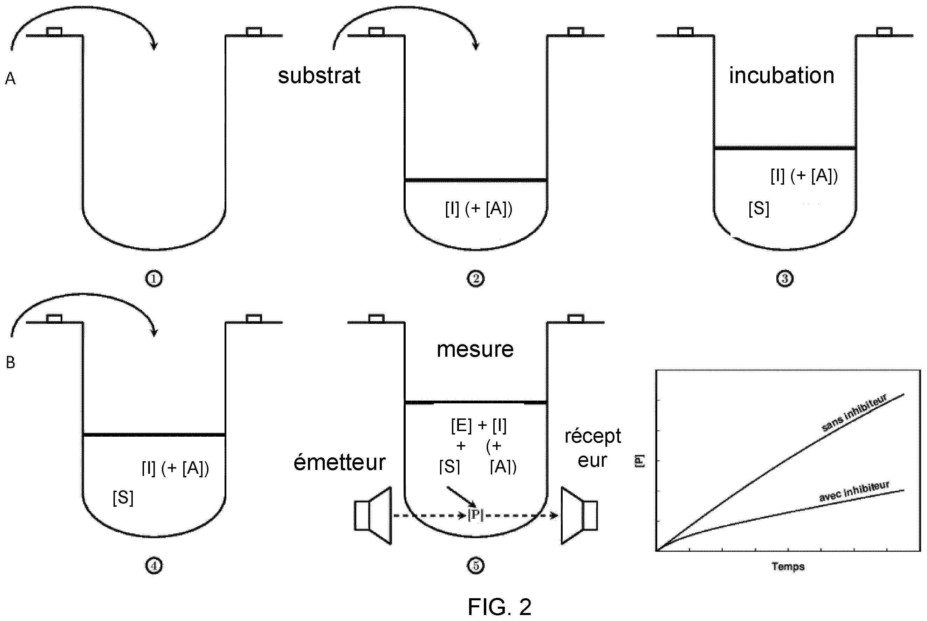

**Figure 2. Principe de la mesure.** (1) un échantillon sanguin (dilué ou non) supposé contenir un inhibiteur I (et si nécessaire) la molécule A) d'une enzyme E de la coagulation est placé au sein d'un consommable. (2) un substrat S spécifique de l'enzyme E est ajouté au mélange réactionnel. (3) une étape d'incubation (généralement plusieurs dizaines de secondes) amène le mélange réactionnel à température (typiquement 37°C). (4) L'enzyme E est ajoutée au mélange réactionnel déclenchant ainsi la compétition entre la réaction d'inhibition et la réaction enzymatique. (5) la mesure proprement dite est alors effectuée (généralement sur une durée de plusieurs dizaines de secondes) ; l'enzyme E transforme le substrat S en produit P tout en étant inhibée en parallèle par l'inhibiteur I (et si nécessaire la molécule A) s'il(s) est(sont) présent(s) dans l'échantillon. Le clivage du substrat S en produit P induit la libération d'un marqueur qu'il est possible de mesurer par un instrument. (6) la mesure aboutit à l'enregistrement d'une cinétique qui est impactée par la présence, le mode d'action et la concentration de l'inhibiteur I (et si nécessaire de la molécule A). Légende : (A) Echantillon supposé contenir un inhibiteur, (B) Enzyme.

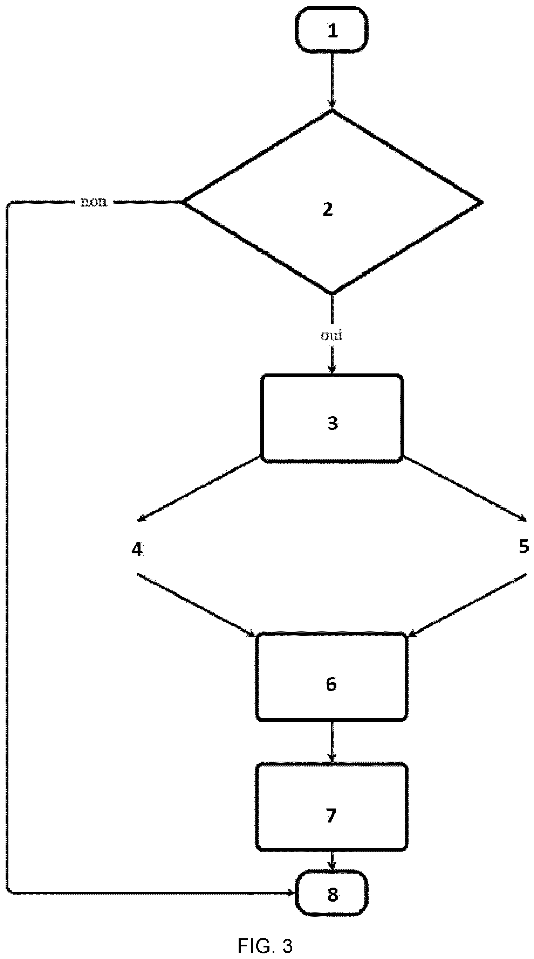

**Figure 3. Cascade de modèles d'apprentissage automatique.** Légende du diagramme : 1. Début, 2. Présence d'un inhibiteur de l'enzyme ?, 3. Catégorie de l'inhibiteur, 4. Direct Réversible, 5. Indirect Irréversible, 6. Identification de l'inhibiteur, 7. Quantification de l'inhibiteur, 8. Fin.

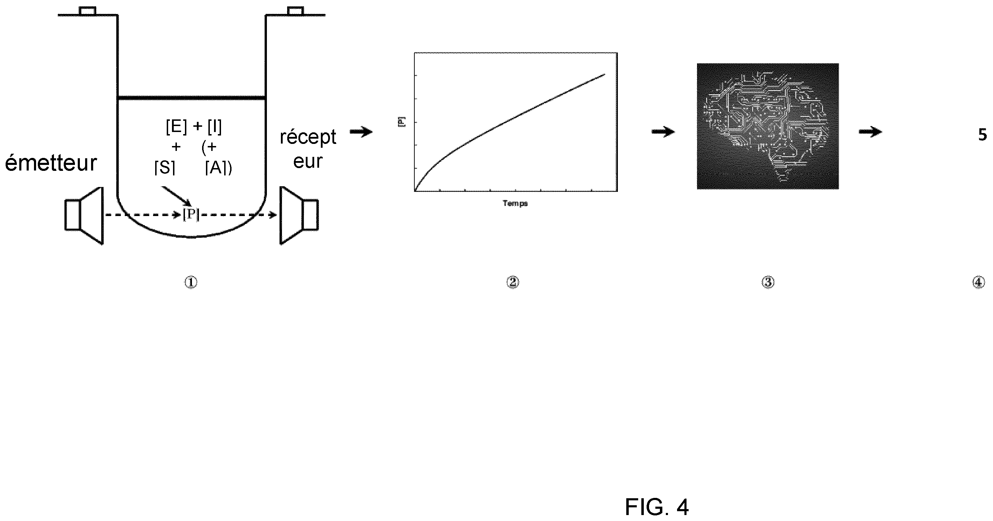

**Figure 4. Principe de l'invention** : détection, identification et quantification d'inhibiteurs d'une enzyme de la coagulation du sang. Légende du diagramme : 1. Mesure, 2. Enregistrement d'une cinétique, 3. Post-traitement par modèles d'apprentissage automatique, 4. Résultat biologique, 5. Détection, identification et quantification d'un inhibiteur d'une enzyme de la coagulation du sang. (1) un échantillon sanguin supposé contenir un inhibiteur I d'une enzyme E de la coagulation est mis en contact avec l'enzyme E et un substrat S spécifique de l'enzyme E au sein d'un même consommable *in vitro.* Le clivage du substrat S par l'enzyme E en un produit P provoque la libération d'un marqueur mesurable par un instrument de laboratoire. (2) La mesure expérimentale aboutit à l'enregistrement d'une courbe proportionnelle à la concentration du produit P au cours du temps. Cette cinétique est impactée par la présence, le mode d'action et la concentration en inhibiteur. (3) La cinétique est analysée et interprétée par une cascade de modèles d'apprentissage automatique qui rend le résultat biologique. (4) Le résultat biologique rendu indique si un inhibiteur de l'enzyme E est présent dans l'échantillon mesuré, et si oui lequel et à quelle concentration.

**Figure 5. Méthodologie universelle anti-Xa** : **sensibilité aux HNF.** La figure illustre la sensibilité de cette méthode à diverses concentrations en HNF : il est observé qu'elle permet de doser des concentrations en HNF comprises entre approximativement [E]*10.0/3.0 et [E]*200.0/3.0 ([E] étant la concentration finale dans le test en enzyme). Les courbes ont été mesurées en utilisant la méthodologie universelle particulière décrite ici ([E]=10 nM, [HNF] (nM) =33,67,133,200,267,333,400,467,533,600 et 670 soit [HNF] (UI/mL)=0.1,0.2,0.4,0.6,0.8,1.0,1.2,1.4,1.6,1.8 et 2.0).

**Figure 6. Méthodologie universelle anti-Xa** : **sensibilité aux HBPM.** La figure illustre la sensibilité de cette méthode à diverses concentrations en HBPM : il est observé qu'elle permet de doser des concentrations en HBPM comprises entre approximativement [E]*20.0 et [E]*400.0 ([E] étant la concentration finale dans le test en enzyme). Les courbes ont été mesurées en utilisant la méthodologie universelle particulière décrite ici ([E]=10 nM, [HBPM] (nM) =200,400,800,1200,1600,2000,2200,2400,2600,2800   et   4000   soit   [HBPM]   (UI   anti-Xa/mL) =0.1,0.2,0.4,0.6,0.8,1.0,1.2,1.4,1.6,1.8 et 2.0).

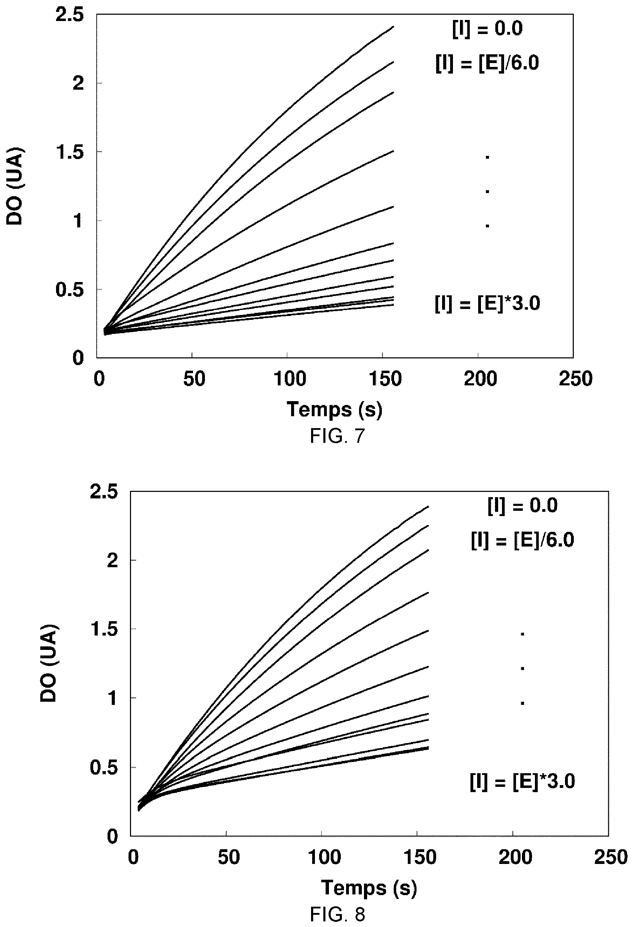

**Figure 7. Méthodologie universelle anti-Xa** : **sensibilité au rivaroxaban.** La figure illustre la sensibilité de cette méthode à diverses concentrations en rivaroxaban : il est observé qu'elle permet de doser des concentrations en rivaroxaban comprises entre approximativement [E]/6.0 et [E]*3.0 ([E] étant la concentration finale dans le test en enzyme). Les courbes ont été mesurées en utilisant la méthodologie universelle particulière décrite ici ([E]=10 nM, [rivaroxaban]   (nM)=1.65,3.3,6.6,9.9,13.2,16.5,19.8,23.1,26.4,29.7   et   33   soit   [rivaroxaban]   (ng/mL) =10,20,40,60,80,100,120,140,160,180 et 200).

**Figure 8. Méthodologie universelle anti-Xa : sensibilité à l'apixaban.** La figure illustre la sensibilité de cette méthode à diverses concentrations en apixaban : il est observé qu'elle permet de doser des concentrations en apixaban comprises entre approximativement [E]/6.0 et [E]*3.0 ([E] étant la concentration finale dans le test en enzyme). Les courbes ont été mesurées en utilisant la méthodologie universelle particulière décrite ici ([E]=10 nM, [apixaban] (nM)=1.56,3.1,6.2,9.3,12.4,15.5,18.6,21.7,24.8,27.9 et 31 soit [apixaban] (ng/mL) =10,20,40,60,80,100,120,140,160,180 et 200).

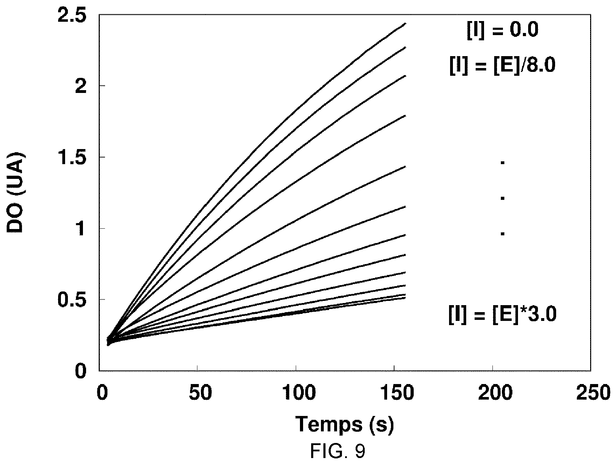

**Figure 9. Méthodologie universelle anti-Xa : sensibilité à l'edoxaban.** La figure illustre la sensibilité de cette méthode à diverses concentrations en edoxaban : il est observé qu'elle permet de doser des concentrations en edoxaban comprises entre approximativement [E]/8.0 et [E]*3.0 ([E] étant la concentration finale dans le test en enzyme). Les courbes ont été mesurées en utilisant la méthodologie universelle particulière décrite ici ([E]=10 nM, [edoxaban] (nM)=1.3,2.6,5.2,7.8,10.4,13.0,15.6,18.2,20.8,23.4 et 26 soit [edoxaban] (ng/mL) =10,20,40,60,80,100,120,140,160,180 et 200).

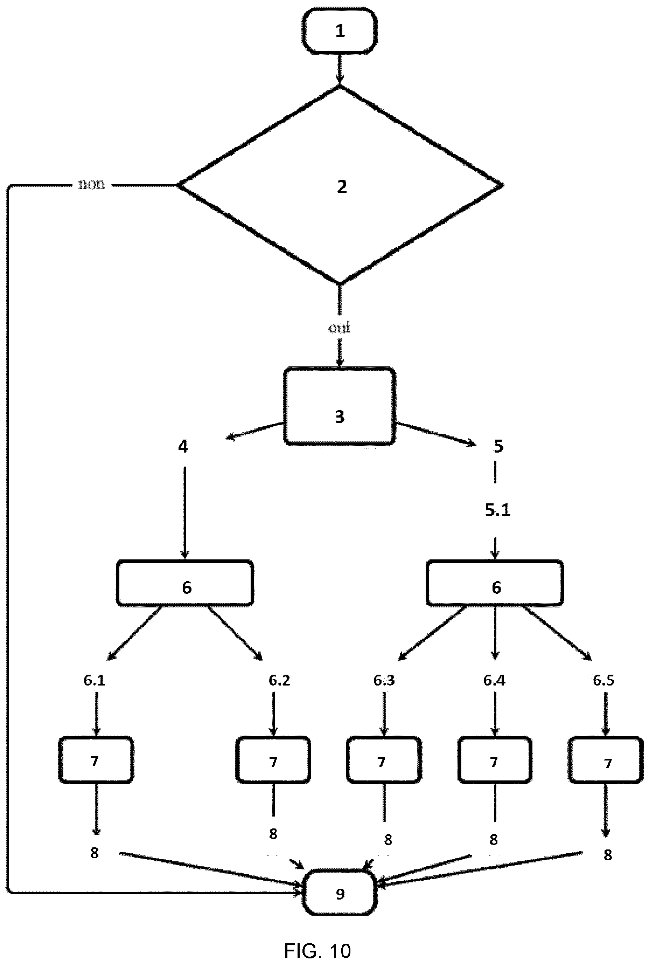

**Figure 10. Cascade de modèles d'apprentissage automatique** pour la détection l'identification et la quantification d'inhibiteurs synthétiques du facteur Xa. Légende du diagramme : 1. Début, 2. Présence d'un inhibiteur du facteur Xa ?, 3. Catégorie de l'anti-Xa, 4. Héparine, 5. AOD, 5.1. Relance expérimentale, 6. Identification, 6.1. HNF, 6.2. HBPM, 6.3. Rivaroxaban, 6.4. Apixaban, 6.5. Edoxaban, 7. Quantification, 8. Taux, 9. Fin.

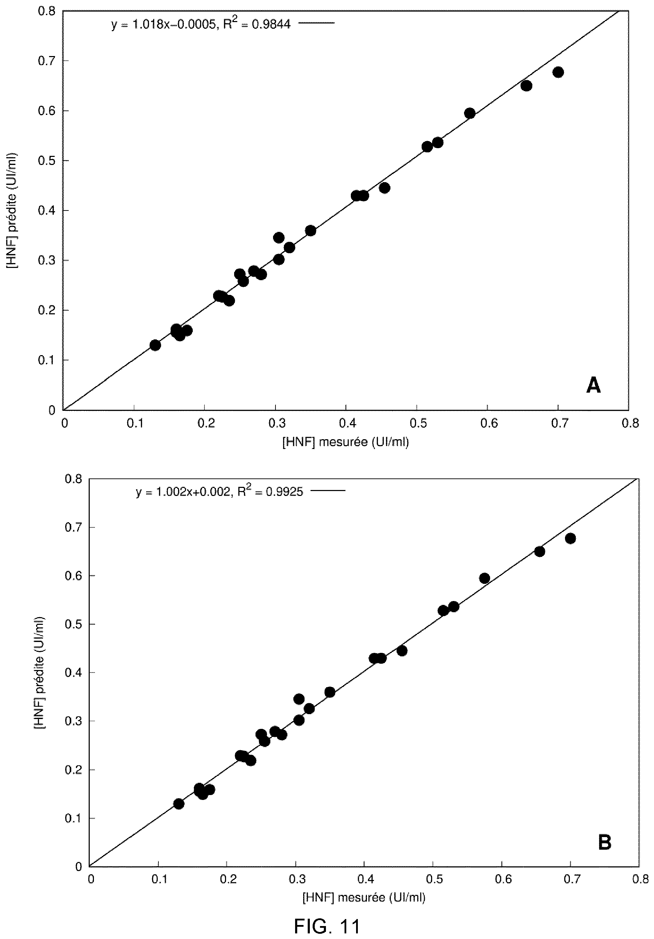

**Figure 11. Dosage des HNF.** A. Analyse en simplicat : les résultats de comparaison des dosages des taux en HNF mesurés en utilisant l'approche décrite ici ([HNF] prédite) aux taux en HNF mesurés en utilisant l'approche standard ([HNF] mesurée) sur les données du jeu de test donnent une droite d'équation y=1.018x-0.0005 et un cœfficient de détermination R2=0.9844. B. Analyse en triplicat : les résultats de comparaison des dosages des taux en HNF mesurés en utilisant l'approche décrite ici ([HNF] prédite) aux taux en HNF mesurés en utilisant l'approche standard ([HNF] mesurée) sur les données du jeu de test donnent une droite d'équation y=1.002x+0.002 et un cœfficient de détermination R2=0.9925.

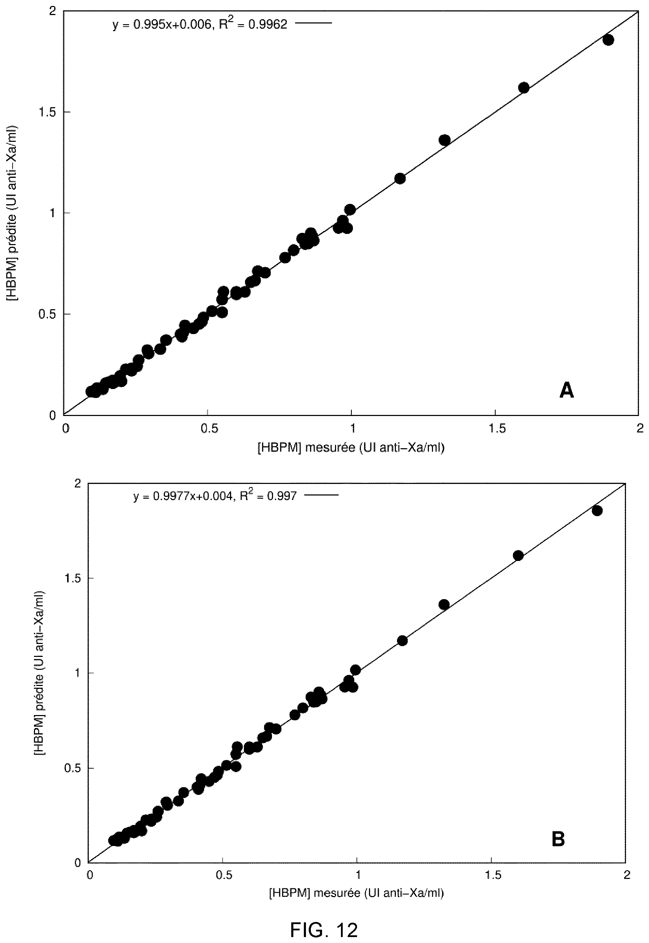

**Figure 12. Dosage des HBPM.** A. Analyse en simplicat : les résultats de comparaison des dosages des taux en HBPM mesurés en utilisant l'approche décrite ici ([HBPM] prédite) aux taux en HBPM mesurés en utilisant l'approche standard ([HBPM] mesurée) sur les données du jeu de test donnent une droite d'équation y=0.995x+0.006 et un cœfficient de détermination R2=0.9962. B. Analyse en triplicat : les résultats de comparaison des dosages des taux en HBPM mesurés en utilisant l'approche décrite ici ([HBPM] prédite) aux taux en HBPM mesurés en utilisant l'approche standard ([HBPM] mesurée) sur les données du jeu de test donnent une droite d'équation y=0.9977x+0.004 et un cœfficient de détermination R2=0.997.

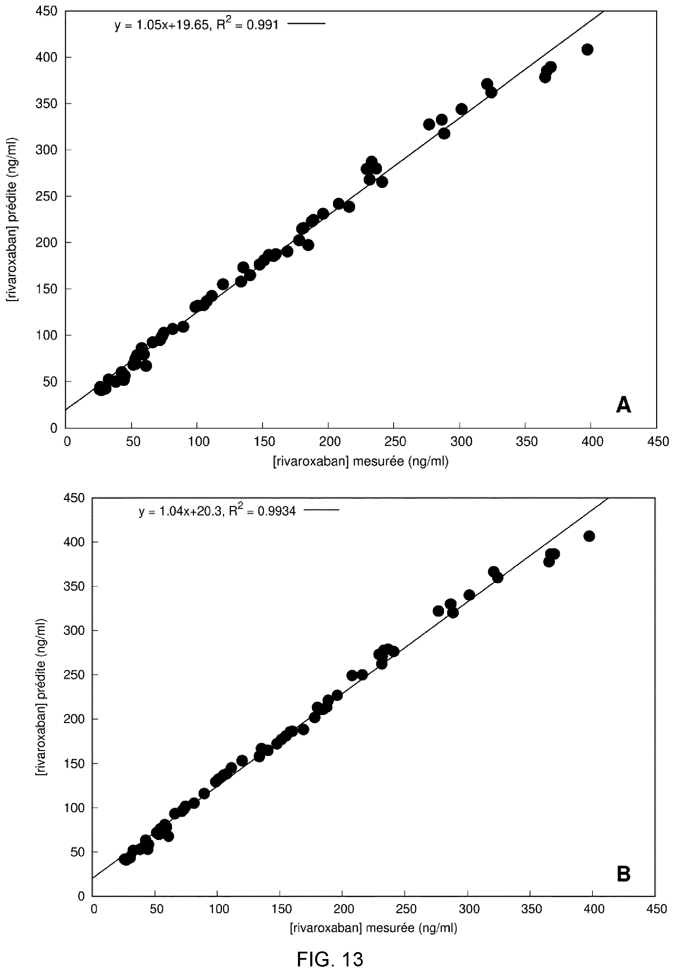

**Figure 13. Dosage du rivaroxaban.** A. Analyse en simplicat : les résultats de comparaison des dosages des taux en rivaroxaban mesurés en utilisant l'approche décrite ici ([rivaroxaban] prédite) aux taux en rivaroxaban mesurés en utilisant l'approche standard ([rivaroxaban] mesurée) sur les données du jeu de test donnent une droite d'équation y=1.05x+19.65 et un cœfficient de détermination R2=0.991. B. Analyse en triplicat : les résultats de comparaison des dosages des taux en rivaroxaban mesurés en utilisant l'approche décrite ici ([rivaroxaban] prédite) aux taux en rivaroxaban mesurés en utilisant l'approche standard ([rivaroxaban] mesurée) sur les données du jeu de test donnent une droite d'équation y=1.04x+20.3 et un cœfficient de détermination R2=0.9934.

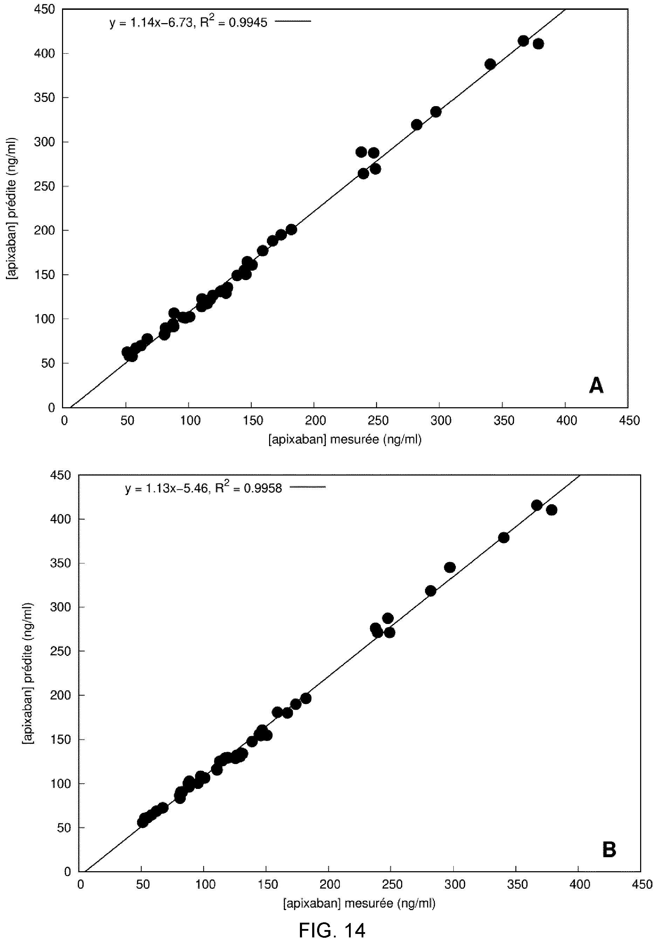

**Figure 14. Dosage de l'apixaban.** A. Analyse en simplicat : les résultats de comparaison des dosages des taux en apixaban mesurés en utilisant l'approche décrite ici ([apixaban] prédite) aux taux en apixaban mesurés en utilisant l'approche standard ([apixaban] mesurée) sur les données du jeu de test donnent une droite d'équation y=1.14x-6.73 et un cœfficient de détermination R2=0.9945. B. Analyse en triplicat : les résultats de comparaison des dosages des taux en apixaban mesurés en utilisant l'approche décrite ici ([apixaban] prédite) aux taux en apixaban mesurés en utilisant l'approche standard ([apixaban] mesurée) sur les données du jeu de test donnent une droite d'équation y=1.13x-5.46 et un cœfficient de détermination R2=0.9958.

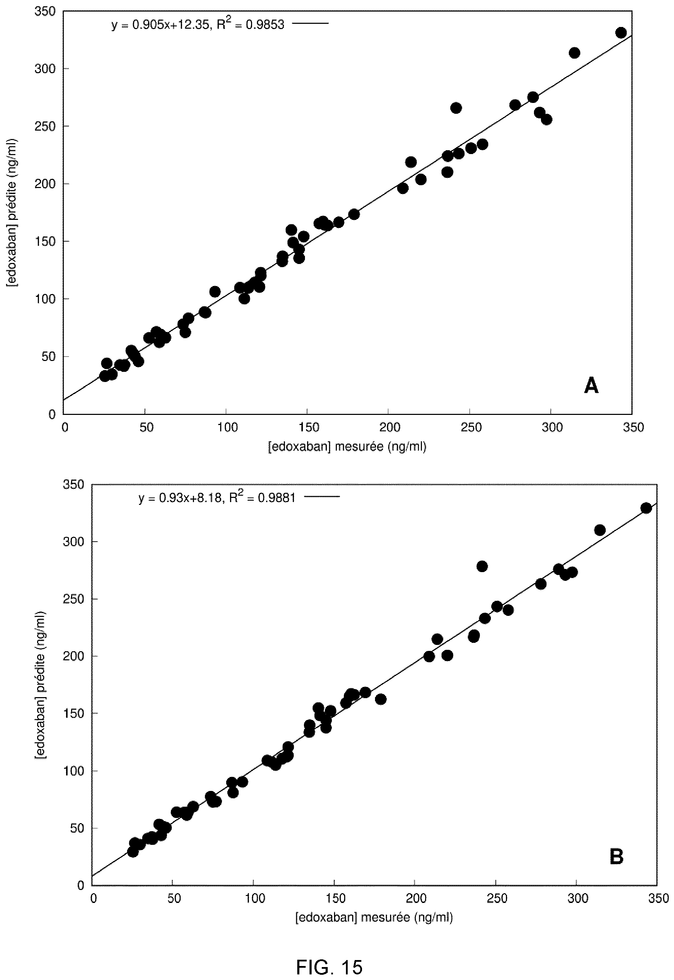

**Figure 15. Dosage de l'edoxaban.** A. Analyse en simplicat : les résultats de comparaison des dosages des taux en edoxaban mesurés en utilisant l'approche décrite ici ([edoxaban] prédite) aux taux en edoxaban mesurés en utilisant l'approche standard ([edoxaban] mesurée) sur les données du jeu de test donnent une droite d'équation y=0.905x+12.35 et un cœfficient de détermination R2=0.9853. B. Analyse en triplicat : les résultats de comparaison des dosages des taux en edoxaban mesurés en utilisant l'approche décrite ici ([edoxaban] prédite) aux taux en edoxaban mesurés en utilisant l'approche standard ([edoxaban] mesurée) sur les données du jeu de test donnent une droite d'équation y=0.93x+8.18 et un cœfficient de détermination R2=0.9881.

**Figure 16. Cascade de modèles d'apprentissage automatique pour la détection, l'identification et la quantification d'inhibiteurs synthétiques du facteur Xa.** Légende du diagramme : 1. Début, 2. Présence d'un inhibiteur du facteur Xa ?, 3. Catégorie de l'anti-Xa, 4. Héparine, 5. AOD, 5.1. Relance expérimentale, 6. Identification, 6.1. HNF, 6.2. HBPM, 6.3. Rivaroxaban, 6.4. Apixaban, 6.5. Edoxaban, 7. Quantification, **7.1. Recalcul de la concentration en utilisant la cinétique mesurée par la méthodologie universelle,** 8. Taux, 9. Fin.

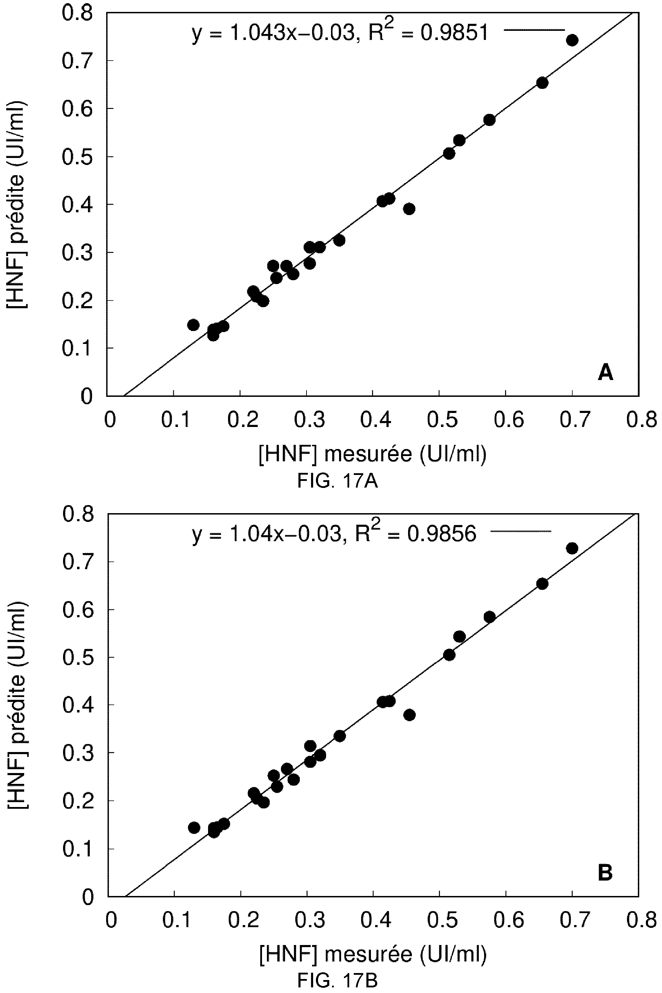

**Figure 17. Dosage des HNF.** A. *Analyse en simplicat* : les résultats de comparaison des dosages des taux en HNF mesurés en utilisant l'approche décrite dans ce document ([HNF] prédite) aux taux en HNF mesurés en utilisant l'approche standard ([HNF] mesurée) sur les données du jeu de test donnent une droite d'équation $y$=1.043x-0.03 et un cœfficient de détermination $R^2$=0.9851. B. *Analyse en triplicat* : les résultats de comparaison des dosages des taux en HNF mesurés en utilisant l'approche décrite dans ce document ([HNF] prédite) aux taux en HNF mesurés en utilisant l'approche standard ([HNF] mesurée) sur les données du jeu de test donnent une droite d'équation $y$=1.04x-0.03 et un cœfficient de détermination $R^2$=0.9856.

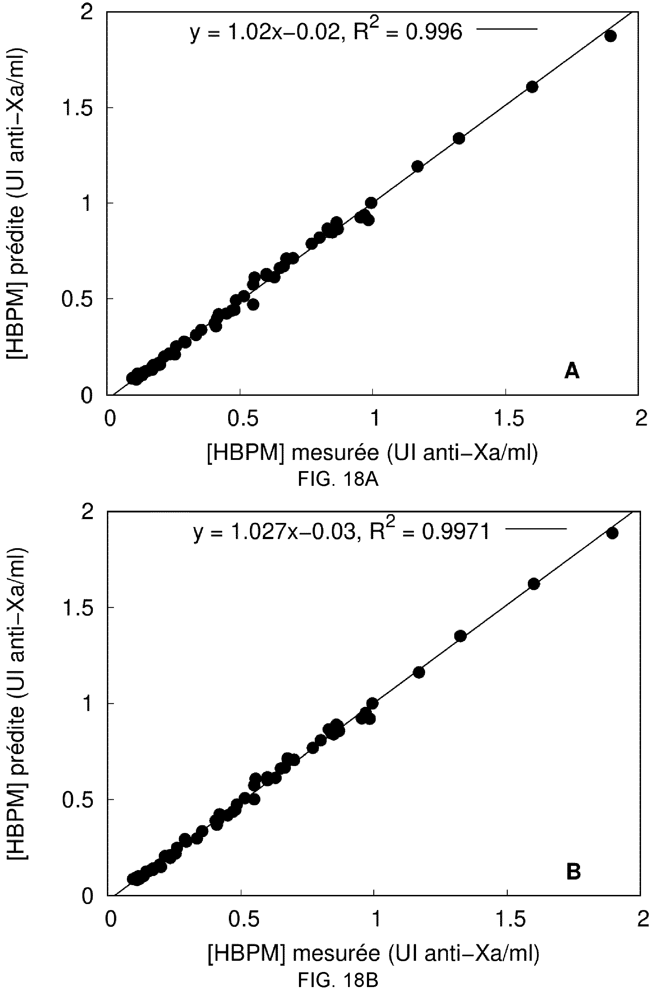

**Figure 18. Dosage des HBPM.** A. *Analyse en simplicat* : les résultats de comparaison des dosages des taux en HBPM mesurés en utilisant l'approche décrite dans ce document ([HBPM] prédite) aux taux en HBPM mesurés en utilisant l'approche standard ([HBPM] mesurée) sur les données du jeu de test donnent une droite d'équation $y$=1.02x-0.02 et un cœfficient de détermination $R^2$=0.996. B. *Analyse en triplicat* : les résultats de comparaison des dosages des taux en HBPM mesurés en utilisant l'approche décrite dans ce document ([HBPM] prédite) aux taux en HBPM mesurés en utilisant l'approche standard ([HBPM] mesurée) sur les données du jeu de test donnent une droite d'équation $y$=1.027x-0.03 et un cœfficient de détermination $R^2$=0.9971.

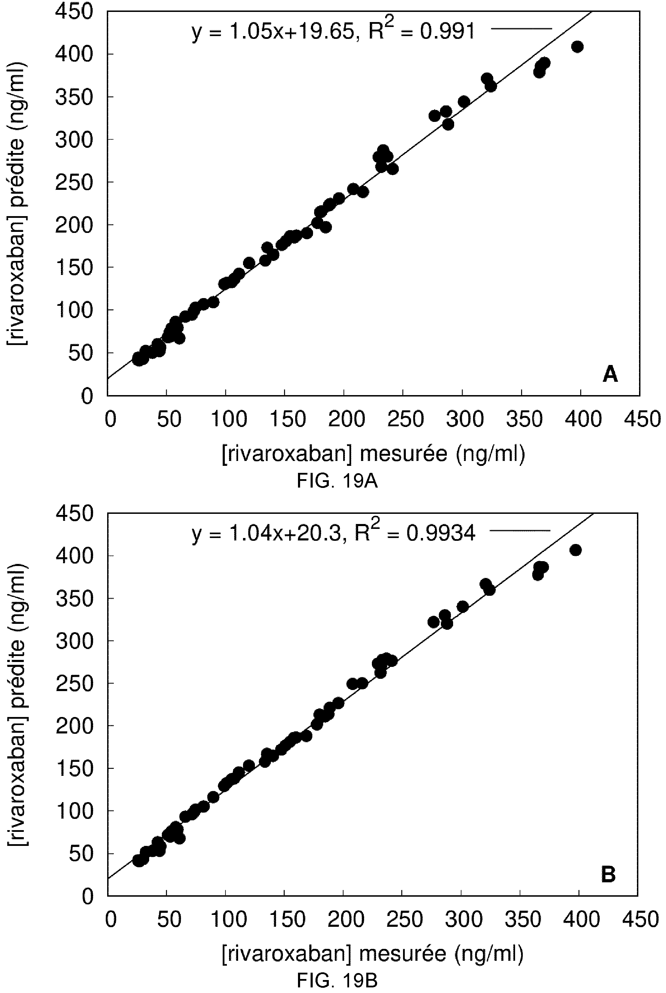

**Figure 19. Dosage du rivaroxaban (méthodologie optimisée AODs).** A. *Analyse en simplicat* : les résultats de comparaison des dosages des taux en rivaroxaban mesurés en utilisant l'approche décrite dans ce document ([rivaroxaban] prédite) aux taux en rivaroxaban mesurés en utilisant l'approche standard ([rivaroxaban] mesurée) sur les données du jeu de test donnent une droite d'équation $y$=1.05x+19.65 et un cœfficient de détermination $R^2$=0.991. B. *Analyse en triplicat* : les résultats de comparaison des dosages des taux en rivaroxaban mesurés en utilisant l'approche décrite dans ce document ([rivaroxaban] prédite) aux taux en rivaroxaban mesurés en utilisant l'approche standard ([rivaroxaban] mesurée) sur les données du jeu de test donnent une droite d'équation $y$=1.04x+20.3 et un cœfficient de détermination $R^2$=0.9934.

**Figure 20. Dosage du rivaroxaban (méthodologie universelle,** ou « méthodologie améliorée sur la base de la méthodologie universelle » ). A. *Analyse en simplicat* : les résultats de comparaison des dosages des taux en rivaroxaban mesurés en utilisant l'approche décrite dans ce document ([rivaroxaban] prédite) aux taux en rivaroxaban mesurés en utilisant l'approche standard ([rivaroxaban] mesurée) sur les données du jeu de test donnent une droite d'équation $y$=1.04x+1.74 et un cœfficient de détermination $R^2$=0.985. B. *Analyse en triplicat* : les résultats de comparaison des dosages des taux en rivaroxaban mesurés en utilisant l'approche décrite dans ce document ([rivaroxaban] prédite) aux taux en rivaroxaban mesurés en utilisant l'approche standard ([rivaroxaban] mesurée) sur les données du jeu de test donnent une droite d'équation $y$=1.032x+1.99 et un cœfficient de détermination $R^2$=0.989.

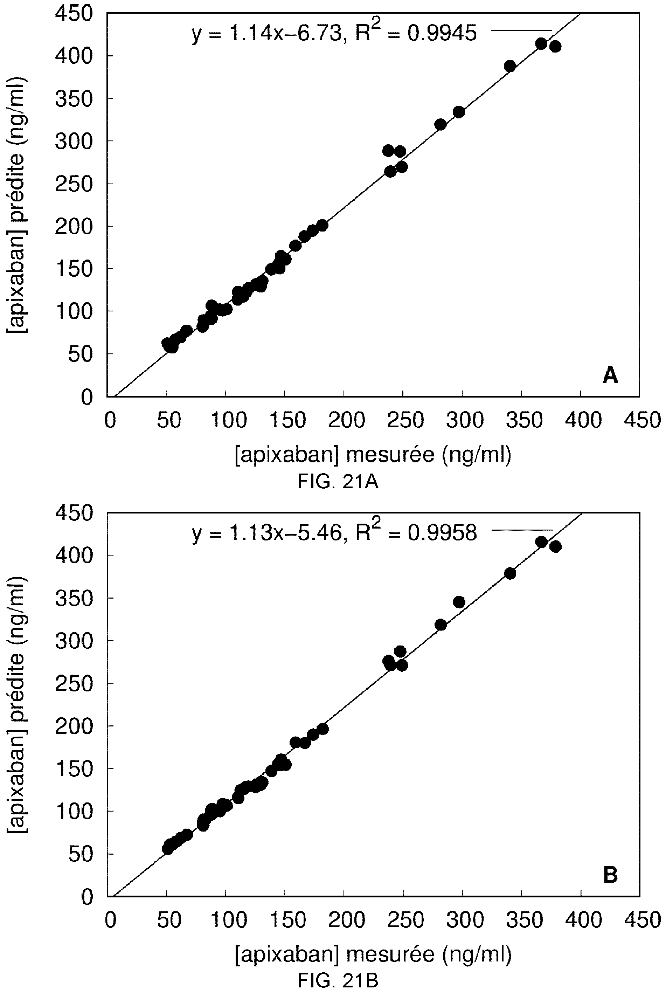

**Figure 21. Dosage de l'apixaban (méthodologie optimisée AODs).** A. *Analyse en simplicat* : les résultats de comparaison des dosages des taux en apixaban mesurés en utilisant l'approche décrite dans ce document ([apixaban] prédite) aux taux en apixaban mesurés en utilisant l'approche standard ([apixaban] mesurée) sur les données du jeu de test donnent une droite d'équation $y$=1.14x-6.73 et un cœfficient de détermination $R^2$=0.9945. B. *Analyse en triplicat* : les résultats de comparaison des dosages des taux en apixaban mesurés en utilisant l'approche décrite dans ce document ([apixaban] prédite) aux taux en apixaban mesurés en utilisant l'approche standard ([apixaban] mesurée) sur les données du jeu de test donnent une droite d'équation $y$=1.13$x$-5.46 et un cœfficient de détermination $R^2$=0.9958.

**Figure 22. Dosage de l'apixaban (méthodologie universelle,** ou « méthodologie améliorée sur la base de la méthodologie universelle »). A. *Analyse en simplicat* : les résultats de comparaison des dosages des taux en apixaban mesurés en utilisant l'approche décrite dans ce document ([apixaban] prédite) aux taux en apixaban mesurés en utilisant l'approche standard ([apixaban] mesurée) sur les données du jeu de test donnent une droite d'équation $y$=1.041$x$-2.66 et un cœfficient de détermination $R^2$=0.9792. B. *Analyse en triplicat* : les résultats de comparaison des dosages des taux en apixaban mesurés en utilisant l'approche décrite dans ce document ([apixaban] prédite) aux taux en apixaban mesurés en utilisant l'approche standard ([apixaban] mesurée) sur les données du jeu de test donnent une droite d'équation $y$=1.041$x$-2.41 et un cœfficient de détermination $R^2$=0.9877.

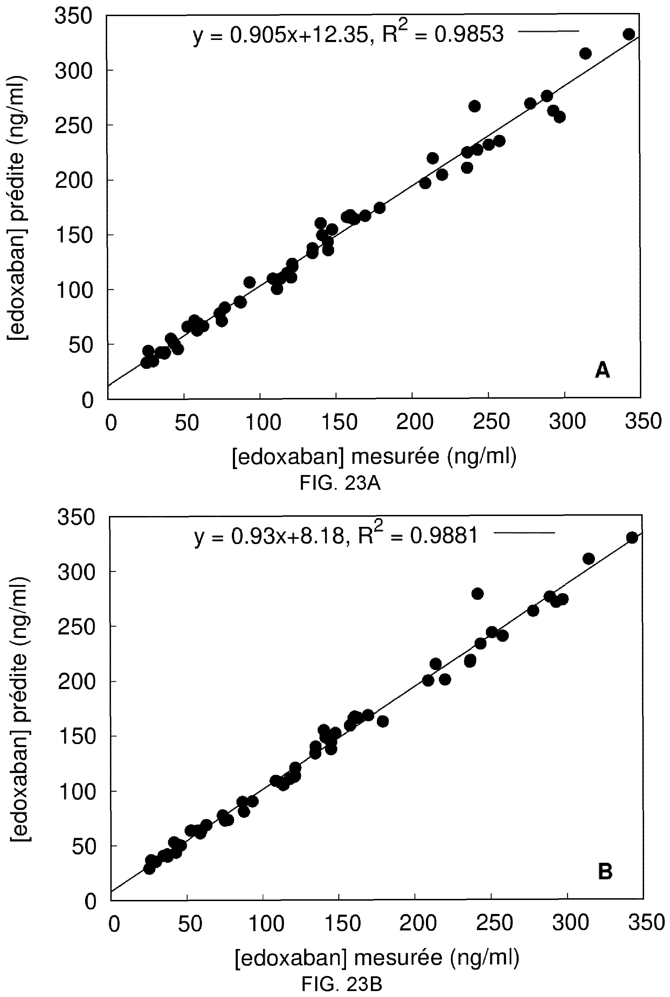

**Figure 23. Dosage de l'edoxaban (méthodologie optimisée AODs).** A. *Analyse en simplicat* : les résultats de comparaison des dosages des taux en edoxaban mesurés en utilisant l'approche décrite dans ce document ([edoxaban] prédite) aux taux en edoxaban mesurés en utilisant l'approche standard ([edoxaban] mesurée) sur les données du jeu de test donnent une droite d'équation $y$=0.905$x$+12.35 et un cœfficient de détermination $R^2$=0.9853. B. *Analyse en triplicat* : les résultats de comparaison des dosages des taux en edoxaban mesurés en utilisant l'approche décrite dans ce document ([edoxaban] prédite) aux taux en edoxaban mesurés en utilisant l'approche standard ([edoxaban] mesurée) sur les données du jeu de test donnent une droite d'équation $y$=0.93$x$+8.18 et un cœfficient de détermination $R^2$=0.9881.

**Figure 24. Dosage de l'edoxaban (méthodologie universelle,** ou « méthodologie améliorée sur la base de la méthodologie universelle »). A. *Analyse en simplicat* : les résultats de comparaison des dosages des taux en edoxaban mesurés en utilisant l'approche décrite dans ce document ([edoxaban] prédite) aux taux en edoxaban mesurés en utilisant l'approche standard ([edoxaban] mesurée) sur les données du jeu de test donnent une droite d'équation $y$=0.9431$x$+4.51 et un cœfficient de détermination $R^2$=0.9968. B. *Analyse en triplicat* : les résultats de comparaison des dosages des taux en edoxaban mesurés en utilisant l'approche décrite dans ce document ([edoxaban] prédite) aux taux en edoxaban mesurés en utilisant l'approche standard ([edoxaban] mesurée) sur les données du jeu de test donnent une droite d'équation $y=0.9442x+4.26$ et un cœfficient de détermination $R^2=0.9962$.

**GENERALITES - METHODOLOGIE EXPERIMENTALE - PRINCIPE DE LA METHODE**

**1. Exposé du principe sous-tendant l'invention**

La présente invention propose une méthode pour détecter, identifier et quantifier dans un échantillon sanguin des inhibiteurs d'enzymes de la coagulation du sang. Pour cela une enzyme de la coagulation du sang, un substrat spécifique de cette enzyme ainsi qu'un échantillon sanguin sont mis en présence au sein d'un même consommable, lequel peut avantageusement un consommable conventionnel, tel que classiquement employé pour les dosages enzymatiques dans le domaine. L'enzyme clive alors le substrat en produit, conduisant ainsi la libération d'un marqueur (le marqueur peut par exemple être chromogène, fluorescent, etc.) ; l'apparition de ce marqueur induit un changement observable des propriétés physiques de l'échantillon sanguin : la mesure consiste alors à enregistrer l'évolution des propriétés physiques de l'échantillon au cours du temps par un instrument (L'instrument peut être un spectrophotomètre si le marqueur est chromogène, il peut être un fluorimètre si le marqueur est fluorescent, etc), i.e. enregistrer une cinétique. Si un inhibiteur de l'enzyme est présent dans l'échantillon sanguin, celui-ci va diminuer l'activité de l'enzyme sur son substrat et impacter en conséquence la cinétique mesurée. Un algorithme de post-traitement interprète cette cinétique et rend le résultat biologique attendu : détection, identification et quantification de l'inhibiteur.

1.1 Méthodologie expérimentale

Les inhibiteurs d'enzymes de la coagulation du sang, qu'ils soient naturels ou synthétiques, appartiennent à deux familles distinctes : les inhibiteurs directs et les inhibiteurs indirects. Un inhibiteur direct se lie directement à l'enzyme pour exercer son action inhibitrice. Un inhibiteur indirect se lie d'abord à une seconde molécule pour former un complexe alors capable d'exercer une action inhibitrice sur l'enzyme. De plus, ces inhibiteurs agissent également selon deux mécanismes réactionnels différents : les schémas cinétiques réversibles et les schémas cinétiques irréversibles. Un inhibiteur réversible se lie à l'enzyme pour former un complexe dissociable contrairement à un inhibiteur irréversible qui se lie à l'enzyme pour former un complexe indissociable. Les inhibiteurs d'enzymes de la coagulation du sang se classent donc en quatre catégories :

- les inhibiteurs directs réversibles,
- les inhibiteurs directs irréversibles,
- les inhibiteurs indirects réversibles,
- les inhibiteurs indirects irréversibles.

Classiquement, le dosage enzymatique de ces inhibiteurs fait intervenir deux réactions biochimiques :

- Une réaction d'inhibition

• si l'inhibiteur est direct réversible, le schéma réactionnel est

$$E + I \rightleftarrows E \cdot I$$

où l'inhibiteur I se lie directement à l'enzyme E pour former le complexe E·I inactif dissociable.
• si l'inhibiteur est direct irréversible, le schéma réactionnel est

$$E + I \rightarrow E \cdot I$$

où l'inhibiteur I se lie directement à l'enzyme E pour former le complexe E·I inactif indissociable.
• si l'inhibiteur est indirect réversible, le schéma réactionnel est

$$E \;+\; A \cdot I \;\rightleftarrows\; E \cdot A \cdot I$$

$$\uparrow\downarrow$$

$$A$$

$$+$$

$$I$$

où l'inhibiteur I s'associe à une seconde molécule A pour former le complexe A·I capable de se lier à l'enzyme E pour former le complexe E·A·I inactif dissociable.

- si l'inhibiteur est indirect irréversible, le schéma réactionnel est

$$E \;+\; A \cdot I \;\rightleftarrows\; E \cdot A \cdot I$$

$$\uparrow\downarrow$$

$$A$$

$$+$$

$$I$$

où l'inhibiteur I s'associe à une seconde molécule A pour former le complexe A·I capable de se lier à l'enzyme E pour former le complexe E·A·I inactif indissociable.

- Une réaction enzymatique

$$E \;+\; S \;\rightleftarrows\; E \cdot S \;\rightarrow\; E \;+\; P$$

où

- E désigne l'enzyme de la coagulation du sang ciblée par l'inhibiteur I supposé présent dans l'échantillon ;
- S représente un substrat spécifique de l'enzyme, généralement composé d'une séquence peptidique spécifique du site actif de l'enzyme et d'un marqueur qui peut être chromogène, fluorescent, électrochimique, ...
- E·S désigne le complexe instable enzyme·substrat ;
- P représente le produit issu de la catalyse du substrat par l'enzyme : dans notre cas, la catalyse aboutit à la libération du marqueur (chromogène, fluorescent, électrochimique, ...).

Notons que les réactions d'inhibition réversibles suivent une cinétique de second ordre pour la formation du complexe enzyme·inhibiteur et une cinétique de premier ordre pour sa dissociation. Les réactions d'inhibition irréversibles suivent une cinétique de second ordre pour la formation du complexe enzyme·inhibiteur. La cinétique de la réaction enzymatique obéit au formalisme de Henri-Michaelis-Menten [Segel, 1993].

Afin de réaliser le dosage enzymatique de ces différents inhibiteurs, les deux réactions biochimiques peuvent être successives ou en compétition. Dans les deux cas, le dosage se divise en deux étapes consécutives : l'incubation et la mesure. Lorsque les deux réactions biochimiques sont successives, l'étape d'incubation consiste à mettre en contact l'échantillon sanguin supposé contenir un inhibiteur I (et si nécessaire la molécule A) avec l'enzyme E présente en excès. L'inhibiteur I (ou le complexe A·I) inhibe alors de manière réversible ou irréversible l'enzyme E ; la durée de l'incubation doit être suffisamment longue pour permettre à la réaction d'inhibition d'atteindre son équilibre biochimique. Ainsi, la concentration résiduelle en enzyme E est inversement proportionnelle à la concentration initiale en inhibiteur I et traduit alors son activité inhibitrice. L'étape de mesure consiste ensuite à ajouter un substrat S spécifique de l'enzyme E au mélange réactionnel ; l'enzyme E, alors présente en quantité résiduelle, transforme le substrat S en un produit P qui induit la libération d'un marqueur au cours du temps permettant l'enregistrement d'une cinétique, cette dernière étant typiquement une droite.

**EP 3 891 752 B1**

Lorsque les deux réactions biochimiques sont en compétition, l'étape d'incubation consiste à mettre en contact l'échantillon sanguin supposé contenir un inhibiteur I (et si nécessaire la molécule A) avec le substrat S : le but de cette étape est uniquement d'élever à 37°C la température du mélange réactionnel. L'étape de mesure consiste ensuite à ajouter l'enzyme E au mélange réactionnel afin de déclencher en parallèle la réaction d'inhibition et la réaction enzymatique : on parle de réactions en compétition car l'inhibiteur I et le substrat S sont en compétition pour l'enzyme E. Ainsi, l'enzyme E clive le substrat S en produit P tout en étant inhibée en parallèle de manière direct ou indirect par l'inhibiteur I. La transformation du substrat S en produit P induit la libération d'un marqueur au cours du temps qui permet l'enregistrement d'une cinétique : la réaction de transformation du substrat en produit étant impactée par l'action de l'inhibiteur sur l'enzyme, la cinétique enregistrée l'est aussi. De ce fait, la concentration de l'inhibiteur et son mode d'action sur l'enzyme modifient la cinétique enregistrée au cours de l'étape de mesure lorsque le dosage enzymatique met en compétition les deux réactions biochimiques.

L'invention ici décrite utilise l'approche compétitive ; la figure 1 synthétise les schémas cinétiques associés au dosage *in vitro* des différents inhibiteurs d'enzymes de la coagulation du sang. La mesure *in vitro* pour la détection, l'identification et la quantification d'inhibiteurs d'enzymes de la coagulation du sang s'effectue donc selon le principe suivant, illustré sur la figure 2 :

- un échantillon sanguin (dilué ou non) supposé contenir un inhibiteur I (et si nécessaire la molécule A) d'une enzyme E de la coagulation est placé au sein d'un consommable ;
- un substrat S spécifique de l'enzyme E est ajouté au mélange réactionnel ;
- une étape d'incubation (généralement plusieurs dizaines de secondes) élève le mélange réactionnel à température (typiquement 37 °C) ;
- l'enzyme E est ajoutée au mélange réactionnel déclenchant ainsi la compétition entre la réaction d'inhibition et la réaction enzymatique ;
- la mesure proprement dite est alors effectuée (généralement sur une durée de plusieurs dizaines de secondes) : l'enzyme E transforme le substrat S en produit P tout en étant inhibée en parallèle par l'inhibiteur I (et si nécessaire la molécule A) s'il(s) est (sont) présent(s) dans l'échantillon. Le clivage du substrat S en produit P induit la libération d'un marqueur qu'il est possible de mesurer par un instrument.
- la mesure aboutit à l'enregistrement d'une cinétique qui est impactée par la présence, le mode d'action et la concentration de l'inhibiteur I (et si nécessaire de la molécule A).

Compte-tenu du champ d'application de la méthode ici décrite, qui vise plus particulièrement la détection et le cas échéant l'identification voire la quantification d'anticoagulants inhibiteurs du facteur Xa et/ou du facteur IIa qui sont soit des inhibiteurs indirects irréversibles (aussi qualifiés comme la catégorie des héparines, comme ici décrites, dans la présente description) ou des inhibiteurs réversibles directs (aussi qualifiés comme la catégorie des AOD, comme ici décrits, dans la présente description), l'enzyme E ajoutée au mélange réactionnel déclenchant la compétition est, selon, du facteur Xa ou du facteur IIa. De façon conventionnelle, cette enzyme doit être ajoutée juste avant le début de la mesure aboutissant à l'enregistrement d'une cinétique.

Puisque que la méthode ici décrite permet, pour la première fois, une détection « en aveugle » d'un inhibiteur du facteur Xa et/ou du facteur IIa supposé être contenu dans l'échantillon analysé, se pose la question de savoir la ou les plages de valeurs de concentration en inhibiteur (du facteur Xa et/ou du facteur IIa) supposé être contenu dans l'échantillon analysé, pour lesquelles la méthode ici décrite sera effective.

La détermination des concentrations en inhibiteur (du facteur Xa et/ou du facteur IIa) que la méthode de mesure cinétique concrètement employée permet de détecter, et le cas échéant d'identifier, voire de quantifier, peut être faite suivant la procédure décrite dans la section « Exemples », appliquée pour les besoins de la démonstration à la recherche d'un inhibiteur du facteur Xa. L'homme du métier comprendra que ces plages de concentration où la détection est possible, peuvent varier selon les types d'enzyme et de substrat employés, de façon classique dans le domaine des tests de détection de la présence d'enzyme, notamment dans des échantillons sanguins. Ceci étant, la méthodologie exposée ci-dessous dans la section « Exemples », permet à l'homme du métier de déterminer sans difficulté si la plage de concentrations en inhibiteur qui peut être recherchée, est adaptée à ses besoins. Par ailleurs, la méthodologie exposée montre également qu'il est possible, par exemple pour un couple enzyme-substrat donné, notamment connu de l'art antérieur, de moduler la plage de sensibilité de détection, identification voire de quantification en fonction de la plage visée. Ceci peut se faire, comme montré de façon expérimentale ci-après sur la base d'exemples d'implémentation, en modulant la dilution de départ de l'échantillon à analyser. C'est ainsi que notamment, le mode de réalisation particulier auquel il est fait référence en tant que « méthodologie optimisée pour les AOD », a été conçu et développé, à titre de variation visant des besoins plus précisément définis. L'homme du métier pourra ainsi adapter le taux de dilution de l'échantillon de départ de sorte à atteindre la plage de sensibilité visée pour les inhibiteurs recherchés en aveugle. Des exemples illustratifs sont inclus dans la présente demande, pour ces modes de réalisation particuliers qui s'inscrivent dans le cadre de la nouvelle méthode de détection en aveugle génériquement exposée ici.

Enfin, un (ou plusieurs) algorithme(s) de post-traitement interprète(nt), notamment suivant une séquence chronologique suivant l'objectif recherché, la cinétique qui est, le cas échéant, impactée par la présence, le mode d'action et la concentration d'un inhibiteur présent dans l'échantillon (et si nécessaire de la molécule A à laquelle il est fait référence ci-dessus), ce qui rend le résultat biologique attendu : détection, et/ou identification et/ou quantification de l'inhibiteur. Ceci est décrit dans la section suivante.

1.2 Post-traitement

La section précédente a décrit une méthodologie expérimentale permettant l'obtention d'une cinétique impactée par la présence, le mode d'action et la concentration d'un inhibiteur d'une enzyme de la coagulation du sang par l'intermédiaire d'un dosage enzymatique compétitif. Cette section présente une méthode de post-traitement, fondée sur des algorithmes d'Intelligence Artificielle (IA), interprétant la cinétique pour rendre le résultat biologique attendu : détection, identification et quantification de l'inhibiteur.

L'Intelligence Artificielle est une discipline informatique née dans les années 1950. Très schématiquement, son but est de mettre au point des algorithmes capables de reproduire les fonctions cognitives du cerveau humain. Dans ces capacités cognitives, l'apprentissage (Ensemble de mécanismes menant à l'acquisition de savoir-faire, de savoirs ou de connaissances) est de loin le champ applicatif le plus étudié dans le contexte de l'IA. Appelé apprentissage automatique, ou encore apprentissage machine (machine learning en anglais), il fait l'objet d'une quantité indénombrable de publications scientifiques mais aussi et surtout, il trouve aujourd'hui de nombreuses applications dans notre quotidien. La présente invention fait usage de modèles d'apprentissage dits supervisés. Contrairement aux algorithmes classiques, les modèles d'apprentissage automatique ne sont pas explicitement programmés pour les tâches qu'ils ont à effectuer mais ils y sont entraînés. En effet, un modèle d'apprentissage automatique établit un lien numérique entre une donnée d'entrée et une donnée de sortie par le biais d'une fonction mathématique empirique. Ainsi pour une donnée d'entrée donnée, le modèle calcule la sortie associée. Lorsque la sortie est un nombre entier, le modèle d'apprentissage automatique répond à un problème de classification (par exemple un patient est sain ou un patient est malade) ; lorsque la sortie est un nombre réel, le modèle d'apprentissage automatique répond à un problème de régression (par exemple un taux d'anticoagulant). Les paramètres de la fonction mathématique empirique sont calculés par entraînement sur une base de données composée de paires (donnée d'entrée, donnée de sortie) : un algorithme d'apprentissage ajuste les paramètres de sorte que pour une donnée d'entrée donnée le modèle recalcule au plus juste la donnée de sortie associée. Parmi les modèles d'apprentissage automatique les plus utilisés citons par exemple les réseaux de neurones, les forêts aléatoires et les machines à vecteurs de support [Géron, 2017]. L'homme du métier a à sa disposition, suivant par exemple la littérature existante (notamment [Géron, 2017] ou Bonaccorso, G. (2017)), des manières conventionnelles d'optimiser des hyper-paramètres d'un modèle d'apprentissage automatique, de façon à les adapter à l'objectif visé.

L'approche classique pour définir, entraîner et évaluer un modèle d'apprentissage automatique s'effectue sur une base de données qui aura été auparavant divisée en trois jeux de données distincts :

- Les données d'entraînement qui vont permettre la détermination des paramètres de la fonction mathématique empirique reliant les paires (donnée d'entrée, donnée de sortie) ;
- Les données de validation qui vont servir à l'optimisation des hyper-paramètres du modèle d'apprentissage automatique, conformément aux connaissances de l'homme du métier - voir par exemple [Géron, 2017]) - du modèle d'apprentissage automatique et à mesurer la capacité de généralisation du modèle d'apprentissage automatique entraîné sur les données d'entraînement ;
- Les données de test qui vont permettre d'évaluer la performance globale et réelle du modèle d'apprentissage automatique entraîné sur les données d'entraînement et optimisé sur les données de validation.

Il est important de noter que la mise en place de modèles d'apprentissage automatique implique obligatoirement d'avoir à disposition une base de données composée de paires (donnée d'entrée, donnée de sortie). Plus la quantité et la qualité de ces données seront élevées, meilleur sera l'entraînement de ces modèles et meilleure sera la capacité de généralisation.

L'invention proposée ici utilise, selon un mode de réalisation particulier allant jusqu'au bout de la chaîne de conclusions possibles et envisageables, une cascade de modèles d'apprentissage automatique pour analyser et interpréter la cinétique mesurée expérimentalement. Cette cascade a pour but de rendre le résultat biologique : elle est décrite sur la figure 3, étant entendu qu'en fonction du résultat et de l'analyse recherchée, l'ensemble des étapes peut ne pas être implémenté.

La cascade particulière la plus complète décrite ici est composée de quatre modèles d'apprentissage automatique ; ces quatre modèles prennent en entrée la (même) cinétique obtenue par la mesure expérimentale et donnent en sortie un résultat :

- Le premier modèle (aussi appelé modèle A) est un modèle de classification : selon l'allure de la cinétique qui lui est donnée en entrée, il détermine si oui ou non l'échantillon analysé contient un inhibiteur de l'enzyme ;
- Le second modèle (aussi appelé modèle B) est aussi un modèle de classification : sachant que l'échantillon analysé contient un inhibiteur de l'enzyme et selon l'allure de la cinétique qui lui est présentée en entrée, il reconnait la catégorie de l'inhibiteur ;
- Le troisième modèle (aussi appelé modèle C) est toujours un modèle de classification : connaissant la catégorie de l'inhibiteur de l'enzyme et selon l'allure de la cinétique qui lui est donnée en entrée, il identifie l'inhibiteur présent dans l'échantillon analysé ;
- Le quatrième et dernier modèle possible (aussi appelé modèle D) est un modèle de régression : connaissant l'inhibiteur présent dans l'échantillon analysé et selon l'allure de la cinétique qui lui est présentée en entrée, il calcule la concentration de cet inhibiteur.

Chacun de ces modèles est un modèle d'apprentissage automatique, par exemple un réseau de neurones, une machine à vecteurs de support ou autres, notamment comme décrit ci-après et détaillé dans la section résultats en ce qui concerne des modes de réalisation particuliers, qui est entraîné par des paires (cinétique, donnée de sortie) de données d'une base de données divisée en trois jeux distincts (jeu d'entraînement, jeu de validation et jeu de test) qui aura été générée au préalable. Le choix du modèle d'apprentissage automatique (réseaux de neurones, machines à vecteurs de support ou autres) se fait selon les performances obtenues sur le jeu de validation. La section expérimentale montre une manière d'analyser les performances obtenues sur un jeu de validation. Le premier modèle est entraîné par des paires (cinétique, présence = oui ou présence = non). Le second modèle est entraîné par des paires (cinétique, catégorie de l'inhibiteur = direct réversible ou catégorie de l'inhibiteur = direct irréversible ou catégorie de l'inhibiteur = indirect réversible ou catégorie de l'inhibiteur = indirect irréversible). Le troisième modèle est entraîné par des paires (cinétique, nom de l'inhibiteur = inhibiteur1 ou nom de l'inhibiteur = inhibiteur2 ou nom de l'inhibiteur = ...). Le quatrième modèle est entraîné par des paires (cinétique, concentration de l'inhibiteur). Enfin, la mise en cascade de ces différents modèles donne le résultat biologique attendu, selon le résultat recherché : détection, identification et quantification d'un inhibiteur d'une enzyme de la coagulation du sang.

### 1.3 Synthèse

Le but de la présente demande est la détection de la présence, l'identification, voire la quantification *in vitro* d'inhibiteurs d'enzymes de la coagulation du sang, comme ici décrits. De façon générale, les inhibiteurs d'enzymes de la coagulation du sang, qu'ils soient naturels ou synthétiques, se classent en quatre catégories : les inhibiteurs directs réversibles, les inhibiteurs directs irréversibles, les inhibiteurs indirects réversibles et les inhibiteurs indirects irréversibles. Chaque catégorie a un mécanisme réactionnel biochimique qui lui est propre. Ainsi, afin de pouvoir effectuer le dosage, un échantillon sanguin supposé contenir un inhibiteur I d'une enzyme E de la coagulation du sang est mis en contact au sein d'un même consommable avec l'enzyme E et un substrat S spécifique de l'enzyme E. La mesure expérimentale consiste à mettre en compétition une réaction d'inhibition (entre E et I) et une réaction enzymatique (entre E et S). Le clivage du substrat S par l'enzyme E en produit P provoque la libération d'un marqueur qui va induire un changement observable des propriétés physiques de l'échantillon qui est enregistré par un instrument de mesure au cours du temps. La cinétique résultante est impactée par la présence, le mécanisme réactionnel biochimique et la concentration en inhibiteur. Cette cinétique est ensuite analysée et interprétée par une cascade de modèles d'apprentissage automatique qui rend le résultat biologique attendu. La figure 4 résume le principe à la base de présente invention.

**EXEMPLES**

**2. Exemple d'application: détection, identification et quantification des inhibiteurs synthétiques du facteur Xa**

### 2.1 Introduction

Le facteur Xa est une enzyme de la coagulation du sang qui, associée au facteur Va sur une membrane phospholipidique et en présence de calcium, forment le complexe enzymatique prothrombinase responsable de l'activation de la prothrombine en thrombine (ou facteur IIa). La prothrombine activée en thrombine est alors capable de transformer le fibrinogène soluble en un caillot de fibrine insoluble, étape ultime de la cascade de la coagulation du sang appelée fibrinoformation. Ainsi le facteur Xa est une enzyme ayant un rôle clé dans le processus de coagulation du sang : promouvoir son activité favorise et amplifie la coagulation du sang, tandis que restreindre son activité diminue et freine la coagulation du sang. De nombreuses thérapeutiques ciblent donc le facteur Xa pour diminuer son activité et préviennent ainsi l'occurence ou la récurrence d'événements thromboemboliques tels la phlébite ou l'embolie pulmonaire. Parmi elles, se trouvent les héparines ou encore les anticoagulants oraux directs anti-Xa.

L'héparine est un médicament anticoagulant s'administrant par voie sous-cutanée ou intraveineuse. Pour inhiber le facteur Xa, l'héparine s'associe à une protéine plasmatique : l'antithrombine. L'antithrombine est un inhibiteur naturel d'enzymes de la coagulation du sang telles que le facteur Ha, le facteur Xa, le facteur IXa et dans de moindres mesures le facteur VIIa (associé ou non au facteur tissulaire), le facteur XIa et le facteur XIIa. Pour exercer son action inhibitrice, l'antithrombine se lie de façon irréversible aux sites actifs de ces différentes enzymes ; associée à l'héparine, son activité inhibitrice est multipliée d'un facteur 1000. L'héparine est donc, entre autres, un inhibiteur indirect irréversible du facteur Xa. Il existe deux grandes familles d'héparines : les héparines non fractionnées (HNF) et les héparines de bas poids moléculaires (HBPM). Il existe une troisième famille d'héparines : les pentasaccharides comme par exemple le fondaparinux ou l'idraparinux. Les HNF accentuent principalement l'action de l'antithrombine sur la thrombine tandis que les HBPM potentialisent l'action de l'antithrombine majoritairement sur le facteur Xa.

Les anticoagulants oraux directs (AODs) sont des médicaments anticoagulants s'administrant par voie orale. Ils se déclinent en deux classes : les anti-Xa inhibiteurs directs du facteur Xa, et les anti-IIa inhibiteurs directs de la thrombine. Dans le contexte de la présente preuve de concept, une focalisation a été faite uniquement sur la famille des anti-Xa dont les molécules disponibles sur le marché sont : le rivaroxaban ou Xarelto® commercialisé par Bayer/Janssen Pharmaceutical, l'apixaban ou Eliquis® commercialisé par Bristol-Myers Squibb/Pfizer et l'edoxaban ou Lixiana®/Savaysa® commercialisé par Daiichi Sankyo. Ces trois molécules inhibent le facteur Xa en se liant directement de manière réversible au site actif de l'enzyme : ces trois molécules sont donc des inhibiteurs directs réversibles du facteur Xa.

A titre de preuve de concept, cette section décrit l'application de la méthode décrite dans la section 1 à la détection, l'identification et la quantification des inhibiteurs synthétiques du facteur Xa. Les éléments ici décrits sont transposables sans difficulté à la détection, l'identification et la quantification des inhibiteurs synthétiques du facteur IIa. Sur ce point, il est par ailleurs relevé que les héparines sont des inhibiteurs indirects irréversibles, à la fois des facteurs Xa et IIa.

2.2 Principe

2.2.1 Méthodologie expérimentale

2.2.1.1 Sensibilité de la méthode selon l'invention à différentes concentrations d'inhibiteur(s) recherché(s), et techniques d'adaptation, si necessaire

Les méthodes commerciales disponibles actuellement pour le dosage des anticoagulants anti-Xa comme les héparines et les AODs, fonctionnent toutes selon le même principe : un dosage enzymatique avec une méthodologie expérimentale dédiée, une calibration dédiée et des contrôles et calibrants dédiés à chaque molécule (HNF, HBPM, rivaroxaban, apixaban et edoxaban). On connait également le kit Stago STA®- Multi-Hep Calibrator qui permet à la fois le dosage des HNF et des HBPM en utilisant une méthodologie commune et une calibration hybride. Ce kit ne permet cependant pas la détection des AOD.

L'approche ici proposée permet de mettre en œuvre une « méthodologie universelle » sensible à la présence de la majorité des anticoagulants anti-Xa, c'est-à-dire aux héparines (HNF et HBPM) et aux AODs (rivaroxaban, apixaban et edoxaban). Pour cela, un volume de plasma de l'échantillon à doser est dilué dans un tampon. Un substrat spécifique du facteur Xa est ajouté au mélange réactionnel puis l'ensemble est mis en incubation pour élévation à 37°C. Enfin l'apport de facteur Xa déclenche la réaction et la mesure s'effectue sur plusieurs secondes.

L'invention concerne un dosage nécessairement compétitif : aussi, l'enzyme doit être le réactif déclenchant et donc être ajoutée en dernier avant d'initier la mesure.

Pour une concentration finale [E] en molaire dans le test en facteur Xa, il a été observé et démontré expérimentalement que la méthodologie ici intitulée « méthodologie universelle » permet de mesurer des concentrations en HNF comprises entre approximativement [E]*10.0/3.0 et approximativement [E]*200.0/3.0, comme illustré sur la figure 5. Les différents volumes en échantillon, tampon et réactifs sont choisis et ajustés afin de doser idéalement l'intervalle [0.1, 2.0] UI/mL.

Pour une concentration finale [E] en molaire dans le test en facteur Xa, il a été observé et démontré expérimentalement que la méthodologie ici intitulée « méthodologie universelle » permet de mesurer des concentrations en HBPM comprises entre approximativement [E]*20.0 et approximativement [E]*400.0, comme illustré sur la figure 6. Les différents volumes en échantillon, tampon et réactifs sont choisis et ajustés afin de doser idéalement l'intervalle [0.1, 2.0] UI anti-Xa/mL.

Pour une concentration finale [E] en molaire dans le test en facteur Xa, il a été observé et démontré expérimentalement que la méthodologie ici intitulée « méthodologie universelle » permet de mesurer des concentrations en rivaroxaban comprises entre approximativement [E]/6.0 et approximativement [E]*3.0, comme illustré sur la figure 7. Les différents volumes en échantillon, tampon et réactifs sont choisis et ajustés afin de doser idéalement l'intervalle [20, 600] ng/mL.

Pour une concentration finale [E] en molaire dans le test en facteur Xa, il a été observé et démontré expérimentalement que la méthodologie ici intitulée « méthodologie universelle » permet de mesurer des concentrations en apixaban comprises entre approximativement [E]/6.0 et approximativement [E]*3.0, comme illustré sur la figure 8. Les différents volumes en échantillon, tampon et réactifs sont choisis et ajustés afin de doser idéalement l'intervalle [20, 600] ng/mL.

Pour une concentration finale [E] en molaire dans le test en facteur Xa, il a été observé et démontré expérimentalement que la méthodologie ici intitulée « méthodologie universelle » permet de mesurer des concentrations en edoxaban comprises entre approximativement [E]/8.0 et approximativement [E]*3.0, comme illustré sur la figure 9. Les différents volumes en échantillon, tampon et réactifs sont choisis et ajustés afin de doser idéalement l'intervalle [20, 600] ng/mL.

La mesure étant compétitive, le substrat doit bien sûr, dans le contexte du mode de réalisation particulier ici décrit, être spécifique du facteur Xa, mais peu affin de ce dernier pour ne pas interférer avec la réaction entre l'enzyme et son inhibiteur ; pour cela, le substrat doit avoir une constante de Michaelis KM élevée, comprise approximativement entre 10 $\mu$M et 1000 $\mu$M. L'affinité entre l'enzyme et le substrat étant volontairement choisie faible, la constante catalytique kcat de l'enzyme pour le substrat doit être suffisamment élevée, par exemple supérieure à approximativement 10 s-1, pour générer le produit P (i.e. le marqueur) et permettre de mesurer un signal. Une constante catalytique élevée permet aussi de minimiser la concentration du complexe enzyme · substrat. Enfin, la concentration initiale en substrat [S]0 doit être suffisante pour permettre la génération du marqueur durant toute la durée d de la mesure, par exemple respecter l'inégalité suivante

$$[S]_0 > \frac{v_{max}[S]_0}{K_M + [S]_0} d$$

Toutefois, elle ne doit pas être trop élevée pour interférer au minimum avec la réaction entre l'enzyme et son inhibiteur : sa valeur peut par exemple être inférieure à KM*10.

La durée de la mesure est choisie suffisamment longue pour permettre à l'anticoagulant, s'il est présent, d'exercer son action inhibitrice sur l'enzyme et que celle-ci soit observable via la mesure ; par exemple une durée comprise entre 10 et 1000 secondes.

2.2.1.2 Exemples d'application particuliers

Les inhibiteurs synthétiques du facteur Xa sont les héparines, qui sont des inhibiteurs indirects irréversibles, et les AODs anti-Xa, qui sont des inhibiteurs directs réversibles.

Au final, un exemple d'application particulier au titre de la méthodologie appelée « méthodologie universelle » a été implémenté avec le kit commercial Stago STA® - Liquid Anti-Xa. L'homme du métier pourra aisément adapter la preuve de concept ici fournie, à l'emploi pour le dosage de départ de tout kit ayant la même visée que celui ici utilisé, ou à la composition d'un tel kit, en particulier pour s'adapter à la nature d'un couple enzyme-substrat donné, la preuve de concept ici fournie n'étant pas limitative en elle-même pour la réalisation de la méthode selon l'invention. Le cas échéant, des éléments permettant, pour plus de facilité d'implémentation, le choix d'un kit approprié sont rapportés ci-dessus. Ces indications peuvent être :

- le substrat employé doit être spécifique du facteur Xa mais peu affin de ce dernier pour ne pas interférer avec la réaction entre l'enzyme et son inhibiteur.
- Par exemple, le substrat employé doit avoir une constante de Michaelis KM considérée élevée, notamment comprise approximativement entre 10 $\mu$M et 1000 $\mu$M ;
- L'affinité entre l'enzyme et le substrat étant volontairement choisie faible, la constante catalytique kcat de l'enzyme pour le substrat doit être suffisamment élevée, par exemple supérieure à approximativement 10 s-1, ou supérieure à 10 s-1;
- La concentration initiale en substrat [S]0 doit être suffisante pour permettre la génération du marqueur durant toute la durée d de la mesure, sans être trop élevée pour interférer au minimum avec la réaction entre l'enzyme et son inhibiteur : sa valeur peut par exemple être inférieure à KM*10 ;
- La durée de la mesure, qui ne fait pas en soi partie d'un kit commercialisé, est préférablement choisie suffisamment longue pour permettre à l'anticoagulant, s'il est présent, d'exercer son action inhibitrice sur l'enzyme et que celle-ci soit observable via la mesure ; par exemple une durée comprise entre 10 et 1000 secondes. Des exemples particuliers sont décrits ci-après.

Selon ce mode de réalisation particulier, la mesure *in vitro* pour la détection, l'identification, et la quantification d'inhibiteurs synthétiques du facteur Xa a été effectuée selon le principe suivant :

- 25 μl d'échantillon plasmatique supposé contenir une héparine ou un AOD anti-Xa sont dilués dans 25 μl de tampon Owren Koller (TOK), puis placés au sein d'un consommable ;
- 150 μl du réactif "Substrate" (MAPA-Gly-Arg-pNA) (réactif du kit commercial STA® - Liquid Anti-Xa) spécifique du facteur Xa sont ajoutés au mélange réactionnel ;
- une étape d'incubation de 240 secondes élève le mélange réactionnel à 37°C ;
- 150 μl du réactif "F. Xa" (facteur Xa bovin) (réactif du kit commercial STA® - Liquid Anti-Xa) sont ajoutés au mélange réactionnel déclenchant ainsi la compétition entre la réaction d'inhibition et la réaction enzymatique ;
- la mesure proprement dite est alors effectuée sur 156 secondes : le facteur Xa transforme le substrat en produit tout en étant inhibé en parallèle par l'héparine ou l'AOD s'ils sont présents dans l'échantillon. Le clivage du substrat en produit induit la libération de paranitroaniline (pNA) qui est mesurée par colorimétrie à 405 nm toutes les deux secondes sur un instrument de type STA-R®.
- la mesure aboutit à l'enregistrement d'une cinétique qui est impactée par la présence, le mode d'action et la concentration de ces inhibiteurs synthétiques du facteur Xa.

De plus, selon un autre mode de réalisation particulier, la méthodologie ci-dessus décrite a été optimisée pour permettre à la fois la détection de la présence d'un inhibiteur synthétique du facteur Xa, la reconnaissance de la catégorie de l'inhibiteur synthétique du facteur Xa, l'identification de l'héparine ainsi que le dosage des HNF et des HBPM. La première méthodologie susnommée « universelle » ci-dessus, ne permettant en effet pas le dosage des AODs sur l'intégralité de la plage de concentration souhaitée (cette plage pouvant, naturellement, varier selon les objectifs), Une seconde méthodologie a été optimisée pour l'identification des AODs ainsi que pour le dosage du rivaroxaban, de l'apixaban et de l'edoxaban. Elle s'effectue selon le principe suivant :

- 6,25 μl d'échantillon plasmatique qui contient un AOD anti-Xa sont dilués dans 43,75 μl de tampon Owren Koller (TOK), puis placés au sein d'un consommable ;
- 150 μl du réactif "Substrate" (MAPA-Gly-Arg-pNA, réactif du kit commercial STA®- Liquid Anti-Xa) spécifique du facteur Xa sont ajoutés au mélange réactionnel ;
- une étape d'incubation de 240 secondes élève le mélange réactionnel à 37°C ;
- 150 μl du réactif "F. Xa" (facteur Xa bovin, réactif du kit commercial STA® - Liquid Anti-Xa) sont ajoutés au mélange réactionnel déclenchant ainsi la compétition entre la réaction d'inhibition et la réaction enzymatique ;
- la mesure proprement dite est alors effectuée sur 86 secondes : le facteur Xa transforme le substrat en produit tout en étant inhibé en parallèle par l'AOD présent dans l'échantillon. Le clivage du substrat en produit induit la libération de paranitroaniline (pNA) qui est mesurée par colorimétrie à 405 nm toutes les deux secondes sur un instrument de type STA-R®.
- la mesure aboutit à l'enregistrement d'une cinétique qui est impactée par la présence, le mode d'action et la concentration de l'AOD anti-Xa.

Notons que la seconde méthodologie est identique à la première méthodologie au facteur de dilution de l'échantillon près. La durée de la mesure diffère aussi : la densité optique est mesurée toutes les deux secondes jusqu'à t = 86 s. Dans la présente description, il pourra être fait référence aux caractéristiques de la première méthodologie comme correspondant à un mode de réalisation particulier d'une « méthodologie universelle » et il pourra être fait référence aux caractéristiques de la seconde méthodologie comme correspondant à un mode de réalisation particulier d'une « méthodologie optimisée AODs ».

De fait, s'agissant de la sensibilité de détection des deux exemples particuliers de méthodologie ici rapportés, selon les modalités d'implémentation ici décrites (notamment couple enzyme-substrat, dilution de l'échantillon et durée de mesure), les vérifications réalisées et montrées en Figures 5 à 9, commentées ci-dessus, permettent de montrer ce qui suit.

Pour la méthodologie universelle, comme appliquée ci-dessus au « facteur Xa », les concentrations finales dans le test sont :

- en enzyme : [E] ≈ 10 nM
- en substrat : [S] ≈ 482 μM
- si l'inhibiteur est
- une HNF : 0 ≤ [I] ≤ 670 nM ou 2 UI/mL ;
- une HBPM : 0 ≤ [I] ≤ 4000 nM ou 2 UI anti-Xa/mL ;
- du rivaroxaban : 0 ≤ [I] ≤ 30 nM ou 180 ng/mL ;
- de l'apixaban : 0 ≤ [I] ≤ 30 nM ou 190 ng/mL ;

- de l'edoxaban : 0 ≤ [I] ≤ 30 nM ou 230 ng/mL ;

De plus, la constante catalytique kcat et la constante de Michaelis KM associées à la réaction enzyme - substrat valent respectivement approximativement 400 s-1 et 500 $\mu$M.

Pour la méthodologie optimisée AODs les concentrations finales dans le test sont :

- en enzyme : idem que précédemment ;
- en substrat : idem que précédemment ;
- si l'inhibiteur est
- du rivaroxaban : 0 ≤ [I] ≤ 30 nM ou 720 ng/mL ;
- de l'apixaban : 0 ≤ [I] ≤ 30 nM ou 760 ng/mL ;
- de l'edoxaban : 0 ≤ [I] ≤ 30 nM ou 920 ng/mL ;

La constante catalytique kcat et la constante de Michaelis KM associées à la réaction enzyme - substrat ont les mêmes valeurs que précédemment.

En résumé, l'approche décrite ici permet en utilisant la méthodologie universelle de doser les HNF, les HBPM, le rivaroxaban, l'apixaban et l'edoxaban sur la plage :

- de 0.0 à 2.0 UI/mL pour les HNF ;
- de 0.0 à 2.0 UI anti-Xa/mL pour les HBPM ;
- de 0.0 à 180.0 ng/mL pour le rivaroxaban ;
- de 0.0 à 190.0 ng/mL pour l'apixaban ;
- de 0.0 à 230.0 ng/mL pour l'edoxaban.

Elle permet en utilisant la méthodologie optimisée AODs comme appliquée ci-dessus au « facteur Xa », de doser le rivaroxaban, l'apixaban et l'edoxaban sur la plage (résultats expérimentaux ici non illustrés, mais obtenus suite à des manipulations identiques à celles ayant permis d'obtenir les résultats des figures 5 à 9, avec les ajustements opérés dans la méthodologie ici appelée méthodologie optimisée AOD):

- de 0.0 à 720.0 ng/mL pour le rivaroxaban ;
- de 0.0 à 760.0 ng/mL pour l'apixaban ;
- de 0.0 à 920.0 ng/mL pour l'edoxaban.

Ainsi, au travers d'une unique méthode et sans calibration, l'approche proposée et décrite dans la présente demande permet de détecter la présence et de doser cinq molécules anti-Xa en contraste avec les méthodes commerciales actuelles qui nécessitent et imposent une méthodologie dédiée et une calibration par molécule. Elle permet en outre d'identifier la molécule, chose impossible aujourd'hui. Un dosage plus particulièrement appliqué à la recherche d'un inhibiteur du facteur IIa peut comprendre les étapes et/ou modalités suivantes :

- Véchantillon = 175$\mu$L
- Dilution au 1/12ème avec le réactif R2 du kit STA-Stachrom Heparin

   ◦ Composition : TRIS EDTA ph 8.4
   ◦ Préparation : Flacon de 15 mL QSP 150mL

- Incubation 240 sec (notamment à une température telle qu'indiquée dans les autres exemples, ou la présente demande)
- Ra = 75$\mu$L = STA-Stachrom ATIII Substrat

   ◦ Composition : Substrat chromogène CBS 61.50, environ 1.4$\mu$mole d'EtM-SPro-Arg-pNA, AcOH par mL de réactif reconstitué.
   ◦ Reconstitution avec 6 mL d'eau distillée

- Rd = 50$\mu$L = STA-Stachrom ATIII Thrombin

   ◦ Composition : Thrombine bovine, environ 11.3 nKat par mL après reconstitution
   ◦ Reconstitution : avec 6 mL de R2 STA-Stachrom Heparin préalablement dilué.

2.2.2 Post-traitement

La figure 10 détaille la cascade de modèles d'apprentissage automatique, représentative du mode particulier de réalisation ici implémenté, qui analyse et interprète la cinétique obtenue par la mesure expérimentale dans le but de rendre le résultat biologique attendu.

La cascade est composée de neuf modèles d'apprentissage automatique ; ces neuf modèles prennent en entrée la cinétique obtenue par la mesure expérimentale et donnent en sortie un résultat :

- Le premier modèle (Modèle A) est un modèle de classification : selon l'allure de la cinétique qui lui est donnée en entrée, il détermine si oui ou non l'échantillon analysé contient un inhibiteur synthétique du facteur Xa ;
- Le second modèle (Modèle B) est aussi un modèle de classification : sachant que l'échantillon analysé contient un inhibiteur synthétique du facteur Xa et selon l'allure de la cinétique qui lui est présentée en entrée, il reconnait la catégorie de l'inhibiteur synthétique anti-Xa : héparine ou AOD ;
- Si l'inhibiteur est une héparine, et selon l'allure de la cinétique qui lui est donnée en entrée, un modèle de classification (Modèle C) identifie s'il s'agit d'une HNF ou d'une HBPM ;
- Si l'inhibiteur est une HNF, et selon l'allure de la cinétique qui lui est donnée en entrée, un modèle de régression calcule la concentration en HNF ;
- Si l'inhibiteur est une HBPM, et selon l'allure de la cinétique qui lui est donnée en entrée, un modèle de régression calcule la concentration en HBPM ;
- Selon un mode particulier de réalisation, si l'inhibiteur est un AOD, une mesure expérimentale est relancée en utilisant la méthodologie optimisée AODs détaillée dans cette description et une nouvelle cinétique est enregistrée puis présentée en entrée d'un modèle de classification (Modèle C) qui identifie si l'inhibiteur est du rivaroxaban, de l'apixaban ou de l'edoxaban ;
- Si l'inhibiteur est du rivaroxaban, et selon l'allure de la cinétique qui lui est présentée en entrée, un modèle de régression calcule la concentration en rivaroxaban ;
- Si l'inhibiteur est de l'apixaban, et selon l'allure de la cinétique qui lui est présentée en entrée, un modèle de régression calcule la concentration en apixaban ;
- Si l'inhibiteur est de l'edoxaban, et selon l'allure de la cinétique qui lui est présentée en entrée, un modèle de régression calcule la concentration en edoxaban.

Enfin, un modèle D de régression peut permettre d'effectuer un dosage.

Les sections suivantes listent respectivement pour chacun de ces modèles d'apprentissage automatique, les jeux de données d'apprentissage et de validation qui ont servi à leur entraînement, ainsi que les algorithmes associés, de façon à illustrer une preuve de concept à la base de la présente invention.

Notons que pour chaque jeu de données, les concentrations en inhibiteurs synthétiques du facteur Xa (HNF, HBPM, rivaroxaban, apixaban et edoxaban) ont été mesurées sur automate STA-R® en utilisant le kit commercial STA-R®-Liquid Anti-Xa, ainsi que les calibrants et méthodologies commerciales associées. Naturellement, l'invention objet de la présente demande peut également être exécutée, le cas échéant conformément aux directives des fabricants, en utilisant le contenu de kits différents pour son implémentation. La présente partie expérimentale détaille des méthodes permettant de s'assurer une transposition correcte, notamment en ce qui concerne les plages de valeurs des inhibiteurs pouvant être détectés. Enfin, on notera que, de façon logique, les conditions dans lesquelles les mesures cinétiques ont été effectuées, doivent coïncider entre les échantillons à analyser par la méthode de l'invention, et ceux qui ont servi à la réalisation des données d'apprentissage et de validation, pour chaque modèle d'apprentissage considéré.

Détection de la présence ou de l'absence d'un anticoagulant anti-Xa

Jeux de données

- Données d'apprentissage
- 3 cinétiques mesurées sur le STA-R® AUT05450 sur un plasma surchargé par 0.0 UI/ml d'HNF calcique (Calciparine®) ;
- 3 cinétiques mesurées sur le STA-R® AUT06366 sur un plasma surchargé par 0.0 UI/ml d'HNF sodique (Héparine Choay®) ;
- 3 cinétiques mesurées sur le STA-R® AUT06366 sur un plasma surchargé par 0.0 UI anti-Xa/ml d'HBPM (Fragmine®) ;
- 3 cinétiques mesurées sur le STA-R® AUT06366 sur un plasma surchargé par 0.0 UI anti-Xa/ml d'HBPM (Lovenox®) ;
- 3 cinétiques mesurées sur le STA-R® AUT06366 sur un plasma surchargé par 0.0 UI anti-Xa/ml d'HBPM (Innohep®) ;
- 3 cinétiques mesurées sur le STA-R® AUT05016 sur un plasma surchargé par 0.0 UI/ml d'HNF calcique (Calciparine®) ;
- 3 cinétiques mesurées sur le STA-R® AUT00603 sur un plasma surchargé par 0.0 UI/ml d'HNF sodique (Héparine Choay®) ;

- 3 cinétiques mesurées sur le STA-R® AUT05016 sur un plasma surchargé par 0.0 UI anti-Xa/ml d'HBPM (Fragmine®) ;
- 3 cinétiques mesurées sur le STA-R® AUT00603 sur un plasma surchargé par 0.0 UI anti-Xa/ml d'HBPM (Lovenox®) ;
- 3 cinétiques mesurées sur le STA-R® AUT00603 sur un plasma surchargé par 0.0 UI anti-Xa/ml d'HBPM (Innohep®) ;
- 3 cinétiques mesurées sur le STA-R® AUT06399 sur un plasma surchargé par 0.0 UI/ml d'HNF calcique (Calciparine®) ;
- 3 cinétiques mesurées sur le STA-R® AUT06399 sur un plasma surchargé par 0.0 UI/ml d'HNF sodique (Héparine Choay®) ;
- 3 cinétiques mesurées sur le STA-R® AUT06399 sur un plasma surchargé par 0.0 UI anti-Xa/ml d'HBPM (Fragmine®) ;
- 3 cinétiques mesurées sur le STA-R® AUT06399 sur un plasma surchargé par 0.0 UI anti-Xa/ml d'HBPM (Lovenox®) ;
- 3 cinétiques mesurées sur le STA-R® AUT06399 sur un plasma surchargé par 0.0 UI anti-Xa/ml d'HBPM (Innohep®) ;
- 3 cinétiques mesurées sur le STA-R® AUT06399 sur un plasma surchargé par 0.0 ng/ml de rivaroxaban (Xarelto®) ;
- 3 cinétiques mesurées sur le STA-R® AUT06399 sur un plasma surchargé par 0.0 ng/ml d'apixaban (Eliquis®) ;
- 3 cinétiques mesurées sur le STA-R® AUT06399 sur un plasma surchargé par 0.0 ng/ml d'edoxaban (Lixiana®/Savaysa®) ;
- 3 cinétiques mesurées sur le STA-R® AUT00603 sur un plasma surchargé par 0.0 ng/ml de rivaroxaban (Xarelto®) ;
- 3 cinétiques mesurées sur le STA-R® AUT00603 sur un plasma surchargé par 0.0 ng/ml d'apixaban (Eliquis®) ;
- 3 cinétiques mesurées sur le STA-R® AUT00603 sur un plasma surchargé par 0.0 ng/ml d'edoxaban (Lixiana®/Savaysa®) ;
- 3 cinétiques mesurées sur le STA-R® AUT06366 sur un plasma surchargé par 0.0 ng/ml de rivaroxaban (Xarelto®) ;
- 3 cinétiques mesurées sur le STA-R® AUT06366 sur un plasma surchargé par 0.0 ng/ml d'apixaban (Eliquis®) ;
- 3 cinétiques mesurées sur le STA-R® AUT06366 sur un plasma surchargé par 0.0 ng/ml d'edoxaban (Lixiana®/Savaysa®) ;
- 3 cinétiques mesurées sur le STA-R® AUT05450 sur un plasma surchargé par 0.0 ng/ml de rivaroxaban (Xarelto®) ;
- 3 cinétiques mesurées sur le STA-R® AUT05450 sur un plasma surchargé par 0.0 ng/ml d'apixaban (Eliquis®) ;
- 3 cinétiques mesurées sur le STA-R® AUT05450 sur un plasma surchargé par 0.0 ng/ml d'edoxaban (Lixiana®/Savaysa®) ;
- Mesures effectuées en triplicats pour chaque surcharge ;
- Méthodologie universelle.

Modèle d'apprentissage automatique: Une machine à vecteurs de supports (One Class SVM) a été entraînée avec le jeu de données décrit précédemment (plasmas non surchargés en inhibiteurs du facteur Xa) de manière semi-supervisée. L'optimisation des hyper-paramètres a été réalisée selon la technique de validation croisée « leave-one-out cross-validation ». Les hyper-paramètres du modèle sont:

- Fonction noyau : Radial Basis Function
- $\gamma \approx 3{,}77 \ 10^{-5}$
- $v \approx 0{,}0131$

Identification de la catégorie de l'anticoagulant anti-Xa

Jeux de données

- Données d'apprentissage
- 60 cinétiques mesurées sur le STA-R® AUT05450 sur un plasma surchargé par 0.12, 0.225, 0.33, 0.43, 0.55, 0.66, 0.75, 0.9, 0.875, 0.995, 1.13, 1.24, 1.315, 1.44, 1.53, 1.63, 1.7, 1.88, 1.955 et 2.005 UI/ml d'HNF calcique (Calciparine®) ;
- 60 cinétiques mesurées sur le STA-R® AUT06366 sur un plasma surchargé par 0.11, 0.21, 0.32, 0.43, 0.545, 0.625, 0.635, 0.76, 0.87, 0.965, 1.14, 1.17, 1.295, 1.425, 1.52, 1.605, 1.695, 1.835, 1.84 et 1.99 UI/ml d'HNF sodique (Héparine Choay®) ;
- 60 cinétiques mesurées sur le STA-R® AUT06366 sur un plasma surchargé par 0.145, 0.245, 0.35, 0.44, 0.535, 0.64, 0.735, 0.85, 0.935, 1.07, 1.14, 1.24, 1.265, 1.44, 1.495, 1.55, 1.66, 1.745, 1.785 et 2.255 UI anti-Xa/ml d'HBPM (Fragmine®) ;
- 60 cinétiques mesurées sur le STA-R® AUT06366 sur un plasma surchargé par 0.105, 0.19, 0.3, 0.4, 0.485, 0.57, 0.685, 0.78, 0.865, 0.945, 1.035, 1.145, 1.23, 1.32, 1.42, 1.495, 1.58, 1.69, 1.755 et 1.805 UI anti-Xa/ml d'HBPM (Lovenox®) ;
- 60 cinétiques mesurées sur le STA-R® AUT06366 sur un plasma surchargé par 0.11, 0.2, 0.295, 0.395, 0.465, 0.535, 0.63, 0.795, 0.885, 0.955, 1.055, 1.21, 1.27, 1.37, 1.455, 1.515, 1.64, 1.81, 1.83 et 1.97 UI anti-Xa/ml d'HBPM (Innohep®) ;

- 60 cinétiques mesurées sur le STA-R® AUT06399 sur un plasma surchargé par 10, 20, 30, 38, 46, 64, 72, 82, 90, 101, 110, 115, 130, 140, 148, 152, 166, 164 et 192 ng/ml de rivaroxaban (Xarelto®) ;
- 60 cinétiques mesurées sur le STA-R® AUT06399 sur un plasma surchargé par 10, 20, 32, 41, 46, 57, 65, 76, 79, 96, 105, 116, 125, 135, 138, 152, 161, 169, 180 et 186 ng/ml d'apixaban (Eliquis®) ;
- 60 cinétiques mesurées sur le STA-R® AUT06399 sur un plasma surchargé par 10, 21, 29, 39, 48, 60, 73, 81, 92, 107, 115, 129, 136, 129, 127, 149, 163, 181, 195 et 200 ng/ml d'edoxaban (Lixiana®/Savaysa®) ;
- 60 cinétiques mesurées sur le STA-R® AUT00603 sur un plasma surchargé par 10, 20, 35, 41, 49, 56, 66, 73, 84, 93, 105, 110, 116, 134, 143, 155, 160, 175, 181 et 198 ng/ml de rivaroxaban (Xarelto®) ;
- 60 cinétiques mesurées sur le STA-R® AUT00603 sur un plasma surchargé par 10, 20, 20, 26, 36, 43, 53, 60, 70, 75, 93, 103, 110, 121, 135, 136, 152, 160, 175, 185 et 191 ng/ml d'apixaban (Eliquis®) ;
- 60 cinétiques mesurées sur le STA-R® AUT00603 sur un plasma surchargé par 10, 21, 27, 40, 49, 64, 73, 90, 99, 108, 122, 132, 144, 130, 136, 153, 169, 192, 194 et 216 ng/ml d'edoxaban (Lixiana®/Savaysa®) ;
- 60 cinétiques mesurées sur le STA-R® AUT06366 sur un plasma surchargé par 10, 20, 25, 34, 43, 50, 62, 74, 84, 93, 104, 108, 118, 133, 143, 159, 158, 176, 173 et 202 ng/ml de rivaroxaban (Xarelto®) ;
- 60 cinétiques mesurées sur le STA-R® AUT06366 sur un plasma surchargé par 10, 20, 25, 34, 44, 50, 61, 70, 74, 94, 103, 110, 122, 135, 138, 147, 162, 173, 182 et 186 ng/ml d'apixaban (Eliquis®) ;
- 60 cinétiques mesurées sur le STA-R® AUT06366 sur un plasma surchargé par 10, 20, 28, 40, 49, 64, 74, 95, 103, 110, 122, 132, 143, 131, 132, 157, 171, 195, 191 et 201 ng/ml d'edoxaban (Lixiana®/Savaysa®) ;
- Mesures effectuées en triplicats pour chaque surcharge ;
- Méthodologie universelle.
- Données de validation
- 60 cinétiques mesurées sur le STA-R® AUT05016 sur un plasma surchargé par 0.095, 0.205, 0.315, 0.44, 0.555, 0.655, 0.77, 0.84, 0.855, 0.98, 1.15, 1.275, 1.335, 1.43, 1.555, 1.6, 1.75, 1.845, 1.92 et 1.96 UI/ml d'HNF calcique (Calciparine®) ;
- 60 cinétiques mesurées sur le STA-R® AUT00603 sur un plasma surchargé par 0.115, 0.23, 0.32, 0.455, 0.535, 0.655, 0.66, 0.78, 0.865, 0.995, 1.175, 1.2, 1.355, 1.47, 1.555, 1.64, 1.735, 1.8, 1.88 et 2.025 UI/ml d'HNF sodique (Héparine Choay®) ;
- 60 cinétiques mesurées sur le STA-R® AUT05016 sur un plasma surchargé par 0.165, 0.255, 0.345, 0.445, 0.54, 0.63, 0.73, 0.82, 0.92, 1.045, 1.125, 1.16, 1.3, 1.355, 1.475, 1.57, 1.685, 1.75, 1.835 et 2.16 UI anti-Xa/ml d'HBPM (Fragmine®) ;
- 60 cinétiques mesurées sur le STA-R® AUT00603 sur un plasma surchargé par 0.1, 0.195, 0.31, 0.4, 0.515, 0.625, 0.71, 0.83, 0.925, 1.015, 1.085, 1.205, 1.28, 1.36, 1.49, 1.575, 1.65, 1.81, 1.85 et 1.965 UI anti-Xa/ml d'HBPM (Lovenox®) ;
- 60 cinétiques mesurées sur le STA-R® AUT00603 sur un plasma surchargé par 0.14, 0.22, 0.305, 0.42, 0.5, 0.56, 0.66, 0.83, 0.905, 0.995, 1.07, 1.25, 1.275, 1.365, 1.47, 1.615, 1.66, 1.805, 1.9 et 1.975 UI anti-Xa/ml d'HBPM (Innohep®) ;
- 60 cinétiques mesurées sur le STA-R® AUT05450 sur un plasma surchargé par 10, 20, 35, 41, 48, 59, 65, 77, 85, 95, 103, 113, 121, 134, 145, 160, 161, 171, 169 et 202 ng/ml de rivaroxaban (Xarelto®) ;
- 60 cinétiques mesurées sur le STA-R® AUT05450 sur un plasma surchargé par 10, 20, 27, 39, 41, 52, 61, 67, 76, 93, 96, 105, 123, 127, 140, 145, 155, 167, 184 et 182 ng/ml d'apixaban (Eliquis®) ;
- 60 cinétiques mesurées sur le STA-R® AUT05450 sur un plasma surchargé par 10, 21, 28, 41, 49, 62, 74, 92, 101, 112, 120, 132, 142, 129, 133, 155, 168, 188, 200 et 206 ng/ml d'edoxaban (Lixiana®/Savaysa®) ;
- Mesures effectuées en triplicats pour chaque surcharge ;
- Méthodologie universelle.

Modèle d'apprentissage automatique

Un modèle des k plus proches voisins a été entraîné avec le jeu de données décrit précédemment. Ses hyper-paramètres sont :

- k = 5 ;
- Métrique : distance euclidienne.

Identification des héparines

Jeux de données

- Données d'apprentissage

- 60 cinétiques mesurées sur le STA-R® AUT05450 sur un plasma surchargé par 0.12, 0.225, 0.33, 0.43, 0.55, 0.66, 0.75, 0.9, 0.875, 0.995, 1.13, 1.24, 1.315, 1.44, 1.53, 1.63, 1.7, 1.88, 1.955 et 2.005 UI/ml d'HNF calcique (Calciparine®) ;
- 60 cinétiques mesurées sur le STA-R® AUT05016 sur un plasma surchargé par 0.095, 0.205, 0.315, 0.44, 0.555, 0.655, 0.77, 0.84, 0.855, 0.98, 1.15, 1.275, 1.335, 1.43, 1.555, 1.6, 1.75, 1.845, 1.92 et 1.96 UI/ml d'HNF calcique (Calciparine®) ;
- 60 cinétiques mesurées sur le STA-R® AUT06366 sur un plasma surchargé par 0.11, 0.21, 0.32, 0.43, 0.545, 0.625, 0.635, 0.76, 0.87, 0.965, 1.14, 1.17, 1.295, 1.425, 1.52, 1.605, 1.695, 1.835, 1.84 et 1.99 UI/ml d'HNF sodique (Héparine Choay®) ;
- 60 cinétiques mesurées sur le STA-R® AUT00603 sur un plasma surchargé par 0.115, 0.23, 0.32, 0.455, 0.535, 0.655, 0.66, 0.78, 0.865, 0.995, 1.175, 1.2, 1.355, 1.47, 1.555, 1.64, 1.735, 1.8, 1.88 et 2.025 UI/ml d'HNF sodique (Héparine Choay®) ;
- 60 cinétiques mesurées sur le STA-R® AUT06366 sur un plasma surchargé par 0.145, 0.245, 0.35, 0.44, 0.535, 0.64, 0.735, 0.85, 0.935, 1.07, 1.14, 1.24, 1.265, 1.44, 1.495, 1.55, 1.66, 1.745, 1.785 et 2.255 UI anti-Xa/ml d'HBPM (Fragmine®) ;
- 60 cinétiques mesurées sur le STA-R® AUT05016 sur un plasma surchargé par 0.165, 0.255, 0.345, 0.445, 0.54, 0.63, 0.73, 0.82, 0.92, 1.045, 1.125, 1.16, 1.3, 1.355, 1.475, 1.57, 1.685, 1.75, 1.835 et 2.16 UI anti-Xa/ml d'HBPM (Fragmine®) ;
- 60 cinétiques mesurées sur le STA-R® AUT06366 sur un plasma surchargé par 0.105, 0.19, 0.3, 0.4, 0.485, 0.57, 0.685, 0.78, 0.865, 0.945, 1.035, 1.145, 1.23, 1.32, 1.42, 1.495, 1.58, 1.69, 1.755 et 1.805 UI anti-Xa/ml d'HBPM (Lovenox®) ;
- 60 cinétiques mesurées sur le STA-R® AUT00603 sur un plasma surchargé par 0.1, 0.195, 0.31, 0.4, 0.515, 0.625, 0.71, 0.83, 0.925, 1.015, 1.085, 1.205, 1.28, 1.36, 1.49, 1.575, 1.65, 1.81, 1.85 et 1.965 UI anti-Xa/ml d'HBPM (Lovenox®) ;
- 60 cinétiques mesurées sur le STA-R® AUT06366 sur un plasma surchargé par 0.11, 0.2, 0.295, 0.395, 0.465, 0.535, 0.63, 0.795, 0.885, 0.955, 1.055, 1.21, 1.27, 1.37, 1.455, 1.515, 1.64, 1.81, 1.83 et 1.97 UI anti-Xa/ml d'HBPM (Innohep®) ;
- 60 cinétiques mesurées sur le STA-R® AUT00603 sur un plasma surchargé par 0.14, 0.22, 0.305, 0.42, 0.5, 0.56, 0.66, 0.83, 0.905, 0.995, 1.07, 1.25, 1.275, 1.365, 1.47, 1.615, 1.66, 1.805, 1.9 et 1.975 UI anti-Xa/ml d'HBPM (Innohep®) ;
- Mesures effectuées en triplicats pour chaque surcharge ;
- Méthodologie universelle.
- Données de validation
- 24 cinétiques mesurées sur le STA-R® AUT06399 sur un plasma surchargé par 0.25, 0.5, 0.75, 1.0, 1.25, 1.5, 1.75 et 2.0 UI/ml d'HNF calcique (Calciparine®) ;
- 24 cinétiques mesurées sur le STA-R® AUT06399 sur un plasma surchargé par 0.25, 0.5, 0.75, 1.0, 1.25, 1.5, 1.75 et 2.0 UI/ml d'HNF sodique (Héparine Choay®) ;
- 24 cinétiques mesurées sur le STA-R® AUT06399 sur un plasma surchargé par 0.25, 0.5, 0.75, 1.0, 1.25, 1.5, 1.75 et 2.0 UI anti-Xa/ml d'HBPM (Fragmine®) ;
- 24 cinétiques mesurées sur le STA-R® AUT06399 sur un plasma surchargé par 0.25, 0.5, 0.75, 1.0, 1.25, 1.5, 1.75 et 2.0 UI anti-Xa/ml d'HBPM (Lovenox®) ;
- 24 cinétiques mesurées sur le STA-R® AUT06399 sur un plasma surchargé par 0.25, 0.5, 0.75, 1.0, 1.25, 1.5, 1.75 et 2.0 UI anti-Xa/ml d'HBPM (Innohep®) ;
- Mesures effectuées en triplicats pour chaque surcharge ;
- Méthodologie universelle.

Modèle d'apprentissage automatique

Un perceptron multi-couches (réseau de neurones) a été entraîné avec le jeu de données décrit précédemment. Ses hyper-paramètres sont :

- 77 neurones dans la couche d'entrée ;
- 27 neurones dans la couche cachée ;
- 3 neurones dans la couche de sortie ;
- Fonction d'activation pour chaque neurone des différentes couches cachées : unité de rectification linéaire (Rectified Linear Unit ou ReLU) ;
- Fonction d'activation pour chaque neurone de la couche de sortie : softmax.

Dosages des héparines non fractionnées

Jeux de données

- Données d'apprentissage
- 63 cinétiques mesurées sur le STA-R® AUT05450 sur un plasma surchargé par 0.0, 0.12, 0.225, 0.33, 0.43, 0.55, 0.66, 0.75, 0.9, 0.875, 0.995, 1.13, 1.24, 1.315, 1.44, 1.53, 1.63, 1.7, 1.88, 1.955 et 2.005 UI/ml d'HNF calcique (Calciparine®) ;
- 63 cinétiques mesurées sur le STA-R® AUT06366 sur un plasma surchargé par 0.0, 0.11, 0.21, 0.32, 0.43, 0.545, 0.625, 0.635, 0.76, 0.87, 0.965, 1.14, 1.17, 1.295, 1.425, 1.52, 1.605, 1.695, 1.835, 1.84 et 1.99 UI/ml d'HNF sodique (Héparine Choay®) ;
- Mesures effectuées en triplicats pour chaque surcharge ;
- Méthodologie universelle.
- Données de validation
- 63 cinétiques mesurées sur le STA-R® AUT05016 sur un plasma surchargé par 0.0, 0.095, 0.205, 0.315, 0.44, 0.555, 0.655, 0.77, 0.84, 0.855, 0.98, 1.15, 1.275, 1.335, 1.43, 1.555, 1.6, 1.75, 1.845, 1.92 et 1.96 UI/ml d'HNF calcique (Calciparine®) ;
- 63 cinétiques mesurées sur le STA-R® AUT00603 sur un plasma surchargé par 0.0, 0.115, 0.23, 0.32, 0.455, 0.535, 0.655, 0.66, 0.78, 0.865, 0.995, 1.175, 1.2, 1.355, 1.47, 1.555, 1.64, 1.735, 1.8, 1.88 et 2.025 UI/ml d'HNF sodique (Héparine Choay®) ;
- Mesures effectuées en triplicats pour chaque surcharge ;
- Méthodologie universelle.

Modèle d'apprentissage automatique

Un perceptron multi-couches (réseau de neurones) a été entraîné avec le jeu de données décrit précédemment. Ses hyper-paramètres sont :

- 77 neurones dans la couche d'entrée ;
- 27 neurones dans la couche cachée ;
- 1 neurone dans la couche de sortie ;
- Fonction d'activation pour chaque neurone des différentes couches cachées : unité de rectification linéaire (Rectified Linear Unit ou ReLU).

Dosages des héparines de bas poids moléculaire

Jeux de données

- Données d'apprentissage
- 63 cinétiques mesurées sur le STA-R® AUT06366 sur un plasma surchargé par 0.0, 0.145, 0.245, 0.35, 0.44, 0.535, 0.64, 0.735, 0.85, 0.935, 1.07, 1.14, 1.24, 1.265, 1.44, 1.495, 1.55, 1.66, 1.745, 1.785 et 2.255 UI anti-Xa/ml d'HBPM (Fragmine®) ;
- 63 cinétiques mesurées sur le STA-R® AUT06366 sur un plasma surchargé par 0.0, 0.105, 0.19, 0.3, 0.4, 0.485, 0.57, 0.685, 0.78, 0.865, 0.945, 1.035, 1.145, 1.23, 1.32, 1.42, 1.495, 1.58, 1.69, 1.755 et 1.805 UI anti-Xa/ml d'HBPM (Lovenox®) ;
- 63 cinétiques mesurées sur le STA-R® AUT06366 sur un plasma surchargé par 0.0, 0.11, 0.2, 0.295, 0.395, 0.465, 0.535, 0.63, 0.795, 0.885, 0.955, 1.055, 1.21, 1.27, 1.37, 1.455, 1.515, 1.64, 1.81, 1.83 et 1.97 UI anti-Xa/ml d'HBPM (Innohep®) ;
- Mesures effectuées en triplicats pour chaque surcharge ;
- Méthodologie universelle.
- Données de validation
- 63 cinétiques mesurées sur le STA-R® AUT05016 sur un plasma surchargé par 0.0, 0.165, 0.255, 0.345, 0.445, 0.54, 0.63, 0.73, 0.82, 0.92, 1.045, 1.125, 1.16, 1.3, 1.355, 1.475, 1.57, 1.685, 1.75, 1.835 et 2.16 UI anti-Xa/ml d'HBPM (Fragmine®) ;
- 63 cinétiques mesurées sur le STA-R® AUT00603 sur un plasma surchargé par 0.0, 0.1, 0.195, 0.31, 0.4, 0.515, 0.625, 0.71, 0.83, 0.925, 1.015, 1.085, 1.205, 1.28, 1.36, 1.49, 1.575, 1.65, 1.81, 1.85 et 1.965 **UI** anti-Xa/ml d'HBPM (Lovenox®) ;
- 63 cinétiques mesurées sur le STA-R® AUT00603 sur un plasma surchargé par 0.0, 0.14, 0.22, 0.305, 0.42, 0.5, 0.56, 0.66, 0.83, 0.905, 0.995, 1.07, 1.25, 1.275, 1.365, 1.47, 1.615, 1.66, 1.805, 1.9 et 1.975 **UI** anti-Xa/ml d'HBPM (Innohep®) ;
- Mesures effectuées en triplicats pour chaque surcharge ;
- Méthodologie universelle.

Modèle d'apprentissage automatique

Un perceptron multi-couches (réseau de neurones) a été entraîné avec le jeu de données décrit précédemment. Ses hyper-paramètres sont :

- 77 neurones dans la couche d'entrée ;
- 27 neurones dans la couche cachée ;
- 1 neurone dans la couche de sortie ;
- Fonction d'activation pour chaque neurone des différentes couches cachées : unité de rectification linéaire (Rectified Linear Unit ou ReLU).

Identification de l'AOD anti-Xa

Jeux de données

- Données d'apprentissage
- 60 cinétiques mesurées sur le STA-R® AUT00460 sur un plasma surchargé par 10, 20, 29, 37, 44, 54, 62, 89, 115, 143, 160, 192, 226, 252, 287, 319, 331, 361, 391 et 407 ng/ml de rivaroxaban (Xarelto®) ;
- 69 cinétiques mesurées sur le STA-R® AUT00460 sur un plasma surchargé par 10, 20, 31, 42, 48, 72, 92, 116, 143, 163, 192, 217, 247, 276, 298, 321, 348, 370, 397, 416, 439, 459 et 473 ng/ml d'apixaban (Eliquis®) ;
- 69 cinétiques mesurées sur le STA-R® AUT00460 sur un plasma surchargé par 16, 21, 30, 39, 51, 76, 100, 128, 142, 155, 187, 215, 245, 269, 284, 322, 347, 359, 380, 393, 410, 426 et 436 ng/ml d'edoxaban (Lixiana®/Savaysa®) ;
- Mesures effectuées en triplicats pour chaque surcharge ;
- Méthodologie optimisée AODs.
- Données de validation
- 60 cinétiques mesurées sur le STA-R® AUT00460 sur un plasma surchargé par 10, 20, 29, 37, 44, 54, 62, 89, 115, 143, 160, 192, 226, 252, 287, 319, 331, 361, 391 et 407 ng/ml de rivaroxaban (Xarelto®) ;
- 69 cinétiques mesurées sur le STA-R® AUT00460 sur un plasma surchargé par 10, 20, 31, 42, 48, 72, 92, 116, 143, 163, 192, 217, 247, 276, 298, 321, 348, 370, 397, 416, 439, 459 et 473 ng/ml d'apixaban (Eliquis®) ;
- 69 cinétiques mesurées sur le STA-R® AUT00460 sur un plasma surchargé par 16, 21, 30, 39, 51, 76, 100, 128, 142, 155, 187, 215, 245, 269, 284, 322, 347, 359, 380, 393, 410, 426 et 436 ng/ml d'edoxaban (Lixiana®/Savaysa®) ;
- Mesures effectuées en triplicats pour chaque surcharge ;
- Méthodologie optimisée AODs.

Modèle d'apprentissage automatique

Un perceptron multi-couches (réseau de neurones) a été entraîné avec le jeu de données décrit précédemment. Ses hyper-paramètres sont :

- 42 neurones dans la couche d'entrée ;
- 29 neurones dans la première couche cachée ;
- 16 neurones dans la seconde couche cachée ;
- 3 neurones dans la couche de sortie ;
- Fonction d'activation pour chaque neurone des différentes couches cachées : unité de rectification linéaire (Rectified Linear Unit ou ReLU) ;
- Fonction d'activation pour chaque neurone de la couche de sortie : softmax.

Dosage du rivaroxaban

Jeux de données

- Données d'apprentissage
- 63 cinétiques mesurées sur le STA-R® AUT00460 sur un plasma surchargé par 0, 10, 20, 29, 37, 44, 54, 62, 89, 115, 143, 160, 192, 226, 252, 287, 319, 331, 361, 391 et 407 ng/ml de rivaroxaban (Xarelto®) ;
- Mesures effectuées en triplicats pour chaque surcharge ;
- Méthodologie optimisée AODs.
- Données de validation
- 63 cinétiques mesurées sur le STA-R® AUT00460 sur un plasma surchargé par 0, 10, 20, 29, 37, 44, 54, 62, 89, 115, 143, 160, 192, 226, 252, 287, 319, 331, 361, 391 et 407 ng/ml de rivaroxaban (Xarelto®) ;
- Mesures effectuées en triplicats pour chaque surcharge ;
- Méthodologie optimisée AODs.

Modèle d'apprentissage automatique

Un perceptron multi-couches (réseau de neurones) a été entraîné avec le jeu de données décrit précédemment. Ses hyper-paramètres sont :

- 42 neurones dans la couche d'entrée ;
- 29 neurones dans la première couche cachée ;
- 16 neurones dans la seconde couche cachée ;
- 1 neurone dans la couche de sortie ;
- Fonction d'activation pour chaque neurone des différentes couches cachées : unité de rectification linéaire (Rectified Linear Unit ou ReLU).

Dosage de l'apixaban

Jeux de données

- Données d'apprentissage
- 72 cinétiques mesurées sur le STA-R® AUT00460 sur un plasma surchargé par 0, 10, 20, 31, 42, 48, 72, 92, 116, 143, 163, 192, 217, 247, 276, 298, 321, 348, 370, 397, 416, 439, 459 et 473 ng/ml d'apixaban (Eliquis®) ;
- Mesures effectuées en triplicats pour chaque surcharge ;
- Méthodologie optimisée AODs.
- Données de validation
- 72 cinétiques mesurées sur le STA-R® AUT00460 sur un plasma surchargé par 0, 10, 20, 31, 42, 48, 72, 92, 116, 143, 163, 192, 217, 247, 276, 298, 321, 348, 370, 397, 416, 439, 459 et 473 ng/ml d'apixaban (Eliquis®) ;
- Mesures effectuées en triplicats pour chaque surcharge ;
- Méthodologie optimisée AODs.

Modèle d'apprentissage automatique

Un perceptron multi-couches (réseau de neurones) a été entraîné avec le jeu de données décrit précédemment. Ses hyper-paramètres sont :

- 42 neurones dans la couche d'entrée ;
- 29 neurones dans la première couche cachée ;
- 16 neurones dans la seconde couche cachée ;
- 1 neurone dans la couche de sortie ;
- Fonction d'activation pour chaque neurone des différentes couches cachées : unité de rectification linéaire (Rectified Linear Unit ou ReLU).

Dosage de l'edoxaban

Jeux de données

- Données d'apprentissage
- 72 cinétiques mesurées sur le STA-R® AUT00460 sur un plasma surchargé par 0, 16, 21, 30, 39, 51, 76, 100, 128, 142, 155, 187, 215, 245, 269, 284, 322, 347, 359, 380, 393, 410, 426 et 436 ng/ml d'edoxaban (Lixiana®/Savaysa®) ;
- Mesures effectuées en triplicats pour chaque surcharge ;
- Méthodologie optimisée AODs.
- Données de validation

- 72 cinétiques mesurées sur le STA-R® AUT00460 sur un plasma surchargé par 0, 16, 21, 30, 39, 51, 76, 100, 128, 142, 155, 187, 215, 245, 269, 284, 322, 347, 359, 380, 393, 410, 426 et 436 ng/ml d'edoxaban (Lixiana®/Savaysa®) ;
- Mesures effectuées en triplicats pour chaque surcharge ;
- Méthodologie optimisée AODs.

Modèle d'apprentissage automatique

Un perceptron multi-couches (réseau de neurones) a été entraîné avec le jeu de données décrit précédemment. Ses hyper-paramètres sont :

- 42 neurones dans la couche d'entrée ;
- 29 neurones dans la première couche cachée ;
- 16 neurones dans la seconde couche cachée ;
- 1 neurone dans la couche de sortie ;
- Fonction d'activation pour chaque neurone des différentes couches cachées : unité de rectification linéaire (Rectified Linear Unit ou ReLU).

2.3 Résultats

Dans cette section, sont listés les résultats des performances obtenues par les différents modèles d'apprentissage automatique de la cascade particulière de la figure 10 sur des données mesurées sur des échantillons vrais en utilisant la méthodologie expérimentale décrite dans la section 2.2.1. Chaque sous-section détaille les données mesurées ainsi que les performances obtenues. Une mesure en simplicat suffit pour rendre un résultat ; toutefois, comme démontré dans cette section, une mesure en triplicat peut améliorer les performances.

2.3.1 Détection de la présence ou de l'absence d'un anticoagulant anti-Xa

Dans cette section, sont donnés les résultats obtenus pour la détection de la présence ou de l'absence d'un anticoagulant anti-Xa en utilisant l'invention.

Données de test

- Analyse en simplicat : 298 cinétiques mesurées sur 298 échantillons vrais (39 plasmas normaux, 259 plasmas de patients sous anticoagulants anti-Xa) ;
- Analyse en triplicat : 894 cinétiques mesurées sur 298 échantillons vrais (39 plasmas normaux, 259 plasmas de patients sous anticoagulants anti-Xa) ;
- L'instrument est le STA-R® AUT06399 ;
- Méthodologie universelle.

[Table 1]

Table 1: Présence ou absence d'un anticoagulant anti-Xa : matrice de confusion. Analyse en simplicat.

| | | Résultat prédit | |
| --- | --- | --- | --- |
| | | Présence d'un anti-Xa | Absence d'anti-Xa |
| **Valeur réelle** | Présence d'un anti-Xa | 257 | 2 |
| | Absence d'anti-Xa | 1 | 38 |

Les résultats donnent une précision de 99,61 % pour la détection de la présence d'un anticoagulant anti-Xa. Les résultats donnent une précision de 97,44 % pour la détection de l'absence d'un anticoagulant anti-Xa.

[Table 2]

Table 2: Présence ou absence d'un anticoagulant anti-Xa : matrice de confusion. Analyse en triplicat.

|  |  | **Résultat prédit** | |
|---|---|---|---|
|  |  | Présence d'un anti-Xa | Absence d'anti-Xa |
| **Valeur réelle** | Présence d'un anti-Xa | 257 | 2 |
|  | Absence d'anti-Xa | 1 | 38 |

Les résultats donnent une précision de 99,61 % pour la détection de la présence d'un anticoagulant anti-Xa. Les résultats donnent une précision de 97,44 % pour la détection de l'absence d'un anticoagulant anti-Xa.

Résultats

Les tableaux 1 et 2 donnent respectivement les matrices de confusion associées à la détection de la présence ou de l'absence d'un anticoagulant anti-Xa lorsque l'analyse est réalisée en simplicat et lorsque l'analyse est réalisée en triplicat sur les données du jeu de test. Les résultats pour la détection de la présence d'un anticoagulant anti-Xa donnent une précision de 99,61 % lorsque l'analyse est effectuée en simplicat et une précision de 99,61 % quand l'analyse est effectuée en triplicat. Les résultats pour la détection de l'absence d'un anticoagulant anti-Xa donnent une précision de 97,44 % lorsque l'analyse est effectuée en simplicat et une précision de 97,44 % quand l'analyse est effectuée en triplicat.

2.3.2 Identification de la catégorie de l'anticoagulant anti-Xa

Dans cette section, sont donnés les résultats obtenus pour l'identification de la catégorie de l'anticoagulant anti-Xa en utilisant le mode particulier de réalisation de l'invention ici décrit.

Dans cette section, sont donnés les résultats obtenus pour l'identification de la catégorie de l'anticoagulant anti-Xa en utilisant le mode particulier de réalisation de l'invention ici décrit.

Données de test

- Analyse en simplicat : 259 cinétiques mesurées sur 259 échantillons vrais (91 plasmas de patients sous héparines, 168 plasmas de patients sous AODs anti-Xa) ;
- Analyse en triplicat : 777 cinétiques mesurées sur 259 échantillons vrais (91 plasmas de patients sous héparines, 168 plasmas de patients sous AODs anti-Xa) ;
- L'instrument est le STA-R® AUT06399 ;
- Méthodologie universelle.

[Table 3]

Table 3: Identification de la catégorie de l'anticoagulant anti-Xa : matrice de confusion. Analyse en simplicat. Les résultats donnent une précision de 98,07 % pour l'identification de la catégorie de l'anticoagulant anti-Xa.

|  |  | **Résultat prédit** | |
|---|---|---|---|
|  |  | Héparine | AOD |
| **Valeur réelle** | Héparine | 86 | 5 |
|  | AOD | 0 | 168 |

[Table 4]

Table 4: Identification de la catégorie de l'anticoagulant anti-Xa : matrice de confusion. Analyse en triplicat. Les résultats donnent une précision de 97,68 % pour l'identification de la catégorie de l'anticoagulant anti-Xa.

| | | **Résultat prédit** | |
| --- | --- | --- | --- |
| | | Héparine | AOD |
| **Valeur réelle** | Héparine | 86 | 5 |
| | AOD | 1 | 167 |

Résultats

Les tableaux 3 et 4 donnent respectivement les matrices de confusion associées à l'identification de la catégorie de l'anticoagulant anti-Xa lorsque l'analyse est réalisée en simplicat et lorsque l'analyse est réalisée en triplicat sur les données du jeu de test. Les résultats pour l'identification de la catégorie de l'anticoagulant anti-Xa donnent une précision de 98,07 % lorsque l'analyse est effectuée en simplicat et une précision de 97,68 % quand l'analyse est effectuée en triplicat.

2.3.3 Identification des héparines

Dans cette section, sont donnés les résultats obtenus pour l'identification des héparines en utilisant le mode particulier de réalisation de l'invention ici décrit.

Données de test

- Analyse en simplicat : 91 cinétiques mesurées sur 91 échantillons vrais (29 échantillons vrais de patients sous HNF, 62 échantillons vrais de patients sous HBPM) ;
- Analyse en triplicat : 273 cinétiques mesurées sur 91 échantillons vrais (29 échantillons vrais de patients sous HNF, 62 échantillons vrais de patients sous HBPM) ;
- L'instrument est le STA-R® AUT06399 ;
- Méthodologie universelle.

[Table 5]

Table 5: Identification des héparines : matrice de confusion. Analyse en simplicat. Les résultats donnent une précision de 92,31 % pour l'identification des héparines.

| | | **Résultat prédit** | |
| --- | --- | --- | --- |
| | | HNF | HBPM |
| **Valeur réelle** | HNF | 25 | 4 |
| | HBPM | 3 | 59 |

[Table 6]

Table 6: Identification des héparines : matrice de confusion. Analyse en triplicat. Les résultats donnent une précision de 93,41 % pour l'identification des héparines.

| | | **Résultat prédit** | |
| --- | --- | --- | --- |
| | | HNF | HBPM |
| **Valeur réelle** | HNF | 26 | 3 |
| | HBPM | 3 | 59 |

Résultats

Les tableaux 5 et 6 donnent respectivement les matrices de confusion associées à l'identification des héparines lorsque l'analyse est réalisée en simplicat et lorsque l'analyse est réalisée en triplicat sur les données du jeu de test. Les résultats pour l'identification des héparines donnent une précision de 92,31 % lorsque l'analyse est effectuée en simplicat et une précision de 93,41 % quand l'analyse est effectuée en triplicat.

2.3.4 Dosages des héparines non fractionnées

Dans cette section, sont donnés les résultats des dosages des taux en HNF sur des échantillons patients obtenus en utilisant le mode particulier de réalisation de l'invention ici décrit.en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination R2 est supérieur ou égal à 0,95 (critères du CLSI EP9-A2).

Données de test

- Analyse en simplicat : 24 cinétiques mesurées sur 24 échantillons vrais de patients sous HNF ;
- Analyse en triplicat : 72 cinétiques mesurées sur 24 échantillons vrais de patients sous HNF ;
- L'instrument est le STA-R® AUT06399 ;
- Méthodologie universelle.

Résultats

La figure 11 donne les résultats de comparaison des dosages des taux en HNF mesurés en utilisant l'approche décrite ici aux taux en HNF mesurés en utilisant l'approche standard sur les données du jeu de test. Ces comparaisons donnent une droite d'équation y=1.018x-0.0005 et un cœfficient de détermination R2=0.9844 lorsque l'analyse est réalisée en simplicat ; elles donnent une droite d'équation y=1.002x+0.002 et un cœfficient de détermination R2=0.9925 lorsque l'analyse est réalisée en triplicat.

2.3.5 Dosages des héparines de bas poids moléculaires

Dans cette section, sont donnés les résultats des dosages des taux en HBPM sur des échantillons patients obtenus en utilisant le mode particulier de réalisation de l'invention ici décrit en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination R2 est supérieur ou égal à 0,95 (critères du CLSI EP9-A2).

Données de test

- Analyse en simplicat : 62 cinétiques mesurées sur 62 échantillons vrais de patients sous HBPM ;
- Analyse en triplicat : 186 cinétiques mesurées sur 62 échantillons vrais de patients sous HBPM ;
- L'instrument est le STA-R® AUT06399 ;
- Méthodologie universelle.

Résultats

La figure 12 donne les résultats de comparaison des dosages des taux en HBPM mesurés en utilisant l'approche décrite ici aux taux en HBPM mesurés en utilisant l'approche standard sur les données du jeu de test. Ces comparaisons donnent une droite d'équation y=0.995x+0.006 et un cœfficient de détermination R2=0.9962 lorsque l'analyse est réalisée en simplicat ; elles donnent une droite d'équation y=0.9977x+0.004 et un cœfficient de détermination R2=0.997 lorsque l'analyse est réalisée en triplicat.

2.3.6 Identification de l'AOD anti-Xa

Dans cette section, sont donnés les résultats obtenus pour l'identification des AODs anti-Xa en utilisant le mode particulier de réalisation de l'invention ici décrit.

Données de test

- Analyse en simplicat : 168 cinétiques mesurées sur 168 échantillons vrais (65 échantillons vrais de patients sous rivaroxaban, 45 échantillons vrais de patients sous apixaban, 58 échantillons vrais de patients sous edoxaban) ;
- Analyse en triplicat : 504 cinétiques mesurées sur 168 échantillons vrais (65 échantillons vrais de patients sous rivaroxaban, 45 échantillons vrais de patients sous apixaban, 58 échantillons vrais de patients sous edoxaban) ;
- L'instrument est le STA-R® AUT06399 ;
- Méthodologie optimisée AODs.

Résultats

Les tableaux 7 et 8 donnent respectivement les matrices de confusion associées à l'identification des AODs anti-Xa lorsque l'analyse est réalisée en simplicat et lorsque l'analyse est réalisée en triplicat sur les données du jeu de test. Les résultats pour l'identification des AODs anti-Xa donnent une précision de 91,67 % lorsque l'analyse est effectuée en simplicat et une précision de 96,43 % quand l'analyse est effectuée en triplicat.

[Table 7]

Table 7: Identification des AODs anti-Xa : matrice de confusion. Analyse en simplicat. Les résultats donnent une précision de 91,67 % pour l'identification des AODs anti-Xa.

| | | Résultat prédit | | |
|---|---|---|---|---|
| | | Rivaroxaban | Apixaban | edoxaban |
| **Valeur réelle** | Rivaroxaban | 61 | 1 | 3 |
| | Apixaban | 0 | 45 | 0 |
| | edoxaban | 10 | 0 | 48 |

[Table 8]

Table 8: Identification des AODs anti-Xa : matrice de confusion. Analyse en triplicat. Les résultats donnent une précision de 96,43 % pour l'identification des AODs anti-Xa.

| | | Résultat prédit | | | |
|---|---|---|---|---|---|
| | | -1 | Rivaroxaban | Apixaban | edoxaban |
| **Valeur réelle** | Rivaroxaban | 1 | 64 | 0 | 0 |
| | Apixaban | 0 | 0 | 45 | 0 |
| | edoxaban | 0 | 5 | 0 | 53 |

2.3.7 Dosage du rivaroxaban

Dans cette section, sont donnés les résultats des dosages des taux en rivaroxaban sur des échantillons patients obtenus en utilisant le mode particulier de réalisation de l'invention ici décrit.en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R2$ est supérieur ou égal à 0,95 (critères du CLSI EP9-A2).

Données de test

- Analyse en simplicat : 62 cinétiques mesurées sur 62 échantillons vrais de patients sous rivaroxaban;
- Analyse en triplicat : 186 cinétiques mesurées sur 62 échantillons vrais de patients sous rivaroxaban;
- L'instrument est le STA-R® AUT06399 ;
- Méthodologie optimisée AODs.

Résultats

La figure 13 donne les résultats de comparaison des dosages des taux en rivaroxaban mesurés en utilisant l'approche du mode particulier de réalisation de l'invention ici décrit aux taux en rivaroxaban mesurés en utilisant l'approche standard sur les données du jeu de test. Ces comparaisons donnent une droite d'équation y=1.05x+19.65 et un cœfficient de détermination R2=0.991 lorsque l'analyse est réalisée en simplicat ; elles donnent une droite d'équation y=1.04x+20.3 et un cœfficient de détermination R2=0.9934 lorsque l'analyse est réalisée en triplicat.

2.3.8 Dosage de l'apixaban

Dans cette section, sont donnés les résultats des dosages des taux en apixaban sur des échantillons patients obtenus en utilisant le mode particulier de réalisation de l'invention ici décrit.en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

Données de test

- Analyse en simplicat : 45 cinétiques mesurées sur 45 échantillons vrais de patients sous apixaban;
- Analyse en triplicat : 135 cinétiques mesurées sur 45 échantillons vrais de patients sous apixaban ;
- L'instrument est le STA-R® AUT06399 ;
- Méthodologie optimisée AODs.

Résultats

La figure 14 donne les résultats de comparaison des dosages des taux en apixaban mesurés en utilisant l'approche du mode particulier de réalisation de l'invention ici décrit aux taux en apixaban mesurés en utilisant l'approche standard sur les données du jeu de test. Ces comparaisons donnent une droite d'équation y=1.14x-6.73 et un cœfficient de détermination $R^2$=0.9945 lorsque l'analyse est réalisée en simplicat ; elles donnent une droite d'équation y=1.13x-5.46 et un cœfficient de détermination $R^2$=0.9958 lorsque l'analyse est réalisée en triplicat.

2.3.9 Dosage de l'edoxaban

Dans cette section, sont donnés les résultats des dosages des taux en edoxaban sur des échantillons patients obtenus en utilisant le mode particulier de réalisation de l'invention ici décrit.en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

Données de test

- Analyse en simplicat : 56 cinétiques mesurées sur 56 échantillons vrais de patients sous edoxaban;
- Analyse en triplicat : 168 cinétiques mesurées sur 56 échantillons vrais de patients sous edoxaban;
- L'instrument est le STA-R® AUT06399 ;
- Méthodologie optimisée AODs.

Résultats

La figure 15 donne les résultats de comparaison des dosages des taux en edoxaban mesurés en utilisant l'approche du mode particulier de réalisation de l'invention ici décrit aux taux en edoxaban mesurés en utilisant l'approche standard sur les données du jeu de test. Ces comparaisons donnent une droite d'équation y=0.905x+12.35 et un cœfficient de détermination $R^2$=0.9853 lorsque l'analyse est réalisée en simplicat ; elles donnent une droite d'équation y=0.93x+8.18 et un cœfficient de détermination $R^2$=0.9881 lorsque l'analyse est réalisée en triplicat.

## 3. Seconde étude de post-traitement de la détection, de l'identification et de la quantification des inhibiteurs synthétiques du facteur Xa

La figure **Erreur ! Source du renvoi introuvable.**6 détaille la cascade (suivie dans le cadre de cette seconde étude) de modèles d'apprentissage automatique qui analyse et interprète la cinétique obtenue par la mesure expérimentale dans le but de rendre le résultat biologique attendu.

La cascade est composée de douze modèles d'apprentissage automatique ; ces douze modèles prennent en entrée la cinétique obtenue par la mesure expérimentale et donnent en sortie un résultat :

- Le premier modèle est un modèle de classification : selon l'allure de la cinétique qui lui est donnée en entrée, il détermine si oui ou non l'échantillon analysé contient un inhibiteur synthétique du facteur Xa ;
- Le second modèle est aussi un modèle de classification : sachant que l'échantillon analysé contient un inhibiteur synthétique du facteur Xa et selon l'allure de la cinétique qui lui est présentée en entrée, il reconnait la catégorie de l'inhibiteur synthétique anti-Xa : héparine ou AOD ;
- Si l'inhibiteur est une héparine, et selon l'allure de la cinétique qui lui est donnée en entrée, un modèle de classification identifie s'il s'agit d'une HNF ou d'une HBPM ;

  - Si l'inhibiteur est une HNF, et selon l'allure de la cinétique qui lui est donnée en entrée, un modèle de régression calcule la concentration en HNF ;
  - Si l'inhibiteur est une HBPM, et selon l'allure de la cinétique qui lui est donnée en entrée, un modèle de régression calcule la concentration en HBPM ;

- Si l'inhibiteur est un AOD, une mesure expérimentale est relancée en utilisant la méthodologie optimisée AODs et une nouvelle cinétique est enregistrée puis présentée en entrée d'un modèle de classification qui identifie si l'inhibiteur est du rivaroxaban, de l'apixaban ou de l'edoxaban :

  - Si l'inhibiteur est du rivaroxaban, et selon l'allure de la cinétique (mesurée par la méthodologie optimisée AODs) qui lui est présentée en entrée, un modèle de régression calcule la concentration en rivaroxaban. Si cette concentration est inférieure à 200 ng/mL, un second modèle de régression recalcule la concentration en rivaroxaban en utilisant cette fois-ci en entrée la cinétique mesurée par la méthodologie universelle : cette dernière permet un rendu de résultat plus précis pour les faibles concentrations en rivaroxaban. Sinon le résultat est rendu directement. (ce mode de réalisation correspond à la méthodologie décrite comme étant la « méthodologie améliorée sur la base de la méthodologie universelle » dans la présente description).
  - Si l'inhibiteur est de l'apixaban, et selon l'allure de la cinétique (mesurée par la méthodologie optimisée AODs) qui lui est présentée en entrée, un modèle de régression calcule la concentration en apixaban. Si cette concentration est inférieure à 200 ng/mL, un second modèle de régression recalcule la concentration en apixaban en utilisant cette fois-ci en entrée la cinétique mesurée par la méthodologie universelle : cette dernière permet un rendu de résultat plus précis pour les faibles concentrations en apixaban. Sinon le résultat est rendu directement. (ce mode de réalisation correspond à la méthodologie décrite comme étant la « méthodologie améliorée sur la base de la méthodologie universelle » dans la présente description).
  - Si l'inhibiteur est de l'edoxaban, et selon l'allure de la cinétique (mesurée par la méthodologie optimisée AODs) qui lui est présentée en entrée, un modèle de régression calcule la concentration en edoxaban. Si cette concentration est inférieure à 200 ng/mL, un second modèle de régression recalcule la concentration en edoxaban en utilisant cette fois-ci en entrée la cinétique mesurée par la méthodologie universelle : cette dernière permet un rendu de résultat plus précis pour les faibles concentrations en edoxaban. Sinon le résultat est rendu directement. (ce mode de réalisation correspond à la méthodologie décrite comme étant la « méthodologie améliorée sur la base de la méthodologie universelle » dans la présente description).

Les sections suivantes listent respectivement pour chacun de ces modèles d'apprentissage automatique, les jeux de données d'apprentissage et de validation qui ont servi à leur entraînement, ainsi que les algorithmes associés.

Notons que pour chaque jeu de données, les concentrations en inhibiteurs synthétiques du facteur Xa (HNF, HBPM, rivaroxaban, apixaban et edoxaban) ont été mesurées sur automate STA-R® en utilisant le kit commercial STA® - Liquid Anti-Xa, ainsi que les calibrants et méthodologies commerciales associées.

### 3.1 Détection de la présence ou de l'absence d'un anticoagulant anti-Xa

### 3.1.1 Jeux de données

Les échantillons sans anticoagulant ont été fabriqués à l'occasion de différents essais. Il s'agit d'une matrice plasmatique diluée dans les mêmes proportions que les échantillons spikés le jour du test :

- 3 cinétiques mesurées le 24.10.2017 sur le STA-R® AUT00603 (soft version 3.04.07) + 3 cinétiques mesurées le 24.10.2017 sur le STA-R® AUT06366 (soft version 3.04.07).

  - un échantillon pool normal de plasmas lot 03.2017 surchargé par 0.00 UI/mL d'HNF sodique (Héparine Choay®), préparé extemporanément et testés simultanément sur les deux automates.
  - STA® - Liquid Anti-Xa lot 251187.

- 3 cinétiques mesurées le 18.07.2017 sur le STA-R® AUT06399 (soft version 3.04.07)

  - un échantillon pool normal de plasmas lot 03.2017 surchargé avec un taux théorique de 0.00 UI/mL d'HNF sodique (Héparine Choay®), préparé extemporanément.
  - STA® - Liquid Anti-Xa lot 251187.

- 3 cinétiques mesurées le 23.10.2019 sur le STA-R® AUT05016 (soft version 3.04.07) + 3 cinétiques mesurées le 23.10.2019 sur le STA-R® AUT05450 (soft version 3.04.07)

  - un échantillon pool normal de plasmas lot 03.2017 surchargé par 0.00 UI/mL d'HNF calcique (Calciparine®), préparé extemporanément et testés simultanément sur les deux automates.
  - STA® - Liquid Anti-Xa lot 251187.

- 3 cinétiques mesurées le 17.07.2017 sur le STA-R® AUT06399 (soft version 3.04.07)

  - un échantillon pool normal de plasmas lot 03.2017 surchargé avec un taux théorique de 0.00 UI/mL d'HNF calcique (Calciparine®), préparé extemporanément.
  - STA® - Liquid Anti-Xa lot 251187.

- 3 cinétiques mesurées le 19.10.2017 sur le STA-R® AUT06366 (soft version 3.04.07) + 3 cinétiques mesurées le 19.10.2017 sur le STA-R® AUT05016 (soft version 3.04.07)

  - un échantillon pool normal de plasmas lot 03.2017 surchargé par 0.00 UI/mL d'HBPM daltéparine sodique (Fragmine®), préparé extemporanément et testés simultanément sur les deux automates.
  - STA® - Liquid Anti-Xa lot 251187.

- 3 cinétiques mesurées le 17.07.2017 sur le STA-R® AUT06399 (soft version 3.04.07)

  - un échantillon pool normal de plasmas lot 03.2017 surchargé avec un taux théorique de 0.00 UI/mL d'HBPM daltéparine sodique (Fragmine®), préparé extemporanément.
  - STA® - Liquid Anti-Xa lot 251187.

- 3 cinétiques mesurées le 26.10.2017 sur le STA-R® AUT06366 (soft version 3.04.07) + 3 cinétiques mesurées le 26.10.2017 sur le STA-R® AUT00603 (soft version 3.04.07)

  - un échantillon pool normal de plasmas lot 03.2017 surchargé par 0.00 UI/mL d'HBPM énoxaparine sodique (Lovenox®), préparé extemporanément et testés simultanément sur les deux automates.
  - STA® - Liquid Anti-Xa lot 251187.

- 3 cinétiques mesurées le 19.07.2017 sur le STA-R® AUT06399 (soft version 3.04.07)

- un échantillon pool normal de plasmas lot 03.2017 surchargé avec un taux théorique de 0.00 UI/mL d'HBPM énoxaparine sodique (Lovenox®), préparé extemporanément.
- STA® - Liquid Anti-Xa lot 251187.

- 3 cinétiques mesurées le 25.10.2017 sur le STA-R® AUT06366 (soft version 3.04.07) + 3 cinétiques mesurées le 25.10.2017 sur le STA-R® AUT00603 (soft version 3.04.07)

- un échantillon pool normal de plasmas lot 03.2017 surchargé par 0.00 UI/mL d'HBPM tinzaparine sodique (InnoHep®), préparé extemporanément et testés simultanément sur les deux automates.
- STA® - Liquid Anti-Xa lot 251187.

- 3 cinétiques mesurées le 18.07.2017 sur le STA-R® AUT06399 (soft version 3.04.07)

- un échantillon pool normal de plasmas lot 03.2017 surchargé avec un taux théorique de 0.00 UI/mL d'HBPM tinzaparine sodique (InnoHep®), préparé extemporanément.
- STA® - Liquid Anti-Xa lot 251187.

- 3 cinétiques mesurées le 18.07.2017 sur le STA-R® AUT06399 (soft version 3.04.07)

- un échantillon pool normal de plasmas lot 03.2017 surchargé avec un taux théorique de 0.00 UI/mL de Fondaparinux (Arixtra®), préparé extemporanément.
- STA® - Liquid Anti-Xa lot 251187.

- 3 cinétiques mesurées le 10.10.2017 sur le STA-R® AUT00603 (soft version 3.04.07) + 3 cinétiques mesurées le 17.10.2017 sur le STA-R® AUT05450 (soft version 3.04.07) + 3 cinétiques mesurées le 17.10.2017 sur le STA-R® AUT06366 (soft version 3.04.07) + 3 cinétiques mesurées le 10.10.2017 sur le STA-R® AUT06399 (soft version 3.04.07)

- un échantillon pool normal de plasmas lot 03.2017 surchargé par 0 ng/mL de Xarelto® (rivaroxaban), préparé et stocké à -80°C.
- STA® - Liquid Anti-Xa lot 251738.

- 3 cinétiques mesurées le 05.02.2018 sur le STA-R AUT06399 (soft version 3.04.07)

- un échantillon pool normal de plasmas lot 03.2017 surchargé par 0 ng/mL de Xarelto® (rivaroxaban), préparé et stocké à -80°C.
- STA® - Liquid Anti-Xa lot 251187.

- 3 cinétiques mesurées le 17.10.2017 sur le STA-R® AUT00603 (soft version 3.04.07) + 3 cinétiques mesurées le 17.10.2017 sur le STA-R® AUT05450 (soft version 3.04.07) + 3 cinétiques mesurées le 21.09.2017 sur le STA-R® AUT06366 (soft version 3.04.07) + 3 cinétiques mesurées le 20.09.2017 sur le STA-R® AUT06399 (soft version 3.04.07)

- un échantillon pool normal de plasmas lot 03.2017 surchargé par 0 ng/mL de Lixiana® (edoxaban), préparé et stocké à -80°C.
- STA® - Liquid Anti-Xa lot 251738.

- 3 cinétiques mesurées le 05.02.2018 sur le STA-R AUT06399 (soft version 3.04.07)

- un échantillon pool normal de plasmas lot 03.2017 surchargé par 0 ng/mL de Lixiana® (edoxaban), préparé et stocké à -80°C.
- STA® - Liquid Anti-Xa lot 251187.

- 3 cinétiques mesurées le 16.10.2017 sur le STA-R® AUT00603 (soft version 3.04.07) + 3 cinétiques mesurées le 05.10.2017 sur le STA-R® AUT06360 (soft version 3.04.07) + 3 cinétiques mesurées le 16.10.2017 sur le STA-R® AUT06366 (soft version 3.04.07) + 3 cinétiques mesurées le 05.10.2017 sur le STA-R® AUT06399 (soft version 3.04.07)

- un échantillon pool normal de plasmas lot 03.2017 surchargé par 0 ng/mL d'Eliquis® (apixaban), préparé et stocké à -80°C.
- STA® - Liquid Anti-Xa lot 251738.

- 3 cinétiques mesurées le 05.02.2018 sur le STA-R AUT06399 (soft version 3.04.07)

 - un échantillon pool normal de plasmas lot 03.2017 surchargé par 0 ng/mL d'Eliquis® (apixaban), préparé et stocké à -80°C.
 - STA® - Liquid Anti-Xa lot 251187.

Concernant l'HNF sodique (Héparine Choay®), les données générées sont :

- 60 cinétiques mesurées le 24.10.2017 sur le STA-R® AUT00603 (soft version 3.04.07)

 - pool normal de plasmas lot 03.2017 surchargé par 0.12, 0.23, 0.32, 0.46, 0.54, 0.66, 0.66, 0.78, 0.86, 1.00, 1.18, 1.20, 1.36, 1.47, 1.56, 1.64, 1.74, 1.80, 1.88 et 2.03 UI/mL ;
 - chaque niveau d'échantillon est testé en *n=3* ;
 - les échantillons ont été préparés extemporanément et testés simultanément sur les deux automates.

- 60 cinétiques mesurées le 24.10.2017 sur le STA-R® AUT06366 (soft version 3.04.07)

 - pool normal de plasmas lot 03.2017 surchargé par 0.11, 0.21, 0.32, 0.43, 0.55, 0.63, 0.64, 0.76, 0.87, 0.97, 1.14, 1.17, 1.30, 1.43, 1.52, 1.61, 1.70, 1.84, 1.84 et 1.99 UI/mL ;
 - chaque niveau d'échantillon est testé en *n=3* ;
 - les échantillons ont été préparés extemporanément et testés simultanément sur les deux automates.

Concernant l'HNF calcique (Calciparine®), les données générées sont :

- 60 cinétiques mesurées le 23.10.2019 sur le STA-R® AUT05016 (soft version 3.04.07)

 - pool normal de plasmas lot 03.2017 surchargé par 0.10, 0.21, 0.32, 0.44, 0.56, 0.66, 0.77, 0.84, 0.86, 0.98, 1.15, 1.28, 1.34, 1.43, 1.56, 1.60, 1.75, 1.85, 1.92 et 1.96 UI/mL ;
 - chaque niveau d'échantillons est testé en *n=3* ;
 - les échantillons ont été préparés extemporanément et testés simultanément sur les deux automates.

- 60 cinétiques mesurées le 23.10.2019 sur le STA-R® AUT05450 (soft version 3.04.07)

 - pool normal de plasmas lot 03.2017 surchargé par 0.12, 0.23, 0.33, 0.43, 0.55, 0.66, 0.75, 0.90, 0.88, 1.00, 1.13, 1.24, 1.32, 1.44, 1.53, 1.63, 1.70, 1.88, 1.96 et 2.01 UI/mL ;
 - chaque niveau d'échantillon est testé en *n=3* ;
 - les échantillons ont été préparés extemporanément et testés simultanément sur les deux automates.

Concernant l'HBPM daltéparine sodique (Fragmine®), les données générées sont :

- 60 cinétiques mesurées le 19.10.2017 sur le STA-R® AUT06366 (soft version 3.04.07)

 - pool normal de plasmas lot 03.2017 surchargé par 0.15, 0.25, 0.35, 0.44, 0.54, 0.64, 0.74, 0.85, 0.94, 1.07, 1.14, 1.24, 1.27, 1.44, 1.50, 1.55, 1.66, 1.75, 1.79, 2.26 UI Anti-Xa/mL ;
 - chaque niveau d'échantillon est testé en *n*=3 ;
 - les échantillons ont été préparés extemporanément et testés simultanément sur les deux automates.

- 60 cinétiques mesurées le 19.10.2017 sur le STA-R® AUT05016 (soft version 3.04.07)

 - pool normal de plasmas lot 03.2017 surchargé par 0.15, 0.25, 0.35, 0.44, 0.54, 0.64, 0.74, 0.85, 0.94, 1.07, 1.14, 1.24, 1.27, 1.44, 1.50, 1.55, 1.66, 1.75, 1.79, 2.26 UI Anti-Xa/mL ;
 - chaque niveau d'échantillon est testé en *n*=3 ;
 - les échantillons ont été préparés extemporanément et testés simultanément sur les deux automates.

Concernant l'HBPM énoxaparine sodique (Lovenox®), les données générées sont :

- 60 cinétiques mesurées le 26.10.2017 sur le STA-R® AUT06366 (soft version 3.04.07)

  - pool normal de plasmas lot 03.2017 surchargé par 0.10, 0.20, 0.31, 0.40, 0.52, 0.63, 0.71, 0.83, 0.93, 1.02, 1.09, 1.21, 1.28, 1.36, 1.49, 1.58, 1.65, 1.81, 1.85, 1.97 UI Anti-Xa/mL ;
  - chaque niveau d'échantillon est testé en *n*=3 ;
  - les échantillons ont été préparés extemporanément et testés simultanément sur les deux automates.

- 60 cinétiques mesurées le 26.10.2017 sur le STA-R® AUT00603 (soft version 3.04.07)

  - pool normal de plasmas lot 03.2017 surchargé par 0.10, 0.20, 0.31, 0.40, 0.52, 0.63, 0.71, 0.83, 0.93, 1.02, 1.09, 1.21, 1.28, 1.36, 1.49, 1.58, 1.65, 1.81, 1.85, 1.97 UI Anti-Xa/mL ;
  - chaque niveau d'échantillon est testé en *n*=3 ;
  - les échantillons ont été préparés extemporanément et testés simultanément sur les deux automates.

Concernant l'HBPM tinzaparine sodique (InnoHep®), les données générées sont :

- 60 cinétiques mesurées le 25.10.2017 sur le STA-R® AUT06366 (soft version 3.04.07)

  - pool normal de plasmas lot 03.2017 surchargé par 0.11, 0.20, 0.30, 0.40, 0.46, 0.54, 0.63, 0.80, 0.89, 0.96, 1.06, 1.21, 1.27, 1.37, 1.46, 1.52, 1.64, 1.81, 1.83, 1.97 UI Anti-Xa/mL ;
  - chaque niveau d'échantillon est testé en *n*=3 ;
  - les échantillons ont été préparés extemporanément et testés simultanément sur les deux automates.

- 60 cinétiques mesurées le 25.10.2017 sur le STA-R® AUT00603 (soft version 3.04.07)

  - pool normal de plasmas lot 03.2017 surchargé par 0.11, 0.20, 0.30, 0.40, 0.46, 0.54, 0.63, 0.80, 0.89, 0.96, 1.06, 1.21, 1.27, 1.37, 1.46, 1.52, 1.64, 1.81, 1.83, 1.97 UI Anti-Xa/mL ;
  - chaque niveau d'échantillon est testé en *n*=3 ;
  - les échantillons ont été préparés extemporanément et testés simultanément sur les deux automates.

Numéro de lot du STA® - Liquid Anti-Xa : 251187, lot utilisé pour la génération des cinétiques en méthodologie universelle et pour le dosage commercial de la surcharge d'héparine.

Concernant le Xarelto® (rivaroxaban), les données générées sont :

- 60 cinétiques mesurées le 10.10.2017 sur le STA-R® AUT00603 (soft version 3.04.07)

  - pool normal de plasmas lot 03.2017 surchargé par 10, 20, 35, 41, 49, 56, 66, 74, 84, 93, 105, 110, 116, 134, 143, 155, 160, 175, 181 et 198 ng/mL ;
  - chaque niveau d'échantillon est testé en *n*=3 ;
  - les échantillons ont été préparés et stockés à -80°C.

- 60 cinétiques mesurées le 17.10.2017 sur le STA-R® AUT05450 (soft version 3.04.07)

  - pool normal de plasmas lot 03.2017 surchargé par 10, 20, 35, 41, 48, 59, 65, 77, 85, 95, 103, 113, 121, 134, 145, 160, 161, 172, 169, 202 ng/mL ;
  - chaque niveau d'échantillon est testé en *n*=3 ;
  - les échantillons ont été préparés et stockés à -80°C.

- 60 cinétiques mesurées le 17.10.2017 sur le STA-R® AUT06366 (soft version 3.04.07)

  - pool normal de plasmas lot 03.2017 surchargé par 10, 20, 25, 34, 43, 50, 62, 74, 84, 93, 104, 109, 118, 133, 143, 159, 158, 176, 173 et 203 ng/mL ;
  - chaque niveau d'échantillon est testé en *n*=3 ;
  - les échantillons ont été préparés et stockés à -80°C.

- 60 cinétiques mesurées le 10.10.2017 sur le STA-R® AUT06399 (soft version 3.04.07)

- pool normal de plasmas lot 03.2017 surchargé par 10, 20, 30, 38, 46, 57, 64, 72, 82, 90, 101, 110, 115, 130, 140, 148, 152, 166, 164 et 192 ng/mL ;
- chaque niveau d'échantillon est testé en *n*=3 ;
- les échantillons ont été préparés et stockés à -80°C.

Concernant l'Eliquis® (apixaban), les données générées sont :

- 60 cinétiques mesurées le 16.10.2017 sur le STA-R® AUT00603 (soft version 3.04.07)

  - pool normal de plasmas lot 03.2017 surchargé par 10, 20, 27, 39, 41, 52, 61, 67, 76, 93, 96, 105, 123, 127, 140, 145, 155, 167, 184 et 182 ng/mL ;
  - chaque niveau d'échantillon est testé en *n*=3 ;
  - les échantillons ont été préparés et stockés à -80°C.

- 60 cinétiques mesurées le 05.10.2017 sur le STA-R® AUT06360 (soft version 3.04.07)

  - pool normal de plasmas lot 03.2017 surchargé par 10, 20, 26, 36, 43, 53, 60, 70, 75, 93, 103, 109, 121, 135, 136, 153, 160, 175, 185 et 192 ng/mL ;
  - chaque niveau d'échantillon est testé en *n*=3 ;
  - les échantillons ont été préparés et stockés à -80°C.

- 60 cinétiques mesurées le 16.10.2017 sur le STA-R® AUT06366 (soft version 3.04.07)

  - pool normal de plasmas lot 03.2017 surchargé par 10, 20, 26, 34, 44, 50, 61, 70, 74, 94, 103, 109, 122, 135, 138, 147, 162, 173, 182 et 186 ng/mL ;
  - chaque niveau d'échantillon est testé en *n*=3.
  - les échantillons ont été préparés et stockés à -80°C.

- 60 cinétiques mesurées le 05.10.2017 sur le STA-R® AUT06399 (soft version 3.04.07)

  - pool normal de plasmas lot 03.2017 surchargé par 10, 20, 33, 41, 46, 57, 65, 76, 79, 96, 105, 116, 125, 135, 138, 153, 161, 169, 180 et 186 ng/mL ;
  - chaque niveau d'échantillon est testé en *n*=3.
  - les échantillons ont été préparés et stockés à -80°C.

Concernant le Lixiana® (edoxaban), les données générées sont :

- 60 cinétiques mesurées le 17.10.2017 sur le STA-R® AUT00603 (soft version 3.04.07)

  - pool normal de plasmas lot 03.2017 surchargé par 10, 20, 28, 40, 49, 64, 74, 95, 103, 109, 122, 132, 144, 131, 132, 157, 171, 195, 191 et 201 ng/mL ;
  - chaque niveau d'échantillon est testé en *n*=3 ;
  - les échantillons ont été préparés et stockés à -80°C.

- 60 cinétiques mesurées le 17.10.2017 sur le STA-R® AUT05450 (soft version 3.04.07)

  - pool normal de plasmas lot 03.2017 surchargé par 10, 21, 28, 41, 49, 62, 74, 92, 102, 112, 120, 133, 142, 129, 133, 155, 168, 188, 200 et 206 ng/mL ;
  - chaque niveau d'échantillon est testé en *n*=3 ;
  - les échantillons ont été préparés et stockés à -80°C.

- 60 cinétiques mesurées le 21.09.2017 sur le STA-R® AUT06366 (soft version 3.04.07)

  - pool normal de plasmas lot 03.2017 surchargé par 10, 21, 27, 40, 49, 64, 73, 90, 99, 108, 122, 132, 144, 130, 136, 153, 169, 192, 194 et 216 ng/mL ;
  - chaque niveau d'échantillon est testé en *n*=3 ;
  - les échantillons ont été préparés et stockés à -80°C.

- 60 cinétiques mesurées le 20.09.2017 sur le STA-R® AUT06399 (soft version 3.04.07)

  - pool normal de plasmas lot 03.2017 surchargé par 10, 21, 29, 39, 48, 60, 73, 81, 92, 107, 115, 129, 136 ,129, 127, 149, 163, 181, 195 et 199 ng/mL ;
  - chaque niveau d'échantillon est testé en $n$=3 ;
  - les échantillons ont été préparés et stockés à -80°C.

Numéro de lot du STA® - Liquid Anti-Xa : 251738, lot utilisé pour la génération des cinétiques en méthodologie universelle et pour le dosage commercial des surcharges d'AODs.

### 3.1.2 Modèle d'apprentissage automatique

### Organisation des données

Les données relatives aux échantillons sans molécule anticoagulante ont été mixées et scindées en deux jeux de données nommés ici ABSENCE-0 et ABSENCE-1.

Le modèle d'apprentissage automatique est entraîné par une validation croisée divisée en deux sous-ensembles de la manière suivante :

- Sous-ensemble 1 :

  - Données d'apprentissage :

    - ABSENCE-0,
    - données HNF sodique (Héparine Choay®) générées sur le STA-R® AUT06366,
    - données HNF calcique (Calciparine®) générées sur le STA-R® AUT05450,
    - données HBPM daltéparine sodique (Fragmine®) générées sur le STA-R® AUT06366,
    - données HBPM énoxaparine sodique (Lovenox®) générées sur le STA-R® AUT06366,
    - données HBPM tinzaparine sodique (InnoHep®) générées sur le STA-R® AUT06366,
    - données rivaroxaban (Xarelto®) générées sur le STA-R® AUT06366,
    - données rivaroxaban (Xarelto®) générées sur le STA-R® AUT05450,
    - données edoxaban (Lixiana®) générées sur le STA-R® AUT06366,
    - données edoxaban (Lixiana®) générées sur le STA-R® AUT05450,
    - données apixaban (Eliquis®) générées sur le STA-R® AUT06366,
    - données apixaban (Eliquis®) générées sur le STA-R® AUT06360.

  - Données de validation :

    - ABSENCE-1,
    - données HNF sodique (Héparine Choay®) générées sur le STA-R® AUT00603,
    - données HNF calcique (Calciparine®) générées sur le STA-R® AUT05016,
    - données HBPM daltéparine sodique (Fragmine®) générées sur le STA-R® AUT05016,
    - données HBPM énoxaparine sodique (Lovenox®) générées sur le STA-R® AUT00603,
    - données HBPM tinzaparine sodique (InnoHep®) générées sur le STA-R® AUT00603,
    - données rivaroxaban (Xarelto®) générées sur le STA-R® AUT00603,
    - données rivaroxaban (Xarelto®) générées sur le STA-R® AUT06399,
    - données edoxaban (Lixiana®) générées sur le STA-R® AUT00603,
    - données edoxaban (Lixiana®) générées sur le STA-R® AUT06399,
    - données apixaban (Eliquis®) générées sur le STA-R® AUT00603,
    - données apixaban (Eliquis®) générées sur le STA-R® AUT06399.

- Sous-ensemble 2 :

  - Données d'apprentissage :

    - ABSENCE-1,
    - données HNF sodique (Héparine Choay®) générées sur le STA-R® AUT00603,
    - données HNF calcique (Calciparine®) générées sur le STA-R® AUT05016,

- données HBPM daltéparine sodique (Fragmine®) générées sur le STA-R® AUT05016,
- données HBPM énoxaparine sodique (Lovenox®) générées sur le STA-R® AUT00603,
- données HBPM tinzaparine sodique (InnoHep®) générées sur le STA-R® AUT00603,
- données rivaroxaban (Xarelto®) générées sur le STA-R® AUT00603,
- données rivaroxaban (Xarelto®) générées sur le STA-R® AUT06399,
- données edoxaban (Lixiana®) générées sur le STA-R® AUT00603,
- données edoxaban (Lixiana®) générées sur le STA-R® AUT06399,
- données apixaban (Eliquis®) générées sur le STA-R® AUT00603,
- données apixaban (Eliquis®) générées sur le STA-R® AUT06399.

- Données de validation :

  - ABSENCE-0,
  - données HNF sodique (Héparine Choay®) générées sur le STA-R® AUT06366,
  - données HNF calcique (Calciparine®) générées sur le STA-R® AUT05450,
  - données HBPM daltéparine sodique (Fragmine®) générées sur le STA-R® AUT06366,
  - données HBPM énoxaparine sodique (Lovenox®) générées sur le STA-R® AUT06366,
  - données HBPM tinzaparine sodique (InnoHep®) générées sur le STA-R® AUT06366,
  - données rivaroxaban (Xarelto®) générées sur le STA-R® AUT06366,
  - données rivaroxaban (Xarelto®) générées sur le STA-R® AUT05450,
  - données edoxaban (Lixiana®) générées sur le STA-R® AUT06366,
  - données edoxaban (Lixiana®) générées sur le STA-R® AUT05450,
  - données apixaban (Eliquis®) générées sur le STA-R® AUT06366,
  - données apixaban (Eliquis®) générées sur le STA-R® AUT06360.

L'apprentissage final se fait sur l'intégralité des données.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 77 neurones
    - Fonctions d'activation : Identité

  - Couche cachée

    - 40 neurones
    - Fonctions d'activation : ReLU

  - Couche de sortie

    - 2 neurones
    - Fonction d'activation : Softmax

- Initialisation de la matrice de poids et de biais : Xavier Glorot
- Méthode numérique pour l'optimisation des poids et des biais : L-BFGS
- Méthode de régularisation : L2, alpha = 0.00001
- Stratégie d'apprentissage : recherche par graines
- Fonction de coût : entropie croisée

**3.2 Identification de la catégorie de l'anticoagulant anti-Xa**

### 3.2.1 Jeux de données

Concernant l'HNF sodique (Héparine Choay®), les données générées sont :

- 60 cinétiques mesurées le 24.10.2017 sur le STA-R® AUT00603 (soft version 3.04.07)

    - pool normal de plasmas lot 03.2017 surchargé par 0.12, 0.23, 0.32, 0.46, 0.54, 0.66, 0.66, 0.78, 0.86, 1.00, 1.18, 1.20, 1.36, 1.47, 1.56, 1.64, 1.74, 1.80, 1.88 et 2.03 UI/mL ;
    - chaque niveau d'échantillon est testé en *n*=3 ;
    - les échantillons ont été préparés extemporanément et testés simultanément sur les deux automates.

- 60 cinétiques mesurées le 24.10.2017 sur le STA-R® AUT06366 (soft version 3.04.07)

    - pool normal de plasmas lot 03.2017 surchargé par 0.11, 0.21, 0.32, 0.43, 0.55, 0.63, 0.64, 0.76, 0.87, 0.97, 1.14, 1.17, 1.30, 1.43, 1.52, 1.61, 1.70, 1.84, 1.84 et 1.99 UI/mL ;
    - chaque niveau d'échantillon est testé en *n*=3 ;
    - les échantillons ont été préparés extemporanément et testés simultanément sur les deux automates.

Concernant l'HNF calcique (Calciparine®), les données générées sont :

- 60 cinétiques mesurées le 23.10.2019 sur le STA-R® AUT05016 (soft version 3.04.07)

    - pool normal de plasmas lot 03.2017 surchargé par 0.10, 0.21, 0.32, 0.44, 0.56, 0.66, 0.77, 0.84, 0.86, 0.98, 1.15, 1.28, 1.34, 1.43, 1.56, 1.60, 1.75, 1.85, 1.92 et 1.96 UI/mL ;
    - chaque niveau d'échantillons est testé en *n*=3 ;
    - les échantillons ont été préparés extemporanément et testés simultanément sur les deux automates.

- 60 cinétiques mesurées le 23.10.2019 sur le STA-R® AUT05450 (soft version 3.04.07)

    - pool normal de plasmas lot 03.2017 surchargé par 0.12, 0.23, 0.33, 0.43, 0.55, 0.66, 0.75, 0.90, 0.88, 1.00, 1.13, 1.24, 1.32, 1.44, 1.53, 1.63, 1.70, 1.88, 1.96 et 2.01 UI/mL ;
    - chaque niveau d'échantillon est testé en *n*=3 ;
    - les échantillons ont été préparés extemporanément et testés simultanément sur les deux automates.

Concernant l'HBPM daltéparine sodique (Fragmine®), les données générées sont :

- 60 cinétiques mesurées le 19.10.2017 sur le STA-R® AUT06366 (soft version 3.04.07)

    - pool normal de plasmas lot 03.2017 surchargé par 0.15, 0.25, 0.35, 0.44, 0.54, 0.64, 0.74, 0.85, 0.94, 1.07, 1.14, 1.24, 1.27, 1.44, 1.50, 1.55, 1.66, 1.75, 1.79, 2.26 UI Anti-Xa/mL ;
    - chaque niveau d'échantillon est testé en *n*=3 ;
    - les échantillons ont été préparés extemporanément et testés simultanément sur les deux automates.

- 60 cinétiques mesurées le 19.10.2017 sur le STA-R® AUT05016 (soft version 3.04.07)

    - pool normal de plasmas lot 03.2017 surchargé par 0.15, 0.25, 0.35, 0.44, 0.54, 0.64, 0.74, 0.85, 0.94, 1.07, 1.14, 1.24, 1.27, 1.44, 1.50, 1.55, 1.66, 1.75, 1.79, 2.26 UI Anti-Xa/mL ;
    - chaque niveau d'échantillon est testé en *n*=3 ;
    - les échantillons ont été préparés extemporanément et testés simultanément sur les deux automates.

Concernant l'HBPM énoxaparine sodique (Lovenox®), les données générées sont :

- 60 cinétiques mesurées le 26.10.2017 sur le STA-R® AUT06366 (soft version 3.04.07)

    - pool normal de plasmas lot 03.2017 surchargé par 0.10, 0.20, 0.31, 0.40, 0.52, 0.63, 0.71, 0.83, 0.93, 1.02, 1.09, 1.21, 1.28, 1.36, 1.49, 1.58, 1.65, 1.81, 1.85, 1.97 UI Anti-Xa/mL ;
    - chaque niveau d'échantillon est testé en *n*=3 ;
    - les échantillons ont été préparés extemporanément et testés simultanément sur les deux automates.

- 60 cinétiques mesurées le 26.10.2017 sur le STA-R® AUT00603 (soft version 3.04.07)

  - pool normal de plasmas lot 03.2017 surchargé par 0.10, 0.20, 0.31, 0.40, 0.52, 0.63, 0.71, 0.83, 0.93, 1.02, 1.09, 1.21, 1.28, 1.36, 1.49, 1.58, 1.65, 1.81, 1.85, 1.97 UI Anti-Xa/mL ;
  - chaque niveau d'échantillon est testé en *n*=3 ;
  - les échantillons ont été préparés extemporanément et testés simultanément sur les deux automates.

Concernant l'HBPM tinzaparine sodique (InnoHep®), les données générées sont :

- 60 cinétiques mesurées le 25.10.2017 sur le STA-R® AUT06366 (soft version 3.04.07)

  - pool normal de plasmas lot 03.2017 surchargé par 0.11, 0.20, 0.30, 0.40, 0.46, 0.54, 0.63, 0.80, 0.89, 0.96, 1.06, 1.21, 1.27, 1.37, 1.46, 1.52, 1.64, 1.81, 1.83, 1.97 UI Anti-Xa/mL ;
  - chaque niveau d'échantillon est testé en *n*=3 ;
  - les échantillons ont été préparés extemporanément et testés simultanément sur les deux automates.

- 60 cinétiques mesurées le 25.10.2017 sur le STA-R® AUT00603 (soft version 3.04.07)

  - pool normal de plasmas lot 03.2017 surchargé par 0.11, 0.20, 0.30, 0.40, 0.46, 0.54, 0.63, 0.80, 0.89, 0.96, 1.06, 1.21, 1.27, 1.37, 1.46, 1.52, 1.64, 1.81, 1.83, 1.97 UI Anti-Xa/mL ;
  - chaque niveau d'échantillon est testé en *n*=3 ;
  - les échantillons ont été préparés extemporanément et testés simultanément sur les deux automates.

Numéro de lot du STA® - Liquid Anti-Xa : 251187, lot utilisé pour la génération des cinétiques en méthodologie universelle et pour le dosage commercial de la surcharge d'héparine.
Concernant le Xarelto® (rivaroxaban), les données générées sont :

- 60 cinétiques mesurées le 10.10.2017 sur le STA-R® AUT00603 (soft version 3.04.07)

  - pool normal de plasmas lot 03.2017 surchargé par 10, 20, 35, 41, 49, 56, 66, 74, 84, 93, 105, 110, 116, 134, 143, 155, 160, 175, 181 et 198 ng/mL ;
  - chaque niveau d'échantillon est testé en *n*=3 ;
  - les échantillons ont été préparés et stockés à -80°C.

- 60 cinétiques mesurées le 17.10.2017 sur le STA-R® AUT05450 (soft version 3.04.07)

  - pool normal de plasmas lot 03.2017 surchargé par 10, 20, 35, 41, 48, 59, 65, 77, 85, 95, 103, 113, 121, 134, 145, 160, 161, 172, 169, 202 ng/mL ;
  - chaque niveau d'échantillon est testé en *n*=3 ;
  - les échantillons ont été préparés et stockés à -80°C.

- 60 cinétiques mesurées le 17.10.2017 sur le STA-R® AUT06366 (soft version 3.04.07)

  - pool normal de plasmas lot 03.2017 surchargé par 10, 20, 25, 34, 43, 50, 62, 74, 84, 93, 104, 109, 118, 133, 143, 159, 158, 176, 173 et 203 ng/mL ;
  - chaque niveau d'échantillon est testé en *n*=3 ;
  - les échantillons ont été préparés et stockés à -80°C.

- 60 cinétiques mesurées le 10.10.2017 sur le STA-R® AUT06399 (soft version 3.04.07)

  - pool normal de plasmas lot 03.2017 surchargé par 10, 20, 30, 38, 46, 57, 64, 72, 82, 90, 101, 110, 115, 130, 140, 148, 152, 166, 164 et 192 ng/mL ;
  - chaque niveau d'échantillon est testé en *n*=3 ;
  - les échantillons ont été préparés et stockés à -80°C.

Concernant l'Eliquis® (apixaban), les données générées sont :

- 60 cinétiques mesurées le 16.10.2017 sur le STA-R® AUT00603 (soft version 3.04.07)

- pool normal de plasmas lot 03.2017 surchargé par 10, 20, 27, 39, 41, 52, 61, 67, 76, 93, 96, 105, 123, 127, 140, 145, 155, 167, 184 et 182 ng/mL ;
- chaque niveau d'échantillon est testé en *n*=3 ;
- les échantillons ont été préparés et stockés à -80°C.

- 60 cinétiques mesurées le 05.10.2017 sur le STA-R® AUT06360 (soft version 3.04.07)

  - pool normal de plasmas lot 03.2017 surchargé par 10, 20, 26, 36, 43, 53, 60, 70, 75, 93, 103, 109, 121, 135, 136, 153, 160, 175, 185 et 192 ng/mL ;
  - chaque niveau d'échantillon est testé en *n*=3 ;
  - les échantillons ont été préparés et stockés à -80°C.

- 60 cinétiques mesurées le 16.10.2017 sur le STA-R® AUT06366 (soft version 3.04.07)

  - pool normal de plasmas lot 03.2017 surchargé par 10, 20, 26, 34, 44, 50, 61, 70, 74, 94, 103, 109, 122, 135, 138, 147, 162, 173, 182 et 186 ng/mL ;
  - chaque niveau d'échantillon est testé en *n*=3.
  - les échantillons ont été préparés et stockés à -80°C.

- 60 cinétiques mesurées le 05.10.2017 sur le STA-R® AUT06399 (soft version 3.04.07)

  - pool normal de plasmas lot 03.2017 surchargé par 10, 20, 33, 41, 46, 57, 65, 76, 79, 96, 105, 116, 125, 135, 138, 153, 161, 169, 180 et 186 ng/mL ;
  - chaque niveau d'échantillon est testé en *n*=3.
  - les échantillons ont été préparés et stockés à -80°C.

Concernant le Lixiana® (edoxaban), les données générées sont :

- 60 cinétiques mesurées le 17.10.2017 sur le STA-R® AUT00603 (soft version 3.04.07)

  - pool normal de plasmas lot 03.2017 surchargé par 10, 20, 28, 40, 49, 64, 74, 95, 103, 109, 122, 132, 144, 131, 132, 157, 171, 195, 191 et 201 ng/mL ;
  - chaque niveau d'échantillon est testé en *n*=3 ;
  - les échantillons ont été préparés et stockés à -80°C.

- 60 cinétiques mesurées le 17.10.2017 sur le STA-R® AUT05450 (soft version 3.04.07)

  - pool normal de plasmas lot 03.2017 surchargé par 10, 21, 28, 41, 49, 62, 74, 92, 102, 112, 120, 133, 142, 129, 133, 155, 168, 188, 200 et 206 ng/mL ;
  - chaque niveau d'échantillon est testé en *n*=3 ;
  - les échantillons ont été préparés et stockés à -80°C.

- 60 cinétiques mesurées le 21.09.2017 sur le STA-R® AUT06366 (soft version 3.04.07)

  - pool normal de plasmas lot 03.2017 surchargé par 10, 21, 27, 40, 49, 64, 73, 90, 99, 108, 122, 132, 144, 130, 136, 153, 169, 192, 194 et 216 ng/mL ;
  - chaque niveau d'échantillon est testé en *n*=3 ;
  - les échantillons ont été préparés et stockés à -80°C.

- 60 cinétiques mesurées le 20.09.2017 sur le STA-R® AUT06399 (soft version 3.04.07)

  - pool normal de plasmas lot 03.2017 surchargé par 10, 21, 29, 39, 48, 60, 73, 81, 92, 107, 115, 129, 136 ,129, 127, 149, 163, 181, 195 et 199 ng/mL ;
  - chaque niveau d'échantillon est testé en *n*=3 ;
  - les échantillons ont été préparés et stockés à -80°C.

Numéro de lot du STA® - Liquid Anti-Xa : 251738, lot utilisé pour la génération des cinétiques en méthodologie universelle et pour le dosage commercial des surcharges d'AODs.

### 3.2.2 Modèle d'apprentissage automatique

### Organisation des données

Le modèle d'apprentissage automatique est entraîné par une validation croisée divisée en deux sous-ensembles de la manière suivante :

- Sous-ensemble 1 :

  - Données d'apprentissage :

    - données HNF sodique (Héparine Choay®) générées sur le STA-R® AUT06366,
    - données HNF calcique (Calciparine®) générées sur le STA-R® AUT05450,
    - données HBPM daltéparine sodique (Fragmine®) générées sur le STA-R® AUT06366,
    - données HBPM énoxaparine sodique (Lovenox®) générées sur le STA-R® AUT06366,
    - données HBPM tinzaparine sodique (InnoHep®) générées sur le STA-R® AUT06366,
    - données rivaroxaban (Xarelto®) générées sur le STA-R® AUT06366,
    - données rivaroxaban (Xarelto®) générées sur le STA-R® AUT05450,
    - données edoxaban (Lixiana®) générées sur le STA-R® AUT06366,
    - données edoxaban (Lixiana®) générées sur le STA-R® AUT05450,
    - données apixaban (Eliquis®) générées sur le STA-R® AUT06366,
    - données apixaban (Eliquis®) générées sur le STA-R® AUT06360.

  - Données de validation :

    - données HNF sodique (Héparine Choay®) générées sur le STA-R® AUT00603,
    - données HNF calcique (Calciparine®) générées sur le STA-R® AUT05016,
    - données HBPM daltéparine sodique (Fragmine®) générées sur le STA-R® AUT05016,
    - données HBPM énoxaparine sodique (Lovenox®) générées sur le STA-R® AUT00603,
    - données HBPM tinzaparine sodique (InnoHep®) générées sur le STA-R® AUT00603,
    - données rivaroxaban (Xarelto®) générées sur le STA-R® AUT00603,
    - données rivaroxaban (Xarelto®) générées sur le STA-R® AUT06399,
    - données edoxaban (Lixiana®) générées sur le STA-R® AUT00603,
    - données edoxaban (Lixiana®) générées sur le STA-R® AUT06399,
    - données apixaban (Eliquis®) générées sur le STA-R® AUT00603,
    - données apixaban (Eliquis®) générées sur le STA-R® AUT06399.

- Sous-ensemble 2 :

  - Données d'apprentissage :

    - données HNF sodique (Héparine Choay®) générées sur le STA-R® AUT00603,
    - données HNF calcique (Calciparine®) générées sur le STA-R® AUT05016,
    - données HBPM daltéparine sodique (Fragmine®) générées sur le STA-R® AUT05016,
    - données HBPM énoxaparine sodique (Lovenox®) générées sur le STA-R® AUT00603,
    - données HBPM tinzaparine sodique (InnoHep®) générées sur le STA-R® AUT00603,
    - données rivaroxaban (Xarelto®) générées sur le STA-R® AUT00603,
    - données rivaroxaban (Xarelto®) générées sur le STA-R® AUT06399,
    - données edoxaban (Lixiana®) générées sur le STA-R® AUT00603,
    - données edoxaban (Lixiana®) générées sur le STA-R® AUT06399,
    - données apixaban (Eliquis®) générées sur le STA-R® AUT00603,
    - données apixaban (Eliquis®) générées sur le STA-R® AUT06399.

  - Données de validation :

    - données HNF sodique (Héparine Choay®) générées sur le STA-R® AUT06366,
    - données HNF calcique (Calciparine®) générées sur le STA-R® AUT05450,
    - données HBPM daltéparine sodique (Fragmine®) générées sur le STA-R® AUT06366,

- données HBPM énoxaparine sodique (Lovenox®) générées sur le STA-R® AUT06366,
- données HBPM tinzaparine sodique (InnoHep®) générées sur le STA-R® AUT06366,
- données rivaroxaban (Xarelto®) générées sur le STA-R® AUT06366,
- données rivaroxaban (Xarelto®) générées sur le STA-R® AUT05450,
- données edoxaban (Lixiana®) générées sur le STA-R® AUT06366,
- données edoxaban (Lixiana®) générées sur le STA-R® AUT05450,
- données apixaban (Eliquis®) générées sur le STA-R® AUT06366,
- données apixaban (Eliquis®) générées sur le STA-R® AUT06360.

L'apprentissage final se fait sur l'intégralité des données.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 77 neurones
    - Fonctions d'activation : Identité

  - Couche cachée

    - 18 neurones
    - Fonctions d'activation : ReLU

  - Couche de sortie

    - 2 neurones
    - Fonction d'activation : Softmax

- Initialisation de la matrice de poids et de biais : Xavier Glorot
- Méthode numérique pour l'optimisation des poids et des biais : L-BFGS
- Méthode de régularisation : L2, alpha = 0.001
- Stratégie d'apprentissage : recherche par graines
- Fonction de coût : entropie croisée

**3.3 Identification des héparines**

**3.3.1 Jeux de données**

Concernant l'HNF sodique (Héparine Choay®), les données générées sont :

- 60 cinétiques mesurées le 24.10.2017 sur le STA-R® AUT00603 (soft version 3.04.07)

  - pool normal de plasmas lot 03.2017 surchargé par 0.12, 0.23, 0.32, 0.46, 0.54, 0.66, 0.66, 0.78, 0.86, 1.00, 1.18, 1.20, 1.36, 1.47, 1.56, 1.64, 1.74, 1.80, 1.88 et 2.03 UI/mL ;
  - chaque niveau d'échantillon est testé en *n=3 ;*
  - les échantillons ont été préparés extemporanément et testés simultanément sur les deux automates.

- 60 cinétiques mesurées le 24.10.2017 sur le STA-R® AUT06366 (soft version 3.04.07)

  - pool normal de plasmas lot 03.2017 surchargé par 0.11, 0.21, 0.32, 0.43, 0.55, 0.63, 0.64, 0.76, 0.87, 0.97, 1.14, 1.17, 1.30, 1.43, 1.52, 1.61, 1.70, 1.84, 1.84 et 1.99 UI/mL ;

- chaque niveau d'échantillon est testé en *n=3* ;
- les échantillons ont été préparés extemporanément et testés simultanément sur les deux automates.

- 24 cinétiques mesurées le 18.07.2017 sur le STA-R® AUT06399 (soft version 3.04.07)

  - pool normal de plasmas lot 03.2017 surchargé avec un taux théorique de 0.25, 0.50, 0.75, 1.00, 1.25, 1.50, 1.75 et 2.00 UI/mL ;
  - chaque niveau d'échantillon est testé en *n=3* ;
  - les échantillons ont été préparés extemporanément.

Concernant l'HNF calcique (Calciparine®), les données générées sont :

- 60 cinétiques mesurées le 23.10.2019 sur le STA-R® AUT05016 (soft version 3.04.07)

  - pool normal de plasmas lot 03.2017 surchargé par 0.10, 0.21, 0.32, 0.44, 0.56, 0.66, 0.77, 0.84, 0.86, 0.98, 1.15, 1.28, 1.34, 1.43, 1.56, 1.60, 1.75, 1.85, 1.92 et 1.96 UI/mL ;
  - chaque niveau d'échantillons est testé en *n=3* ;
  - les échantillons ont été préparés extemporanément et testés simultanément sur les deux automates.

- 60 cinétiques mesurées le 23.10.2019 sur le STA-R® AUT05450 (soft version 3.04.07)

  - pool normal de plasmas lot 03.2017 surchargé par 0.12, 0.23, 0.33, 0.43, 0.55, 0.66, 0.75, 0.90, 0.88, 1.00, 1.13, 1.24, 1.32, 1.44, 1.53, 1.63, 1.70, 1.88, 1.96 et 2.01 UI/mL ;
  - chaque niveau d'échantillon est testé en *n=3* ;
  - les échantillons ont été préparés extemporanément et testés simultanément sur les deux automates.

- 24 cinétiques mesurées le 17.07.2017 sur le STA-R® AUT06399 (soft version 3.04.07)

  - pool normal de plasmas lot 03.2017 surchargé avec un taux théorique de 0.25, 0.50, 0.75, 1.00, 1.25, 1.50, 1.75 et 2.00 UI/mL ;
  - chaque niveau d'échantillon est testé en *n=3* ;
  - les échantillons ont été préparés extemporanément.

Concernant l'HBPM daltéparine sodique (Fragmine®), les données générées sont :

- 60 cinétiques mesurées le 19.10.2017 sur le STA-R® AUT06366 (soft version 3.04.07)

  - pool normal de plasmas lot 03.2017 surchargé par 0.15, 0.25, 0.35, 0.44, 0.54, 0.64, 0.74, 0.85, 0.94, 1.07, 1.14, 1.24, 1.27, 1.44, 1.50, 1.55, 1.66, 1.75, 1.79, 2.26 UI Anti-Xa/mL ;
  - chaque niveau d'échantillon est testé en *n=3* ;
  - les échantillons ont été préparés extemporanément et testés simultanément sur les deux automates.

- 60 cinétiques mesurées le 19.10.2017 sur le STA-R® AUT05016 (soft version 3.04.07)

  - pool normal de plasmas lot 03.2017 surchargé par 0.15, 0.25, 0.35, 0.44, 0.54, 0.64, 0.74, 0.85, 0.94, 1.07, 1.14, 1.24, 1.27, 1.44, 1.50, 1.55, 1.66, 1.75, 1.79, 2.26 UI Anti-Xa/mL ;
  - chaque niveau d'échantillon est testé en *n=3* ;
  - les échantillons ont été préparés extemporanément et testés simultanément sur les deux automates.

- 24 cinétiques mesurées le 17.07.2017 sur le STA-R® AUT06399 (soft version 3.04.07)

  - pool normal de plasmas lot 03.2017 surchargé avec un taux théorique de 0.25, 0.50, 0.75, 1.00, 1.25, 1.50, 1.75 et 2.00 UI/mL ;
  - chaque niveau d'échantillon est testé en *n=3* ;
  - les échantillons ont été préparés extemporanément.

Concernant l'HBPM énoxaparine sodique (Lovenox®), les données générées sont :

- 60 cinétiques mesurées le 26.10.2017 sur le STA-R® AUT06366 (soft version 3.04.07)

  - pool normal de plasmas lot 03.2017 surchargé par 0.10, 0.20, 0.31, 0.40, 0.52, 0.63, 0.71, 0.83, 0.93, 1.02, 1.09, 1.21, 1.28, 1.36, 1.49, 1.58, 1.65, 1.81, 1.85, 1.97 UI Anti-Xa/mL ;
  - chaque niveau d'échantillon est testé en $n$=3 ;
  - les échantillons ont été préparés extemporanément et testés simultanément sur les deux automates.

- 60 cinétiques mesurées le 26.10.2017 sur le STA-R® AUT00603 (soft version 3.04.07)

  - pool normal de plasmas lot 03.2017 surchargé par 0.10, 0.20, 0.31, 0.40, 0.52, 0.63, 0.71, 0.83, 0.93, 1.02, 1.09, 1.21, 1.28, 1.36, 1.49, 1.58, 1.65, 1.81, 1.85, 1.97 UI Anti-Xa/mL ;
  - chaque niveau d'échantillon est testé en $n$=3 ;
  - les échantillons ont été préparés extemporanément et testés simultanément sur les deux automates.

- 24 cinétiques mesurées le 19.07.2017 sur le STA-R® AUT06399 (soft version 3.04.07)

  - pool normal de plasmas lot 03.2017 surchargé avec un taux théorique de 0.25, 0.50, 0.75, 1.00, 1.25, 1.50, 1.75 et 2.00 UI/mL ;
  - chaque niveau d'échantillon est testé en $n$=3 ;
  - les échantillons ont été préparés extemporanément.

Concernant l'HBPM tinzaparine sodique (InnoHep®), les données générées sont :

- 60 cinétiques mesurées le 25.10.2017 sur le STA-R® AUT06366 (soft version 3.04.07)

  - pool normal de plasmas lot 03.2017 surchargé par 0.11, 0.20, 0.30, 0.40, 0.46, 0.54, 0.63, 0.80, 0.89, 0.96, 1.06, 1.21, 1.27, 1.37, 1.46, 1.52, 1.64, 1.81, 1.83, 1.97 UI Anti-Xa/mL ;
  - chaque niveau d'échantillon est testé en $n$=3 ;
  - les échantillons ont été préparés extemporanément et testés simultanément sur les deux automates.

- 60 cinétiques mesurées le 25.10.2017 sur le STA-R® AUT00603 (soft version 3.04.07)

  - pool normal de plasmas lot 03.2017 surchargé par 0.11, 0.20, 0.30, 0.40, 0.46, 0.54, 0.63, 0.80, 0.89, 0.96, 1.06, 1.21, 1.27, 1.37, 1.46, 1.52, 1.64, 1.81, 1.83, 1.97 UI Anti-Xa/mL ;
  - chaque niveau d'échantillon est testé en $n$=3 ;
  - les échantillons ont été préparés extemporanément et testés simultanément sur les deux automates.

- 24 cinétiques mesurées le 18.07.2017 sur le STA-R® AUT06399 (soft version 3.04.07)

  - pool normal de plasmas lot 03.2017 surchargé avec un taux théorique de 0.25, 0.50, 0.75, 1.00, 1.25, 1.50, 1.75 et 2.00 UI/mL ;
  - chaque niveau d'échantillon est testé en $n$=3 ;
  - les échantillons ont été préparés extemporanément.

Numéro de lot du STA® - Liquid Anti-Xa : 251187, lot utilisé pour la génération des cinétiques en méthodologie universelle et pour le dosage commercial de la surcharge d'héparine (lorsque applicable).

### 3.3.2 Modèle d'apprentissage automatique

**Organisation des données**

Le modèle d'apprentissage automatique est entraîné par une validation croisée organisée de la manière suivante :

- Données d'apprentissage :

  - données HNF sodique (Héparine Choay®) générées sur le STA-R® AUT06366,
  - données HNF sodique (Héparine Choay®) générées sur le STA-R® AUT00603,

- données HNF calcique (Calciparine®) générées sur le STA-R® AUT05450,
- données HNF calcique (Calciparine®) générées sur le STA-R® AUT05016,
- données HBPM daltéparine sodique (Fragmine®) générées sur le STA-R® AUT 06366,
- données HBPM daltéparine sodique (Fragmine®) générées sur le STA-R® AUT 05016,
- données HBPM énoxaparine sodique (Lovenox®) générées sur le STA-R® AUT 06366,
- données HBPM énoxaparine sodique (Lovenox®) générées sur le STA-R® AUT 00603,
- données HBPM tinzaparine sodique (InnoHep®) générées sur le STA-R® AUT 06366,
- données HBPM tinzaparine sodique (InnoHep®) générées sur le STA-R® AUT 00603.

- Données de validation :

  - données HNF sodique (Héparine Choay®) générées sur le STA-R® AUT06399,
  - données HNF calcique (Calciparine®) générées sur le STA-R® AUT06399,
  - données HBPM daltéparine sodique (Fragmine®) générées sur le STA-R® AUT 06399,
  - données HBPM énoxaparine sodique (Lovenox®) générées sur le STA-R® AUT 06399,
  - données HBPM tinzaparine sodique (InnoHep®) générées sur le STA-R® AUT 06399.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 77 neurones
    - Fonctions d'activation : Identité

  - Couche cachée

    - 40 neurones
    - Fonctions d'activation : ReLU

  - Couche de sortie

    - 2 neurones
    - Fonction d'activation : Softmax

- Initialisation de la matrice de poids et de biais : Xavier Glorot
- Méthode numérique pour l'optimisation des poids et des biais : L-BFGS
- Méthode de régularisation : L2, alpha = 0.9
- Stratégie d'apprentissage : recherche par graines
- Fonction de coût : entropie croisée

**3.4 Dosages des héparines non fractionnées**

**3.4.1 Jeux de données**

Concernant l'HNF sodique (Héparine Choay®), les données générées sont :

- 63 cinétiques mesurées le 24.10.2017 sur le STA-R® AUT00603 (soft version 3.04.07)

  - pool normal de plasmas lot 03.2017 surchargé par 0.00, 0.12, 0.23, 0.32, 0.46, 0.54, 0.66, 0.66, 0.78, 0.86, 1.00, 1.18, 1.20, 1.36, 1.47, 1.56, 1.64, 1.74, 1.80, 1.88 et 2.03 UI/mL ;
  - chaque niveau d'échantillon est testé en *n=3 ;*

- les échantillons ont été préparés extemporanément et testés simultanément sur les deux automates.

- 63 cinétiques mesurées le 24.10.2017 sur le STA-R® AUT06366 (soft version 3.04.07)

  - pool normal de plasmas lot 03.2017 surchargé par 0.00, 0.11, 0.21, 0.32, 0.43, 0.55, 0.63, 0.64, 0.76, 0.87, 0.97, 1.14, 1.17, 1.30, 1.43, 1.52, 1.61, 1.70, 1.84, 1.84 et 1.99 UI/mL ;
  - chaque niveau d'échantillon est testé en $n$=3 ;
  - les échantillons ont été préparés extemporanément et testés simultanément sur les deux automates.

Concernant l'HNF calcique (Calciparine®), les données générées sont :

- 63 cinétiques mesurées le 23.10.2019 sur le STA-R® AUT05016 (soft version 3.04.07)

  - pool normal de plasmas lot 03.2017 surchargé par 0.00, 0.10, 0.21, 0.32, 0.44, 0.56, 0.66, 0.77, 0.84, 0.86, 0.98, 1.15, 1.28, 1.34, 1.43, 1.56, 1.60, 1.75, 1.85, 1.92 et 1.96 UI/mL ;
  - chaque niveau d'échantillons est testé en $n$=3 ;
  - les échantillons ont été préparés extemporanément et testés simultanément sur les deux automates.

- 63 cinétiques mesurées le 23.10.2019 sur le STA-R® AUT05450 (soft version 3.04.07)

  - pool normal de plasmas lot 03.2017 surchargé par 0.00, 0.12, 0.23, 0.33, 0.43, 0.55, 0.66, 0.75, 0.90, 0.88, 1.00, 1.13, 1.24, 1.32, 1.44, 1.53, 1.63, 1.70, 1.88, 1.96 et 2.01 UI/mL ;
  - chaque niveau d'échantillon est testé en $n$=3 ;
  - les échantillons ont été préparés extemporanément et testés simultanément sur les deux automates.

Numéro de lot du STA® - Liquid Anti-Xa : 251187, lot utilisé pour la génération des cinétiques en méthodologie universelle et pour le dosage commercial de la surcharge d'héparine.

### 3.4.2 Modèle d'apprentissage automatique

### Organisation des données

Le modèle d'apprentissage automatique est entraîné par une validation croisée divisée en quatre sous-ensembles de la manière suivante :

- Sous-ensemble 1 :

  - Données d'apprentissage :

    - données HNF sodique (Héparine Choay®) générées sur le STA-R® AUT06366,
    - données HNF calcique (Calciparine®) générées sur le STA-R® AUT05450.

  - Données de validation :

    - données HNF sodique (Héparine Choay®) générées sur le STA-R® AUT00603,
    - données HNF calcique (Calciparine®) générées sur le STA-R® AUT05016.

- Sous-ensemble 2 :

  - Données d'apprentissage :

    - données HNF sodique (Héparine Choay®) générées sur le STA-R® AUT06366,
    - données HNF calcique (Calciparine®) générées sur le STA-R® AUT05016.

  - Données de validation :

    - données HNF sodique (Héparine Choay®) générées sur le STA-R® AUT00603,
    - données HNF calcique (Calciparine®) générées sur le STA-R® AUT05450.

- Sous-ensemble 3 :

  - Données d'apprentissage :

    - données HNF sodique (Héparine Choay®) générées sur le STA-R® AUT00603,
    - données HNF calcique (Calciparine®) générées sur le STA-R® AUT05450.

  - Données de validation :

    - données HNF sodique (Héparine Choay®) générées sur le STA-R® AUT06366,
    - données HNF calcique (Calciparine®) générées sur le STA-R® AUT05016.

- Sous-ensemble 4 :

  - Données d'apprentissage :

    - données HNF sodique (Héparine Choay®) générées sur le STA-R® AUT00603,
    - données HNF calcique (Calciparine®) générées sur le STA-R® AUT05016.

  - Données de validation :

    - données HNF sodique (Héparine Choay®) générées sur le STA-R® AUT06366,
    - données HNF calcique (Calciparine®) générées sur le STA-R® AUT05450.

L'apprentissage final se fait sur l'intégralité des données.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 77 neurones
    - Fonctions d'activation : Identité

  - Couche cachée

    - 40 neurones
    - Fonctions d'activation : ReLU

  - Couche de sortie

    - 1 neurone
    - Fonction d'activation : Identité

- Initialisation de la matrice de poids et de biais : Xavier Glorot
- Méthode numérique pour l'optimisation des poids et des biais : L-BFGS
- Méthode de régularisation : L2, alpha = 0.01
- Stratégie d'apprentissage : recherche par graines
- Fonction de coût : erreur quadratique moyenne

**3.5 Dosages des héparines de bas poids moléculaires**

### 3.5.1 Jeux de données

Concernant l'HBPM daltéparine sodique (Fragmine®), les données générées sont :

- 63 cinétiques mesurées le 19.10.2017 sur le STA-R® AUT06366 (soft version 3.04.07)

  - pool normal de plasmas lot 03.2017 surchargé par 0.00, 0.15, 0.25, 0.35, 0.44, 0.54, 0.64, 0.74, 0.85, 0.94, 1.07, 1.14, 1.24, 1.27, 1.44, 1.50, 1.55, 1.66, 1.75, 1.79, 2.26 UI Anti-Xa/mL ;
  - chaque niveau d'échantillon est testé en *n=3* ;
  - les échantillons ont été préparés extemporanément et testés simultanément sur les deux automates.

- 63 cinétiques mesurées le 19.10.2017 sur le STA-R® AUT05016 (soft version 3.04.07)

  - pool normal de plasmas lot 03.2017 surchargé par 0.00, 0.15, 0.25, 0.35, 0.44, 0.54, 0.64, 0.74, 0.85, 0.94, 1.07, 1.14, 1.24, 1.27, 1.44, 1.50, 1.55, 1.66, 1.75, 1.79, 2.26 UI Anti-Xa/mL ;
  - chaque niveau d'échantillon est testé en *n=3* ;
  - les échantillons ont été préparés extemporanément et testés simultanément sur les deux automates.

Concernant l'HBPM énoxaparine sodique (Lovenox®), les données générées sont :

- 63 cinétiques mesurées le 26.10.2017 sur le STA-R® AUT06366 (soft version 3.04.07)

  - pool normal de plasmas lot 03.2017 surchargé par 0.00, 0.10, 0.20, 0.31, 0.40, 0.52, 0.63, 0.71, 0.83, 0.93, 1.02, 1.09, 1.21, 1.28, 1.36, 1.49, 1.58, 1.65, 1.81, 1.85, 1.97 UI Anti-Xa/mL ;
  - chaque niveau d'échantillon est testé en *n=3* ;
  - les échantillons ont été préparés extemporanément et testés simultanément sur les deux automates.

- 63 cinétiques mesurées le 26.10.2017 sur le STA-R® AUT00603 (soft version 3.04.07)

  - pool normal de plasmas lot 03.2017 surchargé par 0.00, 0.10, 0.20, 0.31, 0.40, 0.52, 0.63, 0.71, 0.83, 0.93, 1.02, 1.09, 1.21, 1.28, 1.36, 1.49, 1.58, 1.65, 1.81, 1.85, 1.97 UI Anti-Xa/mL ;
  - chaque niveau d'échantillon est testé en *n=3* ;
  - les échantillons ont été préparés extemporanément et testés simultanément sur les deux automates.

Concernant l'HBPM tinzaparine sodique (InnoHep®), les données générées sont :

- 63 cinétiques mesurées le 25.10.2017 sur le STA-R® AUT06366 (soft version 3.04.07)

  - pool normal de plasmas lot 03.2017 surchargé par 0.00, 0.11, 0.20, 0.30, 0.40, 0.46, 0.54, 0.63, 0.80, 0.89, 0.96, 1.06, 1.21, 1.27, 1.37, 1.46, 1.52, 1.64, 1.81, 1.83, 1.97 UI Anti-Xa/mL ;
  - chaque niveau d'échantillon est testé en *n=3* ;
  - les échantillons ont été préparés extemporanément et testés simultanément sur les deux automates.

- 63 cinétiques mesurées le 25.10.2017 sur le STA-R® AUT00603 (soft version 3.04.07)

  - pool normal de plasmas lot 03.2017 surchargé par 0.00, 0.11, 0.20, 0.30, 0.40, 0.46, 0.54, 0.63, 0.80, 0.89, 0.96, 1.06, 1.21, 1.27, 1.37, 1.46, 1.52, 1.64, 1.81, 1.83, 1.97 UI Anti-Xa/mL ;
  - chaque niveau d'échantillon est testé en *n=3* ;
  - les échantillons ont été préparés extemporanément et testés simultanément sur les deux automates.

Numéro de lot du STA® - Liquid Anti-Xa : 251187, lot utilisé pour la génération des cinétiques en méthodologie universelle et pour le dosage commercial de la surcharge d'héparine.

### 3.5.2 Modèle d'apprentissage automatique

### Organisation des données

Le modèle d'apprentissage automatique est entraîné par une validation croisée divisée en deux sous-ensembles de la manière suivante :

- Sous-ensemble 1 :

  - Données d'apprentissage :

    - données HBPM daltéparine sodique (Fragmine®) générées sur le STA-R® AUT05016,
    - données HBPM énoxaparine sodique (Lovenox®) générées sur le STA-R® AUT00603,
    - données HBPM tinzaparine sodique (InnoHep®) générées sur le STA-R® AUT00603.

  - Données de validation :

    - données HBPM daltéparine sodique (Fragmine®) générées sur le STA-R® AUT06366,
    - données HBPM énoxaparine sodique (Lovenox®) générées sur le STA-R® AUT06366,
    - données HBPM tinzaparine sodique (InnoHep®) générées sur le STA-R® AUT06366.

- Sous-ensemble 2 :

  - Données d'apprentissage :

    - données HBPM daltéparine sodique (Fragmine®) générées sur le STA-R® AUT06366,
    - données HBPM énoxaparine sodique (Lovenox®) générées sur le STA-R® AUT06366,
    - données HBPM tinzaparine sodique (InnoHep®) générées sur le STA-R® AUT06366.

  - Données de validation :

    - données HBPM daltéparine sodique (Fragmine®) générées sur le STA-R® AUT05016
    - données HBPM énoxaparine sodique (Lovenox®) générées sur le STA-R® AUT00603,
    - données HBPM tinzaparine sodique (InnoHep®) générées sur le STA-R® AUT00603.

L'apprentissage final se fait sur l'intégralité des données.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 77 neurones
    - Fonctions d'activation : Identité

  - Couche cachée

    - 40 neurones

    - Fonctions d'activation : ReLU

  - Couche de sortie

    - 1 neurone
    - Fonction d'activation : Identité

- Initialisation de la matrice de poids et de biais : Xavier Glorot

- Méthode numérique pour l'optimisation des poids et des biais : L-BFGS
- Méthode de régularisation : L2, alpha = 0.1
- Stratégie d'apprentissage : recherche par graines
- Fonction de coût : erreur quadratique moyenne

**3.6 Identification de l'AOD anti-Xa (méthodologie optimisée AODs)**

**3.6.1 Jeux de données**

Les données relatives au Xarelto® (rivaroxaban) générées sont :

- 60 cinétiques mesurées en janvier 2016 sur le STA-R® AUT00460

  - pool normal de plasmas surchargé par 10, 20, 29, 37, 44, 54, 62, 89, 115, 143, 160, 192, 226, 252, 287, 319, 331, 361, 391 et 407 ng/mL ;
  - chaque niveau d'échantillon est testé en *n=3* ;
  - les échantillons ont été préparés et stockés à -80°C.

- 60 cinétiques mesurées en juin 2016 sur le STA-R® AUT00722

  - pool normal de plasmas surchargé par 10, 20, 29, 37, 44, 54, 62, 89, 115, 143, 160, 192, 226, 252, 287, 319, 331, 361, 391 et 407 ng/mL ;
  - chaque niveau d'échantillon est testé en *n*=3 ;
  - les échantillons ont été préparés et stockés à -80°C.

Les données relatives à Eliquis® (apixaban) générées sont :

- 69 cinétiques mesurées en janvier 2016 sur le STA-R® AUT00460

  - pool normal de plasmas surchargé par 10, 20, 31, 42, 48, 72, 92, 116, 143, 163, 192, 217, 247, 276, 298, 321, 348, 370, 397, 416, 439, 459 et 473 ng/mL ;
  - chaque niveau d'échantillon est testé en *n*=3 ;
  - les échantillons ont été préparés et stockés à -80°C.

- 69 cinétiques mesurées en juin 2016 sur le STA-R® AUT00722

  - pool normal de plasmas surchargé par 10, 20, 31, 42, 48, 72, 92, 116, 143, 163, 192, 217, 247, 276, 298, 321, 348, 370, 397, 416, 439, 459 et 473 ng/mL ;
  - chaque niveau d'échantillon est testé en *n*=3 ;
  - les échantillons ont été préparés et stockés à -80°C.

Les données relatives au Lixiana® (edoxaban) générées sont :

- 69 cinétiques mesurées en janvier 2016 sur le STA-R® AUT00460

  - pool normal de plasmas surchargé par 16, 21, 30, 39, 51, 76, 100, 128, 142, 155, 187, 215, 245, 269, 284, 322, 347, 359, 380, 393, 410, 426 et 436 ng/mL ;
  - chaque niveau d'échantillon est testé en *n*=3 ;
  - les échantillons ont été préparés et stockés à -80°C.

- 69 cinétiques mesurées en juin 2016 sur le STA-R® AUT00722

  - pool normal de plasmas surchargé par 16, 21, 30, 39, 51, 76, 100, 128, 142, 155, 187, 215, 245, 269, 284, 322, 347, 359, 380, 393, 410, 426 et 436 ng/mL ;
  - chaque niveau d'échantillon est testé en *n*=3 ;
  - les échantillons ont été préparés et stockés à -80°C.

**3.6.2 Modèle d'apprentissage automatique**

**Organisation des données**

Le modèle d'apprentissage automatique est entraîné par une validation croisée organisée de la manière suivante :

- Données d'apprentissage :

    - données rivaroxaban (Xarelto®) générées sur le STA-R® AUT00460,
    - données apixaban (Eliquis®) générées sur le STA-R® AUT00460,
    - données edoxaban (Lixiana®) générées sur le STA-R® AUT00460.

- Données de validation :

    - données rivaroxaban (Xarelto®) générées sur le STA-R® AUT00722,
    - données apixaban (Eliquis®) générées sur le STA-R® AUT00722,
    - données edoxaban (Lixiana®) générées sur le STA-R® AUT00722.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

    - Couche d'entrée

        - 42 neurones
        - Fonctions d'activation : Identité

    - Couche cachée

        - 29 neurones
        - Fonctions d'activation : ReLU

    - Couche cachée

        - 16 neurones
        - Fonctions d'activation : ReLU

    - Couche de sortie

        - 3 neurones
        - Fonction d'activation : Softmax

- Initialisation de la matrice de poids et de biais : Xavier Glorot
- Méthode numérique pour l'optimisation des poids et des biais : L-BFGS
- Méthode de régularisation : L2, alpha = 0.01
- Stratégie d'apprentissage : recherche par graines
- Fonction de coût : entropie croisée

**3.7 Dosage du rivaroxaban (méthodologie optimisée AODs)**

**3.7.1 Jeux de données**

Les données relatives au Xarelto® (rivaroxaban) générées sont :

- 63 cinétiques mesurées en janvier 2016 sur le STA-R® AUT00460

  - pool normal de plasmas surchargé par 0, 10, 20, 29, 37, 44, 54, 62, 89, 115, 143, 160, 192, 226, 252, 287, 319, 331, 361, 391 et 407 ng/mL ;
  - chaque niveau d'échantillon est testé en $n$=3 ;
  - les échantillons ont été préparés et stockés à -80°C.

- 63 cinétiques mesurées en juin 2016 sur le STA-R® AUT00722

  - pool normal de plasmas surchargé par 0, 10, 20, 29, 37, 44, 54, 62, 89, 115, 143, 160, 192, 226, 252, 287, 319, 331, 361, 391 et 407 ng/mL ;
  - chaque niveau d'échantillon est testé en $n$=3 ;
  - les échantillons ont été préparés et stockés à -80°C.

**3.7.2 Modèle d'apprentissage automatique**

**Organisation des données**

Le modèle d'apprentissage automatique est entraîné par une validation croisée organisée de la manière suivante :

- Données d'apprentissage : données rivaroxaban (Xarelto®) générées sur le STA-R® AUT00460.
- Données de validation : données rivaroxaban (Xarelto®) générées sur le STA-R® AUT00722.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 42 neurones
    - Fonctions d'activation : Identité

  - Couche cachée

    - 29 neurones
    - Fonctions d'activation : ReLU

  - Couche cachée

    - 16 neurones
    - Fonctions d'activation : ReLU

  - Couche de sortie

    - 1 neurone
    - Fonction d'activation : Identité

- Initialisation de la matrice de poids et de biais : Xavier Glorot
- Méthode numérique pour l'optimisation des poids et des biais : L-BFGS
- Méthode de régularisation : L2, alpha = 0.01
- Stratégie d'apprentissage : recherche par graines
- Fonction de coût : erreur quadratique moyenne

**3.8 Dosages du rivaroxaban (méthodologie universelle ou** « méthodologie améliorée sur la base de la méthodologie universelle »)

**3.8.1 Jeux de données**

Les données relatives au Xarelto® (rivaroxaban) ont été générées lors de deux études distinctes. Concernant la première étude, les données générées sont :

-   63 cinétiques mesurées le 10.10.2017 sur le STA-R® AUT00603 (soft version 3.04.07)

    -   pool normal de plasmas lot 03.2017 surchargé par 0, 10, 20, 35, 41, 49, 56, 66, 74, 84, 93, 105, 110, 116, 134, 143, 155, 160, 175, 181 et 198 ng/mL ;
    -   chaque niveau d'échantillon est testé en $n$=3 ;
    -   les échantillons ont été préparés et stockés à -80°C.

-   63 cinétiques mesurées le 17.10.2017 sur le STA-R® AUT05450 (soft version 3.04.07)

    -   pool normal de plasmas lot 03.2017 surchargé par 0, 10, 20, 35, 41, 48, 59, 65, 77, 85, 95, 103, 113, 121, 134, 145, 160, 161, 172, 169, 202 ng/mL ;
    -   chaque niveau d'échantillon est testé en $n$=3 ;
    -   les échantillons ont été préparés et stockés à -80°C.

-   63 cinétiques mesurées le 17.10.2017 sur le STA-R® AUT06366 (soft version 3.04.07)

    -   pool normal de plasmas lot 03.2017 surchargé par 0, 10, 20, 25, 34, 43, 50, 62, 74, 84, 93, 104, 109, 118, 133, 143, 159, 158, 176, 173 et 203 ng/mL ;
    -   chaque niveau d'échantillon est testé en $n$=3 ;
    -   les échantillons ont été préparés et stockés à -80°C.

-   63 cinétiques mesurées le 10.10.2017 sur le STA-R® AUT06399 (soft version 3.04.07)

    -   pool normal de plasmas lot 03.2017 surchargé par 0, 10, 20, 30, 38, 46, 57, 64, 72, 82, 90, 101, 110, 115, 130, 140, 148, 152, 166, 164 et 192 ng/mL ;
    -   chaque niveau d'échantillon est testé en $n$=3 ;
    -   les échantillons ont été préparés et stockés à -80°C.

Pour cette première étude, le STA® - Liquid Anti-Xa lot 251738 a été utilisé pour la génération des cinétiques en méthodologie universelle et pour le dosage commercial de la surcharge de rivaroxaban. Concernant la deuxième étude, les données générées sont :

-   63 cinétiques mesurées le 14.05.2019 sur le STA-R® AUT05676 (soft version 3.04.07)

    -   pool normal de plasmas lot 19059RD (code 22824) surchargé par 0, 11, 21, 28, 42, 50, 58, 68, 77, 93, 102, 113, 121, 129, 141, 153, 160, 174, 184, 201 et 204 ng/mL ;
    -   chaque niveau d'échantillons est testé en $n$=3 ;
    -   les échantillons ont été préparés et stockés à -80°C, puis testés simultanément sur les trois automates.

-   63 cinétiques mesurées le 14.05.2019 sur le STA-R® AUT05980 (soft version 3.04.07)

    -   pool normal de plasmas lot 19059RD (code 22824) surchargé par 0, 15, 23, 32, 47, 52, 61, 74, 77, 92, 108, 112, 120, 129, 139, 151, 164, 174, 184, 191 et 206 ng/mL ;
    -   chaque niveau d'échantillons est testé en $n$=3 ;
    -   les échantillons ont été préparés et stockés à -80°C, puis testés simultanément sur les trois automates.

-   63 cinétiques mesurées le 14.05.2019 sur le STA-R® AUT06366 (soft version 3.04.07)

    -   pool normal de plasmas lot 19059RD (code 22824) surchargé par 0, 13, 22, 29, 44, 51, 60, 72, 79, 96, 104, 118, 122, 132, 143, 155, 161, 175, 186, 198 et 201 ng/mL ;

- chaque niveau d'échantillons est testé en $n$=3 ;
- les échantillons ont été préparés et stockés à -80°C, puis testés simultanément sur les trois automates.

Pour cette deuxième étude, le STA® - Liquid Anti-Xa lot 253225 a été utilisé pour la génération des cinétiques en méthodologie universelle et pour le dosage commercial de la surcharge de rivaroxaban.

### 3.8.2 Modèle d'apprentissage automatique

**Organisation des données**

Le modèle d'apprentissage automatique est entraîné par une validation croisée divisée en sept sous-ensembles de la manière suivante :

- Sous-ensemble 1 :

  - Données d'apprentissage :

    - données rivaroxaban (Xarelto®) générées sur le STA-R® AUT00603,
    - données rivaroxaban (Xarelto®) générées sur le STA-R® AUT05450,
    - données rivaroxaban (Xarelto®) générées sur le STA-R® AUT05676,
    - données rivaroxaban (Xarelto®) générées sur le STA-R® AUT05980,
    - données rivaroxaban (Xarelto®) générées sur le STA-R® AUT06366,
    - données rivaroxaban (Xarelto®) générées sur le STA-R® AUT06366 (bis).

  - Données de validation : données rivaroxaban (Xarelto®) générées sur le STA-R® AUT06399.

- Sous-ensemble 2 :

  - Données d'apprentissage :

    - données rivaroxaban (Xarelto®) générées sur le STA-R® AUT00603,
    - données rivaroxaban (Xarelto®) générées sur le STA-R® AUT05450,
    - données rivaroxaban (Xarelto®) générées sur le STA-R® AUT05676,
    - données rivaroxaban (Xarelto®) générées sur le STA-R® AUT05980,
    - données rivaroxaban (Xarelto®) générées sur le STA-R® AUT06366 (bis),
    - données rivaroxaban (Xarelto®) générées sur le STA-R® AUT06399.

  - Données de validation : données rivaroxaban (Xarelto®) générées sur le STA-R® AUT06366.

- Sous-ensemble 3 :

  - Données d'apprentissage :

    - données rivaroxaban (Xarelto®) générées sur le STA-R® AUT00603,
    - données rivaroxaban (Xarelto®) générées sur le STA-R® AUT05450,
    - données rivaroxaban (Xarelto®) générées sur le STA-R® AUT05676,
    - données rivaroxaban (Xarelto®) générées sur le STA-R® AUT05980,
    - données rivaroxaban (Xarelto®) générées sur le STA-R® AUT06366,
    - données rivaroxaban (Xarelto®) générées sur le STA-R® AUT06399.

  - Données de validation : données rivaroxaban (Xarelto®) générées sur le STA-R® AUT06366 (bis).

- Sous-ensemble 4 :

  - Données d'apprentissage :

    - données rivaroxaban (Xarelto®) générées sur le STA-R® AUT00603,
    - données rivaroxaban (Xarelto®) générées sur le STA-R® AUT05450,

- données rivaroxaban (Xarelto®) générées sur le STA-R® AUT05676,
- données rivaroxaban (Xarelto®) générées sur le STA-R® AUT06366,
- données rivaroxaban (Xarelto®) générées sur le STA-R® AUT06366 (bis),
- données rivaroxaban (Xarelto®) générées sur le STA-R® AUT06399.

- Données de validation : données rivaroxaban (Xarelto®) générées sur le STA-R® AUT05980.

- Sous-ensemble 5 :

- Données d'apprentissage :

- données rivaroxaban (Xarelto®) générées sur le STA-R® AUT00603,
- données rivaroxaban (Xarelto®) générées sur le STA-R® AUT05450,
- données rivaroxaban (Xarelto®) générées sur le STA-R® AUT05980,
- données rivaroxaban (Xarelto®) générées sur le STA-R® AUT06366,
- données rivaroxaban (Xarelto®) générées sur le STA-R® AUT06366 (bis),
- données rivaroxaban (Xarelto®) générées sur le STA-R® AUT06399.

- Données de validation : données rivaroxaban (Xarelto®) générées sur le STA-R® AUT05676.

- Sous-ensemble 6 :

- Données d'apprentissage :

- données rivaroxaban (Xarelto®) générées sur le STA-R® AUT00603,
- données rivaroxaban (Xarelto®) générées sur le STA-R® AUT05676,
- données rivaroxaban (Xarelto®) générées sur le STA-R® AUT05980,
- données rivaroxaban (Xarelto®) générées sur le STA-R® AUT06366,
- données rivaroxaban (Xarelto®) générées sur le STA-R® AUT06366 (bis),
- données rivaroxaban (Xarelto®) générées sur le STA-R® AUT06399.

- Données de validation : données rivaroxaban (Xarelto®) générées sur le STA-R® AUT05450.

- Sous-ensemble 7 :

- Données d'apprentissage :

- données rivaroxaban (Xarelto®) générées sur le STA-R® AUT05450,
- données rivaroxaban (Xarelto®) générées sur le STA-R® AUT05676,
- données rivaroxaban (Xarelto®) générées sur le STA-R® AUT05980,
- données rivaroxaban (Xarelto®) générées sur le STA-R® AUT06366,
- données rivaroxaban (Xarelto®) générées sur le STA-R® AUT06366 (bis),
- données rivaroxaban (Xarelto®) générées sur le STA-R® AUT06399.

- Données de validation : données rivaroxaban (Xarelto®) générées sur le STA-R® AUT00603.

L'apprentissage final se fait sur l'intégralité des données.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

- Couche d'entrée

- 77 neurones
- Fonctions d'activation : Identité

- Couche cachée

- 40 neurones

- Fonctions d'activation : ReLU

- Couche de sortie

- 1 neurone
- Fonction d'activation : Identité

- Initialisation de la matrice de poids et de biais : Xavier Glorot
- Méthode numérique pour l'optimisation des poids et des biais : L-BFGS
- Méthode de régularisation : L2, alpha = 0.001
- Stratégie d'apprentissage : recherche par graines
- Fonction de coût : erreur quadratique moyenne

**3.9 Dosage de l'apixaban (méthodologie optimisée AODs)**

**3.9.1 Jeux de données**

Les données relatives à Eliquis® (apixaban) générées sont :

- 72 cinétiques mesurées en janvier 2016 sur le STA-R® AUT00460

- pool normal de plasmas surchargé par 0, 10, 20, 31, 42, 48, 72, 92, 116, 143, 163, 192, 217, 247, 276, 298, 321, 348, 370, 397, 416, 439, 459 et 473 ng/mL ;
- chaque niveau d'échantillon est testé en $n=3$ ;
- les échantillons ont été préparés et stockés à -80°C.

- 72 cinétiques mesurées en juin 2016 sur le STA-R® AUT00722

- pool normal de plasmas surchargé par 0, 10, 20, 31, 42, 48, 72, 92, 116, 143, 163, 192, 217, 247, 276, 298, 321, 348, 370, 397, 416, 439, 459 et 473 ng/mL ;
- chaque niveau d'échantillon est testé en $n=3$ ;
- les échantillons ont été préparés et stockés à -80°C.

**3.9.2 Modèle d'apprentissage automatique**

**Organisation des données**

Le modèle d'apprentissage automatique est entraîné par une validation croisée organisée de la manière suivante :

- Données d'apprentissage : données apixaban (Eliquis®) générées sur le STA-R® AUT00460.
- Données de validation : données apixaban (Eliquis®) générées sur le STA-R® AUT00722.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

- Couche d'entrée

  - 42 neurones
  - Fonctions d'activation : Identité

- Couche cachée

  - 29 neurones
  - Fonctions d'activation : ReLU

- Couche cachée

  - 16 neurones
  - Fonctions d'activation : ReLU

- Couche de sortie

  - 1 neurone
  - Fonction d'activation : Identité

- Initialisation de la matrice de poids et de biais : Xavier Glorot
- Méthode numérique pour l'optimisation des poids et des biais : L-BFGS
- Méthode de régularisation : L2, alpha = 0.01
- Stratégie d'apprentissage : recherche par graines
- Fonction de coût : erreur quadratique moyenne

**3.10 Dosages de l'apixaban (méthodologie universelle ou** « méthodologie améliorée sur la base de la méthodologie universelle »)

**3.10.1 Jeux de données**

Les données relatives l'Eliquis® (apixaban) ont été générées lors de deux études distinctes. Concernant la première étude, les données générées sont :

- 63 cinétiques mesurées le 16.10.2017 sur le STA-R® AUT00603 (soft version 3.04.07)

  - pool normal de plasmas lot 03.2017 surchargé par 0, 10, 20, 27, 39, 41, 52, 61, 67, 76, 93, 96, 105, 123, 127, 140, 145, 155, 167, 184 et 182 ng/mL ;
  - chaque niveau d'échantillon est testé en $n$=3 ;
  - les échantillons ont été préparés et stockés à -80°C.

- 63 cinétiques mesurées le 05.10.2017 sur le STA-R® AUT06360 (soft version 3.04.07)

  - pool normal de plasmas lot 03.2017 surchargé par 0, 10, 20, 26, 36, 43, 53, 60, 70, 75, 93, 103, 109, 121, 135, 136, 153, 160, 175, 185 et 192 ng/mL ;
  - chaque niveau d'échantillon est testé en $n$=3 ;
  - les échantillons ont été préparés et stockés à -80°C.

- 63 cinétiques mesurées le 16.10.2017 sur le STA-R® AUT06366 (soft version 3.04.07)

  - pool normal de plasmas lot 03.2017 surchargé par 0, 10, 20, 26, 34, 44, 50, 61, 70, 74, 94, 103, 109, 122, 135, 138, 147, 162, 173, 182 et 186 ng/mL ;
  - chaque niveau d'échantillon est testé en $n$=3.
  - les échantillons ont été préparés et stockés à -80°C.

- 63 cinétiques mesurées le 05.10.2017 sur le STA-R® AUT06399 (soft version 3.04.07)

  - pool normal de plasmas lot 03.2017 surchargé par 0, 10, 20, 33, 41, 46, 57, 65, 76, 79, 96, 105, 116, 125, 135, 138, 153, 161, 169, 180 et 186 ng/mL ;
- chaque niveau d'échantillon est testé en *n*=3.
- les échantillons ont été préparés et stockés à -80°C.

Pour cette première étude, le STA® - Liquid Anti-Xa lot 251738 a été utilisé pour la génération des cinétiques en méthodologie universelle et pour le dosage commercial de la surcharge d'apixaban. Concernant la deuxième étude, les données générées sont :

- 63 cinétiques mesurées le 07.05.2019 sur le STA-R® AUT05676 (soft version 3.04.07)

    - pool normal de plasmas lot 19059RD (code 22824) surchargé par 0, 11, 16, 32, 39, 52, 62, 70, 84, 95, 104, 118, 123, 132, 142, 156, 167, 174, 183, 190 et 198 ng/mL ;
    - chaque niveau d'échantillons est testé en *n*=3 ;
    - les échantillons ont été préparés et stockés à -80°C, puis testés simultanément sur les trois automates.

- 63 cinétiques mesurées le 07.05.2019 sur le STA-R® AUT05980 (soft version 3.04.07)

    - pool normal de plasmas lot 19059RD (code 22824) surchargé par 0, 16, 23, 37, 48, 60, 73, 80, 96, 102, 111, 125, 136, 146, 155, 166, 186, 191, 193, 205 et 211 ng/mL ;
    - chaque niveau d'échantillons est testé en *n*=3 ;
    - les échantillons ont été préparés et stockés à -80°C, puis testés simultanément sur les trois automates.

- 63 cinétiques mesurées le 07.05.2019 sur le STA-R® AUT06366 (soft version 3.04.07)

    - pool normal de plasmas lot 19059RD (code 22824) surchargé par 0, 9, 16, 29, 36, 50, 61, 67, 81, 94, 102, 115, 123, 126, 144, 156, 159, 175, 183, 191 et 192 ng/mL ;
    - chaque niveau d'échantillons est testé en *n*=3 ;
    - les échantillons ont été préparés et stockés à -80°C, puis testés simultanément sur les trois automates.

Pour cette deuxième étude, le STA® - Liquid Anti-Xa lot 253225 a été utilisé pour la génération des cinétiques en méthodologie universelle et pour le dosage commercial de la surcharge d'apixaban.

### 3.10.2 Modèle d'apprentissage automatique

### Organisation des données

Le modèle d'apprentissage automatique est entraîné par une validation croisée divisée en sept sous-ensembles de la manière suivante :

- Sous-ensemble 1 :

    - Données d'apprentissage :

        - données apixaban (Eliquis®) générées sur le STA-R® AUT00603,
        - données apixaban (Eliquis®) générées sur le STA-R® AUT05676,
        - données apixaban (Eliquis®) générées sur le STA-R® AUT05980,
        - données apixaban (Eliquis®) générées sur le STA-R® AUT06360,
        - données apixaban (Eliquis®) générées sur le STA-R® AUT06366,
        - données apixaban (Eliquis®) générées sur le STA-R® AUT06366 (bis).

    - Données de validation : données apixaban (Eliquis®) générées sur le STA-R® AUT06399.

- Sous-ensemble 2 :

    - Données d'apprentissage :

        - données apixaban (Eliquis®) générées sur le STA-R® AUT00603,
        - données apixaban (Eliquis®) générées sur le STA-R® AUT05676,

- données apixaban (Eliquis®) générées sur le STA-R® AUT05980,
- données apixaban (Eliquis®) générées sur le STA-R® AUT06360,
- données apixaban (Eliquis®) générées sur le STA-R® AUT06366 (bis),
- données apixaban (Eliquis®) générées sur le STA-R® AUT06399.

- Données de validation : données apixaban (Eliquis®) générées sur le STA-R® AUT06366.

- Sous-ensemble 3 :

- Données d'apprentissage :

- données apixaban (Eliquis®) générées sur le STA-R® AUT00603,
- données apixaban (Eliquis®) générées sur le STA-R® AUT05676,
- données apixaban (Eliquis®) générées sur le STA-R® AUT05980,
- données apixaban (Eliquis®) générées sur le STA-R® AUT06360,
- données apixaban (Eliquis®) générées sur le STA-R® AUT06366,
- données apixaban (Eliquis®) générées sur le STA-R® AUT06399.

- Données de validation : données apixaban (Eliquis®) générées sur le STA-R® AUT06366 (bis).

- Sous-ensemble 4 :

- Données d'apprentissage :

- données apixaban (Eliquis®) générées sur le STA-R® AUT00603,
- données apixaban (Eliquis®) générées sur le STA-R® AUT05676,
- données apixaban (Eliquis®) générées sur le STA-R® AUT05980,
- données apixaban (Eliquis®) générées sur le STA-R® AUT06366,
- données apixaban (Eliquis®) générées sur le STA-R® AUT06366 (bis),
- données apixaban (Eliquis®) générées sur le STA-R® AUT06399.

- Données de validation : données apixaban (Eliquis®) générées sur le STA-R® AUT06360.

- Sous-ensemble 5 :

- Données d'apprentissage :

- données apixaban (Eliquis®) générées sur le STA-R® AUT00603,
- données apixaban (Eliquis®) générées sur le STA-R® AUT05676,
- données apixaban (Eliquis®) générées sur le STA-R® AUT06360,
- données apixaban (Eliquis®) générées sur le STA-R® AUT06366,
- données apixaban (Eliquis®) générées sur le STA-R® AUT06366 (bis),
- données apixaban (Eliquis®) générées sur le STA-R® AUT06399.

- Données de validation : données apixaban (Eliquis®) générées sur le STA-R® AUT05980.

- Sous-ensemble 6 :

- Données d'apprentissage :

- données apixaban (Eliquis®) générées sur le STA-R® AUT00603,
- données apixaban (Eliquis®) générées sur le STA-R® AUT05980,
- données apixaban (Eliquis®) générées sur le STA-R® AUT06360,
- données apixaban (Eliquis®) générées sur le STA-R® AUT06366,
- données apixaban (Eliquis®) générées sur le STA-R® AUT06366 (bis),
- données apixaban (Eliquis®) générées sur le STA-R® AUT06399.

- Données de validation : données apixaban (Eliquis®) générées sur le STA-R® AUT05676.

- Sous-ensemble 7 :

  - Données d'apprentissage :

    - données apixaban (Eliquis®) générées sur le STA-R® AUT05676,
    - données apixaban (Eliquis®) générées sur le STA-R® AUT05980,
    - données apixaban (Eliquis®) générées sur le STA-R® AUT06360,
    - données apixaban (Eliquis®) générées sur le STA-R® AUT06366,
    - données apixaban (Eliquis®) générées sur le STA-R® AUT06366 (bis),
    - données apixaban (Eliquis®) générées sur le STA-R® AUT06399.

  - Données de validation : données apixaban (Eliquis®) générées sur le STA-R® AUT00603.

L'apprentissage final se fait sur l'intégralité des données.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 77 neurones
    - Fonctions d'activation : Identité

  - Couche cachée

    - 40 neurones

    - Fonctions d'activation : ReLU

  - Couche de sortie

    - 1 neurone

    - Fonction d'activation : Identité

- Initialisation de la matrice de poids et de biais : Xavier Glorot
- Méthode numérique pour l'optimisation des poids et des biais : L-BFGS
- Méthode de régularisation : L2, alpha = 0.001
- Stratégie d'apprentissage : recherche par graines
- Fonction de coût : erreur quadratique moyenne

**3.11 Dosage de l'edoxaban (méthodologie optimisée AODs)**

**3.11.1 Jeux de données**

Les données relatives au Lixiana® (edoxaban) générées sont :

- 72 cinétiques mesurées en janvier 2016 sur le STA-R® AUT00460

  - pool normal de plasmas surchargé par 0, 16, 21, 30, 39, 51, 76, 100, 128, 142, 155, 187, 215, 245, 269, 284, 322, 347, 359, 380, 393, 410, 426 et 436 ng/mL ;
  - chaque niveau d'échantillon est testé en $n$=3 ;

- les échantillons ont été préparés et stockés à -80°C.

- 72 cinétiques mesurées en juin 2016 sur le STA-R® AUT00722

- pool normal de plasmas surchargé par 0, 16, 21, 30, 39, 51, 76, 100, 128, 142, 155, 187, 215, 245, 269, 284, 322, 347, 359, 380, 393, 410, 426 et 436 ng/mL ;
- chaque niveau d'échantillon est testé en $n$=3 ;
- les échantillons ont été préparés et stockés à -80°C.

## 3.11.2 Modèle d'apprentissage automatique

**Organisation des données**

Le modèle d'apprentissage automatique est entraîné par une validation croisée organisée de la manière suivante :

- Données d'apprentissage : données edoxaban (Lixiana®) générées sur le STA-R® AUT00460.
- Données de validation : données edoxaban (Lixiana®) générées sur le STA-R® AUT00722.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 42 neurones
    - Fonctions d'activation : Identité

  - Couche cachée

    - 29 neurones
    - Fonctions d'activation : ReLU

  - Couche cachée

    - 16 neurones
    - Fonctions d'activation : ReLU

  - Couche de sortie

    - 1 neurone
    - Fonction d'activation : Identité

- Initialisation de la matrice de poids et de biais : Xavier Glorot
- Méthode numérique pour l'optimisation des poids et des biais : L-BFGS
- Méthode de régularisation : L2, alpha = 0.01
- Stratégie d'apprentissage : recherche par graines
- Fonction de coût : erreur quadratique moyenne

## 3.12 Dosages de l'edoxaban (méthodologie universelle ou « méthodologie améliorée sur la base de la méthodologie universelle »)

**3.12.1 Jeux de données**

Les données relatives au Lixiana® (edoxaban) ont été générées lors de deux études distinctes. Concernant la première étude, les données générées sont :

- 63 cinétiques mesurées le 17.10.2017 sur le STA-R® AUT00603 (soft version 3.04.07)

    - pool normal de plasmas lot 03.2017 surchargé par 0, 10, 20, 28, 40, 49, 64, 74, 95, 103, 109, 122, 132, 144, 131, 132, 157, 171, 195, 191 et 201 ng/mL ;
    - chaque niveau d'échantillon est testé en $n$=3 ;
    - les échantillons ont été préparés et stockés à -80°C.

- 63 cinétiques mesurées le 17.10.2017 sur le STA-R® AUT05450 (soft version 3.04.07)

    - pool normal de plasmas lot 03.2017 surchargé par 0, 10, 21, 28, 41, 49, 62, 74, 92, 102, 112, 120, 133, 142, 129, 133, 155, 168, 188, 200 et 206 ng/mL ;
    - chaque niveau d'échantillon est testé en $n$=3 ;
    - les échantillons ont été préparés et stockés à -80°C.

- 63 cinétiques mesurées le 21.09.2017 sur le STA-R® AUT06366 (soft version 3.04.07)

    - pool normal de plasmas lot 03.2017 surchargé par 0, 10, 21, 27, 40, 49, 64, 73, 90, 99, 108, 122, 132, 144, 130, 136, 153, 169, 192, 194 et 216 ng/mL ;
    - chaque niveau d'échantillon est testé en $n$=3 ;
    - les échantillons ont été préparés et stockés à -80°C.

- 63 cinétiques mesurées le 20.09.2017 sur le STA-R® AUT06399 (soft version 3.04.07)

    - pool normal de plasmas lot 03.2017 surchargé par 0, 10, 21, 29, 39, 48, 60, 73, 81, 92, 107, 115, 129, 136 ,129, 127, 149, 163, 181, 195 et 199 ng/mL ;
    - chaque niveau d'échantillon est testé en $n$=3 ;
    - les échantillons ont été préparés et stockés à -80°C.

Pour cette première étude, le STA® - Liquid Anti-Xa lot 251738 a été utilisé pour la génération des cinétiques en méthodologie universelle et pour le dosage commercial de la surcharge d'edoxaban. Concernant la deuxième étude, les données générées sont :

- 63 cinétiques mesurées le 06.05.2019 sur le STA-R® AUT05676 (soft version 3.04.07)

    - pool normal de plasmas lot 19059RD (code 22824) surchargé par 0, 12, 19, 27, 36, 50, 59, 70, 81, 89, 100, 112, 119, 136, 145, 142, 163, 176, 186, 198 et 207 ng/mL ;
    - chaque niveau d'échantillons est testé en $n$=3 ;
    - les échantillons ont été préparés et stockés à -80°C, puis testés simultanément sur les trois automates.

- 63 cinétiques mesurées le 06.05.2019 sur le STA-R® AUT05980 (soft version 3.04.07)

    - pool normal de plasmas lot 19059RD (code 22824) surchargé par 0, 13, 20, 29, 37, 50, 61, 71, 83, 88, 95, 108, 115, 134, 141, 137, 161, 169, 176, 188 et 198 ng/mL ;
    - chaque niveau d'échantillons est testé en $n$=3 ;
    - les échantillons ont été préparés et stockés à -80°C, puis testés simultanément sur les trois automates.

- 63 cinétiques mesurées le 06.05.2019 sur le STA-R® AUT06366 (soft version 3.04.07)

    - pool normal de plasmas lot 19059RD (code 22824) surchargé par 0, 14, 20, 29, 35, 52, 58, 70, 77, 85, 97, 107, 114, 131, 140, 136, 165, 170, 177, 189 et 200 ng/mL ;
    - chaque niveau d'échantillons est testé en $n$=3 ;
    - les échantillons ont été préparés et stockés à -80°C, puis testés simultanément sur les trois automates.

Pour cette deuxième étude, le STA® - Liquid Anti-Xa lot 253225 a été utilisé pour la génération des cinétiques en méthodologie universelle et pour le dosage commercial de la surcharge d'edoxaban.

### 3.12.2 Modèle d'apprentissage automatique

### Organisation des données

Le modèle d'apprentissage automatique est entraîné par une validation croisée divisée en sept sousensembles de la manière suivante :

- Sous-ensemble 1 :

  - Données d'apprentissage :

    - données edoxaban (Lixiana®) générées sur le STA-R® AUT00603,
    - données edoxaban (Lixiana®) générées sur le STA-R® AUT05450,
    - données edoxaban (Lixiana®) générées sur le STA-R® AUT05676,
    - données edoxaban (Lixiana®) générées sur le STA-R® AUT05980,
    - données edoxaban (Lixiana®) générées sur le STA-R® AUT06366,
    - données edoxaban (Lixiana®) générées sur le STA-R® AUT06366 (bis).

  - Données de validation : données edoxaban (Lixiana®) générées sur le STA-R® AUT06399.

- Sous-ensemble 2 :

  - Données d'apprentissage :

    - données edoxaban (Lixiana®) générées sur le STA-R® AUT00603,
    - données edoxaban (Lixiana®) générées sur le STA-R® AUT05450,
    - données edoxaban (Lixiana®) générées sur le STA-R® AUT05676,
    - données edoxaban (Lixiana®) générées sur le STA-R® AUT05980,
    - données edoxaban (Lixiana®) générées sur le STA-R® AUT06366,
    - données edoxaban (Lixiana®) générées sur le STA-R® AUT06399.

  - Données de validation : données edoxaban (Lixiana®) générées sur le STA-R® AUT06366 (bis).

- Sous-ensemble 3 :

  - Données d'apprentissage :

    - données edoxaban (Lixiana®) générées sur le STA-R® AUT00603,
    - données edoxaban (Lixiana®) générées sur le STA-R® AUT05450,
    - données edoxaban (Lixiana®) générées sur le STA-R® AUT05676,
    - données edoxaban (Lixiana®) générées sur le STA-R® AUT05980,
    - données edoxaban (Lixiana®) générées sur le STA-R® AUT06366 (bis),
    - données edoxaban (Lixiana®) générées sur le STA-R® AUT06399.

  - Données de validation : données edoxaban (Lixiana®) générées sur le STA-R® AUT06366.

- Sous-ensemble 4 :

  - Données d'apprentissage :

    - données edoxaban (Lixiana®) générées sur le STA-R® AUT00603,
    - données edoxaban (Lixiana®) générées sur le STA-R® AUT05450,
    - données edoxaban (Lixiana®) générées sur le STA-R® AUT05676,
    - données edoxaban (Lixiana®) générées sur le STA-R® AUT06366,
    - données edoxaban (Lixiana®) générées sur le STA-R® AUT06366 (bis),

- données edoxaban (Lixiana®) générées sur le STA-R® AUT06399.

  - Données de validation : données edoxaban (Lixiana®) générées sur le STA-R® AUT05980.

- Sous-ensemble 5 :

  - Données d'apprentissage :

    - données edoxaban (Lixiana®) générées sur le STA-R® AUT00603,
    - données edoxaban (Lixiana®) générées sur le STA-R® AUT05450,
    - données edoxaban (Lixiana®) générées sur le STA-R® AUT05980,
    - données edoxaban (Lixiana®) générées sur le STA-R® AUT06366,
    - données edoxaban (Lixiana®) générées sur le STA-R® AUT06366 (bis),
    - données edoxaban (Lixiana®) générées sur le STA-R® AUT06399.

  - Données de validation : données edoxaban (Lixiana®) générées sur le STA-R® AUT05676.

- Sous-ensemble 6 :

  - Données d'apprentissage :

    - données edoxaban (Lixiana®) générées sur le STA-R® AUT00603,
    - données edoxaban (Lixiana®) générées sur le STA-R® AUT05676,
    - données edoxaban (Lixiana®) générées sur le STA-R® AUT05980,
    - données edoxaban (Lixiana®) générées sur le STA-R® AUT06366,
    - données edoxaban (Lixiana®) générées sur le STA-R® AUT06366 (bis),
    - données edoxaban (Lixiana®) générées sur le STA-R® AUT06399.

  - Données de validation : données edoxaban (Lixiana®) générées sur le STA-R® AUT05450.

- Sous-ensemble 7 :

  - Données d'apprentissage :

    - données edoxaban (Lixiana®) générées sur le STA-R® AUT05450,
    - données edoxaban (Lixiana®) générées sur le STA-R® AUT05676,
    - données edoxaban (Lixiana®) générées sur le STA-R® AUT05980,
    - données edoxaban (Lixiana®) générées sur le STA-R® AUT06366,
    - données edoxaban (Lixiana®) générées sur le STA-R® AUT06366 (bis),
    - données edoxaban (Lixiana®) générées sur le STA-R® AUT06399.

  - Données de validation : données edoxaban (Lixiana®) générées sur le STA-R® AUT00603.

L'apprentissage final se fait sur l'intégralité des données.

**Description du modèle d'apprentissage automatique**

Un perceptron multi-couches (réseau de neurones) a été entraîné selon la stratégie décrite dans la section précédente. Il est défini par les paramètres et hyperparamètres suivants :

- Prétraitement des données : normalisation entre 0 et 1
- Modèle d'apprentissage automatique : Perceptrons multicouches
- Hyper-paramètres :

  - Couche d'entrée

    - 77 neurones
    - Fonctions d'activation : Identité

- Couche cachée

  - 40 neurones
  - Fonctions d'activation : ReLU

- Couche de sortie

  - 1 neurone
  - Fonction d'activation : Identité

- Initialisation de la matrice de poids et de biais : Xavier Glorot
- Méthode numérique pour l'optimisation des poids et des biais : L-BFGS
- Méthode de régularisation : L2, alpha = 0.001
- Stratégie d'apprentissage : recherche par graines
- Fonction de coût : erreur quadratique moyenne

## 4 Résultats de la deuxième étude (données de la section 3. ci-dessus)

Dans cette section, nous listons les résultats des performances obtenues par les différents modèles d'apprentissage automatique de la cascade de la figure **Erreur** ! **Source du renvoi introuvable.6** sur des données mesurées sur des échantillons vrais en utilisant la méthodologie expérimentale décrite ici. Chaque sous-section détaille les données mesurées ainsi que les performances obtenues. Une mesure en simplicat suffit pour rendre un résultat ; toutefois, comme démontré dans cette section, une mesure en triplicat peut améliorer les performances.

### 4.1 Détection de la présence ou de l'absence d'un anticoagulant anti-Xa

Dans cette section, nous donnons les résultats obtenus pour la détection de la présence ou de l'absence d'un anticoagulant anti-Xa en utilisant la méthode décrite dans la section 3. ci-dessus.

### 4.1.1 Données de test

39 échantillons identifiés normaux sur la base de leurs résultats en TP, TCA et Fibrinogène, 24 échantillons congelés de patients traités par héparine non fractionnée (HNF), 62 échantillons congelés de patients traités par héparine de bas poids moléculaire (HBPM), 44 échantillons congelés de patients traités par Xarelto® (rivaroxaban), 37 échantillons congelés de patients traités par Eliquis® (apixaban) et 42 échantillons congelés de patients traités par Lixiana® (edoxaban) ont été testés.

Les données ont été générées sur le STA-R® AUT06399 (soft version 3.04.07) avec le STA® - Liquid Anti-Xa (lot 251187) où chaque échantillon est testé en n=3 avec la méthodologie universelle.

Au final, l'analyse en simplicat se fait sur 248 cinétiques mesurées sur 248 échantillons et l'analyse en triplicat se fait sur 744 cinétiques mesurées sur 248 échantillons.

### 4.1.2 Résultats

Les tableaux 9 et 10 donnent respectivement les matrices de confusion associées à la détection de la présence ou de l'absence d'un anticoagulant anti-Xa lorsque l'analyse est réalisée en simplicat et lorsque l'analyse est réalisée en triplicat sur les données du jeu de test. Les résultats pour la détection de la présence d'un anticoagulant anti-Xa donnent une précision de 100 % que l'analyse soit effectuée en simplicat ou en triplicat. Les résultats pour la détection de l'absence d'un anticoagulant anti-Xa donnent une précision de 97,44 % que l'analyse soit effectuée en simplicat ou en triplicat.

TAB. 9: **Présence ou absence d'un anticoagulant anti-Xa : matrice de confusion. Analyse en simplicat.** Les résultats donnent une précision de 100 % pour la détection de la présence d'un anticoagulant anti-Xa. Les résultats donnent une précision de 97,44 % pour la détection de l'absence d'un anticoagulant anti-Xa.

| | | predicted outcome | |
| --- | --- | --- | --- |
| | | Présence d'un anti-Xa | Absence d'anti-Xa |
| **actual value** | Présence d'un anti-Xa | 209 | 0 |
| | Absence d'anti-Xa | 1 | 38 |

TAB. 10: **Présence ou absence d'un anticoagulant anti-Xa : matrice de confusion. Analyse en triplicat.** Les résultats donnent une précision de 100 % pour la détection de la présence d'un anticoagulant anti-Xa. Les résultats donnent une précision de 97,44 % pour la détection de l'absence d'un anticoagulant anti-Xa.

| | | predicted outcome | |
|---|---|---|---|
| | | Présence d'un anti-Xa | Absence d'anti-Xa |
| **actual value** | Présence d'un anti-Xa | 209 | 0 |
| | Absence d'anti-Xa | 1 | 38 |

### 4.2 Identification de la catégorie de l'anticoagulant anti-Xa

Dans cette section, nous donnons les résultats obtenus pour l'identification de la catégorie de l'anticoagulant anti-Xa en utilisant la méthode décrite dans la section 3. ci-dessus.

### 4.2.1 Données de test

24 échantillons congelés de patients traités par héparine non fractionnée (HNF), 62 échantillons congelés de patients traités par héparine de bas poids moléculaire (HBPM), 44 échantillons congelés de patients traités par Xarelto® (rivaroxaban), 37 échantillons congelés de patients traités par Eliquis® (apixaban) et 42 échantillons congelés de patients traités par Lixiana® (edoxaban) ont été testés.
Les données ont été générées sur le STA-R® AUT06399 (soft version 3.04.07) avec le STA® - Liquid Anti-Xa (lot 251187) où chaque échantillon est testé en n=3 avec la méthodologie universelle.
Au final, l'analyse en simplicat se fait sur 209 cinétiques mesurées sur 209 échantillons et l'analyse en triplicat se fait sur 627 cinétiques mesurées sur 209 échantillons.

### 4.2.2 Résultats

Les tableaux 11 et 12 donnent respectivement les matrices de confusion associées à l'identification de la catégorie de l'anticoagulant anti-Xa lorsque l'analyse est réalisée en simplicat et lorsque l'analyse est réalisée en triplicat sur les données du jeu de test. Les résultats pour l'identification de la catégorie de l'anticoagulant anti-Xa donnent une précision de 100 % lorsque l'analyse est effectuée en simplicat et une précision de 100 % quand l'analyse est effectuée en triplicat.

TAB. 11: **Identification de la catégorie de l'anticoagulant anti-Xa : matrice de confusion. Analyse en simplicat.** Les résultats donnent une précision de 100 % pour l'identification de la catégorie de l'anticoagulant anti-Xa.

| | | predicted outcome | |
|---|---|---|---|
| | | Héparine | AOD |
| **actual value** | Héparine | 86 | 0 |
| | AOD | 0 | 123 |

TAB. 12: **Identification de la catégorie de l'anticoagulant anti-Xa : matrice de confusion. Analyse en triplicat.** Les résultats donnent une précision de 100 % pour l'identification de la catégorie de l'anticoagulant anti-Xa.

| | | predicted outcome | |
|---|---|---|---|
| | | Héparine | AOD |
| **actual value** | Héparine | 86 | 0 |
| | AOD | 0 | 123 |

### 4.3 Identification des héparines

Dans cette section, nous donnons les résultats obtenus pour l'identification des héparines en utilisant la méthode décrite dans la section 3. ci-dessus.

### 4.3.1 Données de test

24 échantillons congelés de patients traités par héparine non fractionnée (HNF) et 62 échantillons congelés de patients traités par héparine de bas poids moléculaire (HBPM) ont été testés.

Les données ont été générées sur le STA-R® AUT06399 (soft version 3.04.07) avec le STA® - Liquid Anti-Xa (lot 251187) où chaque échantillon est testé en n=3 avec la méthodologie universelle.

Au final, l'analyse en simplicat se fait sur 86 cinétiques mesurées sur 86 échantillons et l'analyse en triplicat se fait sur 258 cinétiques mesurées sur 86 échantillons.

### 4.3.2 Résultats

Les tableaux 13 et 14 donnent respectivement les matrices de confusion associées à l'identification des héparines lorsque l'analyse est réalisée en simplicat et lorsque l'analyse est réalisée en triplicat sur les données du jeu de test. Les résultats pour l'identification des héparines donnent une précision de 91,86 % lorsque l'analyse est effectuée en simplicat et une précision de 90,70 % quand l'analyse est effectuée en triplicat.

TAB. 13: **Identification des héparines : matrice de confusion. Analyse en simplicat.** Les résultats donnent une précision de 91,86 % pour l'identification des héparines.

| | | predicted outcome | |
| --- | --- | --- | --- |
| | | HNF | HBPM |
| **actual value** | HNF | 21 | 3 |
| | HBPM | 4 | 58 |

TAB. 14: **Identification des héparines : matrice de confusion. Analyse en triplicat.** Les résultats donnent une précision de 90,70 % pour l'identification des héparines.

| | | predicted outcome | |
| --- | --- | --- | --- |
| | | HNF | HBPM |
| **actual value** | HNF | 21 | 3 |
| | HBPM | 5 | 57 |

### 4.4 Dosages des héparines non fractionnées

Dans cette section, nous donnons les résultats des dosages des taux en HNF sur des échantillons patients obtenus en utilisant la méthode décrite dans la section 3. ci-dessus.en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

### 4.4.1 Données de test

24 échantillons congelés de patients traités par héparine non fractionnée (HNF) ont été testés.

Les données ont été générées sur le STA-R® AUT06399 (soft version3.04.07) avec le STA® - Liquid Anti-Xa (lot 251187) dans les conditions suivantes :

- chaque échantillon est testé en *n*=3 avec la méthodologie universelle ;
- chaque échantillon est testé en *n*=2 avec la méthode de référence (méthodologie commerciale du STA® - Liquid Anti-Xa). Le taux de référence utilisé dans la comparaison de méthodes est donc un taux vérifié après congélation de l'échantillon.

Au final, l'analyse en simplicat se fait sur 24 cinétiques mesurées sur 24 échantillons et l'analyse en triplicat se fait sur 72 cinétiques mesurées sur 24 échantillons.

### 4.4.2 Résultats

La figure 17 donne les résultats de comparaison des dosages des taux en HNF mesurés en utilisant l'approche décrite dans ce document aux taux en HNF mesurés en utilisant l'approche standard sur les données du jeu de test. Ces comparaisons donnent une droite d'équation $y$=1.04x-0.03 et un coefficient de détermination $R^2$=0.9851 lorsque l'analyse est réalisée en simplicat ; elles donnent une droite d'équation $y$=1.04x-0.03 et un coefficient $R^2$=0.9856 lorsque l'analyse est réalisée en triplicat.

**4.5 Dosages des héparines de bas poids moléculaires**

Dans cette section, nous donnons les résultats des dosages des taux en HBPM sur des échantillons patients obtenus en utilisant l'invention décrite dans ce document en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

**4.5.1 Données de test**

62 échantillons congelés de patients traités par héparine de bas poids moléculaire (HBPM) ont été testés.
Les données ont été générées sur le STA-R® AUT06399 (soft version3.04.07) avec le STA® - Liquid Anti-Xa (lot 251187) dans les conditions suivantes :

- chaque échantillon est testé en n=3 avec la méthodologie universelle ;
- chaque échantillon est testé en $n$=2 avec la méthode de référence (méthodologie commerciale du STA® - Liquid Anti-Xa). Le taux de référence utilisé dans la comparaison de méthodes est donc un taux vérifié après congélation de l'échantillon.

Au final, l'analyse en simplicat se fait sur 62 cinétiques mesurées sur 62 échantillons et l'analyse en triplicat se fait sur 186 cinétiques mesurées sur 62 échantillons.

**4.5.2 Résultats**

La figure 18 donne les résultats de comparaison des dosages des taux en HBPM mesurés en utilisant l'approche décrite dans ce document aux taux en HBPM mesurés en utilisant l'approche standard sur les données du jeu de test. Ces comparaisons donnent une droite d'équation $y$=1.02x-0.02 et un cœfficient de détermination $R^2$=0.996 lorsque l'analyse est réalisée en simplicat ; elles donnent une droite d'équation $y$=1.027x-0.03 et un cœfficient de détermination $R^2$=0.9971 lorsque l'analyse est réalisée en triplicat.

**4.6 Identification de l'AOD anti-Xa (méthodologie optimisée AODs)**

Dans cette section, nous donnons les résultats obtenus pour l'identification des AODs anti-Xa en utilisant l'invention décrite dans ce document.

**4.6.1 Données de test**

62 échantillons congelés de patients traités par Xarelto® (rivaroxaban), 45 échantillons congelés de patients traités par Eliquis® (apixaban) et 56 échantillons congelés de patients traités par Lixiana® (edoxaban) ont été testés.
Les données ont été générées sur le STA-R® AUT06399 (soft version 3.04.07) avec le STA® - Liquid Anti-Xa (lot 251187) où chaque échantillon est testé en $n$=3 avec la méthodologie optimisée AODs. Au final, l'analyse en simplicat se fait sur 163 cinétiques mesurées sur 163 échantillons et l'analyse en triplicat se fait sur 489 cinétiques mesurées sur 163 échantillons.

**4.6.2 Résultats**

Les tableaux 15 et 16 donnent respectivement les matrices de confusion associées à l'identification des AODs anti-Xa lorsque l'analyse est réalisée en simplicat et lorsque l'analyse est réalisée en triplicat sur les données du jeu de test. Les résultats pour l'identification des AODs anti-Xa donnent une précision de 92,64 % lorsque l'analyse est effectuée en simplicat et une précision de 97,55 % quand l'analyse est effectuée en triplicat.

TAB. 15: **Identification des AODs anti-Xa : matrice de confusion. Analyse en simplicat.** Les résultats donnent une précision de 92,64 % pour l'identification des AODs anti-Xa.

| | | predicted outcome | | |
|---|---|---|---|---|
| | | rivaroxaban | apixaban | edoxaban |
| **actual value** | rivaroxaban | 59 | 0 | 3 |
| | apixaban | 0 | 45 | 0 |
| | edoxaban | 9 | 0 | 47 |

TAB. 16: **Identification des AODs anti-Xa : matrice de confusion. Analyse en triplicat.** Les résultats donnent une précision de 97,55 % pour l'identification des AODs anti-Xa.

| | | predicted outcome | | |
|---|---|---|---|---|
| | | rivaroxaban | apixaban | edoxaban |
| **actual value** | rivaroxaban | 62 | 0 | 0 |
| | apixaban | 0 | 45 | 0 |
| | edoxaban | 4 | 0 | 52 |

## 4.7 Dosage du rivaroxaban (méthodologie optimisée AODs)

Dans cette section, nous donnons les résultats des dosages des taux en rivaroxaban sur des échantillons patients obtenus en utilisant l'invention décrite dans ce document (méthodologie optimisée AODs) en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

### 4.7.1 Données de test

62 échantillons congelés de patients traités par Xarelto® (rivaroxaban) ont été testés.
Les données ont été générées sur le STA-R® AUT06399 (soft version 3.04.07) avec le STA® - Liquid Anti-Xa (lot 251187) dans les conditions suivantes :

- chaque échantillon est testé en $n$=3 avec la méthodologie optimisée AODs ;
- chaque échantillon est testé en $n$=2 avec la méthode de référence (méthodologie commerciale du STA® - Liquid Anti-Xa). Le taux de référence utilisé dans la comparaison de méthodes est donc un taux vérifié après congélation de l'échantillon.

Au final, l'analyse en simplicat se fait sur 62 cinétiques mesurées sur 62 échantillons et l'analyse en triplicat se fait sur 186 cinétiques mesurées sur 62 échantillons.

### 4.7.2 Résultats

La figure 19 donne les résultats de comparaison des dosages des taux en rivaroxaban mesurés en utilisant l'approche décrite dans ce document aux taux en rivaroxaban mesurés en utilisant l'approche standard sur les données du jeu de test. Ces comparaisons donnent une droite d'équation $y$=1.05x+19.65 et un cœfficient de détermination $R^2$=0.991 lorsque l'analyse est réalisée en simplicat ; elles donnent une droite d'équation $y$=1.04x+20.3 et un cœfficient de détermination $R^2$=0.9934 lorsque l'analyse est réalisée en triplicat.

## 4.8 Dosage du rivaroxaban (méthodologie universelle ou « méthodologie améliorée sur la base de la méthodologie universelle »)

Dans cette section, nous donnons les résultats des dosages des taux en rivaroxaban sur des échantillons patients obtenus en utilisant l'invention décrite dans ce document (méthodologie universelle) en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

**4.8.1 Données de test**

44 échantillons congelés de patients traités par Xarelto® (rivaroxaban) ont été testés.

Les données ont été générées sur le STA-R® AUT06399 (soft version 3.04.07) avec le STA® - Liquid Anti-Xa (lot 251187) dans les conditions suivantes :

- chaque échantillon est testé en n=3 avec la méthodologie universelle ;
- chaque échantillon est testé en *n=2* avec la méthode de référence (méthodologie commerciale du STA® - Liquid Anti-Xa). Le taux de référence utilisé dans la comparaison de méthodes est donc un taux vérifié après congélation de l'échantillon.

Au final, l'analyse en simplicat se fait sur 44 cinétiques mesurées sur 44 échantillons et l'analyse en triplicat se fait sur 132 cinétiques mesurées sur 44 échantillons.

**4.8.2 Résultats**

La figure 20 donne les résultats de comparaison des dosages des taux en rivaroxaban mesurés en utilisant l'approche décrite dans ce document aux taux en rivaroxaban mesurés en utilisant l'approche standard sur les données du jeu de test. Ces comparaisons donnent une droite d'équation $y=1.04x+1.74$ et un coefficient de détermination $R^2=0.985$ lorsque l'analyse est réalisée en simplicat ; elles donnent une droite d'équation $y=1.032x+1.99$ et un coefficient $R^2=0.989$ lorsque l'analyse est réalisée en triplicat.

**4.9 Dosage de l'apixaban (méthodologie optimisée AODs)**

Dans cette section, nous donnons les résultats des dosages des taux en apixaban sur des échantillons patients obtenus en utilisant l'invention décrite dans ce document (méthodologie optimisée AODs) en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

**4.9.1 Données de test**

45 échantillons congelés de patients traités par Eliquis® (apixaban) ont été testés.

Les données ont été générées sur le STA-R® AUT06399 (soft version 3.04.07) avec le STA® - Liquid Anti-Xa (lot 251187) dans les conditions suivantes :

- chaque échantillon est testé en *n*=3 avec la méthodologie optimisée AODs ;
- chaque échantillon est testé en *n=2* avec la méthode de référence (méthodologie commerciale du STA® - Liquid Anti-Xa). Le taux de référence utilisé dans la comparaison de méthodes est donc un taux vérifié après congélation de l'échantillon.

Au final, l'analyse en simplicat se fait sur 45 cinétiques mesurées sur 45 échantillons et l'analyse en triplicat se fait sur 135 cinétiques mesurées sur 45 échantillons.

**4.9.2 Résultats**

La figure 21 donne les résultats de comparaison des dosages des taux en apixaban mesurés en utilisant l'approche décrite dans ce document aux taux en apixaban mesurés en utilisant l'approche standard sur les données du jeu de test. Ces comparaisons donnent une droite d'équation $y=1.14x-6.73$ et un cœfficient de détermination $R^2=0.9945$ lorsque l'analyse est réalisée en simplicat ; elles donnent une droite d'équation $y=1.13x-5.46$ et un cœfficient de détermination $R^2=0.9958$ lorsque l'analyse est réalisée en triplicat.

**4.10 Dosage de l'apixaban (méthodologie universelle ou** « méthodologie améliorée sur la base de la méthodologie universelle »)

Dans cette section, nous donnons les résultats des dosages des taux en apixaban sur des échantillons patients obtenus en utilisant l'invention décrite dans ce document (méthodologie universelle) en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

### 4.10.1 Données de test

37 échantillons congelés de patients traités par Eliquis® (apixaban) ont été testés.
Les données ont été générées sur le STA-R® AUT06399 (soft version 3.04.07) avec le STA® - Liquid Anti-Xa (lot 251187) dans les conditions suivantes :

- chaque échantillon est testé en $n$=3 avec la méthodologie universelle ;
- chaque échantillon est testé en *n=2* avec la méthode de référence (méthodologie commerciale du STA® - Liquid Anti-Xa). Le taux de référence utilisé dans la comparaison de méthodes est donc un taux vérifié après congélation de l'échantillon.

Au final, l'analyse en simplicat se fait sur 37 cinétiques mesurées sur 37 échantillons et l'analyse en triplicat se fait sur 111 cinétiques mesurées sur 37 échantillons.

### 4.10.2 Résultats

La figure 22 donne les résultats de comparaison des dosages des taux en apixaban mesurés en utilisant l'approche décrite dans ce document aux taux en apixaban mesurés en utilisant l'approche standard sur les données du jeu de test. Ces comparaisons donnent une droite d'équation $y$=1.041$x$-2.66 et un coefficient de détermination $R^2$=0.9792 lorsque l'analyse est réalisée en simplicat ; elles donnent une droite d'équation $y$=1.041$x$-2.41 et un coefficient $R^2$=0.9877 lorsque l'analyse est réalisée en triplicat.

### 4.11 Dosage de l'edoxaban (méthodologie optimisée AODs)

Dans cette section, nous donnons les résultats des dosages des taux en edoxaban sur des échantillons patients obtenus en utilisant l'invention décrite dans ce document (méthodologie optimisée AODs) en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

### 4.11.1 Données de test

56 échantillons congelés de patients traités par Lixiana® (edoxaban) ont été testés.
Les données ont été générées sur le STA-R® AUT06399 (soft version 3.04.07) avec le STA® - Liquid Anti-Xa (lot 251187) dans les conditions suivantes :

- chaque échantillon est testé en $n$=3 avec la méthodologie optimisée AODs ;
- chaque échantillon est testé en $n$=2 avec la méthode de référence avec la méthodologie commerciale du STA® - Liquid Anti-Xa. Le taux de référence utilisé dans la comparaison de méthodes est donc un taux vérifié après congélation de l'échantillon.

Au final, l'analyse en simplicat se fait sur 56 cinétiques mesurées sur 56 échantillons et l'analyse en triplicat se fait sur 168 cinétiques mesurées sur 56 échantillons.

### 4.11.2 Résultats

La figure 23 donne les résultats de comparaison des dosages des taux en edoxaban mesurés en utilisant l'approche décrite dans ce document aux taux en edoxaban mesurés en utilisant l'approche standard sur les données du jeu de test. Ces comparaisons donnent une droite d'équation $y$=0.905$x$+12.35 et un cœfficient de détermination $R^2$=0.9853 lorsque l'analyse est réalisée en simplicat ; elles donnent une droite d'équation $y$=0.93$x$+8.18 et un cœfficient de détermination $R^2$=0.9881 lorsque l'analyse est réalisée en triplicat.

**4.12 Dosage de l'edoxaban (méthodologie universelle ou** « méthodologie améliorée sur la base de la méthodologie universelle »)

Dans cette section, nous donnons les résultats des dosages des taux en edoxaban sur des échantillons patients obtenus en utilisant l'invention décrite dans ce document (méthodologie universelle) en comparaison aux taux mesurés en utilisant l'approche standard (kit commercial STA® - Liquid Anti-Xa). Les résultats sont jugés satisfaisants lorsque la pente de la régression linéaire est comprise entre 0,9 et 1,1 et que le cœfficient de détermination $R^2$ est supérieur ou égal à 0,95 (critères du CLSI EP9-A2).

**4.12.1 Données de test**

42 échantillons congelés de patients traités par Lixiana® (edoxaban) ont été testés.
Les données ont été générées sur le STA-R® AUT06399 (soft version 3.04.07) avec le STA® - Liquid Anti-Xa (lot 251187) dans les conditions suivantes :

- chaque échantillon est testé en $n$=3 avec la méthodologie universelle ;
- chaque échantillon est testé en $n$=2 avec la méthode de référence (méthodologie commerciale du STA® - Liquid Anti-Xa). Le taux de référence utilisé dans la comparaison de méthodes est donc un taux vérifié après congélation de l'échantillon.

Au final, l'analyse en simplicat se fait sur 42 cinétiques mesurées sur 42 échantillons et l'analyse en triplicat se fait sur 126 cinétiques mesurées sur 42 échantillons.

**4.12.2 Résultats**

La figure 24 donne les résultats de comparaison des dosages des taux en edoxaban mesurés en utilisant l'approche décrite dans ce document aux taux en edoxaban mesurés en utilisant l'approche standard sur les données du jeu de test. Ces comparaisons donnent une droite d'équation $y$=0.9431$x$+4.51 et un coefficient de détermination $R^2$=0.9968 lorsque l'analyse est réalisée en simplicat ; elles donnent une droite d'équation $y$=0.9442x+4.26 et un coefficient $R^2$=0.9962 lorsque l'analyse est réalisée en triplicat.

## INHIBITEURS

I Inhibiteurs du facteur Xa

Les tableaux 17 et 18 listent respectivement les inhibiteurs naturels et synthétiques du facteur Xa connus à ce jour.

[Table 17]

Table 17 : Inhibiteurs naturels du facteur Xa

|  | Directs | Indirects |
|---|---|---|
| **Réversibles** | TFPI | Protéine S |
| **Irréversibles** | Antithrombine Protéase nexine-1 | |

[Table 18]

Table 18 : Inhibiteurs synthétiques du facteur Xa

|  | Directs | Indirects |
|---|---|---|
| **Réversibles** | Rivaroxaban<br>Apixaban<br>Edoxaban<br>Bétrixaban | |
| **Irréversibles** | | HNF<br>HBPM<br>Pentasaccharides<br>Danaparoïde sodique (Orgaran) |

II Inhibiteurs du facteur IIa

Les tableaux 19 et 20 listent respectivement les inhibiteurs naturels et synthétiques du facteur IIa connus à ce jour.

[Table 19]

Table 19 : Inhibiteurs naturels du facteur IIa

|  | Directs | Indirects |
|---|---|---|
| **Réversibles** | | |
| **Irréversibles** | Antithrombine<br>Cofacteur II de l'héparine<br>Protéase nexine-1<br>$\alpha$2-macroglobuline | |

[Table 20]

Table 20 : Inhibiteurs synthétiques du facteur IIa

|  | Directs | Indirects |
|---|---|---|
| **Réversibles** | Dabigatran<br>Melagatran<br>Argatroban<br>Bivalirudine | |
| **Irréversibles** | Hirudine<br>Lépirudine<br>Désirudine<br>Antithrombine (Aclotine®) | HNF<br><br>HBPM |

**Références**

Géron, A. (2017). Hands-On Machine Learning with Scikit-Learn and TensorFlow: Concepts, Tools, and Techniques to Build Intelligent Systems. O'Reilly.

Segel, I. H. (1993). Enzyme kinetics. Behavior and analysis of rapid equilibrium and steady-state enzyme Systems. Wiley Classics Library.

Bonaccorso, G. (2017). Machine Learning Algorithms: A reference guide to popular algorithms for data science and machine learning. Packt Publishing Limited.

**Revendications**

1. Méthode de détection dans un échantillon biologique, en particulier un échantillon sanguin, de la présence d'un inhibiteur d'une enzyme de la coagulation du sang choisie, indépendamment, parmi le facteur Xa (FXa) et le facteur IIa (FIIa), la méthode comprenant les étapes suivantes:

   a. Mesure d'une ou de plusieurs cinétique(s) de compétition via la réalisation d'un dosage enzymatique compétitif sur un échantillon sanguin préalablement obtenu d'un sujet, ledit dosage étant adapté à la réalisation d'une cinétique compétitive vis-à-vis, soit d'un inhibiteur du facteur Xa, soit d'un inhibiteur du facteur IIa, puis
   b. Fourniture en entrée de la ou des cinétique(s) obtenue(s) dans l'étape a. à un modèle décisionnel de classification A obtenu par entraînement d'un modèle d'apprentissage automatique supervisé, par exemple un modèle choisi parmi l'une des familles suivantes : machine à vecteurs de supports, réseaux de neurones, arbres de décision, méthodes d'ensemble, et modèle des k plus proches voisins, puis

   b.i. Si le modèle décisionnel A exclut la présence d'un inhibiteur de l'enzyme de la coagulation du sang ciblée dans l'échantillon analysé, conclusion de l'absence dudit inhibiteur, optionnellement allocation en sortie par le modèle A, par exemple dans une variable, de l'information correspondante, ou
   b.ii. Si le modèle décisionnel A atteste de la présence d'un inhibiteur de l'enzyme de la coagulation du sang ciblée dans l'échantillon analysé, conclusion de la présence dudit inhibiteur, optionnellement allocation en sortie par le modèle A, par exemple dans une variable, de l'information correspondante.

2. Méthode d'identification dans un échantillon biologique, en particulier un échantillon sanguin, d'un inhibiteur d'une enzyme de la coagulation du sang choisie, indépendamment, parmi le facteur Xa (FXa) et le facteur IIa (FIIa), la méthode comprenant les étapes suivantes:

   1. Mise en œuvre des étapes de la méthode selon la revendication 1, puis
   2. Fourniture en entrée de la ou des cinétique(s) obtenue(s) dans l'étape définie au point a. de la revendication 1, ou étape 1. ci-dessus, et du résultat obtenu en fin d'étape b. ii. de la revendication 1, à un modèle décisionnel de classification B obtenu par entraînement d'un modèle d'apprentissage automatique supervisé, par exemple un modèle choisi parmi l'une des familles suivantes : machine à vecteurs de supports, réseaux de neurones, arbres de décision, méthodes d'ensemble, et modèle des k plus proches voisins, et allocation en sortie par le modèle B de la catégorie de l'inhibiteur à l'une des catégories suivantes: inhibiteur indirect irréversible (héparines), ou inhibiteur direct réversible (AOD), notamment lorsque les jeux de données utilisés pour l'entraînement du modèle B comprennent des données relatives à ces deux catégories d'inhibiteurs, et fourniture en sortie, par exemple par allocation dans une variable, de la catégorie de l'inhibiteur déterminée par le modèle B.

3. Méthode selon la revendication 2, comprenant une étape additionnelle de caractérisation de l'inhibiteur dont la présence a été détectée dans l'étape b. ii de la revendication 1, comme suit: fourniture en entrée de la ou des cinétique(s) obtenue(s) dans l'étape a. de la revendication 1 ou étape 1. de la revendication 2, et de la donnée déterminée, par exemple la variable allouée, à l'étape 2. de la revendication 2 concernant la catégorie d'inhibiteur dont la présence a été détectée, à un modèle décisionnel de classification C obtenu par entraînement d'un modèle d'apprentissage automatique supervisé, par exemple un modèle choisi parmi l'une des familles suivantes : machine à vecteurs de supports, réseaux de neurones, arbres de décision, méthodes d'ensemble, et modèle des k plus proches voisins, et caractérisation en sortie par le modèle C de l'inhibiteur recherché, ce dernier étant identifié parmi:

   a. Dans le cas où la catégorie de l'inhibiteur recherché est celle des héparines : la HNF ou la HBPM, ou
   b. Dans le cas où la catégorie de l'inhibiteur recherché est celle des AOD : le Rivaroxaban, l'Apixaban, l'Edoxaban ou le Dabigatran,

   et fourniture en sortie, par exemple par allocation dans une variable, de la caractérisation de l'inhibiteur déterminée par le modèle C.

4. Méthode selon la revendication 3, comprenant une étape additionnelle de dosage quantitatif de l'inhibiteur caractérisé, dans laquelle sont fournies en entrée à un modèle de régression D, notamment un modèle d'apprentissage automatique supervisé, par exemple un modèle choisi parmi l'une des familles suivantes : machines à vecteurs de support, réseaux de neurones, arbres de décision, méthodes d'ensemble, et modèle des k plus proches voisins, la ou des cinétique(s) obtenue(s) dans l'étape a. de la revendication 1 ou étape 1. de de la revendication 2, et la donnée de caractérisation obtenue suivant la revendication 3 identifiant l'inhibiteur présent dans l'échantillon sanguin analysé, ledit modèle de régression ayant été entrainé sur un jeu de données obtenu dans des conditions de mesure identiques à celles de l'étape a. de la revendication 1 ou étape 1. de la revendication 2, et permettant de déterminer en sortie la concentration de l'inhibiteur identifié dans l'échantillon analysé et optionnellement fourniture en sortie, par exemple par allocation dans une variable, de la concentration déterminée par le modèle D.

5. Méthode selon l'une des revendications 1 à 4, dans laquelle l'inhibiteur recherché est :

    I. un inhibiteur du facteur Xa (FXa) choisi parmi : la HNF, la HBPM, le Rivaroxaban, l'Apixaban, l'Edoxaban, ou
    II. un inhibiteur du facteur IIa (FIIa) choisi parmi : la HNF, la HBPM, le Dabigatran.

6. Méthode selon la revendication 2, appliquée à la recherche d'un inhibiteur du facteur Xa dont la présence a été détectée dans l'étape b. ii de la revendication 1, comprenant l'étape de caractérisation additionnelle suivante:

    I. si la catégorie d'inhibiteur a été allouée, à l'étape 2. de la revendication 2, à la catégorie des héparines, alors :

        I.i. fourniture en entrée à un modèle décisionnel de classification C obtenu par entraînement d'un modèle d'apprentissage automatique supervisé, par exemple un modèle choisi parmi l'une des familles suivantes : machine à vecteurs de supports, réseaux de neurones, arbres de décision, méthodes d'ensemble, et modèle des k plus proches voisins, de ladite donnée déterminée, par exemple la variable allouée, à l'étape 2. de la revendication 2 concernant la catégorie d'inhibiteur dont la présence a été détectée, et de la ou des cinétique(s) compétitives vis-à-vis d'un inhibiteur du facteur Xa, obtenue(s) dans l'étape a. de la revendication 1 ou étape 1. de la revendication 2, et
        I.ii. caractérisation en sortie par le modèle C de l'inhibiteur recherché, ce dernier étant identifié parmi: la HNF ou la HBPM, et fourniture en sortie, par exemple par allocation dans une variable, de la caractérisation de l'inhibiteur déterminée par le modèle C,

    ou, alternativement,
    II. si la catégorie d'inhibiteur a été allouée, à l'étape 2. de la revendication 2, à la catégorie des AOD, alors :

        II.i. réalisation d'une nouvelle mesure d'une ou de plusieurs cinétique(s) de compétition via la réalisation d'un dosage enzymatique compétitif sur un échantillon sanguin obtenu du même sujet, ledit dosage étant adapté à la réalisation d'une cinétique compétitive vis-à-vis d'un inhibiteur du facteur Xa, avec un facteur de dilution de l'échantillon et/ou une durée de mesure adaptés à une situation de compétition impliquant la présence d'AOD inhibant le facteur Xa, en particulier un facteur de dilution et/ou une durée de mesure différent(s) de celui(ceux) employés pour la mesure de la ou des cinétique(s) obtenue(s) dans l'étape a. de la revendication 1 ou étape 1. de de la revendication 2, puis
        II.ii. fourniture en entrée à un modèle décisionnel de classification C obtenu par entraînement d'un modèle d'apprentissage automatique supervisé, par exemple un modèle choisi parmi l'une des familles suivantes : machine à vecteurs de supports, réseaux de neurones, arbres de décision, méthodes d'ensemble, et modèle des k plus proches voisins, de ladite donnée déterminée, par exemple la variable allouée, à l'étape 2. de la revendication 2 concernant la catégorie d'inhibiteur dont la présence a été détectée, et de la ou des cinétique(s) obtenue(s) dans l'étape i. précédente, et
        II.iii. caractérisation en sortie par le modèle C de l'inhibiteur recherché, ce dernier étant identifié parmi: le Rivaroxaban, l'Apixaban, ou l'Edoxaban, et fourniture en sortie, par exemple par allocation dans une variable, de la caractérisation de l'inhibiteur déterminée par le modèle C.

7. Méthode selon la revendication 6, comprenant une étape additionnelle de dosage quantitatif de l'inhibiteur identifié à l'issue des étapes I. ou II. de la revendication 6, dans laquelle, respectivement:

    I. Si l'inhibiteur identifié à l'étape I. de la revendication 6 est une HNF ou une HBPM, alors : fourniture en entrée à un modèle de régression D, notamment un modèle d'apprentissage automatique supervisé, par exemple un modèle choisi parmi l'une des familles suivantes : machines à vecteurs de support, réseaux de neurones, arbres de décision, méthodes d'ensemble, et modèle des k plus proches voisins, ledit modèle ayant été entraîné sur un jeu de données obtenu dans des conditions de mesure identiques à celles de l'étape a. de la revendication 1, ou étape 1. de la revendication 2 de la ou des cinétique(s) obtenue(s) dans l'étape a. de la revendication 1 ou étape 1. de la revendication 2 et de la donnée déterminée à l'étape I.ii de la revendication 6 identifiant l'inhibiteur présent dans l'échantillon sanguin analysé, ledit modèle de régression permettant de déterminer en sortie la concentration en inhibiteur identifié dans l'échantillon analysé, et optionnellement fourniture en sortie, par exemple par allocation dans une variable, de la concentration déterminée par le modèle D, ou

II. Si l'inhibiteur identifié à l'étape II. de la revendication 6 est le Rivaroxaban, l'Apixaban, ou l'Edoxaban, alors : fourniture en entrée à un modèle de régression D, notamment un modèle d'apprentissage automatique supervisé, par exemple un modèle choisi parmi l'une des familles suivantes : machines à vecteurs de support, réseaux de neurones, arbres de décision, méthodes d'ensemble, et modèle des k plus proches voisins, ledit modèle ayant été entrainé sur un jeu de données obtenu dans des conditions de mesure identiques à celles de l'étape II. i. de la revendication 6, de la ou des cinétique(s) obtenue(s) dans l'étape II. i. de la revendication 6 et de la donnée de caractérisation, par exemple la variable allouée, fournie à l'issue de l'étape II. iii. de la revendication 6 identifiant l'inhibiteur présent dans l'échantillon sanguin analysé, ledit modèle de régression permettant de déterminer en sortie la concentration en inhibiteur identifié dans l'échantillon analysé et optionnellement fourniture en sortie, par exemple par allocation dans une variable, de la concentration déterminée par le modèle D.

8. Méthode selon le point II. de la revendication 7, dans laquelle si la concentration en inhibiteur identifié dans l'échantillon analysé, déterminée par le modèle D, est inférieure ou égale à 200 ng/mL, alors fourniture en entrée à un modèle de régression D2, notamment un modèle d'apprentissage automatique supervisé, par exemple un modèle choisi parmi l'une des familles suivantes : machines à vecteurs de support, réseaux de neurones, arbres de décision, méthodes d'ensemble, et modèle des k plus proches voisins, ledit modèle ayant été entrainé sur un jeu de données obtenu dans des conditions de mesure identiques à celles de l'étape a. de la revendication 1, de la ou des cinétique(s) obtenue(s) dans l'étape a. de la revendication 1, ledit modèle de régression D2 permettant de recalculer en sortie la concentration en inhibiteur identifié dans l'échantillon analysé et optionnellement fourniture en sortie, par exemple par allocation dans une variable, de la concentration déterminée par le modèle D2.

9. Méthode selon l'une des revendications 1 à 8, dans laquelle la mesure *in vitro* d'une cinétique de compétition par dosage enzymatique compétitif sur un échantillon sanguin obtenu d'un sujet, comprend les étapes suivantes:

a. fourniture d'un échantillon sanguin dilué ou non, puis
b. ajout à l'échantillon sanguin d'un substrat spécifique soit du facteur Xa soit du facteur IIa, selon l'inhibiteur recherché, notamment un substrat chromogénique ou fluorogénique,
c. incubation avec élévation de la température du mélange obtenu en b. à une température entre 35 et 39 °C, en particulier 37°C,
d. ajout au mélange réactionnel issu de c. de facteur Xa ou de facteur IIa, selon le substrat ajouté à l'étape b., de façon à déclencher la compétition entre une réaction d'inhibition et la réaction enzymatique provoquée,
e. mesure par un instrument, au cours du temps, de la quantité de produit résultant de la transformation du substrat du fait de l'action de l'enzyme analysée sur ce dernier choisi parmi le facteur Xa ou le facteur IIa, le cas échéant via la mesure d'un marqueur associé au substrat, libéré lors de ladite réaction enzymatique et enregistrement de la cinétique obtenue.

10. Méthode selon la revendication 9, dans laquelle le dosage enzymatique compétitif est spécifique du facteur Xa, et où:

a. dans l'étape a. l'échantillon sanguin est un échantillon de plasma dilué au 1/2 dans du tampon Owren Koller,
b. dans l'étape b. le substrat est le réactif MAPA-Gly-Arg-pNA,
c. dans l'étape c. la durée d'incubation est de 240 secondes, à 37°C,
d. le facteur Xa ajouté au mélange dans l'étape d. est du facteur Xa bovin,
e. la mesure de la libération de la paranitroaniline (pNA) dans l'étape e. est réalisée par colorimétrie à 405 nm toutes les deux secondes pendant 156 secondes, sur un instrument approprié, de type STA-R®,

ou, alternativement :

a. dans l'étape a. l'échantillon sanguin est un échantillon de plasma dilué au 1/8ème dans du tampon Owren Koller,
b. dans l'étape b. le substrat est le réactif MAPA-Gly-Arg-pNA,
c. dans l'étape c. la durée d'incubation est de 240 secondes, à 37°C,
d. le facteur Xa ajouté au mélange dans l'étape d. est du facteur Xa bovin,
e. la mesure de la libération de la paranitroaniline (pNA) dans l'étape e. est réalisée par colorimétrie à 405 nm toutes les deux secondes pendant 86 secondes, sur un instrument approprié, de type STA-R®.

11. Méthode selon la revendication 9, dans laquelle le dosage enzymatique compétitif est réalisé sur un dispositif miniaturisé, par exemple un dispositif utilisant la microfluidique, dans un volume réactionnel entre 1 et 20 μL.

12. Méthode selon l'une des revendications 1 à 11, dans laquelle :

a. l'échantillon sanguin est un échantillon de plasma, et/ou

b. la fourniture en entrée à un modèle d'apprentissage automatique supervisé d'une cinétique obtenue expérimentalement consiste à fournir les couples de valeurs constitués par chaque valeur mesurée pour chaque point de mesure discret effectué au cours du temps de mesure.

13. Système de traitement de données ou dispositif pour la détection, dans un échantillon biologique, de la présence ou l'identification d'un inhibiteur du facteur Xa (FXa) ou du facteur IIa (FIIa), ledit système de traitement de données ou dispositif comprenant des moyens pour mettre en œuvre au moins l'étape b. de la revendication 1 et des moyens pour fournir en entrée une ou plusieurs cinétique(s) de compétition obtenues via la réalisation d'un dosage compétitif vis-à-vis, soit d'un inhibiteur du facteur Xa, soit d'un inhibiteur du facteur IIa, sur un échantillon sanguin préalablement obtenu d'un sujet, notamment via un appareil de mesure, et des moyens pour fournir en sortie les variables générées lors de cette étape, et le cas échéant également des moyens pour mettre en œuvre l'étape 2. de la méthode selon la revendication 2, et optionnellement comprenant également des moyens pour fournir en entrée et/ou sortie les variables générées lors de cette étape, et optionnellement comprenant également des moyens pour mettre en œuvre les étapes impliquant les modèles décisionnels de classification ou de régression C, D, et le cas échéant D2, de l'une des revendications 3 à 12, notamment des moyens permettant de faire usage d'une matrice résultant de l'apprentissage ayant permis de fixer les paramètres de ces modèles, pour retourner le résultat de classification ou de régression prenant en compte les variables fournies au(x) modèle(s), et optionnellement comprenant en outre un processeur adapté pour mettre en œuvre les étapes indiquées dans la présente revendication.

14. Programme d'ordinateur comprenant des instructions qui conduisent un système de traitement de données ou un dispositif selon la revendication 13, notamment un dispositif incluant un appareil de mesure de cinétiques telles que définies dans l'une quelconque des revendications 1 ou 6 ou un système de traitement de données ou un dispositif faisant appel à un tel appareil de mesure, notamment distant, à exécuter au moins l'étape b. de la revendication 1, et le cas échéant également l'étape 2. de la méthode selon la revendication 2, et optionnellement conduisant à exécuter les étapes impliquant les modèles décisionnels de classification ou de régression C, D, et le cas échéant D2, de l'une des revendications 3 à 12.

15. Support d'enregistrement lisible par ordinateur comprenant des instructions qui, lorsqu'elles sont exécutées par un ordinateur, conduisent celui-ci à mettre en œuvre au moins l'étape b. de la revendication 1, et le cas échéant également l'étape 2. de la méthode selon la revendication 2, et optionnellement permettent également de récupérer en entrée et/ou fournir en sortie les variables générées lors de de ces étapes, et optionnellement conduisent celui-ci à mettre en œuvre les étapes impliquant les modèles décisionnels de classification ou de régression C, D, et le cas échéant D2, de l'une des revendications 3 à 12.

16. Support de données lisible par ordinateur sur lequel est enregistré le programme d'ordinateur selon la revendication 14, ou signal d'un support de données portant le programme d'ordinateur selon la revendication 14.

17. Kit adapté pour la mise en œuvre d'une méthode selon l'une des revendications 1 à 12, comprenant:

a. Un substrat spécifique du FXa et/ou du FIIa, par exemple le substrat MAPA-Gly-Arg-pNA pour le facteur Xa, par exemple le substrat EtM-SPro-Arg-pNA pour le facteur IIa, et

b. Optionnellement, du FXa et/ou du FIIa, par exemple du facteur Xa bovin ou humain, par exemple du facteur IIa bovin ou humain, et

c. Optionnellement, un ou plusieurs tampons appropriés, par exemple du tampon Owren Koller, par exemple du tampon Tris EDTA et

d. Optionnellement des instructions pour la mise en œuvre de mesure(s) d'une ou de plusieurs cinétique(s) de compétition via la réalisation d'un dosage enzymatique compétitif employant le substrat, et

e. le système et/ou dispositif et/ou programme d'ordinateur et/ou support de données lisible par ordinateur selon l'une des revendications 13 à 15,

f. et optionnellement des instructions permettant de mettre en œuvre la méthode selon l'une des revendications 1 à 12,

g. et optionnellement des instructions relatives à l'utilisation d'un signal d'un support de données selon la revendication 16, pour la mise en œuvre d'une méthode selon l'une des revendications 1 à 12.

# EP 3 891 752 B1

**Patentansprüche**

1. Verfahren zum Nachweis des Vorhandenseins eines Inhibitors eines Blutgerinnungsenzyms, das unabhängig gewählt ist aus dem Faktor Xa (FXa) und dem Faktor IIa (FIIa), in einer biologischen Probe, insbesondere einer Blutprobe, wobei das Verfahren die folgenden Schritte umfasst:

   a. Messung einer oder mehrerer kompetitiver Kinetik(en) mittels Durchführung eines kompetitiven enzymatischen Assays an einer zuvor von einem Subjekt entnommenen Blutprobe, wobei der Assay so ausgelegt ist, dass er die Durchführung einer kompetitiven Kinetik gegenüber entweder einem Inhibitor von Faktor Xa oder einem Inhibitor von Faktor IIa ermöglicht, dann
   b. Eingabe der in Schritt a. erhaltenen Kinetik(en) in ein Entscheidungsmodell A zur Klassifikation, das durch Training eines überwachten maschinellen Lernmodells erhalten wird, z. B. ein Modell, das aus einer der folgenden Familien ausgewählt wird: Support Vector Machine, neuronale Netze, Entscheidungsbäume, Ensemble-Methoden und Modell der k nächsten Nachbarn (k-Nearest Neighbors), dann

   b.i. Wenn das Entscheidungsmodell A das Vorhandensein eines Inhibitors des Enzyms für die gezielte Blutgerinnung in der analysierten Probe ausschließt, Schlussfolgerung auf das Fehlen des besagten Inhibitors und optional Zuweisung der entsprechenden Information als Ausgabe durch Modell A, z. B. in einer Variablen, oder
   b.ii. Wenn das Entscheidungsmodell A das Vorhandensein eines Inhibitors des Enzyms für die gezielte Blutgerinnung in der analysierten Probe attestiert, Schlussfolgerung auf das Vorhandensein des besagten Inhibitors und optional Zuweisung der entsprechenden Information als Ausgabe durch Modell A, z. B. in einer Variablen.

2. Verfahren zur Identifizierung eines Inhibitors eines Blutgerinnungsenzyms, das unabhängig gewählt ist aus dem Faktor Xa (FXa) und dem Faktor IIa (FIIa), in einer biologischen Probe, insbesondere einer Blutprobe, wobei das Verfahren die folgenden Schritte umfasst:

   1. Durchführung der Schritte des Verfahrens nach Anspruch 1, dann
   2. Eingabe der Kinetik(en), die in dem unter a. von Anspruch 1 definierten Schritt oder in obigem Schritt 1. erhalten wurde(n), sowie des am Ende von Schritt b. ii. von Anspruch 1 erhaltenen Ergebnisses, in ein Entscheidungsmodell B zur Klassifikation, das durch Training eines überwachten maschinellen Lernmodells erhalten wird, z.B. ein Modell, das aus einer der folgenden Familien ausgewählt wird: Support Vector Machine, neuronale Netze, Entscheidungsbäume, Ensemble-Methoden und Modell der k nächsten Nachbarn (k-Nearest Neighbors), und Zuweisung der Kategorie des Inhibitors durch Modell B als Ausgabe zu einer der folgenden Kategorien: indirekter irreversibler Inhibitor (Heparin) oder direkter reversibler Inhibitor (DOAK), insbesondere wenn die zum Trainieren von Modell B verwendeten Datensätze Daten zu beiden Inhibitorkategorien enthalten, und Ausgabe der durch Modell B bestimmten Kategorie des Inhibitors, z. B. durch Zuweisung in einer Variablen.

3. Verfahren nach Anspruch 2, das einen zusätzlichen Schritt zur Charakterisierung des in Schritt b. ii von Anspruch 1 nachgewiesenen Inhibitors umfasst, und zwar: die Eingabe der in Schritt a. von Anspruch 1 oder Schritt 1. von Anspruch 2 erhaltenen Kinetik(en) und der in Schritt 2. von Anspruch 2 ermittelten Daten zur Kategorie des nachgewiesenen Inhibitors, z. B. der zugewiesenen Variablen, in ein klassifizierendes Entscheidungsmodell C, das durch Training eines überwachten maschinellen Lernmodells, beispielsweise eines aus einer der folgenden Familien ausgewählten Modells: Support Vector Machine, neuronale Netze, Entscheidungsbäume, Ensemble-Methoden und Modell der k nächsten Nachbarn (k-Nearest Neighbors), erhalten wird, und Charakterisierung des gesuchten Inhibitors durch Modell C als Ausgabe, wobei letzterer identifiziert wird aus:

   a. Für den Fall, dass die Kategorie des gesuchten Inhibitors die der Heparine ist: HNF oder LMWH, oder
   b. Für den Fall, dass die Kategorie des gesuchten Inhibitors die Kategorie der DOAKs ist: Rivaroxaban, Apixaban, Edoxaban oder Dabigatran,

   und Ausgabe der durch Modell C bestimmten Charakterisierung des Inhibitors, z. B. durch Zuweisung in einer Variablen.

4. Verfahren nach Anspruch 3, das einen zusätzlichen Schritt der quantitativen Bestimmung des charakterisierten Inhibitors umfasst, wobei in ein Regressionsmodell D, insbesondere ein überwachtes maschinelles Lernmodell, beispielsweise ein Modell aus einer der folgenden Familien: Support Vector Machines, neuronale Netze, Entscheidungsbäume, Ensemble-Methoden und Modell der k nächsten Nachbarn (k-Nearest Neighbors), die in Schritt a. von Anspruch 1 oder Schritt 1. von Anspruch 2 erhaltene(n) Kinetik(en) eingegeben werden, und die nach Anspruch 3 erhaltenen Charakterisierungsdaten, die den in der analysierten Blutprobe vorhandenen Inhibitor identifizieren, wobei das Regressionsmodell auf einem Datensatz trainiert wurde, der unter identischen Messbedingungen wie jenen von Schritt a. von Anspruch 1 oder Schritt 1. von Anspruch 2 erhalten wurde, und als Ausgabe die Bestimmung der Konzentration des identifizierten Inhibitors in der analysierten Probe, und optional die Ausgabe der durch Modell D bestimmten Konzentration, beispielsweise durch Zuweisung in einer Variablen, ermöglicht.

5. Verfahren nach einem der Ansprüche 1 bis 4, in dem der gesuchte Inhibitor:

    I. ein Inhibitor von Faktor Xa (Fxa), ausgewählt aus: UFH, MPMH, Rivaroxaban, Apixaban, Edoxaban, oder
    II. ein Inhibitor von Faktor IIa (FIIa), ausgewählt aus: HNF, HBPM, Dabigatran, ist.

6. Verfahren nach Anspruch 2, angewandt auf die Suche eines Inhibitors von Faktor Xa, dessen Vorhandensein in Schritt b. ii von Anspruch 1 nachgewiesen wurde, das den folgenden zusätzlichen Charakterisierungsschritt umfasst:

    I. Wenn die Inhibitorkategorie in Schritt 2. von Anspruch 2 der Heparinkategorie zugewiesen wurde, dann:

        I.i. Eingabe der in Schritt 2 von Anspruch 2 ermittelten Daten zur Kategorie des nachgewiesenen Inhibitors, z. B. der zugewiesenen Variablen, und der in Schritt a. von Anspruch 1 oder Schritt 1. von Anspruch 2 erhaltenen kompetitiven Kinetik(en) gegenüber einem Inhibitor von Faktors Xa, in ein Entscheidungsmodell C zur Klassifikation, das durch Training eines überwachten maschinellen Lernmodells, z. B. eines Modells aus einer der folgenden Familien: Support Vector Machines, neuronale Netze, Entscheidungsbäume, Ensemble-Methoden und Modell der k nächsten Nachbarn (k-Nearest Neighbors), erhalten wird, und
        I.ii. Charakterisierung des gesuchten Inhibitors durch Modell C als Ausgabe, wobei letzterer identifiziert wird aus: HNF oder HBPM, und Ausgabe der durch Modell C bestimmten Charakterisierung des Inhibitors, z. B. durch Zuweisung in einer Variablen,

    oder alternativ,
    II. Wenn die Inhibitorkategorie in Schritt 2. von Anspruch 2 der Kategorie der DOAKs zugewiesen wurde, dann:

        II.i. Messung einer oder mehrerer kompetitiver Kinetik(en) mittels Durchführung eines kompetitiven enzymatischen Assays an einer von demselben Subjekt entnommenen Blutprobe, wobei der Assay so ausgelegt ist, dass er die Durchführung einer kompetitiven Kinetik gegenüber einem Inhibitor von Faktor Xa ermöglicht, mit einem Verdünnungsfaktor der Probe und/oder einer Messdauer, die an eine kompetitive Situation mit Vorhandensein von Faktor Xa-hemmenden DOAKs angepasst sind, insbesondere mit einem Verdünnungsfaktor und/oder einer Messdauer, die sich von dem/derjenigen unterscheiden, die für die Messung der in Schritt a. von Anspruch 1 oder Schritt 1. von Anspruch 2 erhaltenen Kinetik(en) verwendet wurden; dann
        II.ii. Eingabe der in Schritt 2. von Anspruch 2 bezüglich der Kategorie des nachgewiesenen Inhibitors ermittelten Daten, z. B. der zugewiesenen Variablen, und der im vorherigen Schritt i. erhaltenen Kinetik(en) in ein Entscheidungsmodell C zur Klassifikation, das durch Training eines überwachten maschinellen Lernmodells, z. B. eines Modells aus einer der folgenden Familien: Support Vector Machines, neuronale Netze, Entscheidungsbäume, Ensemble-Methoden und Modell der k nächsten Nachbarn (k-Nearest Neighbors), erhalten wird und
        II.iii. Charakterisierung des gesuchten Inhibitors durch Modell C als Ausgabe, wobei letzterer identifiziert wird als: Rivaroxaban, Apixaban oder Edoxaban, und Ausgabe der durch Modell C bestimmten Charakterisierung des Inhibitors, z. B. durch Zuweisung in einer Variablen.

7. Verfahren nach Anspruch 6, das einen zusätzlichen Schritt des quantitativen Assays des gemäß Schritt I. oder II. von Anspruch 6 identifizierten Inhibitors umfasst, in dem jeweils folgendes gilt:

    I. Wenn der in Schritt I. von Anspruch 6 identifizierte Inhibitor HNF oder HBPM ist, dann: Eingabe in ein Regressionsmodell D, insbesondere ein überwachtes maschinelles Lernmodell, z. B. ein Modell aus einer der folgenden Familien: Support Vector Machines, neuronale Netze, Entscheidungsbäume, Ensemble-Methoden und Modell der k nächsten Nachbarn (k-Nearest Neighbors), wobei das Modell auf einem Datensatz trainiert wurde, der unter identischen Messbedingungen wie jenen von Schritt a. von Anspruch 1 oder Schritt 1. von Anspruch 2 erhalten wurde, der in Schritt a. von Anspruch 1 oder Schritt 1. von Anspruch 2 erhaltenen Kinetik(en) und der in Schritt I.ii von Anspruch 6 ermittelten Daten, die den in der analysierten Blutprobe vorhandenen Inhibitor identifizieren, wobei das Regressionsmodell als Ausgabe die Bestimmung der Konzentration des in der analysierten Probe identifizierten Inhibitors und optional, die Ausgabe der durch Modell D bestimmten Konzentration ermöglicht, z. B. durch Zuweisung in einer Variablen.

II. Wenn der in Schritt II. von Anspruch 6 identifizierte Inhibitor Rivaroxaban, Apixaban oder Edoxaban ist, dann: Eingabe in ein Regressionsmodell D, insbesondere ein überwachtes maschinelles Lernmodell, z. B. ein Modell aus einer der folgenden Familien: Support Vector Machines, neuronale Netze, Entscheidungsbäume, Ensemble-Methoden und Modell der k nächsten Nachbarn (k-Nearest Neighbors), wobei das Modell auf einem Datensatz trainiert wurde, der unter identischen Messbedingungen wie jenen von Schritt II. i. von Anspruch 6, erhalten wurde, der in Schritt II. i. von Anspruch 6 erhaltenen Kinetik(en) und der Charakterisierungsdaten, z. B. der zugewiesenen Variablen, die am Ende von Schritt II.iii. von Anspruch 6 bereitgestellt werden und den in der analysierten Blutprobe vorhandenen Inhibitor identifizieren, wobei das Regressionsmodell als Ausgabe die Bestimmung der Konzentration des in der analysierten Probe identifizierten Inhibitors und optional die Ausgabe der durch Modell D bestimmten Konzentration ermöglicht, beispielsweise durch Zuweisung in einer Variablen.

8. Verfahren nach Punkt II. von Anspruch 7, in dem, wenn die durch Modell D bestimmte Konzentration des identifizierten Inhibitors in der analysierten Probe kleiner oder gleich 200 ng/mL ist, die in Schritt a. von Anspruch 1 erhaltenen Kinetik(en) in ein Regressionsmodell D2 eingegeben werden, insbesondere ein überwachtes maschinelles Lernmodell, z. B. ein Modell aus einer der folgenden Familien: Support Vector Machines, neuronale Netze, Entscheidungsbäume, Ensemble-Methoden und Modell der k nächsten Nachbarn (k-Nearest Neighbors), wobei das Modell auf einem Datensatz trainiert wurde, der unter identischen Messbedingungen wie jenen von Schritt a. von Anspruch 1 erhalten wurde, wobei das Regressionsmodell als Ausgabe die Bestimmung der Konzentration des in der analysierten Probe identifizierten Inhibitors und optional die Ausgabe der durch Modell D bestimmten Konzentration ermöglicht, beispielsweise durch Zuweisung in einer Variablen.

9. Verfahren nach einem der Ansprüche 1 bis 8, in dem die *in vitro-Messung* einer kompetitiven Kinetik durch einen kompetitiven enzymatischen Assay an einer von einem Subjekt entnommenen Blutprobe die folgenden Schritte umfasst:

a. Bereitstellung einer verdünnten oder unverdünnten Blutprobe, dann

b. Zugabe eines Substrats zur Blutprobe, das je nach gesuchtem Inhibitor entweder für Faktor Xa oder für Faktor IIa spezifisch ist, insbesondere ein chromogenes oder fluorogenes Substrat,

c. Inkubation des in b. erhaltenen Gemischs unter Erhöhung der Temperatur auf 35 bis 39 °C, insbesondere 37 °C,

d. Je nach dem in Schritt b. zugegebenen Substrat Zugabe von Faktor Xa oder Faktor IIa zu dem in c. entstandenen Reaktionsgemisch, um so die Kompetition zwischen einer Hemmungsreaktion und der hervorgerufenen enzymatischen Reaktion auszulösen,

e. Messung der Produktmenge, die aus der Umwandlung des Substrats aufgrund der Wirkung des analysierten Enzyms auf das aus Faktor Xa oder Faktor IIa ausgewählte Substrat resultiert, mittels eines Instruments im Laufe der Zeit, gegebenenfalls über die Messung eines mit dem Substrat assoziierten Markers, der bei der Enzymreaktion freigesetzt wird, und Speicherung der erhaltenen Kinetik.

10. Verfahren nach Anspruch 9, in dem der kompetitive enzymatische Assay spezifisch für Faktor Xa ist, und:

a. die Blutprobe in Schritt a. eine Plasmaprobe ist, die 1:2 in einem Owren-Koller-Puffer verdünnt wurde,

b. das Substrat in Schritt b. das Reagenz MAPA-Gly-Arg-pNA ist,

c. die Inkubationszeit in Schritt c. 240 Sekunden bei 37°C beträgt,

d. der in Schritt d. dem Gemisch zugesetzte Faktor Xa ein boviner Faktor Xa ist,

e. die Messung der Freisetzung von Paranitroanilin (pNA) in Schritt e. durch Kolorimetrie bei 405 nm alle zwei Sekunden für 156 Sekunden auf einem geeigneten Gerät vom Typ STA-R® durchgeführt wird,

oder alternativ:

a. die Blutprobe in Schritt a. eine Plasmaprobe ist, die 1:8 in einem Owren-Koller-Puffer verdünnt wurde,

b. das Substrat in Schritt b. das Reagenz MAPA-Gly-Arg-pNA ist,

c. die Inkubationszeit in Schritt c. 240 Sekunden bei 37°C beträgt,

d. der in Schritt d. dem Gemisch zugesetzte Faktor Xa ein boviner Faktor Xa ist,

e. die Messung der Freisetzung von Paranitroanilin (pNA) in Schritt e. durch Kolorimetrie bei 405 nm alle zwei Sekunden für 86 Sekunden auf einem geeigneten Gerät vom Typ STA-R® durchgeführt wird.

**11.** Verfahren nach Anspruch 9, in dem der kompetitive enzymatische Assay auf einer miniaturisierten Vorrichtung, z. B. einer Vorrichtung, die Mikrofluidik verwendet, in einem Reaktionsvolumen zwischen 1 und 20 μL durchgeführt wird.

**12.** Verfahren nach einem der Ansprüche 1 bis 11, in dem:

    a. die Blutprobe eine Plasmaprobe ist und/oder
    b. die Eingabe einer experimentell gewonnenen Kinetik in ein überwachtes maschinelles Lernmodell darin besteht, die Wertepaare bestehend aus jedem im Messzeitraum gemessenen Wert und dem jeweiligen diskreten Messpunkt bereitzustellen.

**13.** Datenverarbeitungssystem oder Vorrichtung zum Nachweis des Vorhandenseins oder der Identifizierung eines Inhibitors von Faktor Xa (FXa) oder Faktor IIa (FIIa) in einer biologischen Probe, wobei das Datenverarbeitungssystem oder die Vorrichtung Mittel zur Durchführung von mindestens Schritt b. von Anspruch 1 umfasst und Mittel, um als Eingabe eine oder mehrere kompetitive Kinetiken bereitzustellen, die durch die Durchführung eines kompetitiven Assays gegenüber entweder einem Inhibitor von Faktor Xa oder einem Inhibitor von Faktor IIa an einer zuvor von einem Subjekt entnommenen Blutprobe erhalten werden, insbesondere über ein Messgerät, und Mittel, um die in diesem Schritt erzeugten Variablen auszugeben, und gegebenenfalls auch Mittel, um Schritt 2 des Verfahrens nach Anspruch 2 durchzuführen, und optional auch Mittel, um die in diesem Schritt erzeugten Variablen als Eingabe und/oder Ausgabe bereitzustellen, und optional auch Mittel, um die Schritte durchzuführen, die die Entscheidungsmodelle zur Klassifikation oder Regression C, D und gegebenenfalls D2 eines der Ansprüche 3 bis 12 beinhalten, insbesondere Mittel, die die Verwendung einer Matrix ermöglichen, die aus dem Lernen resultiert, das die Festlegung der Parameter dieser Modelle ermöglichte, um das Ergebnis der Klassifikation oder Regression unter Berücksichtigung der dem oder den Modellen zugeführten Variablen zurückzugeben, und optional außerdem einen Prozessor umfassen, der geeignet ist, die in diesem Anspruch angegebenen Schritte auszuführen.

**14.** Computerprogramm mit Anweisungen, die dazu führen, dass ein Datenverarbeitungssystem oder eine Vorrichtung nach Anspruch 13, insbesondere eine Vorrichtung, die ein Messgerät für Kinetiken wie in einem der Ansprüche 1 oder 6 definiert enthält, oder ein Datenverarbeitungssystem oder eine Vorrichtung, bei der ein solches Messgerät insbesondere auf Entfernung benutzt wird, mindestens Schritt b. von Anspruch 1 und gegebenenfalls auch Schritt 2. des Verfahrens nach Anspruch 2 ausführt, und optional dazu führt, die Schritte auszuführen, die die Entscheidungsmodelle zur Klassifikation oder Regression C, D und gegebenenfalls D2 einer der Ansprüche 3 bis 12 beinhalten.

**15.** Computerlesbares Speichermedium mit Anweisungen, die, wenn sie von einem Computer ausgeführt werden, diesen veranlassen, mindestens Schritt b. von Anspruch 1 und gegebenenfalls auch Schritt 2. des Verfahrens nach Anspruch 2 auszuführen, und optional auch das Abrufen und/oder die Ausgabe der in diesen Schritten erzeugten Variablen ermöglichen, und optional diesen veranlassen, die Schritte auszuführen, die die Entscheidungsmodelle zur Klassifikation bzw. Regression C, D und gegebenenfalls D2 einer der Ansprüche 3 bis 12 beinhalten.

**16.** Computerlesbarer Datenträger, auf dem das Computerprogramm nach Anspruch 14 gespeichert ist, oder Signal eines Datenträgers, der das Computerprogramm nach Anspruch 14 trägt.

**17.** Set, das für die Durchführung eines Verfahrens nach einem der Ansprüche 1 bis 12 geeignet ist, und folgendes umfasst:

    a. ein FXa- und/oder FIIa-spezifisches Substrat, z. B. das Substrat MAPA-Gly-Arg-pNA für Faktor Xa oder z. B. das Substrat EtM-SPro-Arg-pNA für Faktor IIa, und
    b. optional FXa und/oder FIIa, z. B. des bovinen oder menschlichen Faktors Xa, z. B. des bovinen oder menschlichen Faktors IIa, und
    c. optional ein oder mehrere geeignete Puffer, z. B. der Owren-Koller-Puffer, z. B. der Puffer Tris EDTA und
    d. optional Anweisungen zur Durchführung einer oder mehrerer Messungen einer oder mehrerer kompetitiver Kinetiken mittels Durchführung eines kompetitiven enzymatischen Assays unter Verwendung des Substrats, und
    e. das System und/oder die Vorrichtung und/oder das Computerprogramm und/oder den computerlesbaren Datenträger nach einem der Ansprüche 13 bis 15,
    f. und optional Anweisungen zur Durchführung des Verfahrens nach einem der Ansprüche 1 bis 12,
    g. und optional Anweisungen zur Verwendung eines Signals eines Datenträgers nach Anspruch 16 zur Durchführung eines Verfahrens nach einem der Ansprüche 1 bis 12.

**EP 3 891 752 B1**

**Claims**

1.  Method of detecting in a biological sample, in particular a blood sample, the presence of an inhibitor of a blood coagulation enzyme selected, independently, from factor Xa (FXa) and factor IIa (FIIa), the method comprising the following steps:

    a. Measurement of one or more competition kinetics by performing a competitive enzymatic assay on a blood sample previously obtained from a subject, said assay being adapted to perform competitive kinetics with respect to either a factor Xa inhibitor or a factor IIa inhibitor, and then

    b. Input of one or more kinetics obtained in step **a.** to a classification decision model A obtained by training a supervised automatic learning model, for example a model chosen from one of the following families: support vector machine, neural networks, decision trees, ensemble methods, and k nearest neighbours model, then

    b.i. If the decision model A excludes the presence of an inhibitor of the targeted blood coagulation enzyme in the sample analysed, conclusion of the absence of said inhibitor, optionally allocation as output by the model A, for example in a variable, of the corresponding information, or
    b.ii. If the decision model A attests to the presence of an inhibitor of the targeted blood coagulation enzyme in the sample analysed, conclusion of the presence of said inhibitor, optionally allocation as output by the model A, for example in a variable, of the corresponding information.

2.  Method of identifying in a biological sample, in particular a blood sample, an inhibitor of a blood coagulation enzyme selected, independently, from factor Xa (FXa) and factor IIa (FIIa), the method comprising the following steps:

    1. Carrying out the steps of the method according to claim 1, then
    2. Inputting of the kinetic(s) obtained in the step defined in point a. of claim 1, or step 1. above, and of the result obtained at the end of step b.ii. of claim 1, to a classification decision model B obtained by training a supervised automatic learning model, for example a model chosen from one of the following families: support vector machine, neural networks, decision trees, ensemble methods, and k nearest neighbours model, and allocation at the output by the model B of the category of the inhibitor to one of the following categories: irreversible indirect inhibitor (heparins), or reversible direct inhibitor (AOD), in particular when the data sets used for training model B comprise data relating to these two categories of inhibitor, and outputting, for example by allocation in a variable, the category of inhibitor determined by model B.

3.  Method according to claim 2, comprising an additional step of characterizing the inhibitor whose presence has been detected in step b.ii of claim 1, as follows: inputting the kinetic(s) obtained in step a. of claim 1 or step 1. of claim 2, and the data determined, for example the allocated variable, in step 2. of claim 2 concerning the category of inhibitor whose presence has been detected, to a classification decision model C obtained by training a supervised automatic learning model, for example a model chosen from one of the following families: support vector machine, neural networks, decision trees, ensemble methods, and k nearest neighbours model, and output characterisation by the model C of the inhibitor sought, the latter being identified from:

    a. If the category of inhibitor sought is that of the heparins: UFH or LMWH, or
    b. If the category of inhibitor sought is that of the AODs: Rivaroxaban, Apixaban, Edoxaban or Dabigatran,

    and output, for example by allocation in a variable, the characterisation of the inhibitor determined by the C model.

4.  Method according to claim 3, comprising an additional step of quantitative assay of the inhibitor **characterised, in** which are supplied as input to a regression model D, in particular a supervised automatic learning model, for example a model chosen from one of the following families: support vector machines, neural networks, decision trees, ensemble methods, and k nearest neighbours model, the kinetic(s) obtained in step a. of claim 1 or step 1. of claim 2, and the characterisation data obtained according to claim 3 identifying the inhibitor present in the blood sample analysed, said regression model having been trained on a set of data obtained under measurement conditions identical to those in step a. of claim 1 or step 1. of claim 2, and making it possible to determine as output the concentration of the inhibitor identified in the sample analysed and optionally to supply as output, for example by allocation in a variable, the concentration determined by model D.

5.  Method according to any one of claims 1 to 4, in which the inhibitor sought is:

I. a factor Xa (FXa) inhibitor chosen from: UFH, LMWH, Rivaroxaban, Apixaban, Edoxaban, or

II. a factor IIa inhibitor (FIIa) chosen from: UFH, LMWH, Dabigatran.

6. Method according to claim 2, applied to the search for a factor Xa inhibitor whose presence has been detected in step b.ii of claim 1, comprising the following additional characterisation step:

I. if the inhibitor class has been allocated in step 2. of claim 2 to the heparin class, then:

I.i. supplying as input to a classification decision model C obtained by training a supervised automatic learning model, for example a model chosen from one of the following families: support vector machine, neural networks, decision trees, ensemble methods, and k nearest neighbours model, said determined data, for example the allocated variable, in step 2. of claim 2 concerning the category of inhibitor whose presence has been detected, and of the competitive kinetic(s) with respect to a factor Xa inhibitor, obtained in step a. of claim 1 or step 1. of claim 2, and

I.ii. output characterisation by model C of the inhibitor sought, the latter being identified from: UFH or LMWH, and output, for example by allocation in a variable, of the characterisation of the inhibitor determined by model C,

or alternatively,

II. if the inhibitor class has been allocated in step 2. of claim 2 to the AODs class, then:

II.i. performance of a new measurement of one or more competition kinetics by performing a competitive enzymatic assay on a blood sample obtained from the same subject, said assay being adapted to the performance of competitive kinetics with respect to a factor Xa inhibitor, with a sample dilution factor and/or a measurement time adapted to a competitive situation involving the presence of AOD inhibiting factor Xa, in particular a dilution factor and/or a measurement time different from those used for measuring the kinetic(s) obtained in step a. of claim 1 or step 1. of claim 2, then

II.ii. supplying as input to a classification decision model C obtained by training a supervised automatic learning model, for example a model chosen from one of the following families: support vector machine, neural networks, decision trees, ensemble methods, and k nearest neighbours model, said determined data, for example the allocated variable, in step 2. of claim 2 concerning the category of inhibitor whose presence has been detected, and of the kinetic(s) obtained in the preceding step i., and

II.iii. output characterisation by model C of the inhibitor sought, the latter being identified from: Rivaroxaban, Apixaban, or Edoxaban, and provision at the output, for example by allocation in a variable, of the characterisation of the inhibitor determined by model C.

7. Method according to claim 6, comprising an additional step of quantitative assay of the inhibitor identified at the end of steps I. or II. of claim 6, in which, respectively:

I. If the inhibitor identified in step I. of claim 6 is an UFH or LMWH, then: supplying as input to a regression model D, in particular a supervised automatic learning model, for example a model chosen from one of the following families: support vector machines, neural networks, decision trees, ensemble methods, and k nearest neighbours model, said model having been trained on a data set obtained under measurement conditions identical to those in step a. of claim 1, or step 1. of claim 2 of the kinetic(s) obtained in step a. of claim 1 or step 1. of claim 2 and of the data determined in step I.ii of claim 6 identifying the inhibitor present in the analysed blood sample, said regression model making it possible to determine as an output the concentration of inhibitor identified in the sample analysed, and optionally supplying as an output, for example by allocation in a variable, the concentration determined by model D, or

II. If the inhibitor identified in step II. of claim 6 is Rivaroxaban, Apixaban, or Edoxaban, then: supplying as input to a regression model D, in particular a supervised automatic learning model, for example a model chosen from one of the following families: support vector machines, neural networks, decision trees, ensemble methods, and k nearest neighbours model, said model having been trained on a data set obtained under measurement conditions identical to those of step II. i. of claim 6, the kinetic(s) obtained in step II. i. of claim 6 and the characterisation data, for example the allocated variable, supplied at the end of step II. iii. of claim 6 identifying the inhibitor present in the analysed blood sample, said regression model making it possible to determine as output the concentration of inhibitor identified in the sample analysed and optionally supplying as output, for example by allocation in a variable, the concentration determined by model D.

8.  Method according to step II. of claim 7, in which if the concentration of inhibitor identified in the sample analysed, as determined by model D, is less than or equal to 200 ng/mL, then input to a regression model D2, in particular a supervised automatic learning model, for example a model chosen from one of the following families: support vector machines, neural networks, decision trees, ensemble methods, and k nearest neighbours model, said model having been trained on a set of data obtained under measurement conditions identical to those in step a. of claim 1, of the kinetic(s) obtained in step a. of claim 1, said regression model D2 making it possible to recalculate as output the concentration of inhibitor identified in the sample analysed and optionally supplying as output, for example by allocation in a variable, the concentration determined by model D2.

9.  Method according to any one of claims 1 to 8, wherein the *in vitro* measurement of competition kinetics by competitive enzyme assay on a blood sample obtained from a subject comprises the following steps:

    a. supply of a blood sample, diluted or undiluted, then
    b. addition to the blood sample of a substrate specific for either factor Xa or factor IIa, depending on the inhibitor sought, in particular a chromogenic or fluorogenic substrate,
    c. incubation with raising the temperature of the mixture obtained in b. to between 35 and 39°C, in particular 37°C,
    d. addition to the reaction mixture obtained from c. of factor Xa or factor IIa, depending on the substrate added in step b., so as to trigger competition between an inhibition reaction and the induced enzymatic reaction,
    e. measurement by an instrument, over time, of the quantity of product resulting from the transformation of the substrate as a result of the action of the enzyme analysed on the latter, chosen from factor Xa or factor IIa, where appropriate by measuring a marker associated with the substrate, released during said enzymatic reaction, and recording of the kinetics obtained.

10. Method according to claim 9, wherein the competitive enzyme assay is specific for factor Xa, and wherein:

    a. in step a. the blood sample is a plasma sample diluted 1/2 in an Owren Koller buffer,
    b. in step b. the substrate is MAPA-Gly-Arg-pNA reagent,
    c. in step c. the incubation time is 240 seconds at 37°C,
    d. factor Xa added to the mixture in step d. is bovine factor Xa,
    e. the release of paranitroaniline (pNA) in step e. is measured by colorimetry at 405 nm every two seconds for 156 seconds, on an appropriate instrument, such as STA-R®,

    or alternatively:

    a. in step a. the blood sample is a plasma sample diluted 1/8th in an Owren Koller buffer,
    b. in step b. the substrate is MAPA-Gly-Arg-pNA reagent,
    c. in step c. the incubation time is 240 seconds at 37°C,
    d. factor Xa added to the mixture in step d. is bovine factor Xa,
    e. the release of paranitroaniline (pNA) in step e. is measured by colorimetry at 405 nm every two seconds for 86 seconds, on an appropriate instrument, such as STA-R®,

11. Method according to claim 9, in which the competitive enzyme assay is carried out on a miniaturised device, for example a device using microfluidics, in a reaction volume of between 1 and 20 $\mu$L.

12. Method according to any one of claims 1 to 11, in which:

    a. the blood sample is a plasma sample, and/or
    b. the input to a supervised automatic learning model of a kinetic obtained experimentally consists of supplying the pairs of values constituted by each value measured for each discrete measurement point taken during the measurement time.

13. Data processing system or device for detecting, in a biological sample, the presence or identification of an inhibitor of factor Xa (FXa) or of factor IIa (FIIa), said data processing system or device comprising means for implementing at least step b. of claim 1 and means for supplying as input one or more competition kinetics obtained by performing a competitive assay against either a factor Xa inhibitor or a factor IIa inhibitor on a blood sample previously obtained from a subject, in particular via a measuring device, and means for supplying as output the variables generated during this step, and where appropriate also means for implementing step 2. of the method according to claim 2, and optionally also comprising means for providing as input and/or output the variables generated during this step, and optionally also comprising means for implementing the steps involving the classification or regression decision models C, D, and where appropriate D2, of one of claims 3 to 12, in particular means making it possible to make use of a matrix resulting from the learning which has made it possible to set the parameters of these models, in order to return the classification or regression result taking into account the variables supplied to the model(s), and optionally also comprising a processor adapted to implement the steps indicated in the present claim.

14. Computer program comprising instructions which lead a data processing system or a device according to claim 13, in particular a device including an apparatus for measuring kinetics as defined in any one of claims 1 or 6 or a data processing system or a device calling upon such a measuring apparatus, in particular a remote one, to execute at least step b. of claim 1, and where appropriate also step 2. of the method according to claim 2, and optionally leading to execution of the steps involving the classification or regression decision models C, D, and where appropriate D2, of one of claims 3 to 12.

15. Computer-readable recording medium comprising instructions which, when executed by a computer, cause the latter to implement at least step b. of claim 1, and where appropriate also step 2. of the method according to claim 2, and optionally also make it possible to recover as input and/or provide as output the variables generated during these steps, and optionally cause the latter to implement the steps involving the classification or regression decision models C, D, and where appropriate D2, of one of claims 3 to 12.

16. Computer-readable data medium on which the computer program according to claim 14 is recorded, or a signal from a data medium carrying the computer program according to claim 14.

17. Kit for implementing a method according to one of claims 1 to 12, comprising:

   a. A substrate specific to FXa and/or FIIa, for example the MAPA-Gly-Arg-pNA substrate for factor Xa, for example the EtM-SPro-Arg-pNA substrate for factor IIa, and
   b. Optionally, FXa and/or FIIa, for example bovine or human factor Xa, for example bovine or human factor IIa, and
   c. Optionally, one or more suitable buffers, e.g. Owren Koller buffer, e.g. Tris EDTA buffer and
   d. Optionally instructions for implementing measurement(s) of one or more competition kinetics by performing a competitive enzymatic assay using the substrate, and
   e. the system and/or device and/or computer program and/or computer-readable data medium according to one of claims 13 to 15,
   f. and optionally instructions allowing to implement the method according to one of claims 1 to 12,
   g. and optionally instructions relating to the use of a signal from a data medium according to claim 16, for the implementation of a method according to one of claims 1 to 12.

Inhibiteurs directs

Inhibiteurs indirects

$$E \quad + \quad S \quad \overset{K_M}{\rightleftarrows} \quad E \cdot S \quad \rightarrow \quad E \quad + \quad P$$

$$+$$

$$I$$

$$k_{i+} \downarrow\uparrow k_{i-}$$

$$E \cdot I$$

$$E \quad + \quad S \quad \overset{K_M}{\rightleftarrows} \quad E \cdot S \quad \rightarrow \quad E \quad + \quad P$$

$$+$$

$$I \quad + \quad A \quad \overset{k_{on}}{\underset{k_{off}}{\rightleftarrows}} \quad A \cdot I$$

$$k_{i+} \downarrow\uparrow k_{i-}$$

$$E \cdot A \cdot I$$

Inhibiteurs réversibles

$$E \quad + \quad S \quad \overset{K_M}{\rightleftarrows} \quad E \cdot S \quad \rightarrow \quad E \quad + \quad P$$

$$+$$

$$I$$

$$\downarrow \quad k_i$$

$$E \cdot I$$

$$E \quad + \quad S \quad \overset{K_M}{\rightleftarrows} \quad E \cdot S \quad \rightarrow \quad E \quad + \quad P$$

$$+$$

$$I \quad + \quad A \quad \overset{k_{on}}{\underset{k_{off}}{\rightleftarrows}} \quad A \cdot I$$

$$\downarrow \quad k_i$$

$$E \cdot A \cdot I$$

Inhibiteurs irréversibles

100

FIG. 1

FIG. 2

FIG. 3

102

FIG. 4

émetteur

récepteur

[E] + [I]
(+ [A])
+
[S]

[P]

Temps

[P]

① ② ③ ④

5

FIG. 5

FIG. 6

FIG. 7

FIG. 8

[I] = 0.0

[I] = [E]/8.0

[I] = [E]*3.0

FIG. 9

FIG. 10

FIG. 11

FIG. 12

FIG. 13

FIG. 14

FIG. 15

FIG. 16

$y = 1.043x - 0.03,\ R^2 = 0.9851$

[HNF] prédite (UI/ml)

[HNF] mesurée (UI/ml)

A

FIG. 17A

$y = 1.04x - 0.03,\ R^2 = 0.9856$

[HNF] prédite (UI/ml)

[HNF] mesurée (UI/ml)

B

FIG. 17B

FIG. 18A

FIG. 18B

FIG. 19A

FIG. 19B

FIG. 20A

FIG. 20B

FIG. 21A

FIG. 21B

FIG. 22A

FIG. 22B

$y = 0.905x+12.35$, $R^2 = 0.9853$

**A**

[edoxaban] prédite (ng/ml)

[edoxaban] mesurée (ng/ml)

FIG. 23A

$y = 0.93x+8.18$, $R^2 = 0.9881$

**B**

[edoxaban] prédite (ng/ml)

[edoxaban] mesurée (ng/ml)

FIG. 23B

y = 0.9431x+4.51, $R^2$ = 0.9968

[edoxaban] prédite (ng/ml)

[edoxaban] mesurée (ng/ml)

FIG. 24A

y = 0.9442x+4.26, $R^2$ = 0.9962

[edoxaban] prédite (ng/ml)

[edoxaban] mesurée (ng/ml)

FIG. 24B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1367135 A1

- WO 2014134223 A1

**Littérature non-brevet citée dans la description**

- **GÉRON, A.** Hands-On Machine Learning with Scikit-Learn and TensorFlow: Concepts, Tools, and Techniques to Build Intelligent Systems. O'Reilly, 2017
- **SEGEL, I. H.** Enzyme kinetics. Behavior and analysis of rapid equilibrium and steady-state enzyme Systems. Wiley Classics Library, 1993

- **BONACCORSO, G.** Machine Learning Algorithms: A reference guide to popular algorithms for data science and machine learning. Packt Publishing Limited, 2017